# EUROPEAN PATENT APPLICATION

(11) **EP 4 670 717 A1**
(43) Date of publication of application: **31.12.2025**
(21) Application number: 24184835.7
(22) Date of filing: 26.06.2024
(51) Int. Cl.: A61K 31/404, A61P 9/00, A61P 15/04, A61P 15/18, A61P 25/16, A61P 25/28, A61P 27/12, A61P 29/00, A61P 33/00, C07D 207/48, C07D 413/12, C07D 403/12, C07F 7/08

(54) **PYRROLIDINE-FUSED MYRTANOL DERIVATIVES AS INHIBITORS AND/OR DEGRADERS OF IDO1 UND USE THEREOF**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to novel pyrrolidine-fused myrtanol derivatives (or 4-azatricyclo[6.1.1.0^{2,6}]decanes) of the general formula (I) or pharmaceutically acceptable salts thereof.

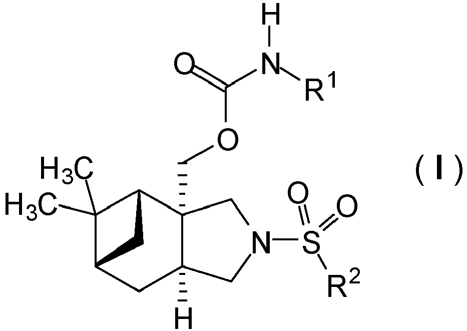

The invention further relates to pharmaceutical compositions comprising such compounds as well as their use as medicaments, especially in methods for the prophylaxis or treatment of a disease and/or a disorder caused by and/or associated with indolamine 2,3-dioxygenase 1 (1001) selected from: an inflammatory condition, an infectious disease, a cancer, a central nervous system disease or disorder, a disease or disorder relating to female reproductive health, coronary heart disease, chronic renal failure, post anaesthesia cognitive dysfunction, and cataracts.

## Description

### Field of the Invention

The present invention relates to novel pyrrolidine-fused myrtanol derivatives (or 4-azatricyclo[6.1.1.0^{2,6}]decanes) of the general formula (I) or pharmaceutically acceptable salts thereof.

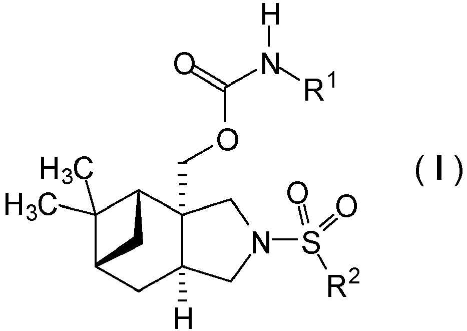

The invention further relates to pharmaceutical compositions comprising such compounds as well as their use as medicaments, especially in methods for the prophylaxis or treatment of a disease and/or a disorder caused by and/or associated with indolamine 2,3-dioxygenase 1 (IDO1) selected from: an inflammatory condition, an infectious disease, a cancer, a central nervous system disease or disorder, a disease or disorder relating to female reproductive health, coronary heart disease, chronic renal failure, post anaesthesia cognitive dysfunction, and cataracts.

### Background of the Invention

Indoleamine 2,3-dioxygenase 1 (IDO1) has a low level of natural expression throughout the body, but is expressed in a broad range of cancers to suppress the immune system (Uyttenhove et al., J. Nat. Med. 2003, 9, 1269). High IDO1 expression in clinical tumours has been shown to correlate with poor patient prognosis in a wide range of cancers including lung, colorectal, breast, melanoma and gynaecologic cancers. Silencing of the IDO1 gene in a murine melanoma cell line resulted in reduced capacity of the cells to form tumours when implanted into mice (Zheng et al., J. Immunol. 2006, 177, 5639), supporting IDO1 inhibition as a method of cancer intervention. A number of groups have pursued the development of small molecule inhibitors of IDO1 as an approach for restoring tumour immunity in cancer patients.

Increased expression of IDO has been shown to be an independent prognostic variable for reduced survival in patients with acute myeloid leukemia (AML), small-cell lung, melanoma, ovarian, colorectal, pancreatic, and endometrial cancers. The level of IDO expression was also shown to correlate with the number of tumour infiltrating lymphocytes in colorectal carcinoma patients.

Investigation of these novel pseudo-natural products in a cell-based assay monitoring the kynurenine (Kyn) pathway which is of high relevance to immune-suppression and diseases like neurodegeneration (D. C. Maddison., Giorgini, F., Semin. Cell Dev. Biol. 2015, 40, p.134-141.), auto-immune diseases (Krupa, A., Kowalska, I., Int. J. Mol. Sci. 2021, 22, 9879) and cancer (Basson, C., Serem, J. C., Hlophe, Y. N., Bipath, P., Cancer Medicine 2023, 12, p.18691-18701; Adams, J. L., Smothers, J., Srinivasan, R., Hoos, A. Nat. Rev. Drug Discovery 2015, 14, 603.) led to the discovery of a new indoleamine-dioxygenase 1 (IDO1) modulator chemotype.

It is object to the present invention to provide novel compounds as Inhibitor and Degrader of Indolamine 2,3-Dioxygenase 1 (IDO1) and/or pharmaceutically acceptable salts thereof, which can be used as pharmaceutically active agents, and use thereof especially for prophylaxis and/or treatment of a disease associated with and/or caused by indolamine 2,3-dioxygenase 1 (IDO1) selected from: an inflammatory condition, an infectious disease, a cancer, a central nervous system disease or disorder, a disease or disorder relating to female reproductive health, coronary heart disease, chronic renal failure, post anaesthesia cognitive dysfunction, and cataracts as well as compositions comprising at least one of those compounds and/or pharmaceutically acceptable salts thereof as pharmaceutically active ingredients.

The objective of the present invention is solved by the teaching of the independent claims. Further advantageous features, aspects and details of the invention are evident from the dependent claims, the description, and the examples of the present application.

### Description of the invention

Thus, the present invention is directed to a compound of the formula (I): 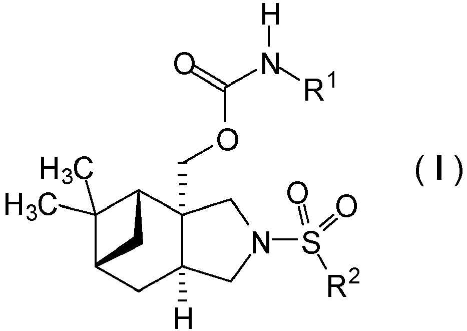 wherein
**R¹** represents 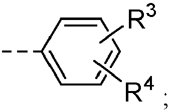
**R²** represents C₃-C₆ alkyl, C₃-C₆ cycloalkyl, 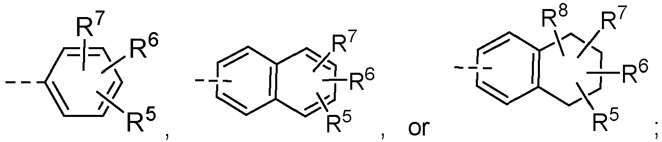
wherein C₃-C₆-cycloalkyl may be substituted with 1 to 4 substituents selected from **R⁵**, **R⁶**, **R⁷** and **R⁸**;
**R³**, and **R⁴** represent independently of each other -H, -Br, -Cl, -I, -CN, -CH₃, -C₂H₅, -CHF₂, -CF₃, -OCH₃, -OCHF₂, -OCF₃, -NH₂, -NHCH₃, -CH=CH₂, -CH₂-CH=CH₂, -C(CH₃)=CH₂, -CH=CH-CH₃, -CH=CH-C₂H₅, -CH=CH-C₂H₄OH, -CH=CH-C₃H₇, -C≡CH, -C≡C-CH₃, -C≡C-C₂H₅, -C≡C-C₂H₄OH, -C≡C-C₃H₇, -CH₂-C≡CH, -CH₂-C≡C-CH₃, -CH₂-C≡C-C₂H₅, -CH(CH₃)-C≡CH, -CH₂-C≡C-C₃H₇, or -C≡C-CH₂-CH(CH₃)₂;
**R⁵**, **R⁶**, **R⁷,** and **R⁸** represent independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -cyclo-C₃H₅, -cyclo-C₄H₇, -cyclo-C₅H₉, -cyclo-C₆H₁₁, -cyclo-C₇H₁₃, -cyclo-C₈H₁₅, -cycloC₃H₅O, -CH₂F, -CHF₂, -CF₃, -CH₂-CH₂F, -CH₂-CHF₂, -CH₂-CF₃, -Ph, -CH₂-Ph, -CH₂-CH₂-Ph, -CH=CH-Ph, -Si(CH₃)₃, -Si(CH₃)₂CH₂CH₃, -Si(CH₃)₂CH(CH₃)₂, -Si(CH₂CH₃)₃, -Si(CH₃)₂(C(CH₃)₃), -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂, -OCF₃, -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CON(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, , 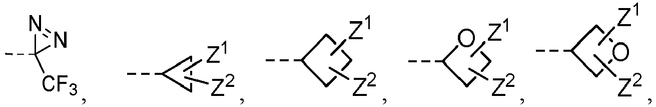 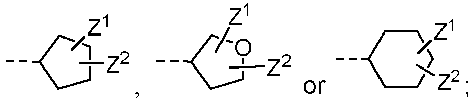 **R⁹** represents -CH₃, -CH₂F, -CHF₂, -CF₃, -C₂H₅, -C₃H₇, -CH(CH₃)₂, -C₄H₉, -CO₂H, -CO₂CH₃, -CO₂CH₂CH₃, -CO₂CH₂CH₂CH₃, -CO₂CH(CH₃)₂, -CO₂C(CH₃)₃, -CO₂-cyclo-C₃H₅, -CO₂-CH₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CONHC(CH₃)₃, -CON(CH₃)₂, -CON(CH₃)(CH₂CH₃), -CON(CH₂CH₃)₂, -CON(CH₂CH₂CH₃)₂, CON(CH(CH₃)₂)₂, -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃, or 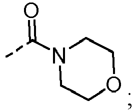
**Z¹**, and **Z²** represent indepenently of each other -H, -F, -Cl, -Br, -CH₃, -C₂H₅, -CH₂F, -CHF₂, -CF₃, -OCH₃, -OC₂H₅, -OCH₂F, -OCHF₂, -OCF₃, -CH₂-CF₃, or-CF₂-CF₃;
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

The term "pharmaceutically acceptable salt" refers to a salt of a compound that does not cause significant irritation to an organism to which it is administered and does not abrogate the biological activity and properties of the compound. The compounds of the present invention may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, china acid, mandelic acid, o-methylmandelic acid, hydrogen-benzenesulfonic acid, picric acid, adipic acid, D-o-tolyltartaric acid, tartronic acid, (o, m, p)-toluic acid, naphthylamine sulfonic acid, trifluoroacetic acid, and other mineral or carboxylic acids well known to those skilled in the art. The salts are prepared by contacting the free base form of the compounds of formula (I) with a sufficient amount of the desired acid to produce a salt in the conventional manner well known to those skilled in the art.

In the case the inventive compounds bear acidic groups, salts could also be formed with inorganic or organic bases. Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) with a solution of an acid, selected out of the group mentioned above.

As used herein, **C₃-C₆ alkyl** refers to -CH₂CH₂CH₃, -CH(CH₃)₂, -CH₂CH₂CH₂CH₃, -CH₂CH(CH₃)₂, -CH(CH₃)(CH₂CH₃), -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃.

As used herein, **C₃-C₆-cycloalkyl** refers to cyclo-C₃H₅, cyclo-C₄H₇, cyclo-C₅H₉, and cyclo-C₆H₁₁, wherein these residues can be substituted with 1 to 4 substituents selected from **R⁵**, **R⁶**, **R⁷** and **R⁸**. However it is clear to a skilled person that the term "can be substituted" refers to the replacement of a hydrogen atom by one of the substituents **R⁵**, **R⁶**, **R⁷** and **R⁸**. The carbon atom number of **C₃-C₆** refers only to the carbon atoms of the cycloalkyl residue and does not include the carbon atoms of the substituents **R⁵** to **R⁸**.

Preferably, in the formula (**I**), **R¹** represents 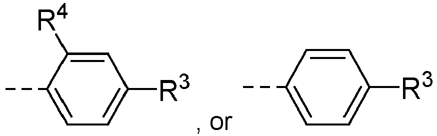 and **R³**, **R⁴**, **R⁵** and **R⁶** have the same meansings as defined in the formula (I).

Preferably, in the formula (**I**), **R²** represents C₃-C₆ alkyl, 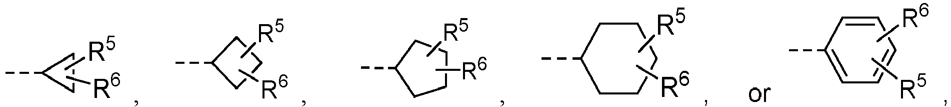 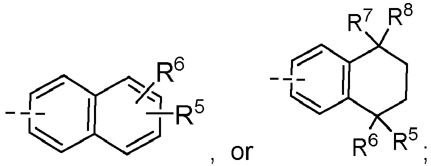 more preferably, **R²** represents C₃-C₆ alkyl, 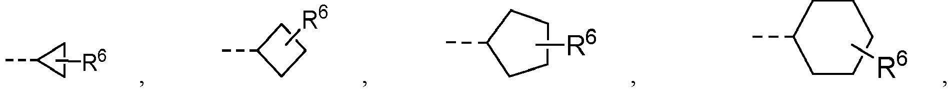 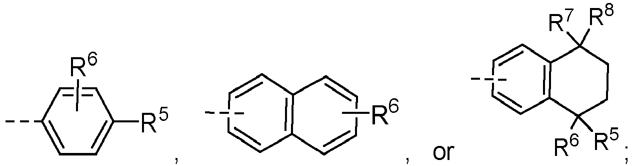 still more preferably, **R²** represents C₃-C₅ alkyl,

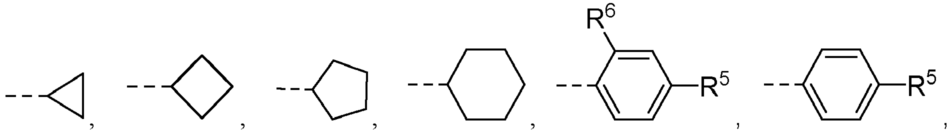

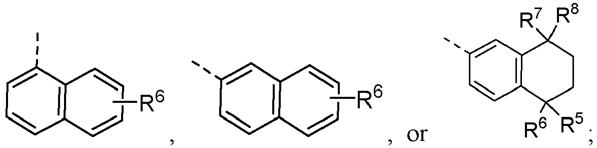

Still more preferably,
**R²** represents -CH(CH₃)₂, -CH₂CH(CH₃)₂, -CH(CH₃)(C₂H₅), 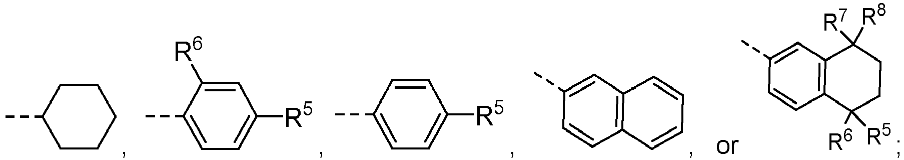 and **R⁵**, **R⁶**, **R⁷**, and **R⁸** have the same meansings as defined in the formula (**I**).

Preferably, in the formula (**I**), **R³** and **R⁴** represents independently of each other -H, -Br, -Cl, -I, -CN, -CH₃, -C₂H₅, -CHF₂, -CF₃, -OCH₃, -OCHF₂, -OCF₃, -NH₂, -CH=CH₂, -CH=CH-CH₃, -CH=CH-C₂H₅, -CH=CH-C₂H₄OH, -C=CH, -C≡C-CH₃, -C≡C-C₂H₅, -C≡C-C₂H₄OH, -CH₂-C≡CH, -CH₂-C≡C-CH₃, or -CH₂-C≡C-C₂H₅; more preferably, -H, -Br, -Cl, -I, -CN, -CH₃, -C₂H₅, -CHF₂, -CF₃, -OCH₃, -OCHF₂, -OCF₃, -NH₂, -CH=CH₂, -CH=CH-CH₃, -CH=CH-C₂H₅, -CH=CH-C₂H₄OH, -C=CH, -C≡C-CH₃, -C≡C-C₂H₅, or -C=C-C₂H₄OH; still more preferably, -H, -Br, -Cl, -I, -CN, -CH₃, -C₂H₅, -CHF₂, -CF₃, -OCH₃, -OCHF₂, -OCF₃, -NH₂, -C=CH, -C≡C-CH₃, -C≡C-C₂H₅, or -C=C-C₂H₄OH; and/or
preferably, in the formula (**I**), **R⁵**, **R⁶**, **R⁷**, and **R⁸** represent independently of each other -H, -F, -Cl, -Br, -I, -CN, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -cyclo-C₃H₅, -cyclo-C₄H₇, -cyclo-C₅H₉, -cyclo-C₆H₁₁, -cycloC₃H₅O, -CH₂F, -CHF₂, -CF₃, -CH₂-CH₂F, -CH₂-CHF₂, -CH₂-CF₃, -Ph, -CH₂-Ph, -CH₂-CH₂-Ph, -CH=CH-Ph, -Si(CH₃)₃, -Si(CH₃)₂CH₂CH₃, -Si(CH₃)₂CH(CH₃)₂, -Si(CH₂CH₃)₃, -Si(CH₃)₂(C(CH₃)₃), -OCH₃, -OCH₂F, -OCHF₂, -OCF₃, -OC₂H₅, -OCH₂CF₃, -OC₃H₇, -O-cyclo-C₃H₅, -OC₄H₉, -OCH₂CH(CH₃)₂, -OCH(CH₃)-C₂H₅, -OCH₂CH(CH₃)₂, -OC(CH₃)₃, 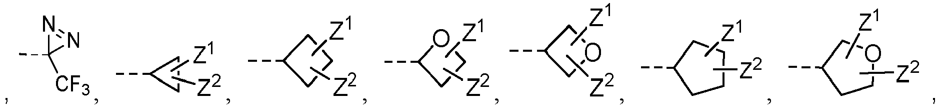 or 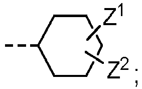
more preferably, **R⁵**, **R⁶**, **R⁷**, and **R⁸** represent independently of each other -H, -F, - Cl, -Br, -CN, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -CH₂C(CH₃)₃, -cyclo-C₃H₅, -cyclo-C₄H₇, -cyclo-C₅H₉, -cyclo-C₆H₁₁, -cycloC₃H₅O, -CH₂F, -CHF₂, -CF₃, -CH₂-CH₂F, -CH₂-CHF₂, -CH₂-CF₃, -Ph, -Si(CH₃)₃, -Si(CH₃)₂CH₂CH₃, -Si(CH₂CH₃)₃, -OCH₃, -OCH₂F, -OCHF₂, -OCF₃, -OC₂H₅, -OCH₂CF₃, -OC₃H₇, -OCH(CH₃)₂, -O-cyclo-C₃H₅, 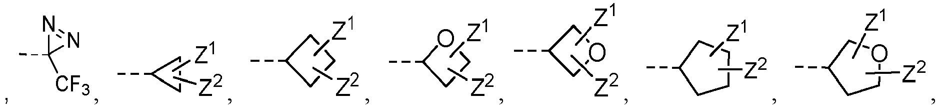 or 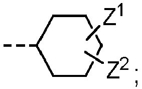
still more preferably, **R⁵**, **R⁶**, **R⁷**, and **R⁸** represent independently of each other -H, -F, -Cl, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -CH₂C(CH₃)₃, -cyclo-C₃H₅, -cyclo-C₄H₇, -cyclo-C₅H₉, -cyclo-C₆H₁₁, -CH₂F, -CHF₂, -CF₃, -CH₂-CH₂F, -CH₂-CHF₂, -CH₂-CF₃, -Ph, -Si(CH₃)₃, -OCH₃, -OCH₂F, -OCHF₂, -OCF₃, -OC₂H₅, -OCH₂CF₃, -OCH(CH₃)₂, -O-cyclo-C₃H₅, , 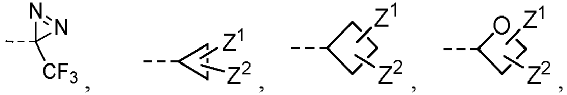 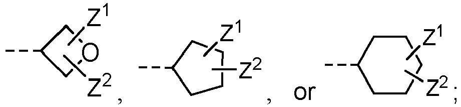
**R⁹** represents -CH₃, -CH₂F, -CHF₂, -CF₃, -C₂H₅, -C₃H₇, -CH(CH₃)₂, -C₄H₉, - CO₂H, -CO₂CH₃, -CO₂CH₂CH₃, -CO₂CH₂CH₂CH₃, -CO₂CH(CH₃)₂, -CO₂C(CH₃)₃, -CO₂-cyclo-C₃H₅, -CO₂-CH₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CONHC(CH₃)₃, -CON(CH₃)₂, -CON(CH₃)(CH₂CH₃), -CON(CH₂CH₃)₂, -CON(CH₂CH₂CH₃)₂, CON(CH(CH₃)₂)₂, -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃, or 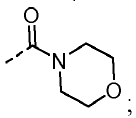 preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃, or 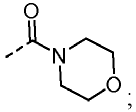
more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CONHCH₃, -CONHCH₂CH₃, -CONHCH₂CH₂CH₃, -CONHCH(CH₃)₂, -CH₂CO₂H, -CH₂CO₂CH₃, -CH₂CO₂CH₂CH₃, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂O(CH₂)₂OCH₃, or 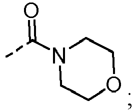
still more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CON(CH₃)₂, -CON(CH₃)(CH₂CH₃), -CON(CH₂CH₃)₂, , -CH₂CO₂H, -CH₂CO₂CH₃, -CH₂CO₂CH₂CH₃, -CH₂N(CH₃)₂, -CH₂N(CH₂CH₃)₂, -CH₂O(CH₂)₂OCH₃, or 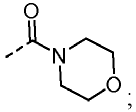
**Z¹,** and **Z²** represent indepenently of each other -H, -F, -Cl, -Br, -CH₃, -C₂H₅, -CH₂F, -CHF₂, -CF₃, -OCH₃, -OC₂H₅, -OCH₂F, -OCHF₂, -OCF₃, -CH₂-CF₃, or -CF₂-CF₃; preferably, -H, -F, -CH₃, -CH₂F, -CHF₂, -CF₃, -OCH₃, -OC₂H₅, -OCH₂F, -OCHF₂, -OCF₃; more preferably, -H, -F, -CH₃, -CH₂F, -CHF₂, -CF₃; more preferably, -H, -F, -CH₃, or -CF₃.

Preferably, the present application is directed to the compound of any one of the formulae (**I-1**) - (**I-4**): 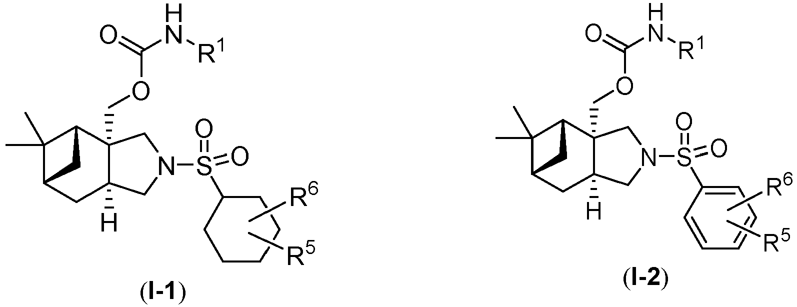 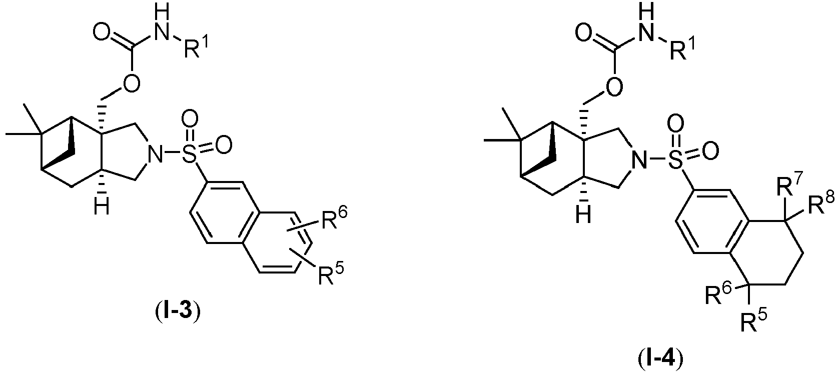
wherein **R¹**, **R⁵**, **R⁶**, **R⁷**, and **R⁸** have the same meansings as defined in the formula (I),
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Preferably, in the formulae (**I-1**) - (**I-4**), **R³** and **R⁴** represents independently of each other -H, -Br, -Cl, -I, -CN, -CH₃, -C₂H₅, -CHF₂, -CF₃, -OCH₃, -OCHF₂, -OCF₃, -CH=CH₂, -CH=CH-CH₃, -CH=CH-C₂H₅, -CH=CH-C₂H₄OH, -C=CH, -C≡C-CH₃, -C≡C-C₂H₅, -C≡C-C₂H₄OH, -CH₂-C≡CH, -CH₂-C≡C-CH₃, or -CH₂-C≡C-C₂H₅; more preferably, -H, -Br, -Cl, -I, -CN, -CH₃, -C₂H₅, -CHF₂, -CF₃, -OCH₃, -OCHF₂, -OCF₃, -CH=CH₂, -CH=CH-CH₃, -CH=CH-C₂H₅, -CH=CH-C₂H₄OH, -C=CH, -C≡C-CH₃, -C≡C-C₂H₅, or -C=C-C₂H₄OH; still more preferably, -H, -Br, -Cl, -I, -CN, -CH₃, -C₂H₅, -CHF₂, -CF₃, -OCH₃, -OCHF₂, -OCF₃, -C=CH, -C≡C-CH₃, -C≡C-C₂H₅, or -C≡C-C₂H₄OH; still more preferably, -H, -Br, -Cl, -I, -C=CH, -CH₃; most preferably, -I, -C≡CH, and/or
preferably, in the formulae (**I-1**) -( **I-4), R⁵**, **R⁶**, **R⁷**, and **R⁸** represent independently of each other -H, -F, -Cl, -Br, -I, -CN, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -cyclo-C₃H₅, -cyclo-C₄H₇, -cyclo-C₅H₉, -cyclo-C₆H₁₁, -cycloC₃H₅O, -CH₂F, -CHF₂, -CF₃, -CH₂-CH₂F, -CH₂-CHF₂, -CH₂-CF₃, -Ph, -CH₂-Ph, -CH₂-CH₂-Ph, -CH=CH-Ph, -Si(CH₃)₃, -Si(CH₃)₂CH₂CH₃, -Si(CH₃)₂CH(CH₃)₂, -Si(CH₂CH₃)₃, -Si(CH₃)₂(C(CH₃)₃), -OCH₃, -OCH₂F, -OCHF₂, -OCF₃, -OC₂H₅, -OCH₂CF₃, -OC₃H₇, -O-cyclo-C₃H₅, -OC₄H₉, -OCH₂CH(CH₃)₂, -OCH(CH₃)-C₂H₅, -OCH₂CH(CH₃)₂, -OC(CH₃)₃, 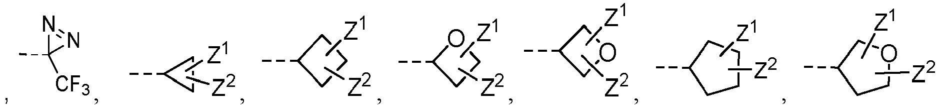 or 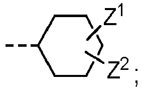
more preferably, **R⁵**, **R⁶**, **R⁷**, and **R⁸** represent independently of each other -H, -F, - Cl, -Br, -CN, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -CH₂C(CH₃)₃, -cyclo-C₃H₅, -cyclo-C₄H₇, -cyclo-C₅H₉, -cyclo-C₆H₁₁, -cycloC₃H₅O, -CH₂F, -CHF₂, -CF₃, -CH₂-CH₂F, -CH₂-CHF₂, -CH₂-CF₃, -Ph, -Si(CH₃)₃, -Si(CH₃)₂CH₂CH₃, -Si(CH₂CH₃)₃, -OCH₃, -OCH₂F, -OCHF₂, -OCF₃, -OC₂H₅, -OCH₂CF₃, -OC₃H₇, -OCH(CH₃)₂, -O-cyclo-C₃H₅, 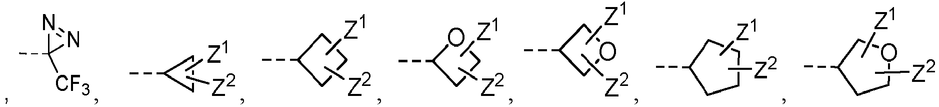 or 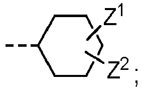
still more preferably, **R⁵**, **R⁶**, **R⁷**, and **R⁸** represent independently of each other -H, -F, -Cl, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -CH₂C(CH₃)₃, -cyclo-C₃H₅, -cyclo-C₄H₇, -cyclo-C₅H₉, -cyclo-C₆H₁₁, -CH₂F, -CHF₂, -CF₃, -CH₂-CH₂F, -CH₂-CHF₂, -CH₂-CF₃, -Ph, -Si(CH₃)₃, -OCH₃, -OCH₂F, -OCHF₂, -OCF₃, -OC₂H₅, -OCH₂CF₃, -OCH(CH₃)₂, -O-cyclo-C₃H₅, , 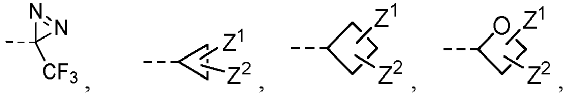 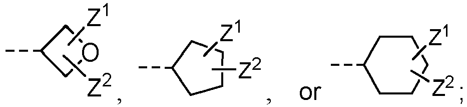
**R⁹** represents -CH₃, -CH₂F, -CHF₂, -CF₃, -C₂H₅, -C₃H₇, -CH(CH₃)₂, -C₄H₉, - CO₂H, -CO₂CH₃, -CO₂CH₂CH₃, -CO₂CH₂CH₂CH₃, -CO₂CH(CH₃)₂, -CO₂C(CH₃)₃, -CO₂-cyclo-C₃H₅, -CO₂-CH₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CONHC(CH₃)₃, -CON(CH₃)₂, -CON(CH₃)(CH₂CH₃), -CON(CH₂CH₃)₂, -CON(CH₂CH₂CH₃)₂, CON(CH(CH₃)₂)₂, -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃, or 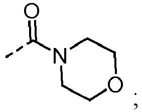
preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃,
   or 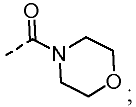
more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CONHCH₃, -CONHCH₂CH₃, -CONHCH₂CH₂CH₃, -CONHCH(CH₃)₂, -CH₂CO₂H, -CH₂CO₂CH₃, -CH₂CO₂CH₂CH₃, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂O(CH₂)₂OCH₃, or 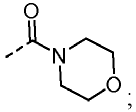
still more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CON(CH₃)₂, -CON(CH₃)(CH₂CH₃), -CON(CH₂CH₃)₂, -CH₂CO₂H, -CH₂CO₂CH₃, -CH₂CO₂CH₂CH₃, -CH₂N(CH₃)₂, -CH₂N(CH₂CH₃)₂, -CH₂O(CH₂)₂OCH₃, or 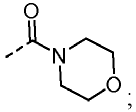
**Z¹**, and **Z²** represent indepenently of each other -H, -F, -Cl, -Br, -CH₃, -C₂H₅, -CH₂F, -CHF₂, -CF₃, -OCH₃, -OC₂H₅, -OCH₂F, -OCHF₂, -OCF₃, -CH₂-CF₃, or -CF₂-CF₃; preferably, -H, -F, -CH₃, -CH₂F, -CHF₂, -CF₃, -OCH₃, -OC₂H₅, -OCH₂F, -OCHF₂, -OCF₃; more preferably, -H, -F, -CH₃, -CH₂F, -CHF₂, -CF₃; more preferably, -H, -F, -CH₃, or -CF₃.

Preferably, the present application is directed to the compound of the formula (**II**): 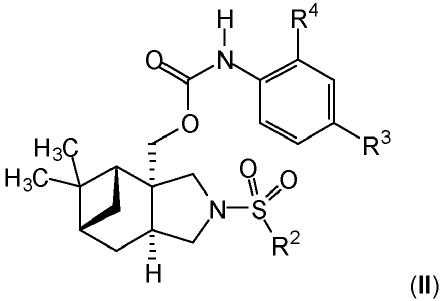 and **R²**, **R³** and **R⁴** have the same meanings as defined in the formula (**I**),
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Preferably, in the formula (**II**), **R²** represents C₃-C₆ alkyl, 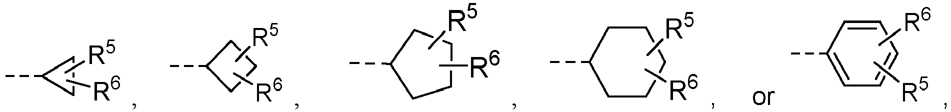 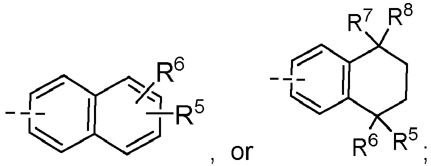 more preferably, **R²** represents C₃-C₆ alkyl, 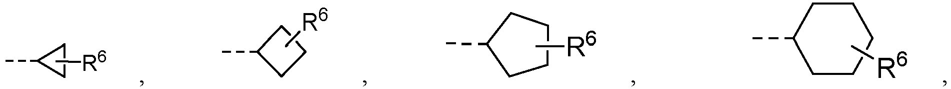 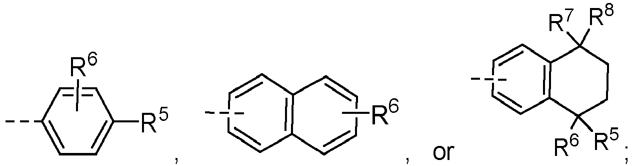 still more preferably, **R²** represents C₃-C₅ alkyl,

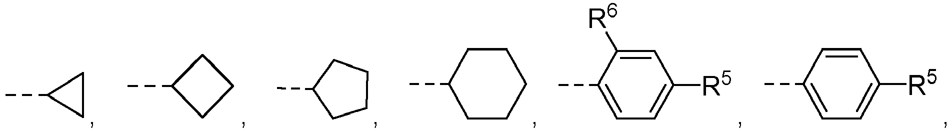

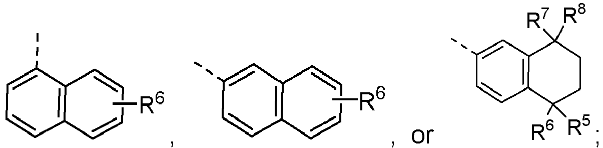

Still more preferably,
**R²** represents -CH(CH₃)₂, -CH₂CH(CH₃)₂, -CH(CH₃)(C₂H₅), 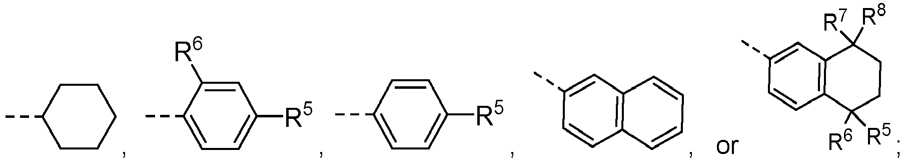
and **R⁵**, **R⁶**, **R⁷**, and **R⁸** have the same meansings as defined in the formula (**I**).

Preferably, the present application is directed to the compound of any one of the formulae (**II-1**) - (**II-4**): 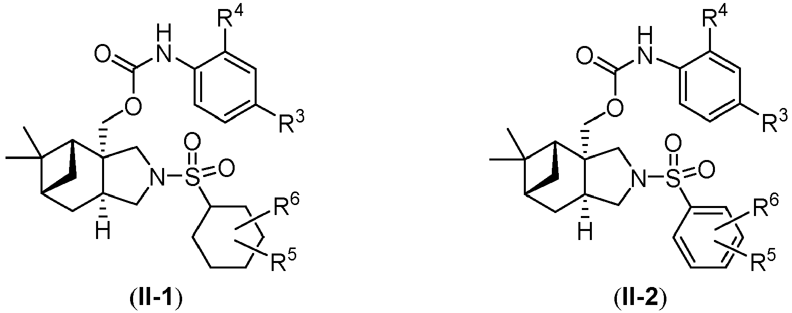 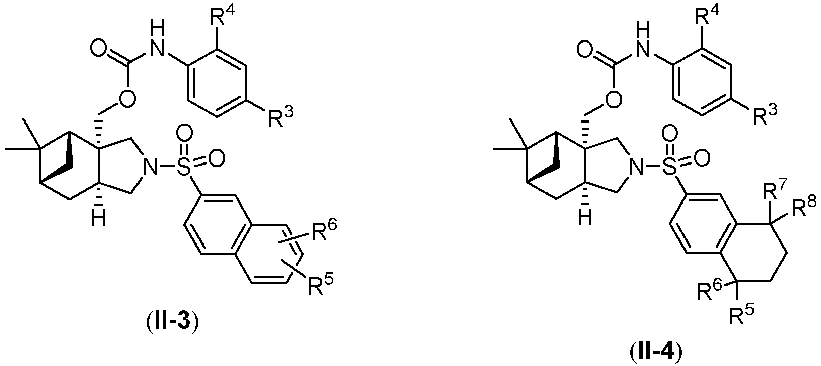
wherein **R³**, **R⁴**, **R⁵**, **R⁶**, **R⁷**, and **R⁸** have the same meansings as defined in the formula (**I**),
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Preferably, in the formulae (**II-1**) **-**(**II-4), R³** and **R⁴** represents independently of each other -H, -Br, -Cl, -I, -CN, -CH₃, -C₂H₅, -CHF₂, -CF₃, -OCH₃, -OCHF₂, -OCF₃, -CH=CH₂, -CH=CH-CH₃, -CH=CH-C₂H₅, -CH=CH-C₂H₄OH, -C≡CH, -C≡C-CH₃, -C≡C-C₂H₅, -C≡C-C₂H₄OH, -CH₂-C≡CH, -CH₂-C≡C-CH₃, or -CH₂-C≡C-C₂H₅; more preferably, -H, -Br, -Cl, -I, -CN, -CH₃, -C₂H₅, -CHF₂, -CF₃, -OCH₃, -OCHF₂, -OCF₃, -CH=CH₂, -CH=CH-CH₃, -CH=CH-C₂H₅, -CH=CH-C₂H₄OH, -C=CH, -C≡C-CH₃, -C≡C-C₂H₅, or -C=C-C₂H₄OH; still more preferably, -H, -Br, -Cl, -I, -CN, -CH₃, -C₂H₅, -CHF₂, -CF₃, -OCH₃, -OCHF₂, -OCF₃, -C=CH, -C≡C-CH₃, -C≡C-C₂H₅, or -C≡C-C₂H₄OH; still more preferably, -H, -Br, -Cl, -I, -C≡CH, -CH₃; most preferably, -I, -C≡CH, and/or
preferably, in the formula (**II-1**) or (**II-2), R⁵**, **R⁶**, **R⁷**, and **R⁸** represent independently of each other -H, -F, -Cl, -Br, -I, -CN, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -cyclo-C₃H₅, -cyclo-C₄H₇, -cyclo-C₅H₉, -cyclo-C₆H₁₁, -cycloC₃H₅O, -CH₂F, -CHF₂, -CF₃, -CH₂-CH₂F, -CH₂-CHF₂, -CH₂-CF₃, -Ph, -CH₂-Ph, -CH₂-CH₂-Ph, -CH=CH-Ph, -Si(CH₃)₃, -Si(CH₃)₂CH₂CH₃, -Si(CH₃)₂CH(CH₃)₂, -Si(CH₂CH₃)₃, -Si(CH₃)₂(C(CH₃)₃), -OCH₃, -OCH₂F, -OCHF₂, -OCF₃, -OC₂H₅, -OCH₂CF₃, -OC₃H₇, -O-cyclo-C₃H₅, -OC₄H₉, -OCH₂CH(CH₃)₂, -OCH(CH₃)-C₂H₅, -OCH₂CH(CH₃)₂, -OC(CH₃)₃, 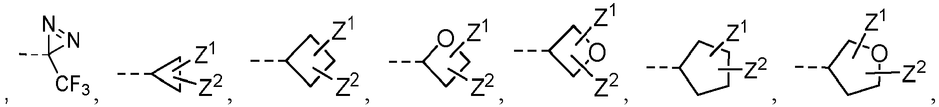 or 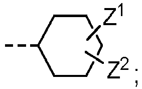
more preferably, **R⁵**, **R⁶**, **R⁷**, and **R⁸** represent independently of each other -H, -F, - Cl, -Br, -CN, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -CH₂C(CH₃)₃, -cyclo-C₃H₅, -cyclo-C₄H₇, -cyclo-C₅H₉, -cyclo-C₆H₁₁, -cycloC₃H₅O, -CH₂F, -CHF₂, -CF₃, -CH₂-CH₂F, -CH₂-CHF₂, -CH₂-CF₃, -Ph, -Si(CH₃)₃, -Si(CH₃)₂CH₂CH₃, -Si(CH₂CH₃)₃, -OCH₃, -OCH₂F, -OCHF₂, -OCF₃, -OC₂H₅, -OCH₂CF₃, -OC₃H₇, -OCH(CH₃)₂, -O-cyclo-C₃H₅, 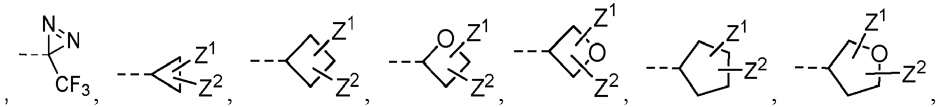 or 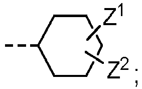
still more preferably, **R⁵**, **R⁶**, **R⁷**, and **R⁸** represent independently of each other -H, -F, -Cl, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -CH₂C(CH₃)₃, -cyclo-C₃H₅, -cyclo-C₄H₇, -cyclo-C₅H₉, -cyclo-C₆H₁₁, -CH₂F, -CHF₂, -CF₃, -CH₂-CH₂F, -CH₂-CHF₂, -CH₂-CF₃, -Ph, -Si(CH₃)₃, -OCH₃, -OCH₂F, -OCHF₂, -OCF₃, -OC₂H₅, -OCH₂CF₃, -OCH(CH₃)₂, -O-cyclo-C₃H₅, , 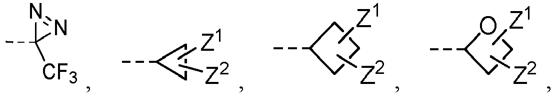 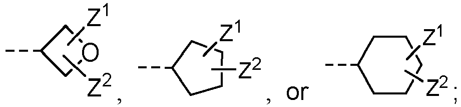
**R⁹** represents -CH₃, -CH₂F, -CHF₂, -CF₃, -C₂H₅, -C₃H₇, -CH(CH₃)₂, -C₄H₉, - CO₂H, -CO₂CH₃, -CO₂CH₂CH₃, -CO₂CH₂CH₂CH₃, -CO₂CH(CH₃)₂, -CO₂C(CH₃)₃, -CO₂-cyclo-C₃H₅, -CO₂-CH₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CONHC(CH₃)₃, -CON(CH₃)₂, -CON(CH₃)(CH₂CH₃), -CON(CH₂CH₃)₂, -CON(CH₂CH₂CH₃)₂, CON(CH(CH₃)₂)₂, -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃, or 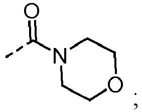 preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃,
   or 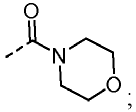
more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CONHCH₃, -CONHCH₂CH₃, -CONHCH₂CH₂CH₃, -CONHCH(CH₃)₂, -CH₂CO₂H, -CH₂CO₂CH₃, -CH₂CO₂CH₂CH₃, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂O(CH₂)₂OCH₃, or 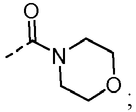
still more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CON(CH₃)₂, -CON(CH₃)(CH₂CH₃), -CON(CH₂CH₃)₂, -CH₂CO₂H, -CH₂CO₂CH₃, -CH₂CO₂CH₂CH₃, -CH₂N(CH₃)₂, -CH₂N(CH₂CH₃)₂, -CH₂O(CH₂)₂OCH₃, or 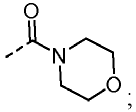
**Z¹**, and **Z²** represent indepenently of each other -H, -F, -Cl, -Br, -CH₃, -C₂H₅, -CH₂F, -CHF₂, -CF₃, -OCH₃, -OC₂H₅, -OCH₂F, -OCHF₂, -OCF₃, -CH₂-CF₃, or -CF₂-CF₃; preferably, -H, -F, -CH₃, -CH₂F, -CHF₂, -CF₃, -OCH₃, -OC₂H₅, -OCH₂F, -OCHF₂, -OCF₃; more preferably, -H, -F, -CH₃, -CH₂F, -CHF₂, -CF₃; more preferably, -H, -F, -CH₃, or -CF₃.

Preferably, in the formula (**II-1**) or (**II-2**), **R⁵**, **R⁶**, **R⁷**, and **R⁸** represent independently of each other -H, -F, -Cl, -Br, -I, -CN, -CH₃, -CH₂F, -CHF₂, -CF₃, -C₂H₅, -C₃H₇, -CH₂-C(CH₃)₃, or -cyclo-C₃H₅; more preferably -H, -F, -Cl, -CH₃, -CH₂F, -CHF₂, -CF₃, or -C₂H₅; still more preferably -H, -F, -CH₃, -CH₂F, -CHF₂, or -CF₃; still more preferably -H, or -CH₃.

Preferably, the present application is directed to the compound of the formula (**III**): 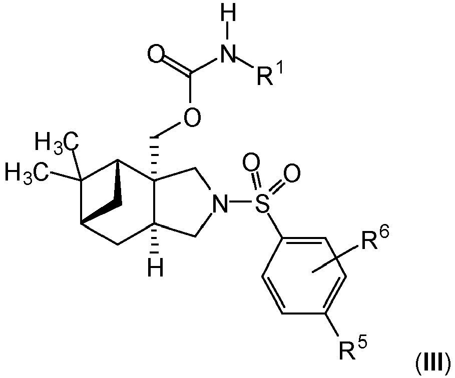 and **R¹**, **R⁵** and **R⁶** have the same meanings as defined in the formula (**I**), or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Preferably, in the formula (**III**), **R¹** represents 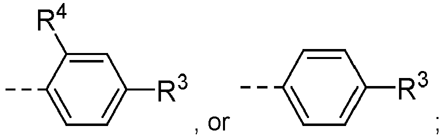 and **R³**, and **R⁶** have the same meansings as defined in the formula (**I**).

More preferably, the present application is directed to the compound of the formula (**IV**): 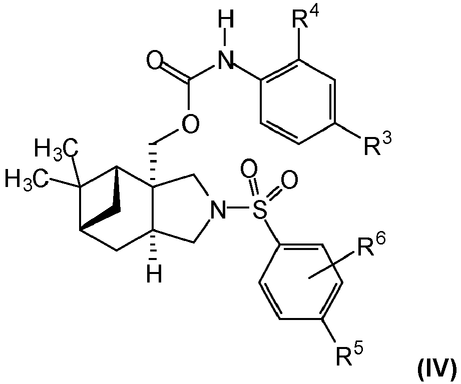 and **R³**, **R⁴**, **R⁵** and **R⁶** have the same meanings as defined in the formula (**I**),
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Still more preferably, the present application is directed to the compound of any one of the formulae (**IV-1**) to (**IV-4**): 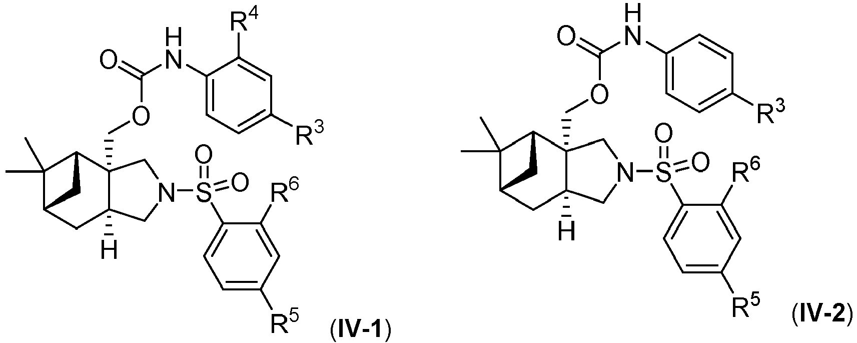 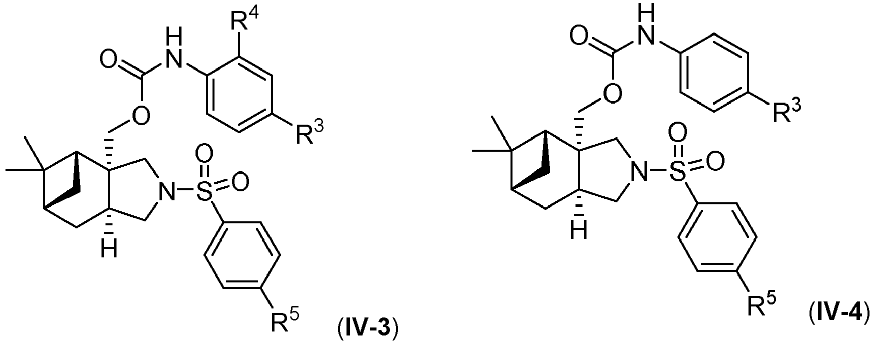 and **R³**, **R⁴**, **R⁵** and **R⁶** have the same meanings as defined in the formula (**I**), or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Preferably, in the formulae **(IV), (IV-1) -(IV-4), R³** and **R⁴** represents independently of each other -H, -Br, -Cl, -I, -CN, -CH₃, -C₂H₅, -CHF₂, -CF₃, -OCH₃, -OCHF₂, -OCF₃, -CH=CH₂, -CH=CH-CH₃, -CH=CH-C₂H₅, -CH=CH-C₂H₄OH, -C=CH, -C≡C-CH₃, -C≡C-C₂H₅, -C≡C-C₂H₄OH, -CH₂-C≡CH, -CH₂-C≡C-CH₃, or -CH₂-C≡C-C₂H₅; more preferably, -H, -Br, -Cl, -I, -CN, -CH₃, -C₂H₅, -CHF₂, -CF₃, -OCH₃, -OCHF₂, -OCF₃, -CH=CH₂, -CH=CH-CH₃, -CH=CH-C₂H₅, -CH=CH-C₂H₄OH, -C=CH, -C≡C-CH₃, -C≡C-C₂H₅, or -C=C-C₂H₄OH; still more preferably, -H, -Br, -Cl, -I, -CN, -CH₃, -C₂H₅, -CHF₂, -CF₃, -OCH₃, -OCHF₂, -OCF₃, -C=CH, -C≡C-CH₃, -C≡C-C₂H₅, or -C≡C-C₂H₄OH; still more preferably, -H, -Br, -Cl, -I, -C=CH, -CH₃; most preferably, -I, -C=CH, and/or
preferably, in the formula formulae (**IV**), (**IV-1**) -(**IV-4**), **R⁵**, and **R⁶** represent independently of each other -H, -F, -Cl, -Br, -I, -CN, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -cyclo-C₃H₅, -cyclo-C₄H₇, -cyclo-C₅H₉, -cyclo-C₆H₁₁, -cycloC₃H₅O, -CH₂F, -CHF₂, -CF₃, -CH₂-CH₂F, -CH₂-CHF₂, -CH₂-CF₃, -Ph, -CH₂-Ph, -CH₂-CH₂-Ph, -CH=CH-Ph, -Si(CH₃)₃, -Si(CH₃)₂CH₂CH₃, -Si(CH₃)₂CH(CH₃)₂, -Si(CH₂CH₃)₃, -Si(CH₃)₂(C(CH₃)₃), -OCH₃, -OCH₂F, -OCHF₂, -OCF₃, -OC₂H₅, -OCH₂CF₃, -OC₃H₇, -O-cyclo-C₃H₅, -OC₄H₉, -OCH₂CH(CH₃)₂, -OCH(CH₃)-C₂H₅, -OCH₂CH(CH₃)₂, -OC(CH₃)₃, 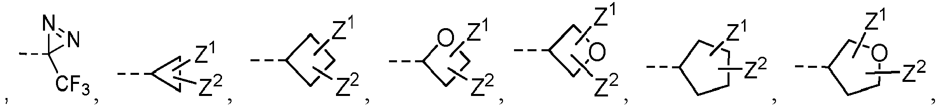 or 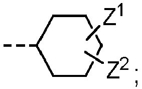
more preferably, **R⁵**, and **R⁶** represent independently of each other -H, -F, -Cl, -Br, -CN, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -CH₂C(CH₃)₃, -cyclo-C₃H₅, -cyclo-C₄H₇, -cyclo-C₅H₉, -cyclo-C₆H₁₁, -cycloC₃H₅O, -CH₂F, -CHF₂, -CF₃, -CH₂-CH₂F, -CH₂-CHF₂, -CH₂-CF₃, -Ph, -Si(CH₃)₃, -Si(CH₃)₂CH₂CH₃, -Si(CH₂CH₃)₃, -OCH₃, -OCH₂F, -OCHF₂, -OCF₃, -OC₂H₅, -OCH₂CF₃, -OC₃H₇, -OCH(CH₃)₂, -O-cyclo-C₃H₅, 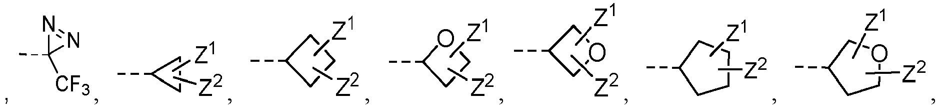 or 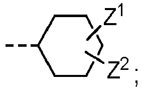
still more preferably, **R⁵**, and **R⁶** represent independently of each other -H, -F, -Cl, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -CH₂C(CH₃)₃, -cyclo-C₃H₅, -cyclo-C₄H₇, -cyclo-C₅H₉, -cyclo-C₆H₁₁, -CH₂F, -CHF₂, -CF₃, -CH₂-CH₂F, -CH₂-CHF₂, -CH₂-CF₃, -Ph, -Si(CH₃)₃, -OCH₃, -OCH₂F, -OCHF₂, -OCF₃, -OC₂H₅, -OCH₂CF₃, -OCH(CH₃)₂, -O-cyclo-C₃H₅, , 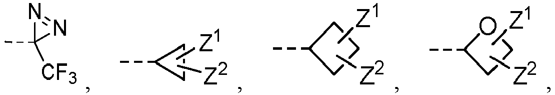 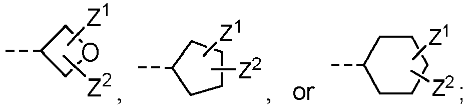
**R⁹** represents -CH₃, -CH₂F, -CHF₂, -CF₃, -C₂H₅, -C₃H₇, -CH(CH₃)₂, -C₄H₉, - CO₂H, -CO₂CH₃, -CO₂CH₂CH₃, -CO₂CH₂CH₂CH₃, -CO₂CH(CH₃)₂, -CO₂C(CH₃)₃, -CO₂-cyclo-C₃H₅, -CO₂-CH₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CONHC(CH₃)₃, -CON(CH₃)₂, -CON(CH₃)(CH₂CH₃), -CON(CH₂CH₃)₂, -CON(CH₂CH₂CH₃)₂, CON(CH(CH₃)₂)₂, -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃, or 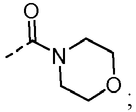 preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃,
   or 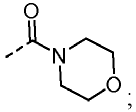
more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CONHCH₃, -CONHCH₂CH₃, -CONHCH₂CH₂CH₃, -CONHCH(CH₃)₂, -CH₂CO₂H, -CH₂CO₂CH₃, -CH₂CO₂CH₂CH₃, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂O(CH₂)₂OCH₃, or 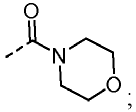
still more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CON(CH₃)₂, -CON(CH₃)(CH₂CH₃), -CON(CH₂CH₃)₂, -CH₂CO₂H, -CH₂CO₂CH₃, -CH₂CO₂CH₂CH₃, -CH₂N(CH₃)₂, -CH₂N(CH₂CH₃)₂, -CH₂O(CH₂)₂OCH₃, or 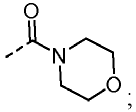
still more preferably, **R⁹** represents -CH₃, -CH₂F, -CHF₂, -CF₃, -CO₂H, -CO₂CH₃, -CO₂CH₂CH₃, -CH₂CO₂H, -CH₂CO₂CH₃, -CH₂CO₂CH₂CH₃; most preferably, **R⁹** represents -CH₃, -CO₂H, -CO₂CH₃, -CH₂CO₂H, or -CH₂CO₂CH₃;
**Z¹**, and **Z²** represent indepenently of each other -H, -F, -Cl, -Br, -CH₃, -C₂H₅, -CH₂F, -CHF₂, -CF₃, -OCH₃, -OC₂H₅, -OCH₂F, -OCHF₂, -OCF₃, -CH₂-CF₃, or -CF₂-CF₃; preferably, -H, -F, -CH₃, -CH₂F, -CHF₂, -CF₃, -OCH₃, -OC₂H₅, -OCH₂F, -OCHF₂, -OCF₃; more preferably, -H, -F, -CH₃, -CH₂F, -CHF₂, -CF₃; more preferably, -H, -F, -CH₃, or -CF₃.

Still more preferably, the present application is directed to the compound of any one of the formulae (**V-1**) - (**V-4**): 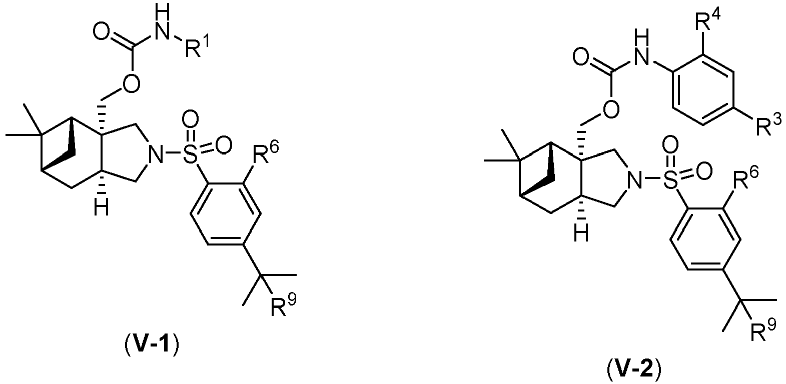 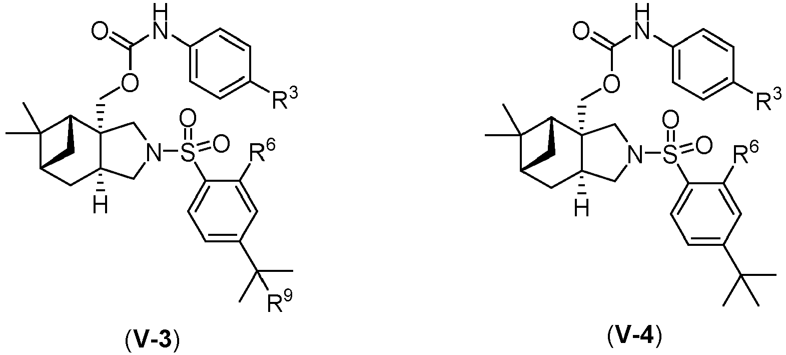 wherein **R¹**, **R³**, **R⁴**, **R⁶**, **R⁶** and **R⁹** have the same meanings as defined in the formula (**I**),
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Preferably, in the formula (**V-1**), **R¹** represents 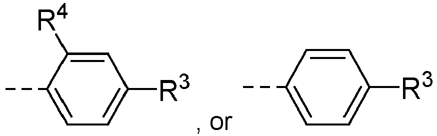 and **R³**, and **R⁶** have the same meansings as defined in the formula (I).

Preferably, in the formulae (**V-2**) - (**V-4**), **R³** and **R⁴** represents independently of each other -H, -Br, -Cl, -I, -CN, -CH₃, -C₂H₅, -CHF₂, -CF₃, -OCH₃, -OCHF₂, -OCF₃, -CH=CH₂, -CH=CH-CH₃, -CH=CH-C₂H₅, -CH=CH-C₂H₄OH, -C≡CH, -C≡C-CH₃, -C≡C-C₂H₅, -C≡C-C₂H₄OH, -CH₂-C≡CH, -CH₂-C≡C-CH₃, or -CH₂-C≡C-C₂H₅; more preferably, -H, -Br, -Cl, -I, -CN, -CH₃, -C₂H₅, -CHF₂, -CF₃, -OCH₃, -OCHF₂, -OCF₃, -CH=CH₂, -CH=CH-CH₃, -CH=CH-C₂H₅, -CH=CH-C₂H₄OH, -C=CH, -C≡C-CH₃, -C≡C-C₂H₅, or -C=C-C₂H₄OH; still more preferably, -H, -Br, -Cl, -I, -CN, -CH₃, -C₂H₅, -CHF₂, -CF₃, -OCH₃, -OCHF₂, -OCF₃, -C≡CH, -C≡C-CH₃, -C≡C-C₂H₅, or -C≡C-C₂H₄OH; still more preferably, -H, -Br, -Cl, -I, -C=CH, -CH₃; most preferably, -I, -C=CH; and/or
preferably, in the formula formulae (**V-1**) - (**V-4**), **R⁶** represents -H, -F, -Cl, -Br, -I, -CN, -CH₃, -C₂H₅, -C₃H₇, -CH(CH₃)₂, -C₄H₉, -CH₂-CH(CH₃)₂, -CH(CH₃)-C₂H₅, -cyclo-C₃H₅, -cyclo-C₄H₇, -cycloC₃H₅O, -CH₂F, -CHF₂, -CF₃, -CH₂-CH₂F, -CH₂-CHF₂, -CH₂-CF₃, -OCH₃, -OCH₂F, -OCHF₂, -OCF₃, -OC₂H₅, -OCH₂CF₃, -OC₃H₇, or -O-cyclo-C₃H₅;
more preferably, **R⁶** represents -H, -F, -Cl, -CN, -CH₃, -C₂H₅, -C₃H₇, -CH(CH₃)₂, -cyclo-C₃H₅, -cycloC₃H₅O, -CH₂F, -CHF₂, -CF₃, -OCH₃, -OCH₂F, -OCHF₂, -OCF₃;
preferably, in the formula formulae (**V-1**) - (**V-3), R⁹** represents -CH₃, -CH₂F, -CHF₂, -CF₃, -C₂H₅, -C₃H₇, -CH(CH₃)₂, -C₄H₉, -CO₂H, -CO₂CH₃, -CO₂CH₂CH₃, -CO₂CH₂CH₂CH₃, -CO₂CH(CH₃)₂, -CO₂C(CH₃)₃, -CO₂-cyclo-C₃H₅, -CO₂-CH₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CONHC(CH₃)₃, -CON(CH₃)₂, -CON(CH₃)(CH₂CH₃), -CON(CH₂CH₃)₂, -CON(CH₂CH₂CH₃)₂, CON(CH(CH₃)₂)₂, -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃, or 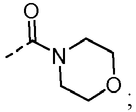
preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CON(CH₃)₂, -CON(CH₃)(CH₂CH₃), -CON(CH₂CH₃)₂, , -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃,
   or 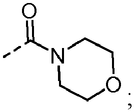
more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CONHCH₃, -CONHCH₂CH₃, -CONHCH₂CH₂CH₃, -CONHCH(CH₃)₂, -CH₂CO₂H, - CH₂CO₂CH₃, -CH₂CO₂CH₂CH₃, -CON(CH₃)₂, -CON(CH₃)(CH₂CH₃), - CON(CH₂CH₃)₂, , -CH₂N(CH₃)₂, -CH₂N(CH₃)(CH₂CH₃), -CH₂N(CH₂CH₃)₂, - CH₂O(CH₂)₂OCH₃, or 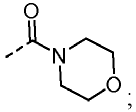
still more preferably, **R⁹** represents -CH₃, -CH₂F, -CHF₂, -CF₃, -C₂H₅, -C₃H₇, -CH(CH₃)₂, -CO₂H, -CO₂CH₃, -CO₂CH₂CH₃, -CO₂CH(CH₃)₂, -CO₂-cyclo-C₃H₅, -CON(CH₃)₂, -CON(CH₃)(CH₂CH₃), -CON(CH₂CH₃)₂, -CH₂CO₂H, -CH₂CO₂CH₃, -CH₂CO₂CH₂CH₃, -CH₂N(CH₃)₂, -CH₂N(CH₂CH₃)₂, -CH₂O(CH₂)₂OCH₃, or 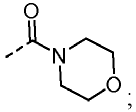
still more preferably, **R⁹** represents -CH₃, -CH₂F, -CHF₂, -CF₃, -CO₂H, -CO₂CH₃, -CO₂CH₂CH₃, -CH₂CO₂H, -CH₂CO₂CH₃, -CH₂CO₂CH₂CH₃, ; most preferably, **R⁹** represents -CH₃, -CO₂H, -CO₂CH₃, -CH₂CO₂H, or -CH₂CO₂CH₃.

Still more preferably, the present invention is directed to the compound of the fomula (I), 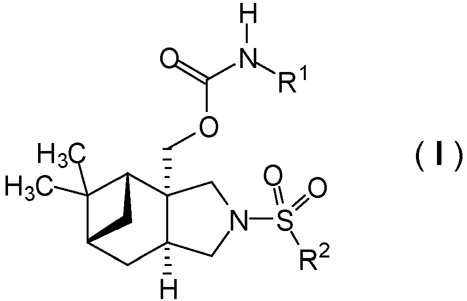 wherein
**R¹** represents 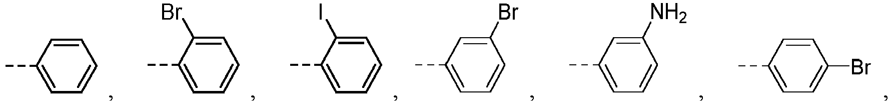 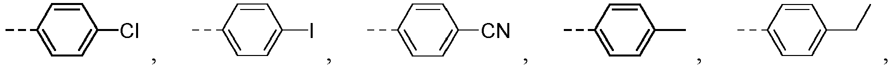 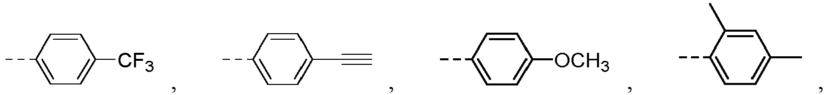 or 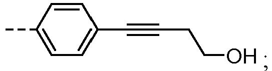 more preferably, 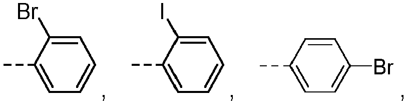 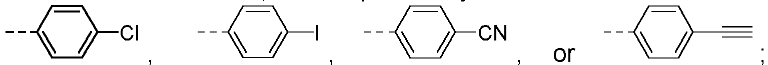 still more preferably 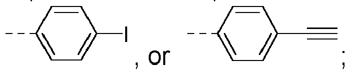
**R²** represents 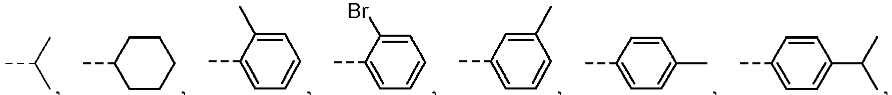 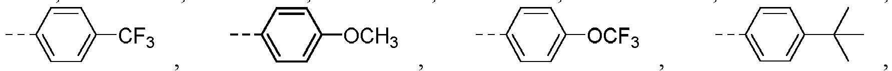 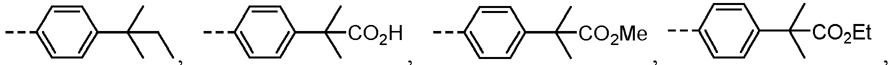 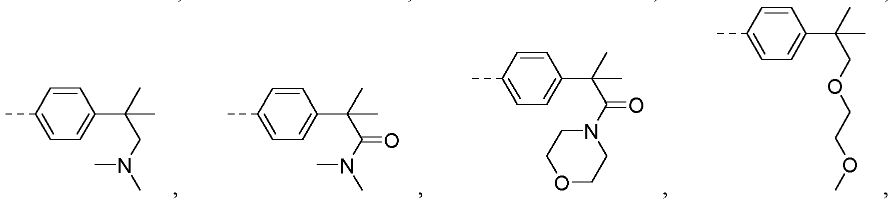 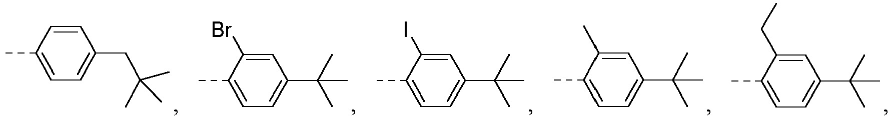 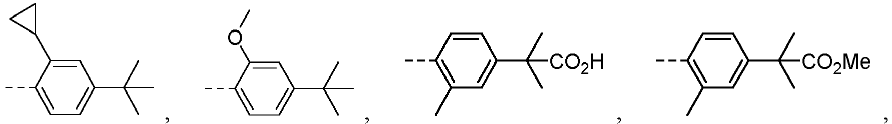 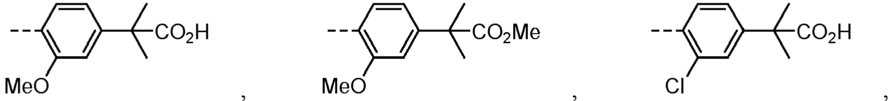 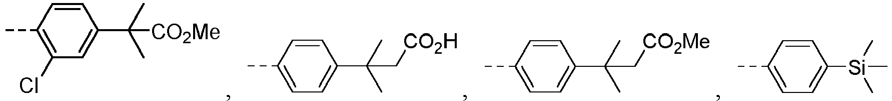 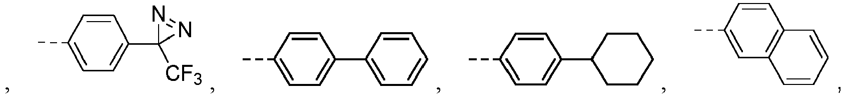 or 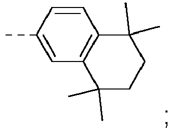
more preferably, 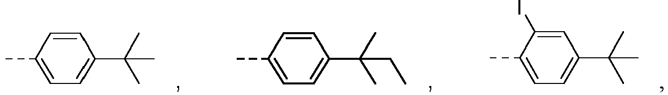 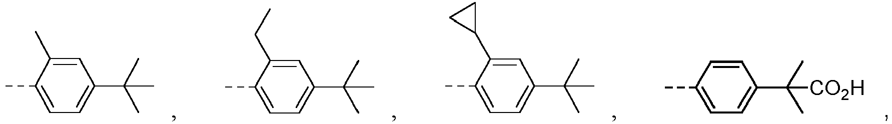 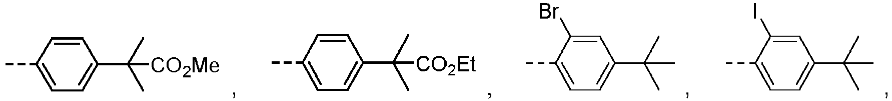 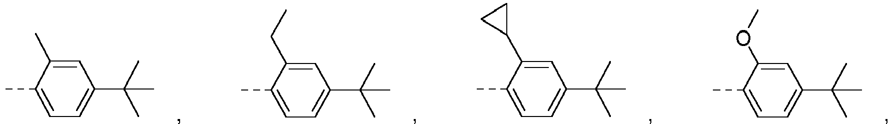 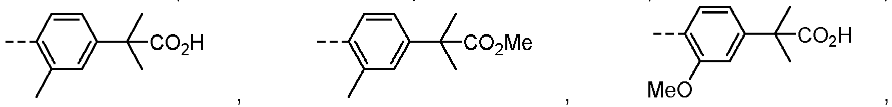 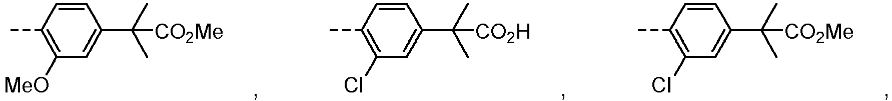 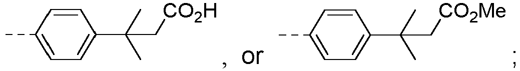
still more preferably, 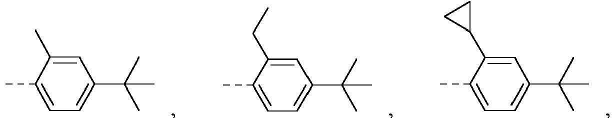 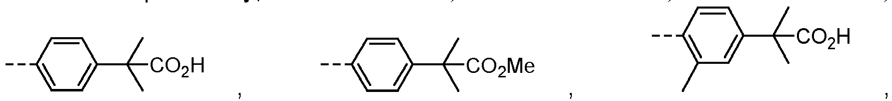 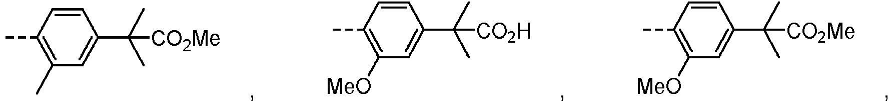 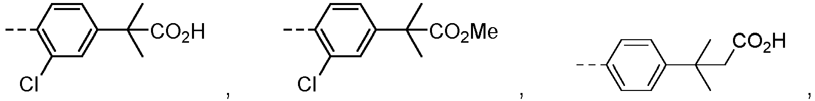 or 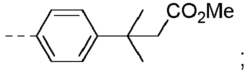
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

Especially, the present invention is directed to a compound selected from the group consisting of:

| **Cpd No.** | **Structure** | **IUPAC-Name** |
|---|---|---|
| **1** | 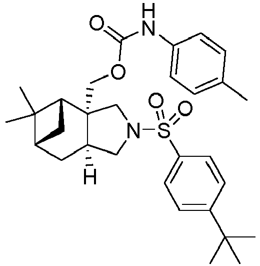 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl *p-*tolylcarbamate |
| **2** | 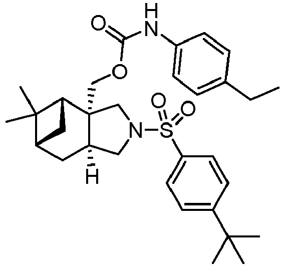 | ((3aS,4R,6R,7aS)-2-((4-(tert-butyl)phenyl)sulfonyl)-5,5-dimethyloctahydro-3a*H*-4,6-methanoisoindol-3a-yl)methyl (4-ethylphenyl)carbamate |
| **3** | 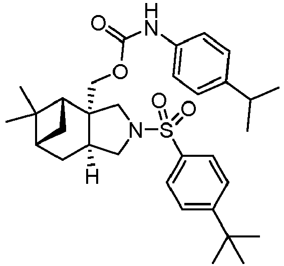 | ((3aS,4R,6R,7aS)-2-((4-(tert-butyl)phenyl)sulfonyl)-5,5-dimethyloctahydro-3a*H*-4,6-methanoisoindol-3a-yl)methyl (4-isopropylphenyl)carbamate |
| **4** | 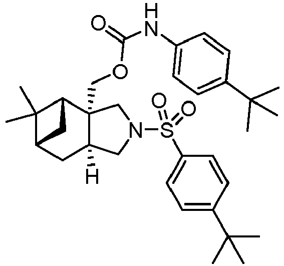 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-(tert-butyl)phenyl)carbamate |
| **5** | 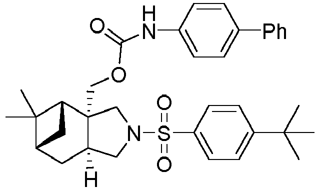 | ((3a*S*,4*R*,6*R*,7a*S*)-2-((4-(*tert-*butyl)phenyl)sulfonyl)-5,5-dimethyloctahydro-3aH-4,6-methanoisoindol-3a-yl)methyl [1,1'-biphenyl]-4-ylcarbamate |
| **6** | 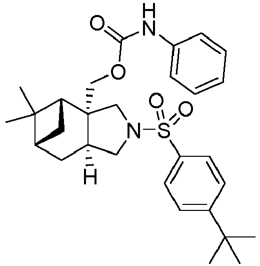 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl phenylcarbamate |
| **7** | 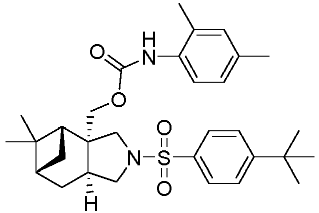 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (2,4-dimethylphenyl)carbamate |
| **8** | 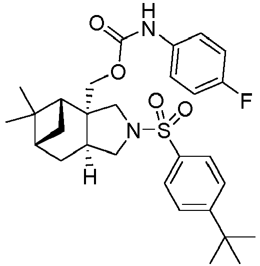 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-fluorophenyl)carbamate |
| **9** | 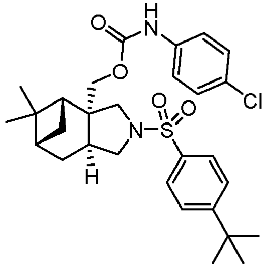 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-chlorophenyl)carbamate |
| **10** | 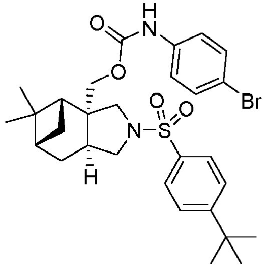 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-bromophenyl)carbamate |
| **11)** | 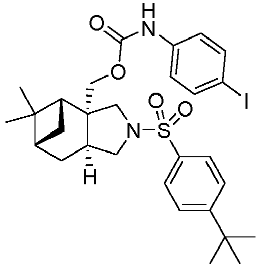 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-iodophenyl)carbamate |
| **12** | 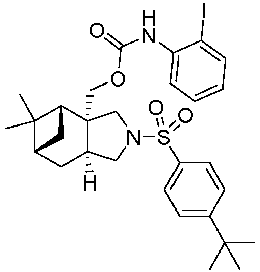 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (2-iodophenyl)carbamate |
| **13** | 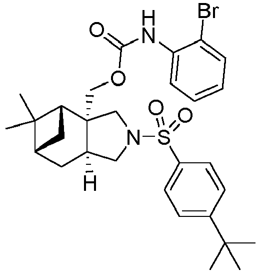 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (2-bromophenyl)carbamate |
| **14** | 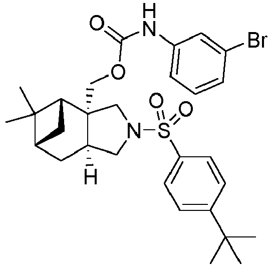 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (3-bromophenyl)carbamate |
| **15** | 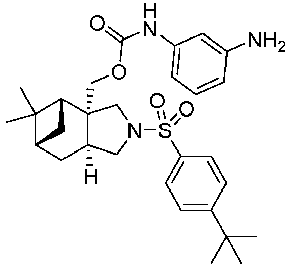 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (3-aminophenyl)carbamate |
| **18** | 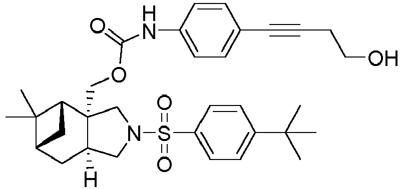 | ((3a*S*,4*R*,6*R*,7a*S*)-2-((4-(tert-butyl)phenyl)sulfonyl)-5,5-dimethyloctahydro-3a*H*-4,6-methanoisoindol-3a-yl)methyl (4-(4-hydroxybut-1-yn-1-yl)phenyl)carbamate |
| **19** | 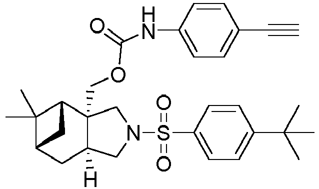 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-(4-ethynylphenyl)carbamate |
| **20** | 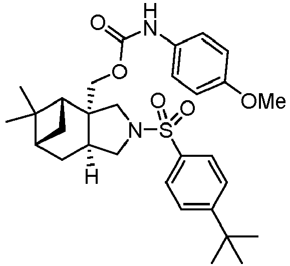 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-methoxyphenyl)carbamate |
| **21** | 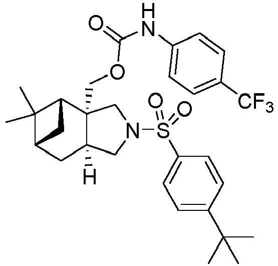 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-(trifluoromethyl)phenyl)carbamate |
| **23** | 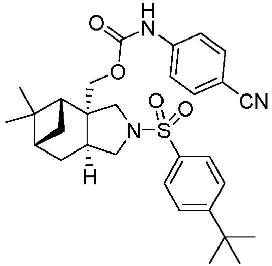 | ((3a*S*,4*R*,6*R*,7a*S*)-2-((4-(*tert-*butyl)phenyl)sulfonyl)-5,5-dimethyloctahydro-3a*H*-4,6-methanoisoindol-3a-yl)methyl (4-cyanophenyl)carbamate |
| **30** | 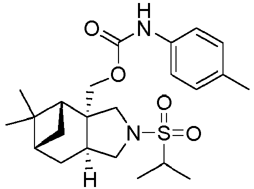 | ((3a*S*,4*R*,6*R*,7a*S*)-2-(isopropylsulfonyl)-5,5-dimethyloctahydro-3a*H*-4,6-methanoisoindol-3a-yl) methyl p-tolylcarbamate |
| **31** | 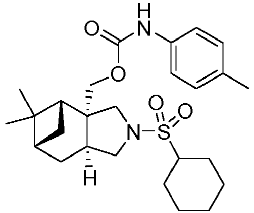 | ((3a*S*,4*R*,6*R*,7a*S*)-2-(cyclohexylsulfonyl)-5,5-dimethyloctahydro-3a*H*-4,6-methanoisoindol-3a-yl)methyl *p-*tolylcarbamate |
| **32** | 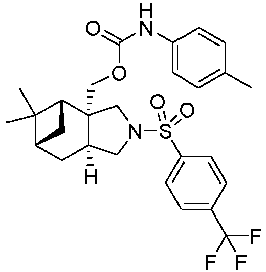 | ((3a*S*,4*R*,6*R*,7a*S*)-5,5-dimethyl-2-((4-(trifluoromethyl)phenyl)sulfonyl)oct ahydro-3aH-4,6-methanoisoindol-3a-yl)methyl *p*-tolylcarbamate |
| **34** | 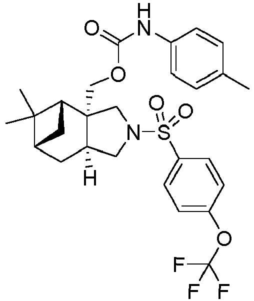 | ((3a*S*,4*R*,6*R*,7a*S*)-5,5-dimethyl-2-((4-(trifluoromethoxy)phenyl)sulfonyl)o ctahydro-3a*H*-4,6-methanoisoindol-3a-yl)methyl *p-*tolylcarbamate |
| **35** | 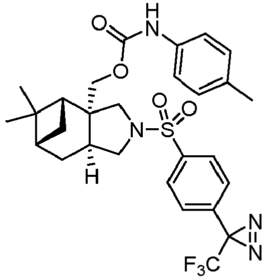 | ((3a*S*,4*R*,6*R*,7a*S*)-5,5-dimethyl-2-((4-(3-(trifluoromethyl)-3H-diazirin-3-yl)phenyl)sulfonyl)octahydro-3aH-4,6-methanoisoindol-3a-yl)methyl *p*-tolylcarbamate |
| **36** | 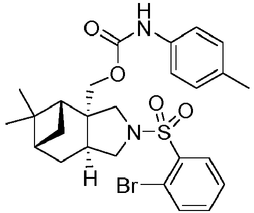 | ((3a*S*,4*R*,6*R*,7a*S*)-2-((2-bromophenyl)sulfonyl)-5,5-dimethyloctahydro-3a*H*-4,6-methanoisoindol-3a-yl)methyl *p-*tolylcarbamate |
| **37** | 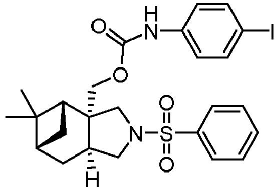 | ((3aS,4R,6R,7aS)-5,5-dimethyl-2-(phenylsulfonyl)octahydro-3aH-4,6-methanoisoindol-3a-yl)methyl (4-iodophenyl)carbamate |
| **38** | 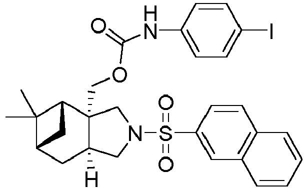 | ((3a*S*,4*R*,6*R*,7a*S*)-5,5-dimethyl-2-(naphthalen-2-ylsulfonyl)octahydro-3aH-4,6-methanoisoindol-3a-yl)methyl (4-iodophenyl)carbamate |
| **39** | 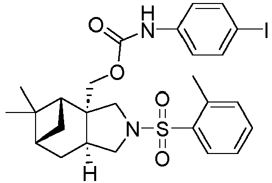 | ((3a*S*,4*R*,6*R*,7a*S*)-5,5-dimethyl-2-(o-tolylsulfonyl)octahydro-3aH-4,6-methanoisoindol-3a-yl)methyl (4-iodophenyl)carbamate |
| **40** | 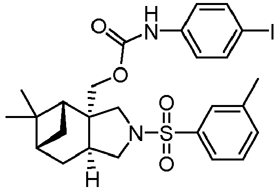 | ((3a*S*,4*R*,6*R*,7a*S*)-5,5-dimethyl-2-(m-tolylsulfonyl)octahydro-3aH-4,6-methanoisoindol-3a-yl)methyl (4-iodophenyl)carbamate |
| **41** | 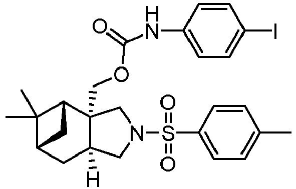 | ((3a*S*,4*R*,6*R*,7a*S*)-5,5-dimethyl-2-tosyloctahydro-3aH-4,6-methanoisoindol-3a-yl)methyl (4-iodophenyl)carbamate |
| **43** | 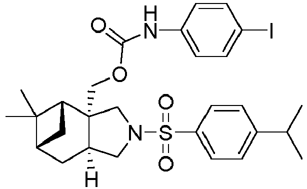 | ((3a*S*,4*R*,6*R*,7a*S*)-2-((4-isopropylphenyl)sulfonyl)-5,5-dimethyloctahydro-3a*H*-4,6-methanoisoindol-3a-yl)methyl (4-iodophenyl)carbamate |
| **44** | 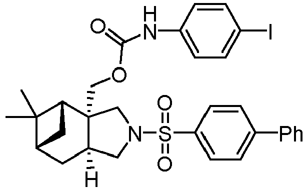 | ((3a*S*,4*R*,6*R*,7a*S*)-2-([1,1'-biphenyl]-4-ylsulfonyl)-5,5-dimethyloctahydro-3a*H*-4,6-methanoisoindol-3a-yl)methyl (4-iodophenyl)carbamate |
| **45** | 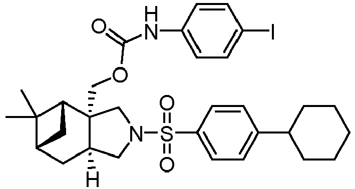 | ((3a*S*,4*R*,6*R*,7a*S*)-2-((4-cyclohexylphenyl)sulfonyl)-5,5-dimethyloctahydro-3aH-4,6-methanoisoindol-3a-yl)methyl (4-iodophenyl)carbamate |
| **46** | 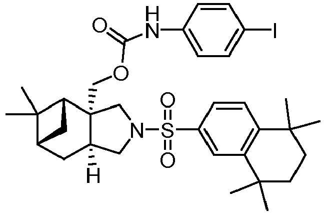 | ((3a*S*,4*R*,6*R*,7a*S*)-5,5-dimethyl-2-((5,5,8,8-tetramethyl-5,6,7,8-tetrahydronaphthalen-2-yl)sulfonyl)octahydro-3aH-4,6-methanoisoindol-3a-yl)methyl (4-iodophenyl)carbamate |
| **47** | 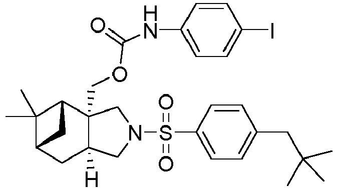 | ((3a*S*,4*R*,6*R*,7a*S*)-5,5-dimethyl-2-((4-neopentylphenyl)sulfonyl)octahydr o-3aH-4,6-methanoisoindol-3a-yl)methyl (4-iodophenyl)carbamate |
| **48** | 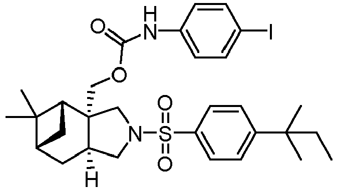 | ((3a*S*,4*R*,6*R*,7a*S*)-5,5-dimethyl-2-((4-(*tert-*pentyl)phenyl)sulfonyl)octahydro-3aH-4,6-methanoisoindol-3a-yl)methyl (4-iodophenyl)carbamate |
| **50** | 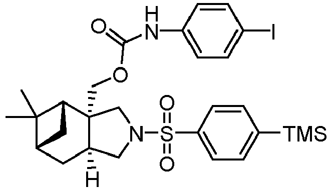 | ((3a*S*,4*R*,6*R*,7a*S*)-5,5-dimethyl-2-((4-(trimethylsilyl)phenyl)sulfonyl)octah ydro-3aH-4,6-methanoisoindol-3a-yl)methyl (4-iodophenyl)carbamate |
| **52** | 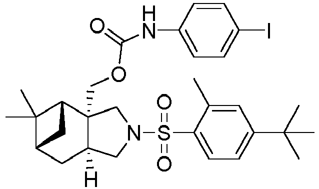 | ((3a*S*,4*R*,6*R*,7a*S*)-2-((4-(*tert*-butyl)-2-methylphenyl)sulfonyl)-5,5-dimethyloctahydro-3a*H*-4,6-methanoisoindol-3a-yl)methyl (4-iodophenyl)carbamate |
| **53** | 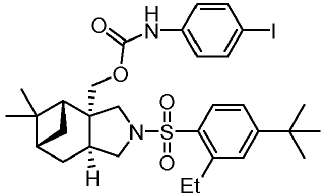 | ((3a*S*,4*R*,6*R*,7a*S*)-2-((4-(tert-butyl)-2-ethylphenyl)sulfonyl)-5,5-dimethyloctahydro-3aH-4,6-methanoisoindol-3a-yl)methyl (4-iodophenyl)carbamate |
| **54** | 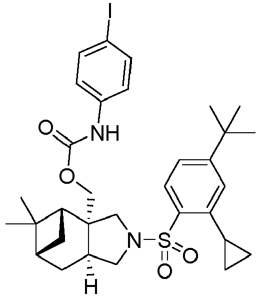 | ((3a*S*,4*R*,6*R*,7a*S*)-2-((4-(*tert*-butyl)-2-cyclopropylphenyl)sulfonyl)-5,5-dimethyloctahydro-3a*H*-4,6-methanoisoindol-3a-yl)methyl (4-iodophenyl)carbamate |
| **55** | 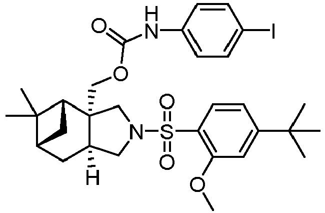 | ((3a*S*,4*R*,6*R*,7a*S*)-2-((4-(*tert*-butyl)-2-methoxyphenyl)sulfonyl)-5,5-dimethyloctahydro-3a*H*-4,6-methanoisoindol-3a-yl)methyl (4-iodophenyl)carbamate |
| **56** | 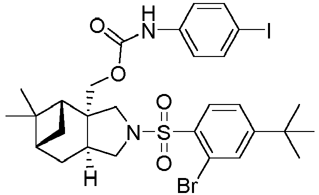 | ((3a*S*,4*R*,6*R*,7a*S*)-2-((2-bromo-4-(tert-butyl)phenyl)sulfonyl)-5,5-dimethyloctahydro-3a*H*-4,6-methanoisoindol-3a-yl)methyl (4-iodophenyl)carbamate |
| **57** | 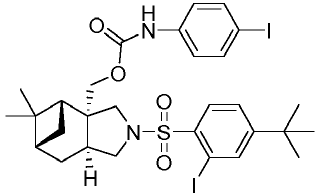 | ((3a*S*,4*R*,6*R*,7a*S*)-2-((4-(tert-butyl)-2-iodophenyl)sulfonyl)-5,5-dimethyloctahydro-3aH-4,6-methanoisoindol-3a-yl)methyl (4-iodophenyl)carbamate |
| **58** | 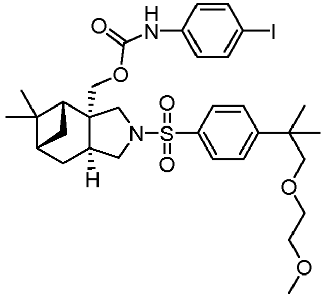 | ((3a*S*,4*R*,6*R*,7a*S*)-2-((4-(1-(2-methoxyethoxy)-2-methylpropan-2-yl)phenyl)sulfonyl)-5,5-dimethyloctahydro-3a*H*-4,6-methanoisoindol-3a-yl)methyl (4-iodophenyl)carbamate |
| **59** | 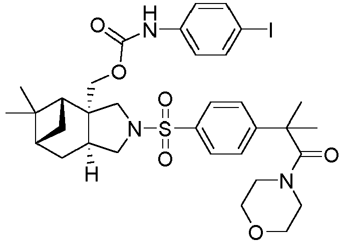 | ((3a*S*,4*R*,6*R*,7a*S*)-5,5-dimethyl-2-((4-(2-methyl-1-morpholino-1-oxopropan-2-yl)phenyl)sulfonyl)octahydro-3aH-4,6-methanoisoindol-3a-yl)methyl (4-iodophenyl)carbamate |
| **60** | 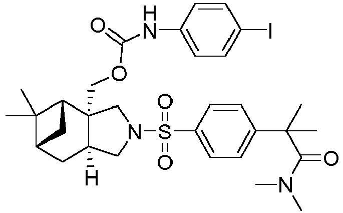 | ((3a*S*,4*R*,6*R*,7a*S*)-2-((4-(1-(dimethylamino)-2-methyl-1 - oxopropan-2-yl)phenyl)sulfonyl)-5,5-dimethyloctahydro-3aH-4,6-methanoisoindol-3a-yl)methyl (4-iodophenyl)carbamate |
| **61** | 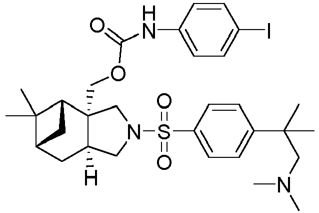 | ((3a*S*,4*R*,6*R*,7a*S*)-2-((4-(1-(dimethylamino)-2-methylpropan-2-yl)phenyl)sulfonyl)-5,5-dimethyloctahydro-3a*H*-4,6-methanoisoindol-3a-yl)methyl (4-iodophenyl)carbamate |
| **64** | 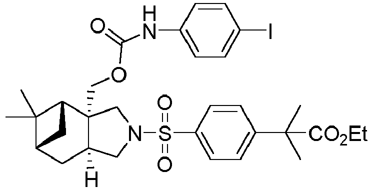 | Ethyl 2-(4-(((3a*S*,4*R*,6*R*,7a*S*)-3a-((((4-iodophenyl)carbamoyl)oxy)methyl)-5,5-dimethyloctahydro-2*H*-4,6-methanoisoindol-2-yl)sulfonyl)phenyl)-2-methylpropanoate |
| **65** | 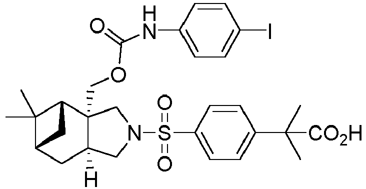 | 2-(4-(((3a*S*,4*R*,6*R*,7a*S*)-3a-((((4-iodophenyl)carbamoyl)oxy)methyl)-5,5-dimethyloctahydro-2H-4,6-methanoisoindol-2-yl)sulfonyl)phenyl)-2-methylpropanoic acid |
| **66** | 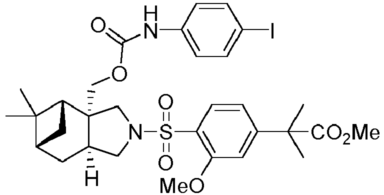 | Methyl 2-(4-(((3a*S*,4*R*,6*R*,7a*S*)-3a-((((4-iodophenyl)carbamoyl)oxy)methyl)-5,5-dimethyloctahydro-2*H*-4,6-methanoisoindol-2-yl)sulfonyl)-3-methoxyphenyl)-2-methylpropanoate |
| **67** | 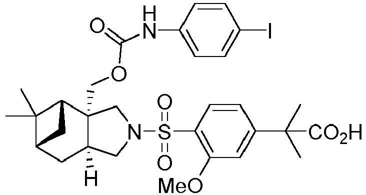 | 2-(4-(((3a*S*,4*R*,6*R*,7a*S*)-3a-((((4-iodophenyl)carbamoyl)oxy)methyl)-5,5-dimethyloctahydro-2H-4,6-methanoisoindol-2-yl)sulfonyl)-3-methoxyphenyl)-2-methylpropanoic acid |
| **68** | 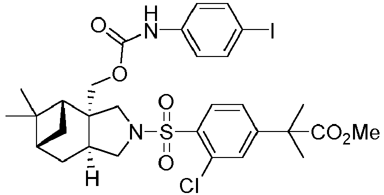 | Methyl 2-(3-chloro-4-(((3aS,4R,6R,7aS)-3a-(((((4-iodophenyl)carbamoyl)oxy)methyl)-5,5-dimethyloctahydro-2H-4,6-methanoisoindol-2-yl)sulfonyl)phenyl)-2-methylpropanoate |
| **69** | 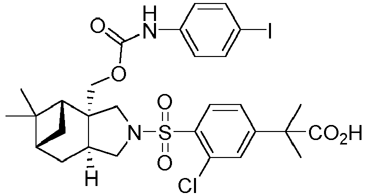 | 2-(3-chloro-4-(((3a*S*,4*R*,6*R*,7a*S*)-3a-((((4-iodophenyl)carbamoyl)oxy)methyl)-5,5-dimethyloctahydro-2H-4,6-methanoisoindol-2-yl)sulfonyl)phenyl)-2-methylpropanoic acid |
| **70** | 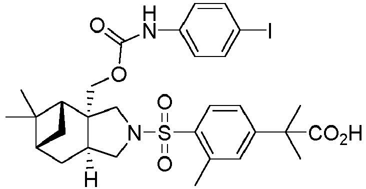 | 2-(4-(((3a*S*,4*R*,6*R*,7a*S*)-3a-((((4-iodophenyl)carbamoyl)oxy)methyl)-5,5-dimethyloctahydro-2H-4,6-methanoisoindol-2-yl)sulfonyl)-3-methylphenyl)-2-methylpropanoic acid |
| **71** | 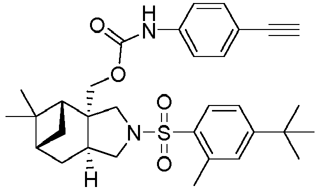 | ((3a*S*,4*R*,6*R*,7a*S*)-2-((4-(*tert*-butyl)-2-methylphenyl) sulfonyl)-5,5-dimethyloctahydro-3a*H*-4,6-methanoisoindol-3a-yl)methyl (4-ethynylphenyl)carbamate |
| **72** | 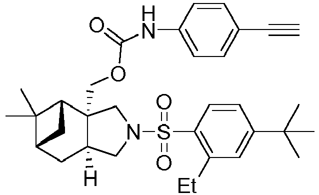 | ((3a*S*,4*R*,6*R*,7a*S*)-2-((4-(t*ert*-butyl)-2-ethylphenyl)sulfonyl)-5,5-dimethyloctahydro-3a*H*-4,6-methanoisoindol-3a-yl)methyl (4-ethynylphenyl)carbamate |
| **73** | 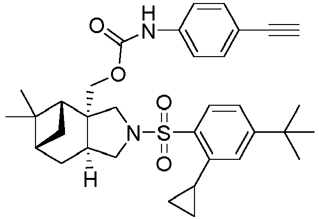 | ((3a*S*,4*R*,6*R*,7a*S*)-2-((4-(tert-butyl)-2-cyclopropylphenyl)sulfonyl)-5,5-dimethyloctahydro-3aH-4,6-methanoisoindol-3a-yl)methyl (4-ethynylphenyl)carbamate |
| **74** | 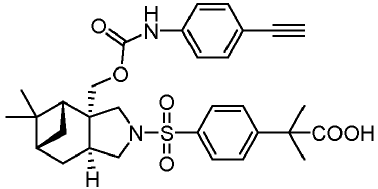 | 2-(4-(((3a*S*,4*R*,6*R*,7a*S*)-3a-((((4-ethynylphenyl)carbamoyl)oxy)meth yl)-5,5-dimethyloctahydro-2*H*-4,6-methanoisoindol-2-yl)sulfonyl)phenyl)-2-methylpropanoic acid |
| **75** | 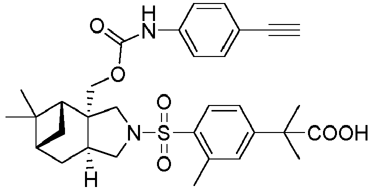 | 2-(4-(((3a*S*,4*R*,6*R*,7a*S*)-3a-((((4-ethynylphenyl)carbamoyl)oxy)meth yl)-5,5-dimethyloctahydro-2*H*-4,6-methanoisoindol-2-yl)sulfonyl)-3-methylphenyl)-2-methylpropanoic acid |
| **76** | 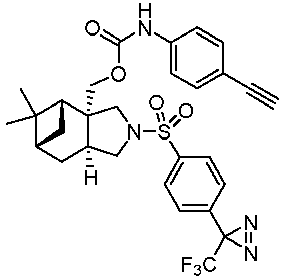 | ((3a*S*,4*R*,6*R*,7a*S*)-5,5-dimethyl-2-((4-(3-(trifluoromethyl)-3*H*-diazirin-3-yl)phenyl)sulfonyl)octahydro-3aH-4,6-methanoisoindol-3a-yl)methyl (4-ethynylphenyl)carbamate |

or an enantiomer, a diastereomer, a tautomer, a mixture of enantiomers, a mixture of diastereomers, a mixture of tautomers, a hydrate, a solvate, a pharmaceutically acceptable salt thereof.

In a preferred embodiment, the present invention is directed to a compound selected from compounds **1**, **11**, **19**, **52**, **65**, **71**, **72**, **74**, **75**, or a pharmaceutically acceptable salt thereof.

### Synthetic Method

In another aspect, the present invention refers to a method for producting a compound of the formula (**I**) comprising:
**Step A1-1**) performing a condensation reaction between a compound **1a*** and an isocyanate comopund **2*** (O=C=N-R¹) 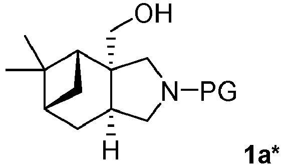
   to obtain a compound **3a*** 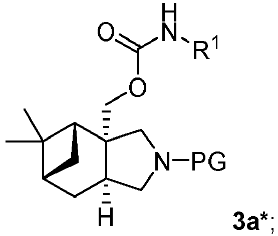
   wherein PG is an amine protecting group;
**Step A1-2**) deprotecting the amine protecting group PG from the compound **3a*** to obtain a compound **3b*** 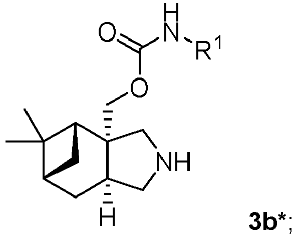
**Step B1**) performing a coupling reaction of compound **3b*** with a sulfonyl chloride compound **4*** (Cl-SO₂-R²) to obtain a compound of the formula (**I**). 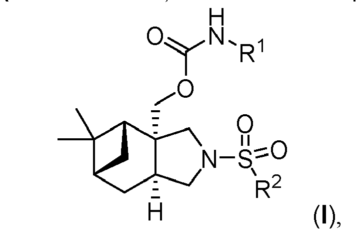 wherein **R¹** and **R²** have the same meanings as defiend in claim 1;
   or
**Step A2**) performing a coupling reaction of compound **1b*** with a sulfonyl chloride compound **4*** (Cl-SO₂-R²) 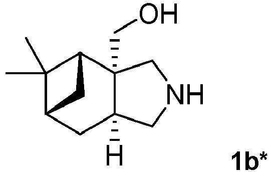 to obtain a sulfonyl amide compound 3c*; 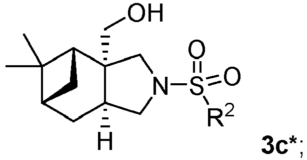
**Step B2**) performing a condensation reaction between a compound **3c*** and an isocyanate comopund **4*** (O=C=N-R¹) to obtain a compound of the formula (**I**). 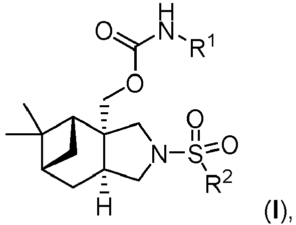 wherein **R¹** and **R²** have the same meanings as defiend in claim 1.

In a further aspect, the presnt invention relates to an intermediate compound selelected from compounds **3a*, 3b***, and **3c***; 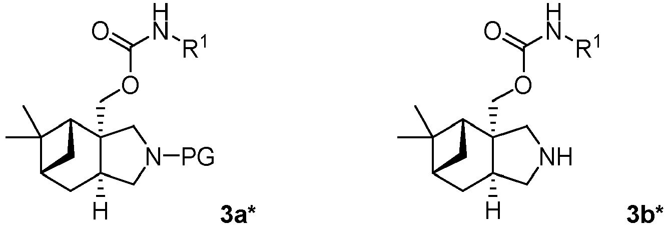 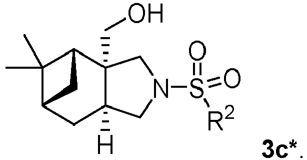 wherein, **R¹** and **R²** have the same meinaings as defined in the formula (I), and PG is an amine protecting group.

The term "protecting group (PG)" as used herein refers to commonly used protection groups in organic synthesis, preferably for amine protecting group. Preferably, PG may be selected from the group consisting of or comprising: acetyl, benzyl, benzoyl, benzyloxycarbonyl (Cbz), tert-butylcarbonyl, tert-butyloxycarbonyl (Boc), and fluorenylmethylenoxy group (Fmoc), preferably benzyl, benzoyl, benzyloxycarbonyl (Cbz), tert-butylcarbonyl, tert-butyloxycarbonyl (Boc); more preferably, benzyl, benzoyl, benzyloxycarbonyl (Cbz), or tert-butyloxycarbonyl (Boc); still more preferably, benzyl group.

### Pharmaceutical Composition

Furthermore, the present invention relates to a pharmaceutical composition comprising at least one compound according to the general formula (I), as an active ingredient or a pharmacologically acceptable salts thereof as an active ingredient, together with at least one pharmacologically acceptable carrier, excipient and/or diluent.

Preferably, the pharmaceutical composition further comprises at least one active agent selected from anti-microtubule agents, platinum coordination complexes, alkylating agents, antibiotic agents, topoisomerase II inhibitors, antimetabolites, topoisomerase I inhibitors, hormones and hormone analogues, signal transduction pathway inhibitors, nonreceptor tyrosine kinase angiogenesis inhibitors, immunotherapeutic agents (such as an anti- tumour vaccine, an oncolytic virus, an immune stimulatory antibody such as anti-CTLA4, anti- PD1 , anti-PDL-1 , anti-OX40, anti-41BB, anti-CD27, anti-CD40, anti-LAG3, anti-TIM3, and anti-GITR, a novel adjuvant, a peptide, a cytokine, a chimeric antigen receptor T cell therapy (CAR-T), a small molecule immune modulator, tumour microenvironment modulators, and anti-angiogenic agents), proapoptotic agents and cell cycle signalling inhibitors.

The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, gels, elixirs, dispersable granules, syrups, suspensions, and the like, and consistent with conventional pharmaceutical practices. For example, for oral administration in the form of tablets or capsules, the active drug component may be combined with any oral non-toxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95-weight % of the compounds of the formula (I) or the respective pharmaceutically active salt as active ingredient.

Suitable binders include starch, gelatin, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and opacifiers for oral solutions, suspensions, and emulsions. Liquid form preparations may also include solutions for intranasal administration.

Aerosol preparations suitable for inhalation may include solutions and solids in powder form, which may be present in combination with a pharmaceutically acceptable carrier such as an inert, compressed gas, e.g. nitrogen.

For preparing suppositories, a low melting wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted, shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methylcellulose, polyvinyl alcohols, or denatured gelatins or starch for holding or containing compositions comprising the active ingredient(s). Capsules with hard shells are typically made of blended of relatively high gel strength gelatins from bones or pork skin. The capsule itself may contain small amounts of dyes, opaquing agents, plasticisers and/or preservatives.

Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix.

Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight % of the composition, more preferably from about 5 to ca. 10 weight %.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatin and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight % of the composition, preferably from about 3 to about 10 weight %, and more preferably from about 3 to about 6 weight %.

Lubricants refer to a class of substances which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D,L-leucine. Lubricants are usually added at the very last step before compression, since they must be present at the surface of the granules. The amount of lubricant in the composition may range from about 0.2 to about 5 weight % of the composition, preferably from about 0.5 to about 2 weight %, and more preferably from about 0.3 to about 1.5 weight % of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight % of the final composition, preferably from about 0.5 to about 2 weight %.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight % of the composition, preferably from about 0.1 to about 1 weight %.

### Medical Use

Surprisingly, it was found that the compounds of the present invention effectively inhibit the activity of indoleamine-1,2-dioxygenase 1 (IDO1).

In the biological example **B-1**, Kyn assay has been performed with inventive compounds in BxPC-3 cells. Cells were incubated with IFN-γ, Trp and the inventive compounds for 48 h prior to detection of Kyn levels (**Table B-1**)**.** It was proven that the inventive compounds effectively inhibit the activity of indoleamine-1,2-dioxygenase 1 (IDO1).

Subsequent in-depth investigation of the mode of action and identification of the cellular target revealed that compound 1 targets the heme-free apo-form of indoleamine-1,2-dioxygenase 1 (IDO1), inhibits the enzyme and induces degradation of IDO1 through the ubiquitin-proteasome system by supercharging the native degradation pathway employing the cullin-RING E3 ligase CRL2^{KLHDC3}. Thus the inventinve compunds are found as IDO1 degraders and inhibitors, in particualr, they are defined as a novel monovalent degrader chemotype.

As shown in **Table B-2** in the biological example **B-2**, it was found that some oft he inventive compounds also function as degraders of IDO1.

Since compound **11** was 7-fold more potent in the Kyn assay than compound **1**, the inventors characterized degradation which revealed a maximum degradation level of 37% for compound **11.** compound **19**; *tert*-butyl-phenyl sulfonamide combined with alkinyl-phenyl urethane), and compound **52**; (*ortho*-methyl-*para-tert*-butyl-phenyl sulfonamide combined with iodo-phenyl urethane) were weak inhibitors but better degraders with Dₘₐₓ >60%). Variation of the *ortho*-substituent in the *para-tert-*butylphenyl sulfonamide in compounds **53-57** diminished both, enzyme inhibition and degradation efficiency (Dₘₐₓ 55-59%). Replacement of one methyl group in the *tert-*butylphenyl sulfonamide in compound **52** by a carboxylic acid yielded more potent inhibitors, including comopund **65**, but degradation efficiency was lower (compounds **63** and **65**; Dₘₐₓ of 36-46%), and introduction of a further ortho-substituent into the sulfonamide aryl group did not improve activity (compounds **67-69**). Notably, compounds **69** and **70** are potent inhibitors, but are not efficient in the cellular assay monitoring IDO1 degradation, which may in part be due to possibly limited cell penetration of these carboxylic acids. This may also hold true for compounds **74** and **75.** Highest degradation efficiency was displayed by compounds **71**, and **72** and their close analog **73.** These compounds embody an alkinyl-phenyl urethane and an ortho-substituted *para-tert*-butylphenyl sulfonamide, and were not identified as particularly potent inhibitors. For compounds **71** and **72**, the inventors determined that their half maximal degradation concentration is in the low nanomolar range, hence they are efficient degraders.

This analysis revealed compound **72** as compound with the best balance of properties. It shows an IC₅₀ value of 11 nM in the Kyn assay, inhibits the enzyme *in vitro* with IC₅₀ = 4 µM, reached a degradation efficiency of 75% at 100 nM concentration and its half maximal degradation concentration is 3 nM.

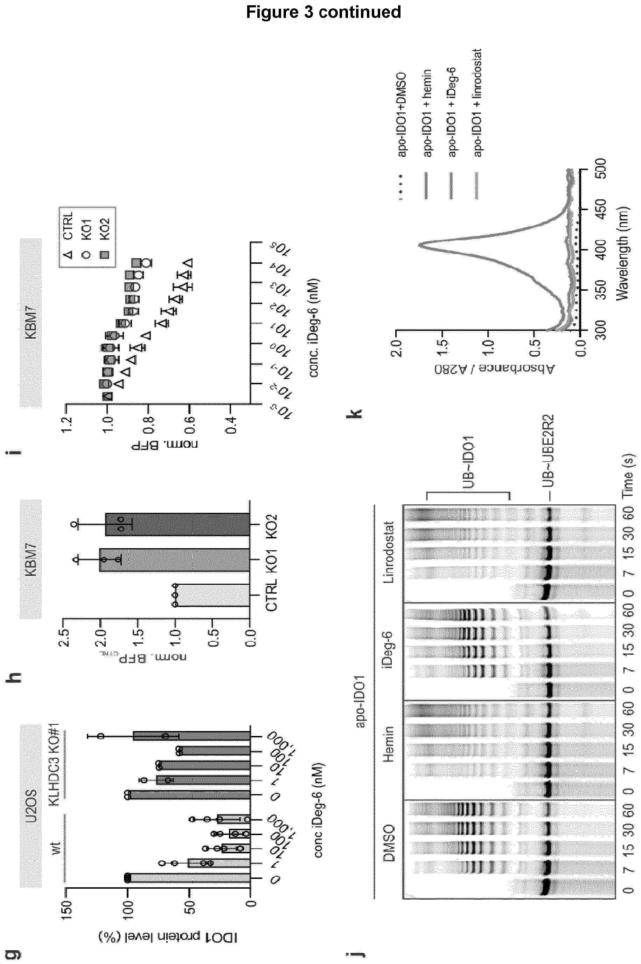

Surprisingly, it was found that *in vitro,* apo-IDO1 was rapidly ubiquitinated by CRL2^{KLHDC3}, which was less pronounced in the presence of heme (Fig. 3j). IDO1 was also ubiquitinated upon treatment of apo-IDO1 with compound 71 (= iDeg-6) (Fig. 3j). In contrast, apo-IDO1 inhibitor linrodostat rather suppressed IDO1 ubiquitination under these conditions (Fig. 3j-k). Hence, in a biochemical setting, apo-IDO1 is a better substrate for KLHDC3 than holo-IDO1. These findings suggest a possible model for regulation of IDO1 levels in cells. Freshly translated apo-IDO1 is rapidly cleared in cells by physiological degradation mediated by KLHDC3 in order to keep its concentration low when not needed. In contrast, after an external stimulus, IDO1 expression is upregulated, apo-IDO1 exceeds predominant degradation and is charged with heme to form holo-IDO1. The holo-pool escapes degradation and performs the desired function in response to the stimulus.

Therefore, another aspect of the present invention relates to compounds according to the general formula (**I**), or a pharmaceutical composition comprising at least one compound according to the general formula (I) as described above as medicine as well as their use in medicine. Especially preferred is the use as inhibitors and degraders of IDO1.

The compounds according to general formula (I) described herein are especially suitable for the treatment and prophylaxis of diseases or disoders associated with and/or caused by IDO1.

Another aspect of the present invention is the use of the inventive compounds of the general formula (I), or the pharmaceutical composition thereof as descirbed herein in the the prophylaxis or treatment of a disease and/or a disorder caused by and/or associated with indolamine 2,3-dioxygenase 1 (IDO1),
wherein the disease and/or the disorder selected from: an inflammatory condition, an infectious disease, a cancer, a central nervous system disease or disorder, a disease or disorder relating to female reproductive health, coronary heart disease, chronic renal failure, post anaesthesia cognitive dysfunction, and cataracts.

In similar words, the present invention is directed to a inventive compound of the formula (I), for use, a pharmaceutically acceptable salt thereof for use, or the pharmaceutical composition thereof as descirbed herein for use in the prophylaxis or treatment of a disease and/or a disorder caused by and/or associated with indolamine 2,3-dioxygenase 1 (IDO1),
wherein the disease and/or the disorder selected from: an inflammatory condition, an infectious disease, a cancer, a central nervous system disease or disorder, a disease or disorder relating to female reproductive health, coronary heart disease, chronic renal failure, post anaesthesia cognitive dysfunction, and cataracts.

As used herein the term "inhibiting" or "inhibition" refers to the ability of a compound to downregulate, decrease, reduce, suppress, inactivate, or inhibit at least partially the activity of an enzyme, or the expression of an enzyme or protein.

The term "effective amount" means an amount of compound that, when administered to a patient in need of such treatment, is sufficient to
(i) treat or prevent a particular disease, condition, or disorder which can be treated with IDO1 inhibitor;
(ii) attenuate, ameliorate, or eliminate one or more symptoms of the particular disease, condition, or disorder; or
(iii) prevent or delay the onset of one or more symptoms of the particular disease, condition, or disorder described herein.

### Infections diseases and inflammation

Infection by bacteria, parasites, or viruses induces a strong IFN-y-dependent inflammatory response. IDO can dampen protective host immunity, thus indirectly leading to increased pathogen burdens. For example, IDO activity attenuates Toxoplasma gondii replication in the lung, and the inflammatory damage is significantly decreased by the administration of the IDO inhibitor 1MT after infection. Also, in mice infected with murine leukaemia virus (MuLV), IDO was found to be highly expressed, and ablation of IDO enhanced control of viral replication and increased survival. In a model of influenza infection, the immunosuppressive effects of IDO could predispose lungs to secondary bacterial infection. In Chagas Disease, which is caused by the Trypanosoma cruzi parasite, kynurenine is increased in patients and correlates with disease severity. Therefore, IDO inhibitors could be used to improve the outcomes of patients with a wide variety of infectious diseases and inflammatory conditions.

IDO plays a role in regulating mucosal immunity to the intestinal microbiota. IDO has been shown to regulate commensal induced antibody production in the gut; IDO-deficient mice had elevated baseline levels of immunoglobulin A (IgA) and immunoglobulin G (IgG) in the serum and increased IgA in intestinal secretions. Due to elevated antibody production, IDO deficient mice were more resistant to intestinal colonization by the gram-negative enteric bacterial pathogen Citrobacter rodentium than WT mice. IDO-deficient mice also displayed enhanced resistance to the colitis caused by infection with C. rodentium.

Therefore, pharmacological targeting of IDO activity may represent a new approach to manipulating intestinal immunity and controlling the pathology caused by enteric pathogens including colitis (Harrington et al, 2008).

### HIV infection

Patients infected with HIV have chronically reduced levels of plasma tryptophan and increased levels of kynurenine, and increased IDO expression. In HIV patients the upregulation of IDO acts to suppress immune responses to HIV antigens contributing to the immune evasion of the virus. HIV triggers high levels of IDO expression when it infects human macrophages in vitro, and simian immunodeficiency virus (SIV) infection of the brain in vivo induces IDO expression by cells of the macrophage lineage.

The pathogenesis of HIV is characterized by CD4+ T cell depletion and chronic T cell activation, leading ultimately to AIDS. CD4+ T helper (TH) cells provide protective immunity and immune regulation through different immune cell functional subsets, including TH1 , TH2, T regulatory (Treg), and TH 17 cells. Progressive HIV is associated with the loss of TH17 cells and a reciprocal increase in the fraction of the immunosuppressive Treg cells. The loss of TH17/Treg balance is associated with induction of IDO by myeloid antigen- presenting dendritic cells. In vitro, the loss of TH 17 Treg balance is mediated directly by the proximal tryptophan catabolite from IDO metabolism, 3- hydroxyanthranilic acid. Therefore in progressive HIV, induction of IDO contributes to the inversion of the TH17/Treg balance and maintenance of a chronic inflammatory state. Therefore, IDO inhibitors could have utility in addressing the TH 17/Treg balance in HIV. Sepsis-induced hypotension

Systemic inflammation such as sepsis is characterized by arterial hypotension and systemic inflammatory response syndrome. The associated increase in circulating pro-inflammatory cytokines, including interferon-γ (IFN-γ), leads to the unchecked production of effector molecules such as reactive oxygen and nitrogen species that themselves can contribute to pathology.

The metabolism of tryptophan to kynurenine by IDO expressed in endothelial cells contributes to arterial vessel relaxation and the control of blood pressure (Wang et al, 2010). Infection of mice with malarial parasites {Plasmodium berghei), and experimental induction of endotoxemia, caused endothelial expression of IDO, resulting in decreased plasma tryptophan, increased kynurenine, and hypotension. Pharmacological inhibition of IDO increased blood pressure in systemically inflamed mice, but not in mice deficient for IDO or interferon-γ, which is required for IDO induction. Arterial relaxation by kynurenine was mediated by activation of the adenylate and soluble guanylate cyclase pathways. Therefore, inhibitors of IDO could have utility in treating sepsis-induced hypotension.

### CNS disorders

In the central nervous system both fates of TRP which act as a precursor to kynurenine and serotonin are pathways of interest and importance. Metabolites produced by the kynurenine pathway have been implicated to play a role in the pathomechanism of neuroinflammatory and neurodegenerative disorder. The first stable intermediate from the kynurenine pathway is KYN. Subsequently, several neuroactive intermediates are generated. They include kynurenic acid (KYN A), 3-hydroxykynurenine (3-HK), and quinolinic acid (QUIN). 3-HK and QUIN are neurotoxic by distinct mechanisms; 3-HK is a potent free-radical generator, whereas QUIN is an excitotoxic N-methyl-D-aspartate (NMD A) receptor agonist. KYNA, on the other hand, has neuroprotective properties as an antagonist of excitatory amino acid receptors and a free-radical scavenger. Changes in the concentration levels of kynurenines can shift the balance to pathological conditions. The ability to influence the metabolism towards the neuroprotective branch of the kynurenine pathway, i.e. towards kynurenic acid (K.YNA) synthesis, may be one option in preventing neurodegenerative diseases.

In the CNS, the kynurenine pathway is present to varying extents in most cell types, Infiltrating macrophages, activated microglia and neurons have the complete repertoire of kynurenine pathway enzymes. On the other hand, neuroprotective astrocytes and oligodendrocytes lack the enzyme, kynurenine 3-monooxygenase (KMO) and IDO respectively, and are incapable of synthesizing the excitotoxin, quinolinic acid (QUIN).

### Amyotrophic lateral sclerosis

Amyotrophic lateral sclerosis (ALS), or Lou Gehrig's disease, is a progressive and fatal neurodegenerative disease targeting the motor system. ALS results in the selective attacking and destruction of motor neurons in the motor cortex, brainstem and spinal cord.

Although multiple mechanisms are likely to contribute to ALS, the kynurenine pathway activated during neuroinfl animation is emerging as a contributing factor. Initial inflammation may inflict a nonlethal injury to motor neurons of individuals with a susceptible genetic constitution, in turn triggering a progressive inflammatory process which activates microglia to produce neurotoxic kynurenine metabolites that further destroy motor neurons.

In the brain and spinal cord of ALS patients large numbers of activated microglia, reactive astrocytes, T cells and infiltrating macrophages have been observed. These cells release inflammatory and neurotoxic mediators, among others IFN-γ, the most potent inducer of IDO. The neuronal and microglial expression of IDO is increased in ALS motor cortex and spinal cord. It has been proposed that the release of immune activating agents activates the rate- limiting enzyme of the P, IDO, which generates metabolites such as the neurotoxin QUIN. Therefore, inhibition of IDO would reduce the synthesis of neurotoxic QUIN, which has been clearly implicated in the pathogenesis of ALS.

### Huntington 's disease

Huntington's disease (HD) is a genetic autosomal dominant neurodegenerative disorder caused by expansion of the CAG repeats in the huntingtin (htt) gene. Patients affected by HD display progressive motor dysfunctions characterized by abnormality of voluntary and involuntary movements (choreoathetosis) and psychiatric and cognitive disturbances. In-life monitoring of metabolites within the YN pathway provides one of the few biomarkers that correlates with the number of CAG repeats and hence the severity of the disorder. Post mortem very high levels of QUIN are found located in areas of neurodegeneration, while striatal glutamatergic neurones, on which QUIN acts as an excitotoxin, are a principal class lost in the disease.

### Alzheimer's disease

Alzheimer's disease (AD) is an age-related neurodegenerative disorder characterised by neuronal loss and dementia. The histopathology of the disease is manifested by the accumulation of intracellular β- amyloid (Aβ) and subsequent formation of neuritic plaques as well as the presence of neurofibrillary tangles in specific brain regions associated with learning and memory. The pathological mechanisms underlying this disease are still controversial, however, there is growing evidence implicating KP metabolites in the development and progression of AD.

It has been shown that Aβ (1-42) can activate primary cultured microglia and induce IDO expression. Furthermore, IDO over-expression and increased production of QUIN have been observed in microglia associated with the amyloid plaques in the brain of AD patients. QUIN has been shown to lead to tau hyperphosphorylation in human cortical neurons. Thus, overexpression of IDO and over-activation of the KP in microglia are implicated in the pathogenesis of AD,

### Psychiatric disorders and pain

Most tryptophan is processed through the kynurenine pathway. A small proportion of tryptophan is processed to 5-HT and hence to melatonin, both of which are also substrates for IDO. It has long been known that amongst other effects acute tryptophan depletion can trigger a depressive episode and produces a profound change in mood even in healthy individuals. These observations link well with the clinical benefits of serotonergic drags both to enhance mood and stimulate neurogenesis.

Schizophrenic patients exhibit elevated KYN levels both in CSF and brain tissue, particularly the frontal cortex. This has been associated with the "hypofrontality" observed in schizophrenia. Indeed rodents treated with neuroleptics show a marked reduction in frontal KYN levels. These changes have been associated with reduced KMO and 3HAO. Evidence includes an association between a KMO polymorphism, elevated CSF KYN and schizophrenia. Taken together there is potential for manipulations in this pathway to be both pro-cognate and neuroleptic.

Pain and depression are frequently comorbid disorders. It has been shown that IDO plays a key role in this comorbidity. Recent studies have shown that IDO activity is linked to (a) decreased serotonin content and depression and (b) increased kynurenine content and neuroplastic changes through the effect of its derivatives such as quinolinic acid on glutamate receptors.

In rats chronic pain induced depressive behaviour and IDO upregulation in the bilateral hippocampus. Upregulation of IDO resulted in the increased kynurenine/tryptophan ratio and decreased serotonin/tryptophan ratio in the bilateral hippocampus. Furthermore, IDO gene knockout or pharmacological inhibition of hippocampal IDO activity attenuated both nociceptive and depressive behaviour. Since proinflammatory cytokines have been implicated in the pathophysiology of both pain and depression, the regulation of brain IDO by proinflammatory cytokines serves as a critical mechanistic link in the comorbid relationship between pain and depression through the regulation of tryptophan metabolism.

### Multiple sclerosis

Multiple sclerosis (MS) is an autoimmune disease characterized by inflammatory lesions in the white matter of the nervous system, consisting of a specific immune response to the myelin sheet resulting in inflammation and axonal loss. Accumulation of neurotoxic kynurenine metabolites caused by the activation of the immune system is implicated in the pathogenesis of MS. QUIN was found to be selectively elevated in the spinal cords of rats with EAE, an autoimmune animal model of MS. The origin of the increased QUIN in EAE was suggested to be the macrophages. QUIN is an initiator of lipid peroxidation and high local levels of QUIN near myelin may contribute to the demyelination in EAE and possibly MS.

Interferon beta lb (IFN-p ib) induces KP metabolism in macrophages at concentrations comparable to those found in the sera of IFN-b treated patients, this which may be a limiting factor in its efficacy in the treatment of MS. After IFN-β administration, increased kynurenine levels and kynurenine/tryptophan ratio were found in the plasma of MS patients receiving IFN-b injection compared to healthy subjects indicating an induction of IDO by IFN-β. IFN-p Ib, leads to production of QLJIN at concentrations sufficient to disturb the ability of neuronal dendrites to integrate incoming signals and kill oligodendrocytes. In I FN-β I b-treated patients concomitant blockade of the KP with an IDO inhibitor may improve its efficacy of IFN-p ib.

### Parkinson's disease

Parkinson's disease (PD) is a common neurodegenerative disorder characterised by loss of dopaminergic neurons and localized neuroinfl animation.

Parkinson's disease is associated with chronic activation of microglia. Microglia activation release neurotoxic substances including reactive oxygen species (ROS) and proinflammatory cytokines such as iNF-γ, a potent activator of KP via induction of IDO expression. KP in activated microglia leads to upregulation of 3HK and QUIN. 3HK is toxic primarily as a result of conversion to ROS. The combined effects of ROS and NMDA receptor-mediated excitotoxicity by QUIN contribute to the dysfunction of neurons and their death. However, picolinie acid (PIC) produced through KP activation in neurons, has the ability to protect neurons against QUIN-induced neurotoxicity, being NMDA agonist. Microglia can become overactivated, by proinflammatory mediators and stimuli from dying neurons and cause perpetuating cycle of further microglia activation microgliosis. Excessive microgliosis will cause neurotoxicity to neighbouring neurons and resulting in neuronal death, contributing to progression of Parkinson's disease.

Therefore, PD is associated with an imbalance between the two main branches of the P within the brain. KY' A synthesis by astrocytes is decreased and concomitantly, QUIN production by microglia is increased.

### HIV

HIV patients, particularly those with HIV-linked dementia, often have significantly elevated KYN levels in CSF. These levels are directly related to the development of neurocognitive decline and often the presence of sever psychotic symptoms.

### Cancer

It is clear that tumours can induce tolerance to their own antigens. Tryptophan catabolism in cancer is increasingly being recognized as an important micro-environmental factor that suppresses antitumor immune responses. Depletion of tryptophan and accumulation of immunosuppressive tryptophan catabolites such as kynurenine create an immunosuppressive milieu in tumours and in tumour-draining lymph nodes by inducing T-cell anergy and apoptosis. Such immunosuppression in the tumour microenvironment may help cancers evade the immune response and enhance tumorigenicity.

Recently, IDO have been found to be overexpressed in various cancers, furthermore, several cancers overexpress IDO. IDO mediate immunosuppressive effects through the metabolization of Trp to kynurenine, triggering downstream signalling through GCN2, mTOR and AHR that can affect differentiation and proliferation of T cells. Also, expression of IDO by activated dendritic cells can serve to activate regulatory T cells (Tregs) and inhibit rumor-specific effector CD8+ T cells, thereby constituting a mechanism by which the immune system can restrict excessive lymphocyte reactivity.

### Cataracts

A cataract is a clouding of the lens inside the eye that leads to a decrease in vision. Recent studies suggest that kynurenines might chemically alter protein structure in the human lens leading to cataract formation. In the human lens IDO activity is present mainly in the anterior epithelium. Several kynurenines, such as kynurenine ( Y ), 3- hydroxykynurenine (30HKYN), and 3-hydroxykynurenine glucoside (30H G) have been detected in the lens; where they were thought to protect the retina by absorbing UV light and therefore are commonly referred to as UV filters. However, several recent studies show that kynurenines are prone to deamination and oxidation to form α,β-unsaturated ketones that chemically react and modify lens proteins. Kynurenine mediated modification could contribute to the lens protein modifications during aging and cataractogenesis. They may also reduce the chaperone function of α-crystallin, which is necessary for maintaining lens transparency.

Transgenic mouse lines that overexpress human IDO in the lens developed bilateral cataracts within 3 months of birth. It was demonstrated that IDO-mediated production o kynurenines results in defects in fibre cell differentiation and their apoptosis. Therefore inhibition of IDO may slow the progression of cataract formation.

### Female reproductive health

### Endometriosis

Endometriosis, the presence of endometrium outside the uterine cavity, is a common gynaecological disorder, causing abdominal pain, dyspareunia and infertility. IDO expression was found to be higher in eutopic endometrium from women with endometriosis by microarray analysis. Furthermore, IDO was shown to enhance the survival and invasiveness of endometrial stromal cells. Therefore, an IDO inhibitor could be used as a treatment for endometriosis.

### Contraception and abortion

The process of implantation of an embryo requires mechanisms that prevent allograft rejection; and tolerance to the fetal allograft represents an important mechanism for maintaining a pregnancy. Cells expressing IDO in the foeto-maternal interface protect the allogeneic foetus from lethal rejection by maternal immune responses. Inhibition of IDO by exposure of pregnant mice to 1 -methyl-tryptophan induced a T cell-mediated rejection of allogeneic concepti, whereas syngeneic concepti were not affected; this suggests that IDO expression at the foetal- maternal interface is necessary to prevent rejection of the foetal allograft. Accumulating evidence indicates that IDO production and normal function at the foetal- maternal interface may play a prominent role in pregnancy tolerance. Therefore, an IDO inhibitor could be used as a contraceptive or abortive agent.

On the above basis, the inventors have determined that a strong rationale exists for the therapeutic utility of inventive compounds which block the activity of IDO, in treating the above- mentioned diseases, conditions and disorders.

The amount of a compound of general formula (I) that will correspond to such an amount will vary depending upon factors such as the particular compound, disease condition and its severity, the identity (e.g., weight) of the patient in need of treatment, but can nevertheless be routinely determined by one skilled in the art.

Preferably, the inventive compounds, the pharmaceutically acceptable salt thereof, or the inventive pharmaceutical compositions as described above are useful in the prophylaxis or treatment of a disease and/or a disorder caused by and/or associated with indolamine 2,3-dioxygenase 1 (IDO1), wherein the disease and/or the disorder selected from: an inflammatory condition, an infectious disease, a cancer, a central nervous system disease or disorder, a disease or disorder relating to female reproductive health, coronary heart disease, chronic renal failure, post anaesthesia cognitive dysfunction, and cataracts.

The inflammatory condition is a condition relating to immune B cell, T cell, dendritic cell, natural killer cell, macrophage, and/or neutrophil dysregulation;

The infectious disease is selected from a bacterial infection and a viral infection, preferably a gut infection, sepsis, and sepsis induced hypotension.

The cancer is a cancer selected from: a solid or liquid tumour including cancer of the eye, brain (such as gliomas, glioblastomas, meduUablastomas, craniopharyngioma, ependymoma, and astrocytoma), spinal cord, kidney, mouth, lip, throat, oral cavity, nasal cavity, small intestine, colon, parathyroid gland, gall bladder, head and neck, breast, bone, bile duct, cervix, heart, hypopharyngeal gland, lung, bronchus, liver, skin, ureter, urethra, testicles, vagina, anus, laryngeal gland, ovary, thyroid, oesophagus, nasopharyngeal gland, pituitary gland, salivary gland, prostate, pancreas, adrenal glands; an endometrial cancer, oral cancer, melanoma, neuroblastoma, gastric cancer , an angiomatosis, a hemangioblastoma, a pheochromocytoma, a pancreatic cyst, a renal cell carcinoma, Wilms' tumour, squamous cell carcinoma, sarcoma, osteosarcoma, Kaposi sarcoma, rhabdomyosarcoma, hepatocellular carcinoma, PTEN Hamartoma-Tumor Syndromes (PHTS) (such as Lhermitte-Duclos disease, Cowden syndrome, Proteus syndrome, and Proteus-like syndrome), leukaemias and lymphomas (such as acute lymphoblastic leukaemia, chronic lymphocytic leukaemia, acute myelogenous leukaemia, chronic myelogenous leukaemia, hairy cell leukaemia, T-cell prolymphocytic leukemia (T-PLL), large granular lymphocytic leukemia, adult T-cell leukemia, juvenile myelomonocytic leukaemia, Hodgkin lymphoma, non-Hodgkin lymphoma, mantle lymphoma, follicular lymphoma, primary effusion lymphoma, AIDS-related lymphoma, Hodgkin lymphoma, diffuse B cell lymphoma, Burkitt lymphoma, and cutaneous T-cell lymphoma);

Prefearably, the cancer is selected from acute myeloid leukemia (AML), a small-cell lung cancer, a melanoma, an ovarian cancer, a colorectal cancer, a pancreatic cancer, an endometrial cancer, and a skin papilloma.

The central nervous system (CNS) disease or disorder is selected from amyotrophic lateral sclerosis (AML), Huntington's disease, Alzheimer's disease, pain, a psychiatric disorder, multiple sclerosis, Parkinson's disease, and HIV related neurocognitive decline.

The disease or disorder relating to female reproductive health is endometriosis, or the condition relating to female reproductive health is contraception or abortion.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Description of Figures

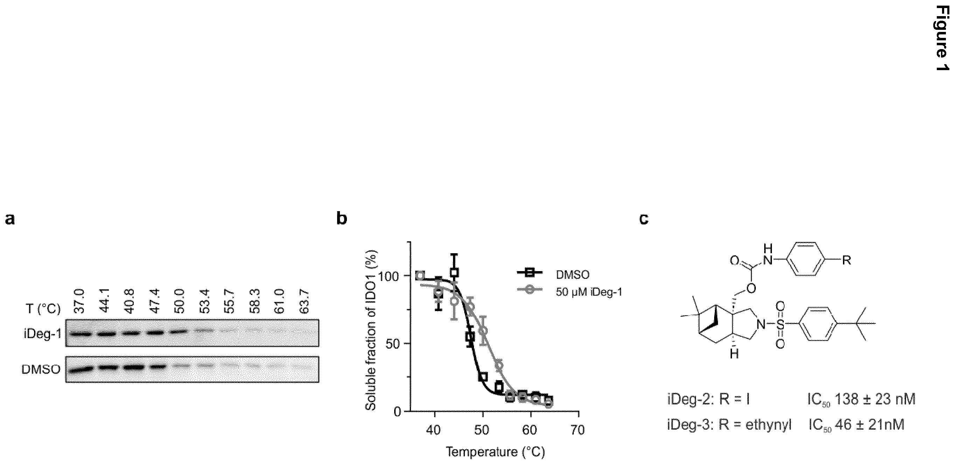

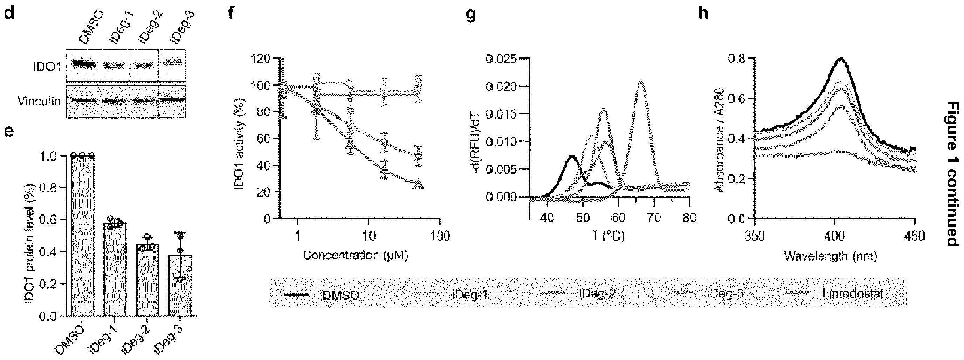

**Figure. 1****:** inventive compounds interact with IDO1. a,b, CETSA in intact SKOV3 cells treated with 50 µM compound **1** (iDeg-1) or DMSO for 1 h followed by heat treatment and immunoblotting.
   **a**, Representative immunoblot for IDO1.
   **b**, Quantification of band intensities from **a.** Mean values ± SD, n = 3.
   **c**, Structures of compounds **11** (iDeg-2) and **19** (iDeg-3) and IC₅₀ values in the Kyn assay in BxPC3 cells. Mean values ± SD, n = 3.
   **d,e,** Influence of compounds **1, 11**, **19** (iDeg-1, 2 and 3) on IDO1 protein levels in BxPC3 cells. Cells were treated with IFN-γ and the compounds (3.33 µM) for 24 h prior to immunoblotting (d). Quantification of band intensities shown in **e.** Mean values ± SD, n = 3.
   **f**, Influence on the *in vitro* rhIDO1 activity. rhIDO1 was pre-incubated with the compounds at 37°C for 40 min prior to detection of Kyn levels using p-DMAB. Mean values ± SD, n = 3.
   **g**, rhIDO1 thermal stability in presence of 50 µM compounds **1, 11**, or **19** (iDeg-1, 2 or 3) or DMSO and the apo-IDO1 inhibitor linrodostat (50 µM) using nanoDSF. rhIDO1 and compounds were pre-incubated for 3 h min at 37°C prior to the measurement. Representative result (n = 3).
   **h**, Detection of heme-bound IDO1 by means of UV/Vis spectroscopy in presence of compounds **1, 11**, or **19** (iDeg-1, 2 or 3) (50 µM), DMSO or linrodostat (50 µM). Incubation at 37°C for 3 h. Representative data for n = 3..

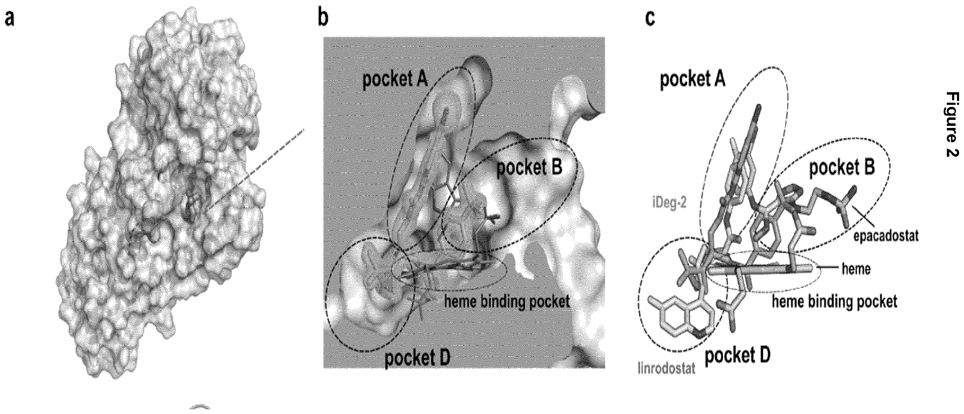

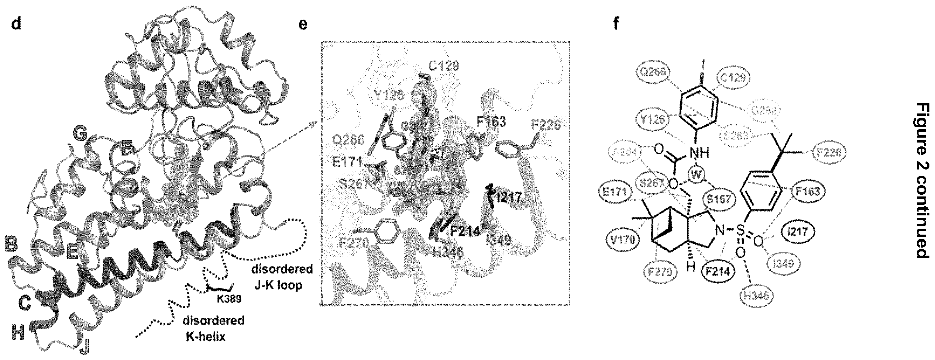

**Figure.2****. Co-crystal structure of IDO1 with compound 11** (**iDeg-2**).
   **a,** Surface representation of apo-IDO1 with iDeg-2 as sticks bound in the catalytic cleft.
   **b,** Surface model in the cut-in side view of **compound 11** (**iDeg-2**) in the active site. The electron density (2Fo-Fc) around **compound 11** (**iDeg-2**) in blue contoured at 1.6 σ. Superposition with cyanide- and L-Trp-bound holo-IDO1 (PDB ID: 5wmu). The hydrophobic pockets A, B, D and the heme binding site are indicated.
   **c,** Overlay of **compound 11** (**iDeg-2**) (violet) with apo-IDO1 inhibitor linrodostat (PDB ID: 6dpr-B) (grey) and holo-IDO1 inhibitor epacadostat in complex with heme (PDB ID: 5wn8) (pink).
   **d,** Apo-IDO1 **compound 11** (**iDeg-2**) structure shown as cartoon (violet). The J-K loop and the K-helix are disordered. Helices that show conformational changes after compound binding are colored. **e*,*** Zoom-in view of the amino acids involved in **compound 11** (**iDeg-2**) binding. Hydrogen bonds are indicated by black dotted lines. Helices that show a conformational change and amino acids located in these helices are colored like in Fig. 2d.
   **f,** Schematic illustration of the interactions. Hydrogen bonds, van der Waals interactions and π-π stacking interactions are represented by dotted lines in blue, grey and green, respectively. Side- and main-chain interactions are indicated by solid and dashed circles, respectively.

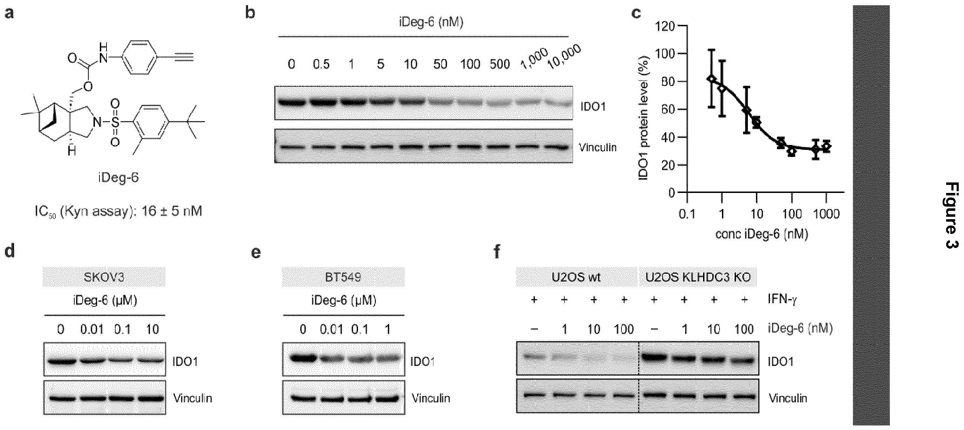

**Figure.3****: Validation of KLHDC3 as E3 ligase regulating IDO1.**
   **a,** Structure of compound **71** (iDeg-6) and IC₅₀ value in the Kyn assay in BxPC3 cells. Mean value ± SD, n = 3.
   **b,c,** Reduction of IDO1 protein levels by compound **71** (iDeg-6). Degradation efficiency was assessed in a modified setup including IFN-γ washout prior to compound addition. BxPC3 cells were treated with 50 ng/mL IFN-γ for 24 h prior to washout, addition of compound **71** (iDeg-6) for 24 h and immunoblotting (b). **c,** Quantification of IDO1 protein levels from b (mean values ± SD, n = 4 except for 50 and 500 nM (n = 3)).
   **d,e,** IDO1 levels in SKOV3 (d) and BT549 (e) cells. Cells were treated with iDeg-6 for 24 h prior to immunoblotting.
   **f,g,** IDO1 protein levels in wildtype (wt) U2OS or KLHDC3 knockout (KO) U2OS cells. Cells were stimulated with 5 ng/mL IFN-γ for 24 h prior to washout, treatment with compound **71** (iDeg-6) or DMSO for 24 h and immunoblotting (f). Representative data of n = 4 (U2OS wt) or n = 2 (KO cells).
   **g,** Quantification of band intensities from f. Mean values ± SD, n = 3 or n = 2.
   h,i, IDO1 protein levels in KBM7-BFP-IDO1 (CTRL) or KBM7-BFP-IDO1 KLHDC3 KO1 or KO2 cells in absence (h, normalized to CTRL) or presence of iDeg-6 (i, normalized to respective genotype). Mean values ± SD, n = 3.
   **j,** Fluorescent scan of pulse-chase assay monitoring ubiquitination of apo-IDO1 with or without supplementation with small molecules by CRL2^{KLHDC3}. Apo-IDO1 was incubated with the indicated compounds (30 µM) for 90 min at 37°C prior to incubation with CRL2^{KLHDC3} E3 complex. Ubiquitination reactions were initiated by the addition of the pre-formed thioester-linked E2~ubiquitin conjugate (UBE2R2-UB) with the ubiquitin fluorescently-labeled.
   **k,** Detection of heme load for the conditions in j detected by means of UV/Vis spectroscopy. Representative data for n = 2.

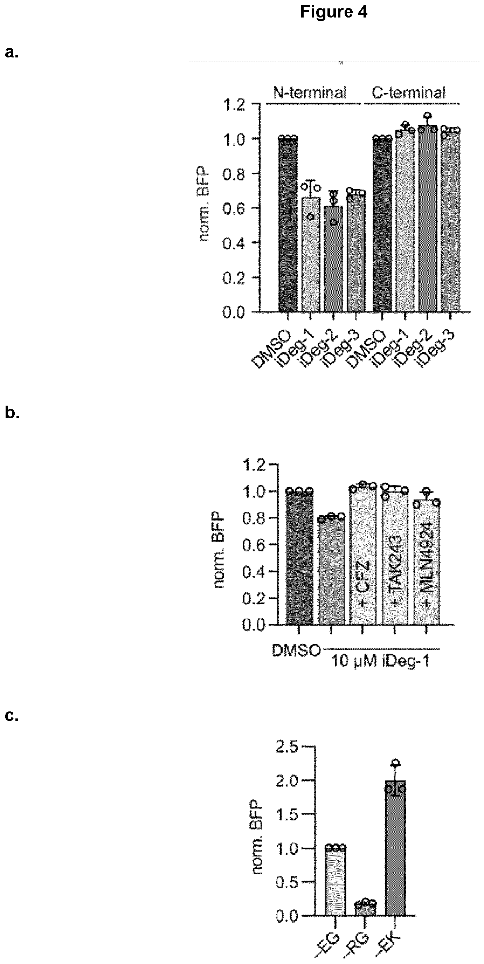

**Fig. 4****: KLHDC3 is involved in iDeg-mediated IDO1 degradation.**
   **a,** Detection of IDO1 levels using the IDO1 stability reporters. KBM7 IDO1 reporter cells were treated with iDeg-1, 2 or 3 (1 µM) for 24 h prior to detection of IDO1 levels using flow cytometry. Normalized BFP to mCherry ratios (norm. BFP) were calculated per genotype respectively. Mean values ± SD, n = 3.
   **b,** IDO1 depletion is rescued by 10 h co-treatment with either CFZ, TAK243 or MLN4924 (1 µM each). Mean values ± SD, n = 3.
   **c,** IDO1 stability reporter variants in KBM7 cells measured by flow cytometry and depicted normalized to the wt (i.e., -EG) reporter. Mean values ± SD, n = 3.

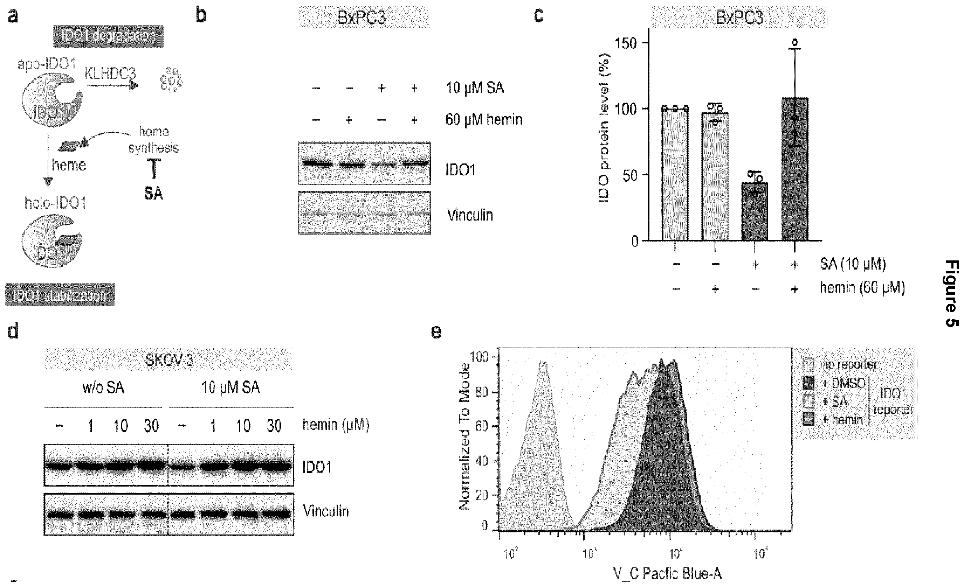

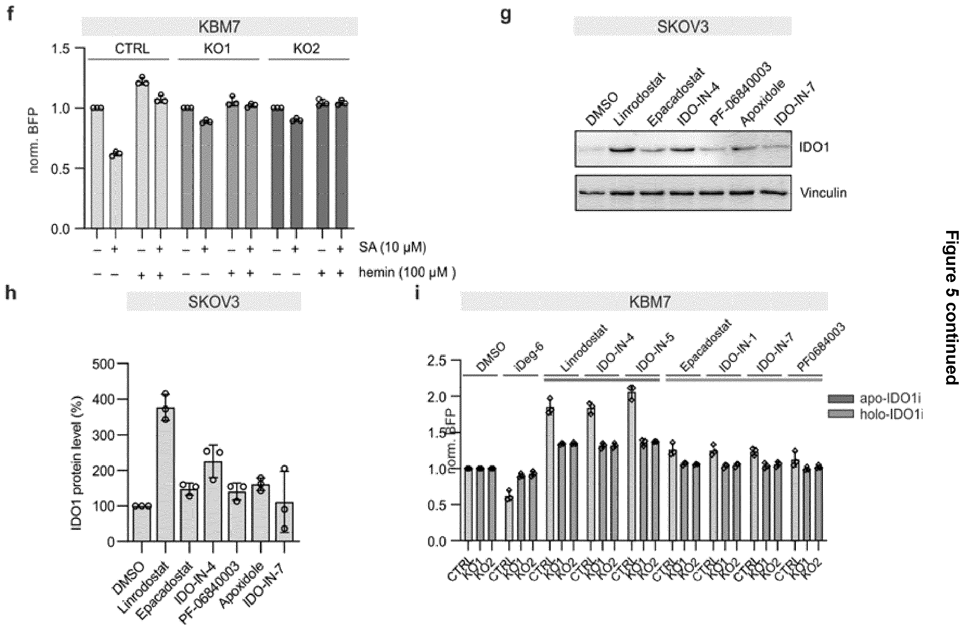

**Fig. 5****. Apo-IDO and not holo-IDO1 is the preferential substrate for degradation.**
   **a,** Regulation of IDO1 by heme synthesis and succinyl acetone (SA) as an inhibitor of heme synthesis.
   **b,c,** Influence of SA and heme on IDO1 protein levels in BxPC3 cells as detected using immunoblotting. Cells were treated with 50 ng/mL IFN-γ with or without 10 µM SA for 24 h followed by the addition of hemin for further 24 h. c, Quantification of band intensities from b. Mean values ± SD, n = 3.
   **d,** Influence of SA and heme on IDO1 levels in SKOV3 cells. Cells were treated with SA for 48 h prior to the addition of hemin for another 24 h and immunoblotting.
   **e,f,** Influence of SA and hemin on IDO1 abundance in KBM7-BFP-IDO1 (e) or KBM7-BFP-IDO1 KLHDC3 knockout cells (f). Cells were pre-treated with 10 µM SA for 24 h followed by a treatment for 48 h with 100 µM hemin or DMSO as a control. IDO1 levels were quantified using flow cytometry. Mean values ± SD, n = 3.
   **g,h,** Influence of known IDO1 inhibitors on IDO1 protein levels in SKOV3 cells. Cells were incubated with the compounds (5 µM) for 48 h prior to immunoblotting (g).
   **h,** Quantification of band intensities from g. Mean values ± SD (n = 3). **i,** Influence of IDO1 inhibitors (IDO1i, 1 µM) on IDO1 protein levels in KBM7-BFP-IDO1 or KBM7-BFP-IDO1 KLHDC3 knockout cells. Cells were treated with the compounds for 24 h followed by quantification of IDO1 using flow cytometry. Mean values ± SD, n = 3.

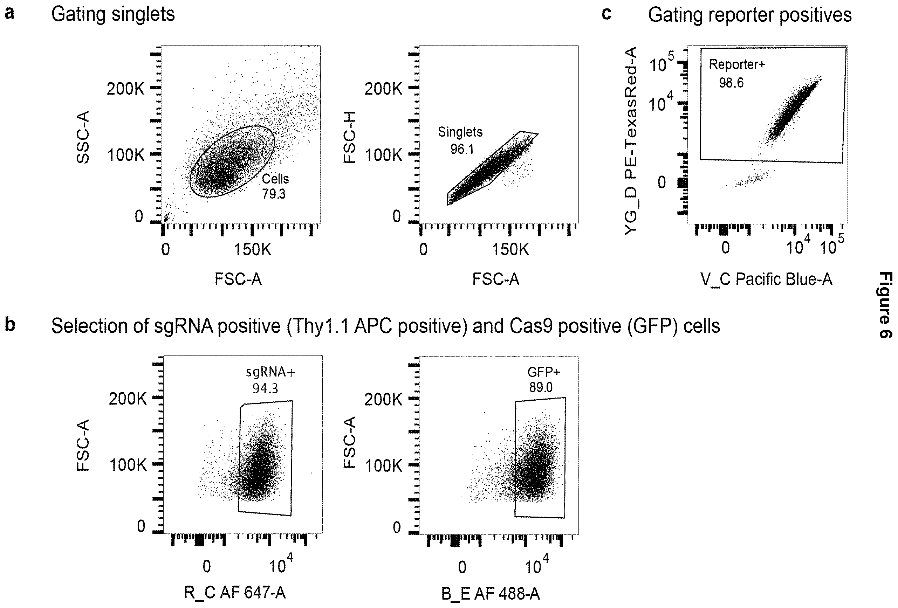

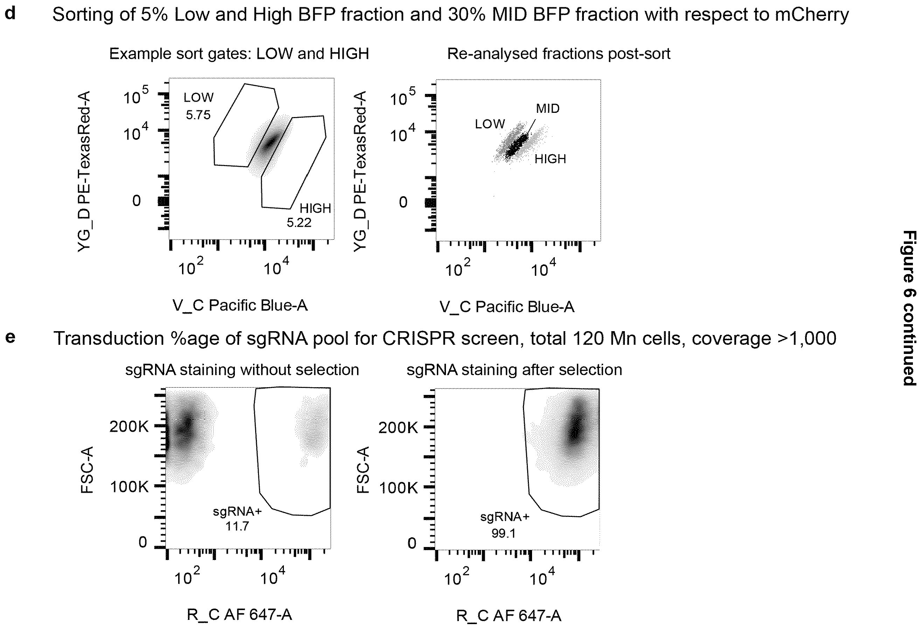

**Figure. 6****. Gating strategy for flow cytometric assays and FACS.**
   **a,** The identification of viable cells was performed using the forward and side scatter area (FSC-A and SSC-A, respectively). Singlets were selected using the FSC-A and FSC-H (height) as indicated.
   **b,** sgRNA transduction was assessed by staining for the Thy1.1 surface antigen for both single and pooled sgRNA experiments. GFP expression monitored Cas9 induction.
   **c,** Reporter-positive cells were selected in the BFP and mCherry channel.
   **d,** For the CRISPR-Cas9 screen the highest (HIGH) and lowest 5% (LOW) of BFP expressing cells were sorted and compared against the 30 % mid-fraction (MID). Reanalysis of sorted fractions was routinely performed to confirm sorting accuracy.
   **e,** The sgRNA pool for the CRISPR-Cas9 screen was transduced at 11.7 % for a total of 120 million cells and selected with G-418 (see Materials and Methods) prior to screening.

### Examples

### General Information

Unless otherwise noted, all commercially available compounds were used as provided without further purifications. Solvents for chromatography were technical grade. Analytical thin-layer chromatography (TLC) was performed using petroleum-ether and ethyl acetate as gradient eluents, based on Merck aluminium TLC sheets (silica gel 60F254). Compounds were visualized by irradiation with UV light. Column chromatography was performed using silica gel Merck 60 (particle size 0.040-0.063 mm), or Flash column chromatography (FC) was performed using silica gel Merck 60 (particle size 0.040-0.063 mm). Further purification was achieved using preparative HPLC (column: NUCLEODUR C18 Gravity, 250 mm × 21 mm, particle size 10 µm). Compounds were visualized by irradiation with UV light. ¹H-NMR- and ¹³C-NMR spectra were recorded on a Bruker DRX400 (400 MHz), Bruker DRX500 (500 MHz), INOVA500 (500 MHz) and Bruker DRX700 using CD₂Cl₂, CDCl₃, or (CD₃)₂SO as solvent. Data are reported in the following order: chemical shift (δ) values are reported in ppm with the solvent resonance as internal standard (CD₂Cl₂: δ = 5.32 ppm for ¹H-spectra, δ = 53.84 ppm for ¹³C-spectra; CDCl₃: δ = 7.26 ppm for 1H-spectra, δ = 77.16 ppm for ¹³C-spectra); multiplicities are indicated s (singlet), d (doublet), t (triplet), q (quartet), m (multiplet); coupling constants (*J*) are given in Hertz (Hz). High resolution mass spectra were recorded on a LTQ Orbitrap mass spectrometer coupled to an Accela HPLC-System (HPLC column: Hypersyl GOLD, 50 mm × 1 mm, particle size 1.9 µm, ionization method: electron spray ionization).

For the synthesis of inventive compounds, (1*R*)-(-)-myrtenal was subjected to a 1,3-dipolar cycloaddition with the azomethine ylide generated in situ from *N-*(methoxymethyl)-*N*-(trimethylsilylmethyl) benzylamine by treatment with trifluoroacetic acid to yield the corresponding pyrrolidine cycloadduct (**Scheme 1a**) analogous to previously reported comparable cycloadditions (Mutti *et al.* and Sheffler. *et al.*).¹⁻² Subsequently, the aldehyde **Int-1** was reduced to the corresponding alcohol **Int-2** (**Scheme 1b**).

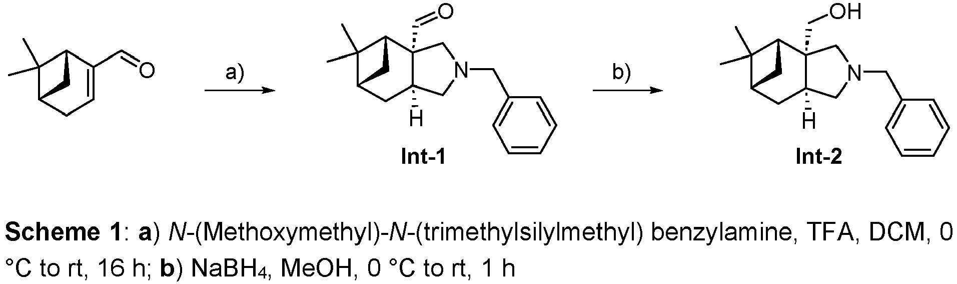

The alcohol **Int-2** was further functionalized as urethane, followed by *N-*debenzylation and sulfonamide formation (**Scheme 2**).

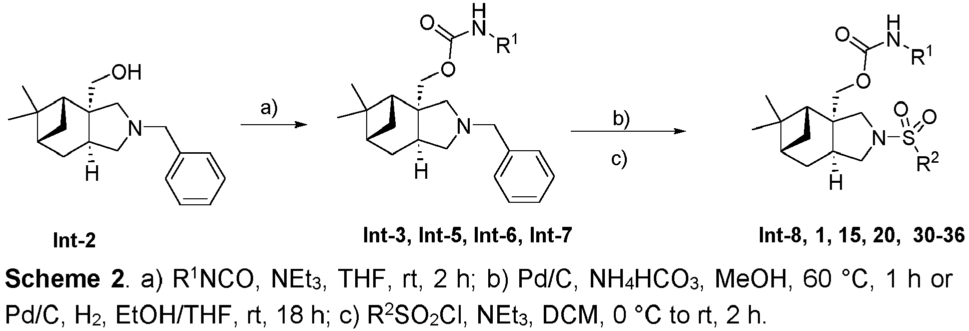

For installing of iodide-phenyl carbamate, the *N*-benzyl protecting group of **Int-2** was removed to yield amine **Int-9** followed by sulfonamide formation and functionalization of the alcohol as urethane (**Scheme 3**). For ethynyl carbamate formation, the aryl iodide was subjected to a Pd(0) mediated Sonogashira coupling reaction with TMS-acetylene, followed by desilylation (**Scheme 3**).

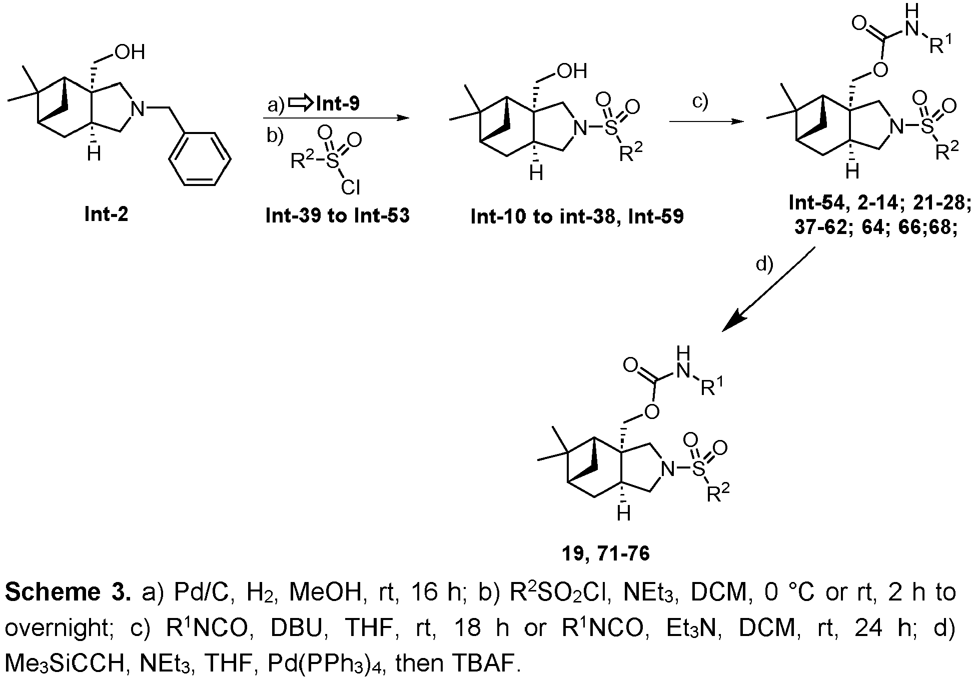

### General procedure A: carbamate formation

### Procedure A1

To a solution of the alcohol (1.0 equiv.) in anhydrous THF (0.2 M) was added NEts (2.0 equiv.) and the respective isocyanate (1.3 equiv.). The solution was stirred at room temperature for 2 h. The solvent was removed under reduced pressure. Column chromatography afforded the carbamate.

### Procedure A2

To a solution of the alcohol (1.0 equiv.) in anhydrous THF (0.1-0.2 M) was added DBU (1.5 equiv.) and the respective isocyanate (1.2 equiv.) at 0 °C. The solution was stirred at room temperature for 18-22 h. The solvent was removed under reduced pressure. Column chromatography afforded the carbamate.

### Procedure A3

To a solution of the alcohol (1.0 equiv.) in anhydrous DCM (0.2 M) was added NEts (3.0 equiv.) and the respective isocyanate (1.3 equiv.). The solution was stirred at room temperature for 24 h and then water was added. The mixture was then extracted with DCM (3 times) and the combined organic phases was dried over anhydrous MgSO₄, filtered and concentrated under reduced pressure. Column chromatography afforded the carbamate.

### General procedure B: benzyl cleavage

### Procedure B1

To a degassed solution of the *N*-benzyl amine (1.0 equiv.) in MeOH (0.1-0.2 M) was added ammonium formate (5.0 equiv.) and Pd/C (10 wt.%), and the suspension was stirred at 60 °C for 1 h. After cooling to room temperature, the reaction mixture was filtered over Celite, washed with EtOAc and the solvent was removed under reduced pressure to give the amine.

### Procedure B2

To a degassed solution of the *N*-benzyl protected amine (1.0 equiv.) in EtOH/THF (4:1, 0.1-0.2 M) was added Pd/C (10 wt.%), and the suspension was stirred under a H₂ atmosphere for 16-22 h. The reaction mixture was filtered over Celite, washed with EtOAc and the solvent was removed under reduced pressure to give the amine.

### Procedure B3

To a degassed solution of the *N*-benzyl protected amine in MeOH (0.1M) was added Pd/C (10 wt%, 0.2 equiv.), and the mixture was stirred under a H₂ atmosphere for 16 h. The reaction mixture was filtered over Celite, washed with MeOH and the solvent was removed under reduced pressure to give amine.

### General procedure C: sulfonamide formation

### Procedure C1

To a solution of the amine (1.0 equiv.) in DCM (0.2 M) at 0 °C was added NEts (2.0 equiv.) and the respective sulfonyl chloride (1.0 -1.2 equiv.) and the solution was stirred at room temperature for 2 h. The solvent was removed under reduced pressure. Column chromatography afforded the sulfonamide.

### Procedure C2

To a solution of **Int-9** (1.0 equiv.) in THF (0.1M) at 0 °C was added NEts (1.2 equiv.) and arenesulfonyl chloride (1.0 equiv.). The mixture was stirred at 0 °C for 2 h to overnight. The solvent was removed under reduced pressure. Column chromatography afforded a colorless sulfonamide.

### General procedure D: ethynyl group installation

To a degassed solution of Ar-I (1.0 equiv.) in THF (0.1 M) was added NEts (5.0 equiv.), trimethylsilylacetylene (2.0 equiv.), Cul (0.2 equiv.) and Pd(PPh₃)₄ (0.1 equiv.). The solution was stirred at room temperature for 22 h. The reaction mixture was poured into sat. aq. NaCl solution, extracted with DCM twice, dried over MgSO₄ and the solvent was removed under reduced pressure. Column chromatography afforded the product.

To a solution of the above alkynylated product in THF (0.1 M) was added TBAF (1.2 equiv.), and the mixture was stirred at room temperature for 1 h. The solvent was removed under reduced pressure. Column chromatography afforded a colorless solid.

### Synthesis of compounds 1, 15- 20, 30-36

### Synthesis of intermediates Int-1 to Int-8

### (3aS,4R,6R,7aS)-2-benzyl-5,5-dimethyloctahydro-3aH-4,6-methanoisoindole-3a-carbaldehyde (Int-1)

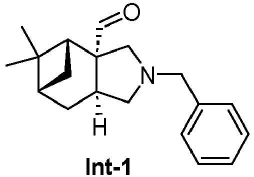

To a solution of myrtenal (2.0 g, 13.3 mmol, 1 equiv.) and *N*-(methoxymethyl)-*N-*trimethylsilylmethyl) benzylamine (4.5 mL, 15.9 mmol, 1.2 equiv., 90% pure) in 65 mL dry CH₂Cl₂ under argon atmosphere in an ice bath, a solution of TFA in dry DCM (0.2 mL, 2.6 mmol, 0.2 equiv., 0.1 M) was added dropwise over 1 min. The mixture was stirred at 5 to 10 °C for 12 h. Progress of the reaction was monitored by TLC. After complete consumption of the starting material the reaction was quenched by the addition of aqueous saturated sodium bicarbonate and the mixture was stirred for 5 min. The layers were separated and the aqueous layer was extracted with dichloromethane. The combined organic layers were dried over Na₂SO₄, filtered, and concentrated under reduced pressure to give a crude residue, which was purified by chromatography (PE/EtOAc 4:1). The product was obtained as colorless oil (2.79 g, 9.84 mmol, 74 % yield).

**¹H NMR** (400 MHz, CDCl₃): δ 9.69 (s, 1H), 7.38 - 7.13 (m, 5H), 3.65 (d, *J =* 13.1 Hz, 1H), 3.50 (d, *J* = 13.1 Hz, 1H), 3.18 (t, *J* = 8.4 Hz, 1H), 2.92 (dtd, *J* = 10.7, 7.8, 3.0 Hz, 1H), 2.69 (d, *J* = 10.2 Hz, 1H), 2.43 (d, *J* = 10.2 Hz, 1H), 2.35 - 2.08 (m, 4H), 1.88 (tt, *J* = 6.0, 3.1 Hz, 1H), 1.69 - 1.57 (m, 2H), 1.24 (s, 3H), 0.69 (s, 3H).

**¹³C NMR** (101 MHz, CDCl₃): δ 206.1, 138.9, 128.6, 128.4, 127.1, 66.2, 61.2, 60.9, 59.7, 45.8, 40.5, 38.7, 32.2, 31.0, 26.6, 26.5, 23.7.

**HRMS** (ESI) for C₁₉H₂₆ON: calculated 284.2009 [M+H]⁺, found: 284.2015.

### ((3aS,4R,6R,7aS)-2-benzyl-5,5-dimethyloctahydro-3aH-4,6-methanoisoindol-3a-yl)methanol (Int-2)

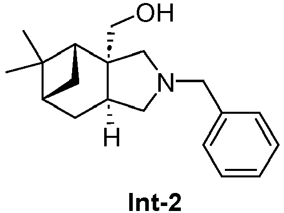

To a solution of **Int-1** (1.0 g, 3.5 mmol, 1 equiv.) in MeOH (10 mL) at 0 °C. NaBH₄ (173 mg, 4.5 mmol, 1.3 equiv.) was added in portions. After stirring for 1 h, the reaction was quenched with acetone (5 mL). The solvents were removed under reduced pressure and the residue was dissolved in water. The aqueous layer was extracted with ethyl acetate twice, the combined organic layers were dried over Na₂SO₄ and concentrated under reduced pressure. The crude product was purified by chromatography (PE/EtOAc 4:1) and the product was obtained as colorless **oil Int-2** (0.92 g, 3.22 mmol, 92 % yield).

**¹H NMR** (500 MHz, CDCl₃) δ 7.32 (d, *J* = 4.5 Hz, 4H), 7.30 - 7.25 (m, 1H), 3.81 (d, *J* = 9.9 Hz, 1H), 3.68 (t, *J* = 10.8 Hz, 2H), 3.39 (d, *J* = 9.9 Hz, 1H), 3.36 (d, *J* = 8.6 Hz, 1H), 2.86 (d, *J* = 9.3 Hz, 1H), 2.59 - 2.52 (m, 1H), 2.50 (d, *J* = 9.5 Hz, 1H), 2.23 - 2.17 (m, 1H), 2.18-2.11 (m, 1H), 2.11 (d, *J* = 13.9 Hz, 1H), 1.89 (dq, *J* = 5.9, 3.0 Hz, 1H), 1.75 (dd, *J* = 6.4, 4.9 Hz, 1H), 1.56 (d, *J* = 13.5 Hz, 1H), 1.40 (d, *J* = 10.4 Hz, 1H), 1.22 (s, 3H), 0.95 (s, 3H).

**¹³C NMR** (126 MHz, CDCl₃) δ 137.4, 129.0, 128.7, 127.7, 71.4, 66.4, 64.2, 59.7, 51.0, 46.2, 41.3, 38.9, 32.9, 32.1, 27.6, 27.6, 24.4.

**HRMS** (ESI) for C₁₉H₂₈ON; calculated 286.2165 [M+H]⁺, found 286.2167.

[α]²⁰_{D} = -53.7° (CHCl₃, c = 1.0)

### ((3aS,4R,6R,7aS)-2-benzyl-5,5-dimethyloctahydro-3aH-4,6-methanoisoindol-3a-yl) methyl p-tolylcarbamate (Int-3)

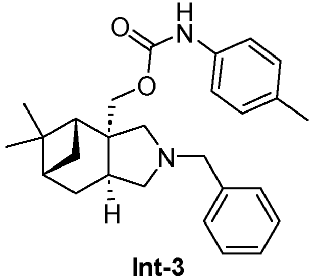

Following general procedure A1: From **Int-2** (100 mg, 0.350 mmol) and p-tolyl isocyanate. Purification by column chromatography (PE/EtOAc 9:1) afforded a colorless foam **Int-3** (122 mg, 0.291 mmol, 83 % yield).

**¹H NMR** (CDCl₃, 500 MHz): δ = 1.03 (s, 3 H, CH₃), 1.23 (s, 3 H, CH₃), 1.60 (dt, *J* = 3.5, 13.3 Hz, 1 H, CH), 1.82-1.89 (m, 2 H, 2 × CH), 1.95 (t, *J* = 5.7 Hz, 1 H, CH), 2.06-2.19 (m, 2 H, 2 × CH), 2.21-2.28 (m, 1 H, CH), 2.30 (s, 3 H, CH₃), 2.34 (d, *J* = 9.4 Hz, 1 H, CH), 2.46-2.52 (m, 1 H, CH), 2.55 (d, *J* = 9.5 Hz, 1 H, CH), ), 2.57-2.62 (m, 1 H, CH), 3.55 (d, *J* = 13.1 Hz, 1 H, CH_{A}), 3.63 (d, *J* = 13.3 Hz, 1 H, CH_{B}), 4.05 (d, *J* = 10.3 Hz, 1 H, CH_{A}), 4.21 (d, *J* = 10.3 Hz, 1 H, CH_{B'}), 6.46 (s_{br}, 1 H, NH), 7.10 (d, *J* = 8.1 Hz, 2 H, 2 × Ar-H), 7.20-7.39 (m, 7 H, 7 × Ar-H).

**¹³C NMR** (CDCl₃, 125 MHz): δ = 20.9, 23.6, 26.7, 27.1, 27.6, 29.9, 34.6, 34.9, 39.5, 40.6, 46.3, 50.5, 64.2, 71.0, 118.7, 126.8, 128.3, 128.6, 129.7, 133.01, 135.5, 139.9, 154.0.

**MS** (ESI) *m*/*z* (%) = 419.4 (100) [M+H]⁺.

**HRMS** (ESI) for C₂₇H₃₅N₂O₂: calculated 419.2693 [M+H]⁺, found 419.2692.

### ((3aS,4R,6R,7aS)-5,5-dimethyloctahydro-3aH-4,6-methanoisoindol-3a-yl) methyl p-tolylcarbamate (Int-4)

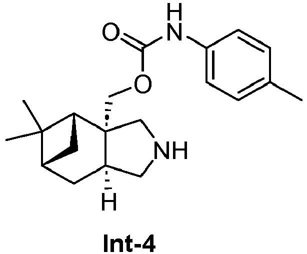

Following general procedure B1: From **Int-3** (1.0 g, 2.39 mmol) afforded a colorless solid **Int-4** (745.5 mg, 2.27 mmol, 95 % yield).

**¹H NMR** (CDCl₃, 400 MHz): δ = 7.33 (d, *J* = 8.2 Hz, 2H), 7.08 (d, *J* = 8.2 Hz, 2H), 4.28 (d, *J* = 10.7 Hz, 1H), 4.07 (d, *J* = 10.7 Hz, 1H), 3.75 - 3.61 (m, 1H), 3.41 - 3.28 (m, 2H), 3.11 (d, *J* = 12.3 Hz, 1H), 2.95 (dd, *J* = 11.8, 7.5 Hz, 1H), 2.51 - 2.22 (m, 3H), 2.27 (s, 3H), 2.04 (t, *J* = 5.6 Hz, 1H), 1.96 (d, *J* = 5.4 Hz, 1H), 1.65 (dt, *J* = 13.8, 3.2 Hz, 1H), 1.28 (d, 3H), 1.06 (s, 3H).

**HRMS** (ESI) for C₂₀H₂₉N₂O₂: calculated 329.220 [M+H]⁺, found: 329.224.

### ((3aS,4R,6R,7aS)-2-benzyl-5,5-dimethyloctahydro-3aH-4,6-methanoisoindol-3a-yl)methyl (3-nitrophenyl)carbamate (Int-5)

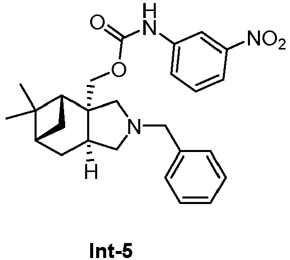

Following general procedure A1: From **Int-2** (72.0 mg, 0.252 mmol) and 3-nitrophenyl isocyanate. Purification by column chromatography (PE/EtOAc 9:1) afforded a yellow oil **Int-5** (98.0 mg, 0.218 mmol, 86 %yield).

**¹H NMR** (CDCl₃, 500 MHz): δ = 1.03 (s, 3 H, CH₃), 1.24 (s, 3 H, CH₃), 1.62 (dt, *J* = 3.5, 13.3 Hz, 1 H, CH), 1.78-1.90 (m, 2 H, 2 × CH), 1.91-1.97 (m, 1 H, CH), 2.06-2.21 (m, 2 H, 2 × CH), 2.22-2.29 (m, 1 H, CH), 2.32-2.42 (m, 1 H, CH), 2.46-2.59 (m, 2 H, 2 × CH), 2.60-2.71 (m, 1 H, CH), 3.52-3.67 (m, 2 H, 2 × CH), 4.06-4.15 (m, 1 H, CH), 4.19-4.29 (m, 1 H, CH), 6.85-6.96 (m, 1 H, NH), 7.19-7.40 (m, 5 H, 5 × Ar-H), 7.46 (t, *J* = 8.2 Hz, 1 H, Ar-H), 7.71-7.81 (m, 1 H, Ar-H), 7.88-7.92 (m, 1 H, Ar-H), 8.22-8.34 (m, 1 H, Ar-H).

**¹³C NMR** (CDCl₃, 125 MHz): δ = 23.6, 26.7, 27.1, 27.6, 34.3, 34.8, 39.4, 40.5, 46.2, 49.9, 60.6, 64.0, 64.4, 71.6, 113.4, 118.0, 120.7, 124.2, 127.0, 128.4, 128.6, 130.0, 139.6, 148.8, 153.5.

**MS** (ESI) *m*/*z* (%) = 450.4 (100) [M+H]⁺.

**HRMS** (ESI) for C₂₆H₃₂N₃O₄: calculated 450.2387 [M+H]⁺, found 450.2386.

### ((3aS,4R,6R,7aS)-2-benzyl-5,5-dimethyloctahydro-3aH-4,6-methanoisoindol-3a-yl) methyl (4-methoxyphenyl) carbamate (Int-6)

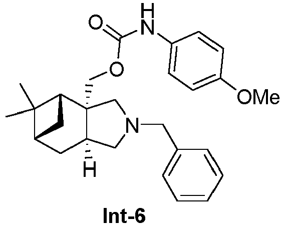

Following general procedure A1: From **Int-2** (285 mg, 1.00 mmol) and 4-methoxyphenyl isocyanate. Purification by column chromatography (PE/EtOAc= 8:1) afforded a slightly yellow semicrystalline solid **Int-6** (314 mg, 0.723 mmol, 72 % yield).

**¹H NMR** (CDCl₃, 500 MHz): δ = 1.03 (s, 3 H, CH₃), 1.23 (s, 3 H, CH₃), 1.60 (dt, *J* = 3.5, 13.3 Hz, 1 H, CH), 1.83-1.90 (m, 2 H, 2 × CH), 1.91-1.98 (m, 1 H, CH), 2.06-2.17 (m, 2 H, 2 × CH), 2.21-2.28 (m, 1 H, CH), 2.33 (d, *J* = 9.4 Hz, 1 H, CH), 2.45-2.64 (m, 3 H, 3 × CH), 3.55 (d, *J* = 13.1 Hz, 1 H, CH_{A}), 3.63 (d, *J* = 13.4 Hz, 1 H, CH_{B}), 3.78 (s, 3 H, OMe), 4.04 (d, *J* = 10.4 Hz, 1 H, CH_{A'}), 4.19 (d, *J* = 10.3 Hz, 1 H, CH_{B'}), 6.44 (s_{br}, 1 H, NH), 6.82-6.88 (m, 2 H, 2 × Ar-H), 7.20-7.39 (m, 7 H, 7 × Ar-H). **¹³C NMR** (CDCl₃, 125 MHz): δ = 23.6, 26.7, 27.1, 27.6, 29.8, 34.6, 34.8, 39.4, 40.6, 46.3, 50.0, 55.6, 60.0, 64.2, 64.2, 114.3, 120.6, 126.8, 128.3, 128.6, 139.9, 153.9.

**MS** (ESI) *m*/*z* (%) = 435.3 (100) [M+H]⁺.

**HRMS** (ESI) for C₂₇H₃₅N₂O₃: calculated 435.2642 [M+H]⁺, found 435.2640.

[α]²⁰_{D} = -13.5° (CHCl₃, *c* = 1.0).

### ((3aS,4R,6R,7aS)-2-benzyl-5,5-dimethyloctahydro-3aH-4,6-methanoisoindol-3a-yl) methyl (2-nitrophenyl)carbamate (Int-7)

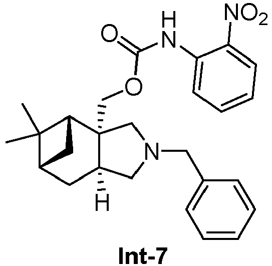

Following general procedure A1: From **Int-2** (75.0 mg, 0.263 mmol) and 2-nitrophenyl isocyanate. Purification by column chromatography (PE/EtOAc 9:1) afforded a yellow oil **Int-7** (98.0 mg, 0.218 mmol, 83 %yield).

**¹H NMR** (CDCl₃, 500 MHz): δ = 1.04 (s, 3 H, CH₃), 1.24 (s, 3 H, CH₃), 1.58-1.65 (m, 1 H, CH), 1.83-1.90 (m, 2 H, 2 × CH), 1.96-1.99 (m, 1 H, CH), 2.08-2.14 (m, 1 H, CH), 2.15-2.20 (m, 1 H, CH), 2.22-2.30 (m, 1 H, CH), 2.37 (d, *J* = 9.5 Hz, 1 H, CH), 2.49 (dd, *J* = 3.0, 8.9 Hz, 1 H, CH), 2.59 (d, *J* = 9.5 Hz, 1 H, CH), 2.63 (dd, *J* = 6.6, 8.8 Hz, 1 H, CH), 3.57 (d, *J* = 13.2 Hz, 1 H, CH_{A}), 3.64 (d, *J* = 13.2 Hz, 1 H, CH_{B}), 4.13 (d, *J* = 10.4 Hz, 1 H, CH_{A'}), 4.26 (d, *J* = 10.4 Hz, 1 H, CH_{B'}), 7.13 (ddd, *J* = 1.3, 7.2, 8.5 Hz, 1 H, Ar-H), 7.20-7.24 (m, 1 H, Ar-H), 7.27-7.32 (m, 2 H, 2 × Ar-H), 7.33-7.37 (m, 2 H, 2 × Ar-H), 7.64 (ddd, *J* = 1.6, 7.2, 8.8 Hz, 1 H, Ar-H), 8.21 (dd, *J* = 1.6, 8.5 Hz, 1 H, Ar-H), 8.57 (dd, *J=* 1.3, 8.6 Hz, 1 H, Ar-H), 9.81 (s, 1 H, NH).

**¹³C NMR** (CDCl₃, 125 MHz): δ = 23.6, 26.8, 27.1, 27.6, 34.4, 34.9, 39.4, 40.5, 46.2, 50.0, 60.0, 64.0, 64.3, 71.7, 120.9, 122.4, 126.0, 126.8, 128.3, 128.6, 135.7, 136.1, 136.1, 139.8, 153.5.

**MS** (ESI) *m*/*z* (%) = 450.4 (100) [M+H]⁺.

**HRMS** (ESI) for C₂₆H₃₂N₃O₄: calculated 450.2387 [M+H]⁺, found 450.2386.

### ((3aS,4R,6R,7aS)-2-((4-(tert-butyl)phenyl)sulfonyl)-5,5-dimethyloctahydro-3aH-4,6-methanoisoindol-3a-yl)methyl (2-aminophenyl)carbamate (Int-8)

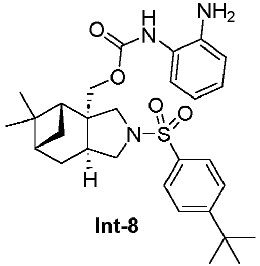

Following general procedure B2: From **Int-7** (100 mg, 0.222 mmol) afforded an orange oil which was used in next step without further purification (50.0 mg, 0.152 mmol, 68 % yield).

Following general procedure C1: From the crude amine obtained from the step described above (50.0 mg, 0.152 mmol) and 4-tert-butylbenzenesulfonyl chloride. Purification by column chromatography (PE/EtOAc = 3:1) afforded a colorless oil **Int-8** (41.0 mg, 78.0 µmol, 51 % yield).

**¹H NMR** (CDCl₃, 500 MHz): δ = 0.99 (s, 3 H, CH₃), 1.21 (s, 3 H, CH₃), 1.30 (s, 9 H, 3 × CH₃), 1.31-1.34 (m, 1 H, CH), 1.56-1.64 (m, 1 H, CH), 1.83-1.97 (m, 2 H, 2 × CH), 2.12-2.35 (m, 3 H, 3 × CH), 2.91-3.30 (m, 4 H, 4 × CH), 3.97-4.10 (m, 2 H, 2 × CH), 6.32 (s, 1 H, NH), 6.78-6.90 (m, 2 H, 2 × Ar-H), 6.99-7.08 (m, 2 H, 2 × Ar-H), 7.50-7.58 (m, 2 H, 2 × Ar-H), 7.70-7.80 (m, 2 H, 2 × Ar-H).

**¹³C NMR** (CDCl₃, 125 MHz): δ = 23.5, 27.1, 27.4, 31.2, 34.1, 34.5, 35.3, 39.1, 40.2, 46.6, 50.8, 56.9, 58.3, 70.5, 126.1, 128.1, 156.8.

**MS** (ESI) *m*/*z* (%) = 526.3 (100) [M+H]⁺.

**HRMS** (ESI) for C₂₉H₄₀N₃O₄S: calculated 526.2734 [M+H]⁺, found 526.2733.

### ((3aS,4R,6R,7aS)-2-((4-(tert-butyl)phenyl)sulfonyl)-5,5-dimethyloctahydro-3aH-4,6-methanoisoindol-3a-yl)methyl p-tolylcarbamate (1) (iDeg-1)

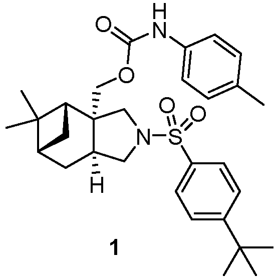

Following general procedure B1: From **Int-3** (110 mg, 0.263 mmol) afforded a colorless solid **Int-4** (81.0 mg, 0.247 mmol, 94 % yield).

Following general procedure C1: From amine **Int-4** (81.0 mg, 0.247 mmol) and 4-*tert-*butylbenzenesulfonyl chloride. Purification by column chromatography (PE/EtOAc 8:1 → 4:1) afforded a colorless foam of compound **1 (iDeg-1)** (115 mg, 0.219 mmol, 89 % yield).

**¹H NMR** (600 MHz, CD₂Cl₂) δ 7.71 (d, *J* = 8.5 Hz, 2H), 7.56 (d, *J* = 8.6 Hz, 2H), 7.19 (d, *J* = 7.6 Hz, 2H), 7.10 (d, *J* = 7.9 Hz, 2H), 6.36 (s, 1H), 4.06 (d, *J* = 10.7 Hz, 1H), 3.92 (d, *J=* 10.6 Hz, 1H), 3.15 (dd, *J* = 9.2, 7.1 Hz, 1H), 3.05 (d, *J* = 9.9 Hz, 1H), 3.02 (dd, *J* = 9.2, 3.1 Hz, 1H), 2.99 (d, *J* = 9.9 Hz, 1H), 2.33 (ddd, *J* = 10.5, 3.3, 2.1 Hz, 1H), 2.30 (s, 3H), 2.25 (tdd, *J* = 7.4, 4.2, 2.2 Hz, 1H), 2.20 (ddt, *J* = 8.4, 6.2, 3.1 Hz, 1H), 1.98 (dd, *J* = 6.5, 5.0 Hz, 1H), 1.87 (tt, *J* = 5.6, 3.0 Hz, 1H), 1.63 - 1.59 (m, 1H), 1.32 (d, *J =* 10.4 Hz, 1H), 1.30 (s, 9H), 1.22 (s, 3H), 1.01 (s, 3H).

**¹³C NMR** (151 MHz, CD₂Cl₂) δ 157.2, 153.7, 135.6, 133.6, 132.4, 129.9, 128.3, 126.4, 119.2, 70.1, 58.5, 57.4, 54.2, 54.0, 53.8, 53.7, 53.5, 50.9, 46.7, 40.6, 39.4, 35.4, 34.7, 34.5, 31.2, 27.4, 27.3, 23.5, 20.8.

**MS** (ESI) *m*/*z* (%) = 525.2 (100) [M+H]⁺.

**HRMS** (ESI) for C₃₀H₄₁N₂O₄S: calculated 525.2782 [M+H]⁺, found 525.2785.

[α]²⁰_{D} = -16.0° (CHCl₃, c = 0.5).

### ((3aS,4R,6R,7aS)-2-((4-(tert-butyl)phenyl)sulfonyl)-5,5-dimethyloctahydro-3aH-4,6-methanoisoindol-3a-yl)methyl (3-aminophenyl)carbamate (15)

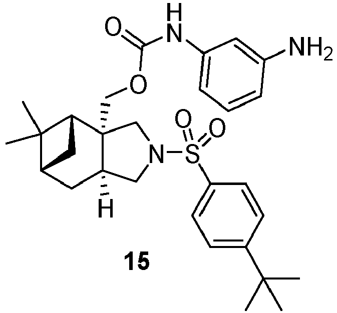

Following general procedure B2: From **Int-5** (85.0 mg, 0.139 mmol) afforded an orange oil, which was used in next step without further purification (58.0 mg, 0.176 mmol, 93 %yield).

Following general procedure C1: From the crude amine obtained from the step described above (58.0 mg, 0.176 mmol) and 4-tert-butylbenzenesulfonyl chloride. Purification by column chromatography (PE/EtOAc 3:1) afforded a colorless oil **15** (43.0 mg, 81.8 µmol, 46 %yield).

**¹H NMR** (CDCl₃, 500 MHz): δ = 1.00 (s, 3 H, CH₃), 1.21 (s, 3 H, CH₃), 1.31 (s, 9 H, 3 × CH₃), 1.31-1.35 (m, 1 H, CH), 1.57-1.65 (m, 1 H, CH), 1.85-1.89 (m, 1 H, CH), 1.93-1.97 (m, 1 H, CH), 2.15-2.22 (m, 1 H, CH), 2.22-2.33 (m, 2 H, 2 × CH), 3.01-3.11 (m, 3 H, 3 × CH), 3.16-3.23 (m, 1 H, CH), 3.95 (d, *J* = 10.7 Hz, 1 H, CH_{A}), 4.07 (d, *J* = 10.5 Hz, 1 H, CH_{B}), 6.42-6.51 (m, 2 H, 2 × Ar-H), 6.58-6.66 (m, 1 H, Ar-H), 6.94 (s, 1 H, NH), 7.06 (t, *J* = 8.0 Hz, 1 H, Ar-H), 7.51-7.56 (m, 2 H, 2 × Ar-H), 7.72-7.76 (m, 2 H, 2 × Ar-H).

**¹³C NMR** (CDCl₃, 125 MHz): δ = 23.5, 27.1, 27.4, 31.2, 34.2, 35.3, 39.1, 40.2, 46.4, 50.6, 57.0, 58.2, 69.9, 111.1, 126.1, 127.1, 128.1, 130.0, 132.2, 138.8, 156.9.

**MS** (ESI) *m*/*z* (%) = 526.2 (100) [M+H]⁺.

**HRMS** (ESI) for C₂₉H₄₀N₃O₄S: calculated 526.2734 [M+H]⁺, found 526.2732.

### ((3aS,4R,6R,7aS)-2-((4-(tert-butyl)phenyl)sulfonyl)-5,5-dimethyloctahydro-3aH-4,6-methanoisoindol-3a-yl)methyl (4-methoxyphenyl)carbamate (20)

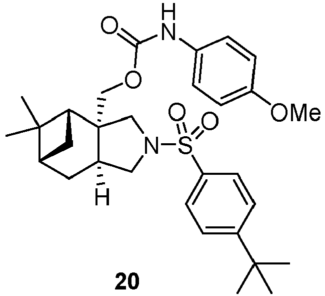

Following general procedure B1: From **Int-6** (100 mg, 0.230 mmol) afforded a colorless solid -which was used in next step without further purification (79.0 mg, 0.229 mmol, quant.).

Following general procedure C1: From the crude amine obtained from the step described above (79.0 mg, 0.229 mmol) and 4-tert-butylbenzenesulfonyl chloride. Purification by column chromatography (PE/EtOAc 4:1) afforded a colorless solid **20** (90.0 mg, 0.166 mmol, 72 % yield).

**¹H NMR** (CDCl₃, 600 MHz): δ = 1.00 (s, 3 H, CH₃), 1.22 (s, 3 H, CH₃), 1.30 (s, 9 H, 3 × CH₃), 1.37 (d, *J* = 10.4 Hz, 1 H, CH), 1.60-1.67 (m, 2 H, 2 × CH), 1.86-1.90 (m, 1 H, CH), 1.92-1.97 (m, 1 H, CH), 2.16-2.34 (m, 3 H, 3 × CH), 2.97-3.21 (m, 4 H, 4 × CH), 3.79 (s, 3 H, OMe), 3.95-4.06 (m, 2 H, 2 × CH), 6.23 (s, 1 H, NH), 6.82-6.87 (m, 2 H, 2 × Ar-H), 7.17-7.25 (s_{br}, 2 H, 2 × Ar-H), 7.51-7.56 (m, 2 H, 2 × Ar-H), 7.74 (d, *J* = 8.1 Hz, 2 H, 2 × Ar-H).

**¹³C NMR** (CDCl₃, 150 MHz): δ = 23.5, 27.2, 27.4, 29.8, 31.2, 34.4, 34.5, 35.3, 39.2, 40.2, 46.6, 50.7, 55.7, 57.1, 58.2, 70.1, 114.4, 121.0, 126.0, 128.1, 130.7, 132.3, 153.8, 156.3, 156.8.

**MS** (ESI) *m*/*z* (%) = 541.1 (100) [M+H]⁺.

**HRMS** (ESI) for C₃₀H₄₁N₂O₅S: calculated 541.2731 [M+H]⁺, found 541.2736.

### ((3aS,4R,6R,7aS)-2-(isopropylsulfonyl)-5,5-dimethyloctahydro-3aH-4,6-methanoisoindol-3a-yl) methyl p-tolylcarbamate (30)

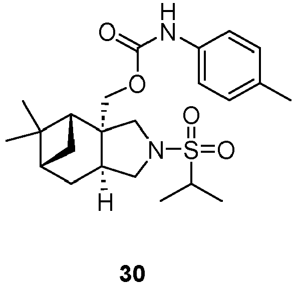

Following general procedure C1: From amine **Int-4** (50 mg, 0.15 mmol) and propane-2-sulfonyl chloride. Purification by column chromatography (PE/EtOAc 4:1) afforded a colorless solid **30** (39.70 mg, 0.090 mmol, 60 % yield).

**¹H NMR** (CDCl₃, 400 MHz): δ = 7.31 - 7.21 (m, 2H), 7.10 (d, *J* = 8.5 Hz, 2H), 6.80 (s, 1H), 4.22 (d, *J* = 10.7 Hz, 1H), 4.12 (dd, *J* = 14.1, 8.9 Hz, 1H), 3.68 (s, 1H), 3.52 (d, *J* = 10.3 Hz, 1H), 3.27 - 3.18 (m, 1H), 3.14 (dd, *J* = 9.9, 3.4 Hz, 1H), 2.39 - 2.24 (m, 5H), 2.00 - 1.96 (m, 1H), 1.93 (dd, *J* = 8.5, 4.9 Hz, 1H), 1.60 (dd, *J* = 9.2, 2.8 Hz, 1H), 1.37 (dt, *J* = 6.8, 3.4 Hz, 6H), 1.25 (s, 3H), 1.06 (s, 3H).

**¹³C NMR** (CDCl₃, 100 MHz): δ = 153.8, 135.3, 129.7, 119.0, 70.2, 58.9, 57.2, 53.7, 51.1, 46.6, 40.4, 39.2, 34.9, 34.2, 27.5, 27.4, 23.7, 20.9, 16.9, 16.8.

**HRMS** (ESI) for C₂₃H₃₅O₄N₂S: calculated 435.231 [M+H]⁺, found 435.233.

[α]²⁰_{D} = -105.1° (CHCl₃, c = 1.0)

### ((3aS,4R,6R,7aS)-2-(cyclohexylsulfonyl)-5,5-dimethyloctahydro-3aH-4,6-methanoisoindol-3a-yl)methyl p-tolylcarbamate (31)

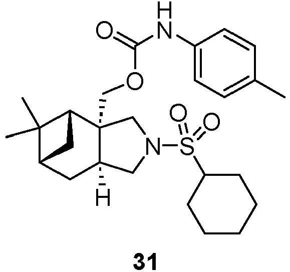

Following general procedure C1: From amine **Int-4** (50 mg, 0.150 mmol) and cyclohexylsulfonyl chloride. Purification by column chromatography (PE/EtOAc 4:1) afforded a colorless foam **31** (65 mg, 0.140 mmol, 90 %yield).

**¹H NMR** (CDCl₃, 400 MHz): δ = 7.26 - 7.13 (m, 2H), 7.04 (d, *J* = 8.5 Hz, 2H), 6.69 (s, 1H), 4.16 (d, *J* = 10.7 Hz, 1H), 4.04 (d, *J* = 10.6 Hz, 1H), 3.61 (s, 1H), 3.45 (d, *J* = 10.3 Hz, 1H), 3.14 (d, *J* = 10.4 Hz, 1H), 3.07 (dd, *J* = 9.7, 3.1 Hz, 1H), 2.87 (tt, *J* = 12.0, 3.2 Hz, 1H), 2.32 - 2.16 (m, 6H), 2.08 (s, 2H), 1.92 (t, *J* = 5.6 Hz, 1H), 1.84 (dd, *J* = 18.7, 8.1 Hz, 3H), 1.63 (d, *J* = 11.6 Hz, 1H), 1.57 - 1.40 (m, 4H), 1.31 (d, *J* = 10.4 Hz, 1H), 1.25 - 1.07 (m, 7H), 0.99 (s, 3H).

**¹³C NMR** (CDCl₃, 100 MHz): δ = 153.8, 135.3, 129.7, 119.0, 70.3, 61.7, 58.8, 57.2, 51.1, 46.6, 40.4, 39.2, 34.9, 34.1, 27.5, 27.4, 26.7, 26.6, 25.4, 25.4, 23.7, 20.9. **HRMS** (ESI) for C₂₆H₃₉N₂O₄S: calculated 475.262 [M+H]⁺, found 475.265

[α]²⁰_{D} = -97.2° (CHCl₃, c = 1.0)

### ((3aS,4R,6R,7aS)-5,5-dimethyl-2-((4-(trifluoromethyl)phenyl)sulfonyl)octahydro-3aH-4,6-methanoisoindol-3a-yl)methyl p-tolylcarbamate (32)

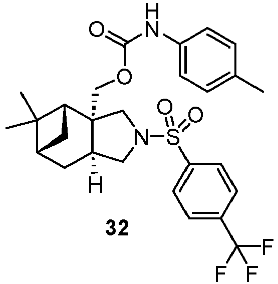

Following general procedure C1: From amine **Int-4** (50 mg, 0.15 mmol) and 4-(trifluoromethyl)phenyl)sulfonyl chloride, Purification by column chromatography (PE/EtOAc 4:1) afforded a colorless solid **32** (73.52 mg, 0.137 mmol, 90 % yield).

**¹H NMR** (CDCl₃, 400 MHz): δ = 7.87 (d, *J* = 8.2 Hz, 2H), 7.73 (d, *J* = 8.3 Hz, 2H), 7.09 (s, 2H), 7.03 (d, *J* = 8.3 Hz, 2H), 6.23 (s, 1H), 3.99 (dt, *J* = 29.2, 9.0 Hz, 2H), 3.14 - 2.90 (m, 4H), 2.31 - 2.21 (m, 5H), 2.19 - 2.09 (m, 1H), 1.90 (t, *J* = 5.7 Hz, 1H), 1.83 (dd, *J* = 8.5, 5.3 Hz, 1H), 1.60 - 1.50 (m, 1H), 1.24 (d, *J* = 10.5 Hz, 1H), 1.16 (s, 3H), 0.95 (s, 3H).

**¹³C NMR** (CDCl₃, 100 MHz): δ = 139.2, 135.0, 134.9, 134.5, 133.5, 129.7, 128.6, 126.3, 126.3, 124.7, 69.9, 58.3, 57.0, 50.9, 46.6, 40.2, 39.2, 34.6, 34.4, 27.4, 27.2, 23.5, 20.9.

**HRMS** (ESI) for C₂₇H₃₂O₄N₂F₃S: calculated 537.203 [M+H]⁺, found 537.205.

[α]²⁰_{D} = -155.2° (CHCl₃, c = 1.0)

### ((3aS,4R,6R,7aS)-2-((4-methoxyphenyl)sulfonyl)-5,5-dimethyloctahydro-3aH-4,6-methanoisoindol-3a-yl)methyl p-tolylcarbamate (33)

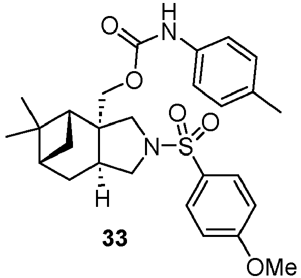

Following general procedure C1: From **Int-4** (130 mg, 0.396 mmol) and 4-methoxybenzenesulfonyl chloride. Purification by column chromatography (PE/EtOAc 4:1) afforded a colorless solid **33** (153 mg, 0.307 mmol, 78 %yield).

**¹H NMR** (CDCl₃, 400 MHz): δ = 1.00 (s, 3 H, CH₃), 1.23 (s, 3 H, CH₃), 1.43 (d, *J* = 10.3 Hz, 1 H, CH), 1.63-1.68 (m, 1 H, CH), 1.87-1.94 (m, 2 H, 2 × CH), 2.19-2.34 (m, 3 H, 3 × CH), 2.31 (s, 3 H, CH₃), 2.93 (d, *J* = 9.8 Hz, 1 H, CH), 3.00-3.06 (m, 1 H, CH), 3.07-3.14 (m, 2 H, 2 × CH), 3.75 (s, 3 H, OMe), 3.92-4.03 (m, 2 H, 2 × CH), 6.06 (s_{br}, 1 H, NH), 6.96-7.02 (m, 2 H, 2 × Ar-H), 7.08-7.13 (m, 2 H, 2 × Ar-H), 7.15-7.21 (m, 2 H, 2 × Ar-H), 7.73-7.79 (m, 2 H, 2 × Ar-H).

**¹³C NMR** (CDCl₃, 100 MHz): δ = 20.9, 23.6, 27.1, 27.4, 27.4, 34.7, 34.8, 39.1, 40.2, 46.9, 50.7, 55.6, 57.0, 58.5, 70.2, 114.1, 118.8, 126.4, 129.7, 130.6, 133.3, 135.0, 163.1.

**MS** (ESI) *m*/*z* (%) = 499.1 (100) [M+H]⁺.

**HRMS** (ESI) for C₂₇H₃₅N₂O₅S: calculated 499.2261 [M+H]⁺, found 499.2264.

### ((3aS,4R,6R,7aS)-5,5-dimethyl-2-((4-(trifluoromethoxy)phenyl)sulfonyl) octahydro-3aH-4,6-methanoisoindol-3a-yl)methyl p-tolylcarbamate (34)

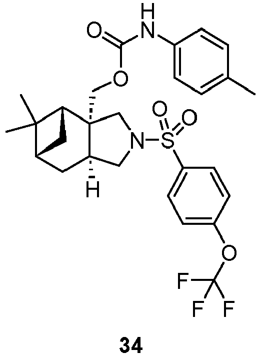

Following general procedure C1: From amine **Int-4** (50 mg, 0.15 mmol) and 4-(trifluoromethoxy)phenylsulfonyl chloride. Purification by column chromatography (PE/EtOAc 4:1) afforded a colorless solid **34** (77.00 mg, 0.140 mmol, 91 %yield).

**¹H NMR** (CDCl₃, 400 MHz): δ = 7.78 (d, *J* = 8.6 Hz, 2H), 7.26 (d, *J* = 8.0 Hz, 2H), 7.10 (s, 2H), 7.03 (d, *J* = 8.5 Hz, 2H), 6.27 (s, 1H), 4.01 (d, *J* = 10.8 Hz, 1H), 3.91 (d, *J* = 10.7 Hz, 1H), 3.02 (dd, *J* = 13.8, 9.6 Hz, 3H), 2.93 (d, *J* = 9.7 Hz, 1H), 2.32 - 2.20 (m, 5H), 2.17 - 2.09 (m, 1H), 1.88 (t, *J* = 5.7 Hz, 1H), 1.84 - 1.76 (m, 1H), 1.62 - 1.45 (m, 1H), 1.26 (d, *J* = 10.5 Hz, 1H), 1.15 (s, 3H), 0.94 (s, 3H).

**¹³C NMR** (CDCl₃, 100 MHz): δ = 153.5, 152.5, 152.4, 135.0, 133.7, 133.4, 130.3, 129.7, 121.6, 120.8, 119.1, 118.9, 69.9, 58.3, 57.0, 50.9, 46.7, 40.2, 39.2, 34.6, 34.4, 27.4, 27.2, 23.5, 20.9.

**HRMS** (ESI) for C₂₇H₃₂O₅N₂F₃S: calculated 553.198 [M+H]⁺, found 553.200.

[α]²⁰_{D} = -139.1° (CHCl₃, c = 1.0)

### ((3aS,4R,6R,7aS)-5,5-dimethyl-2-((4-(3-(trifluoromethyl)-3H-diazirin-3-yl)phenyl)sulfonyl)octahydro-3aH-4,6-methanoisoindol-3a-yl)methyl p-tolylcarbamate (35)

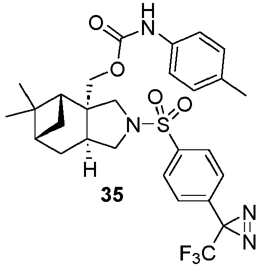

Following general procedure C1: From amine **Int-4** (35.0 mg, 0.108 mmol) and 4-[3-(trifluoromethyl)-3*H*-diazirin-3-yl]benzene-1-sulfonyl chloride. Purification by column chromatography (PE/EtOAc 8:1) afforded a colorless solid **35** (38.0 mg, 65.9 µmol, 62 % yield).

**¹H NMR** (CDCl₃, 400 MHz): δ = 1.01 (s, 3 H, CH₃), 1.23 (s, 3 H, CH₃), 1.31 (d, *J* = 10.5 Hz, 1 H, CH), 1.58-1.65 (m, 1 H, CH), 1.87-1.92 (m, 1 H, CH), 1.94-2.00 (m, 1 H, CH), 2.18-2.34 (m, 3 H, 3 × CH), 2.32 (s, 3 H, CH₃), 3.00 (d, *J* = 9.8 Hz, 1 H, CH), 3.04-3.17 (m, 3 H, 3 × CH), 3.97 (d, *J* = 10.8 Hz, 1 H, CH_{A}), 4.08 (d, *J* = 10.8 Hz, 1 H, CH_{B}), 6.30 (s_{br}, 1 H, NH), 7.09-7.15 (m, 2 H, 2 × Ar-H), 7.15-7.24 (m, 2 H, 2 × Ar-H), 7.32 (d, *J* = 8.2 Hz, 2 H, 2 × Ar-H), 7.84 (d, *J* = 8.3 Hz, 2 H, 2 × Ar-H).

**¹³C NMR** (CDCl₃, 100 MHz): δ = 20.9, 23.5, 27.2, 27.4, 34.3, 34.6, 39.2, 40.1, 46.5, 50.8, 57.0, 58.2, 69.8, 127.0, 127.0, 128.5, 129.8, 134.1, 135.0, 136.9.

**MS** (ESI) *m*/*z* (%) = 577.0 (100) [M+H]⁺.

**HRMS** (ESI) for C₂₈H₃₂F₃N₄O₄S: calculated 577.2091 [M+H]⁺, found 577.2096.

### ((3aS,4R,6R,7aS)-2-((2-bromophenyl)sulfonyl)-5,5-dimethyloctahydro-3aH-4,6-methanoisoindol-3a-yl)methyl p-tolylcarbamate (36)

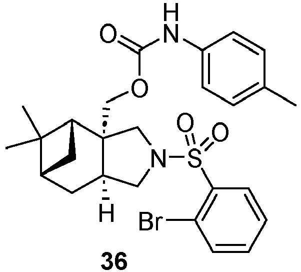

Following general procedure C1: From amine **Int-4** (50 mg, 0.15 mmol) and 2-bromophenyl sulfonyl chloride. Purification by column chromatography (PE/EtOAc 4:1) afforded a colorless solid (70.85 mg, 0.130 mmol, 85 %yield).

**¹H NMR** (CDCl₃, 400 MHz): δ = 8.03 (dd, *J* = 7.9, 1.7 Hz, 1H), 7.65 (dd, *J* = 7.8, 1.3 Hz, 1H), 7.36 (ddd, *J* = 7.9, 7.5, 1.3 Hz, 1H), 7.27 (td, *J* = 7.7, 1.8 Hz, 1H), 7.16 (d, *J* = 7.5 Hz, 2H), 7.03 (d, *J* = 8.2 Hz, 2H), 6.55 (s, 1H), 4.03 (dd, *J* = 34.5, 10.7 Hz, 2H),

3.53 (t, *J* = 9.0 Hz, 1H), 3.30 (d, *J* = 10.2 Hz, 1H), 3.17 - 3.08 (m, 2H), 2.34 - 2.09 (m, 6H), 1.88 - 1.80 (m, 2H), 1.61 - 1.54 (m, 1H), 1.28 (d, *J* = 10.5 Hz, 1H), 1.16 (s, *J* = 6.5 Hz, 3H), 0.96 (s, 3H).

**¹³C-NMR** (CDCl₃, 100 MHz): δ = 153.6, 137.6, 135.9, 135.3, 133.9, 132.4, 129.7, 127.7, 120.9, 119.0, 70.3, 58.5, 56.8, 51.2, 46.7, 40.4, 39.1, 34.9, 34.1, 27.5, 27.4, 23.7, 20.9.

**HRMS** (ESI) for C₂₆H₃₂O₄N₂BrS: calculated 547.126 [M+H]⁺, found 547.129.

### Synthesis of compounds 2-14, 21-28

### Synthesis of intermediate Int-10

### ((3aS,4R,6R,7aS)-2-((4-(tert-butyl)phenyl)sulfonyl)-5,5-dimethyloctahydro-3aH-4,6-methanoisoindol-3a-yl)methanol (Int-10)

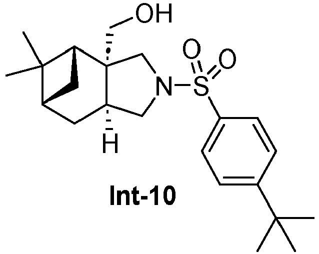

Following general procedure B3: To a degassed solution of **Int-2** (389 mg, 1.36 mmol) in MeOH (4 mL) was added Pd/C (10 wt%, 40 mg), and the mixture was stirred under a H₂ atmosphere for 16 h. The reaction mixture was filtered over Celite, washed with MeOH and the solvent was evaporated under reduced pressure to give a colorless solid **Int-9** which was used without further purification (242 mg, 1.24 mmol, 91 %yield).

Following general procedure C2: To a solution of **Int-9** (242 mg, 1.24 mmol) in THF (10 mL) at 0 °C was added NEt₃ (198 µL, 1.49 mmol, 1.2 equiv.) and 4-*tert-*butylbenzenesulfonyl chloride (288 mg, 1.24 mmol, 1.0 equiv.) and it was stirred at 0 °C for 2 h. The solvent was removed under reduced pressure. Column chromatography (PE/EtOAc 4:1 → 2:1) afforded a colorless solid **Int-10** (415 mg, 1.06 mmol, 86 %yield).

**¹H NMR** (500 MHz, CDCl₃) δ 7.72 (d, *J* = 8.6 Hz, 2H), 7.53 (d, *J* = 8.6 Hz, 2H), 3.51 (d, *J* = 10.5 Hz, 1H), 3.42 (d, *J* = 10.6 Hz, 1H), 3.19 (dd, *J* = 9.1, 7.3 Hz, 1H), 3.06 (d, *J* = 9.7 Hz, 1H), 3.01 - 2.95 (m, 2H), 2.31 - 2.23 (m, 1H), 2.21 - 2.14 (m, 2H), 1.96 (dd, *J* = 6.5, 5.0 Hz, 1H), 1.86 (dq, *J* = 5.4, 2.8 Hz, 1H), 1.61 (ddd, *J* = 13.4, 4.4, 2.8 Hz, 1H), 1.35 (s, 9H), 1.31 (d, *J* = 10.4 Hz, 1H), 1.21 (s, 3H), 0.95 (s, 3H).

**¹³C NMR** (126 MHz, CDCl₃) δ 156.6, 132.0, 128.1, 126.0, 68.2, 58.5, 56.9, 52.3, 45.8, 40.4, 39.2, 35.3, 34.2, 34.1, 31.2, 27.5, 27.2, 23.7.

**MS** (ESI) *m*/*z* (%) = 392.2 (100) [M+H]⁺.

**HRMS** (ESI) for C₂₂H₃₄NO₃S: calculated 392.2254 [M+H]⁺, found 392.2254.

### [α]²⁰_{D} = -25.3° (CHCl₃, c = 1.0 )

### ((3aS,4R,6R,7aS)-2-((4-(tert-butyl)phenyl)sulfonyl)-5,5-dimethyloctahydro-3aH-4,6-methanoisoindol-3a-yl)methyl (4-ethylphenyl)carbamate (2)

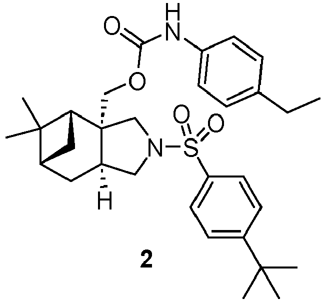

Following general procedure A2: From **Int-10** (20.0 mg, 51.1 µmol) and 4-ethylphenyl isocyanate. Purification by column chromatography (PE/EtOAc 6:1) afforded a colorless solid **2** (21.0 mg, 40.0 µmol, 78 %yield).

**¹H NMR** (CDCl₃, 500 MHz): δ = 1.01 (s, 3 H, CH₃), 1.20-1.23 (m, 6 H, 2 × CH₃), 1.30 (s, 9 H, 3 × CH₃), 1.38 (d, *J* = 10.4 Hz, 1 H, CH), 1.63-1.66 (m, 1 H, CH), 1.86-1.92 (m, 1 H, CH), 1.94-1.98 (m, 1 H, CH), 2.17-2.35 (m, 3 H, 3 × CH), 2.60 (q, *J* = 7.6 Hz, 2 H, CH₂), 3.01 (d, *J* = 9.8 Hz, 1 H, CH), 3.05-3.17 (s, 3 H, 3 × CH), 3.97 (d, *J* = 10.7 Hz, 1 H, CH_{A}), 4.05 (d, *J =* 10.6 Hz, 1 H, CH_{B}), 6.32 (s, 1 H, NH), 7.11-7.14 (m, 2 H, 2 × Ar-H), 7.18-7.27 (m, 2 H, 2 × Ar-H), 7.51-7.56 (m, 2 H, 2 × Ar-H), 7.71-7.77 (m, 2 H, 2 × Ar-H).

**¹³C NMR** (CDCl₃, 125 MHz): δ = 15.9, 23.5, 24.0, 27.1, 27.4, 28.4, 31.2, 34.3, 34.5, 35.3, 39.2, 40.2, 46.5, 50.6, 57.1, 58.2, 70.0, 115.4, 119.1, 126.0, 128.1, 128.5, 128.7, 132.2, 156.8.

**MS** (ESI) *m*/*z* (%) = 539.1 (100) [M+H]⁺.

**HRMS** (ESI) for C₃₁H₄₃N₂O₄S: calculated 539.2938 [M+H]⁺, found 539.2941.

### ((3aS,4R,6R,7aS)-2-((4-(tert-butyl)phenyl)sulfonyl)-5,5-dimethyloctahydro-3aH-4,6-methanoisoindol-3a-yl)methyl (4-isopropylphenyl)carbamate (3)

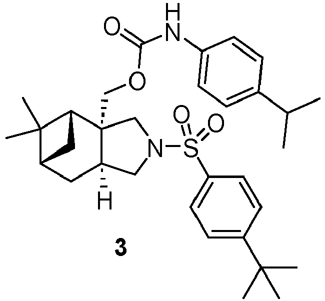

Following general procedure A2: From **Int-10** (20.0 mg, 51.1 µmol) and 4-isopropylphenyl isocyanate. Purification by column chromatography (PE/EtOAc 6:1) afforded a colorless solid **3** (22.0 mg, 40.0 µmol, 78 %yield).

**¹H NMR** (CDCl₃, 500 MHz): δ = 1.01 (s, 3 H, CH₃), 1.22 (s, 6 H, 2 × CH₃), 1.23 (m, 3 H, CH₃), 1.29 (s, 9 H, 3 × CH₃), 1.38 (d, *J* = 10.5 Hz, 1 H, CH),1.63-1.66 (m, 1 H, CH), 1.86-1.91 (m, 1 H, CH), 1.94-1.98 (m, 1 H, CH), 2.17-2.35 (m, 3 H, 3 × CH), 2.87 (p, *J* = 6.9 Hz, 1 H, CH), 3.00 (d, *J* = 9.8 Hz, 1 H, CH), 3.05-3.17 (s, 3 H, 3 × CH), 3.97 (d, *J* = 10.7 Hz, 1 H, CH_{A}), 4.06 (d, *J* = 10.7 Hz, 1 H, CH_{B}), 6.31 (s, 1 H, NH), 7.13-7.18 (m, 2 H, 2 × Ar-H), 7.19-7.25 (m, 2 H, 2 × Ar-H), 7.51-7.56 (m, 2 H, 2 × Ar-H), 7.71-7.77 (m, 2 H, 2 × Ar-H).

**¹³C NMR** (CDCl₃, 125 MHz): δ = 23.5, 24.2, 27.1, 27.4, 31.2, 33.6, 34.4, 34.5, 35.3, 39.2, 40.2, 46.5, 50.7, 57.1, 58.2, 70.0, 115.3, 119.1, 126.0, 127.1, 128.1, 132.2, 135.3, 153.5, 156.8.

**MS** (ESI) *m*/*z* (%) = 553.0 (100) [M+H]⁺.

**HRMS** (ESI) for C₃₂H₄₅N₂O₄S: calculated 553.3095 [M+H]⁺, found 553.3100.

### ((3aS,4R,6R,7aS)-2-((4-(tert-butyl)phenyl)sulfonyl)-5,5-dimethyloctahydro-3aH-4,6-methanoisoindol-3a-yl)methyl (4-(tert-butyl)phenyl)carbamate (4)

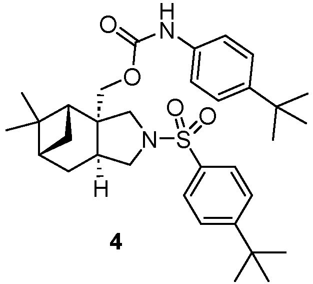

Following general procedure A2: From **Int-10** (20.0 mg, 51.1 µmol) and 4-(*tert*-butyl) phenyl isocyanate. Purification by column chromatography (PE/EtOAc 6:1) afforded an orange oil **4** (26.0 mg, 45.9 µmol, 90 % yield).

**¹H NMR** (CDCl₃, 400 MHz): δ = 1.01 (s, 3 H, CH₃), 1.22 (m, 3 H, CH₃), 1.29 (s, 9 H, 3 × CH₃), 1.30 (s, 9 H, 3 × CH₃), 1.38 (d, *J* = 10.4 Hz, 1 H, CH), 1.60-1.67 (m, 1 H, CH), 1.85-1.92 (m, 1 H, CH), 1.93-1.98 (m, 1 H, CH), 2.16-2.36 (m, 3 H, 3 × CH), 3.01 (d, *J* = 9.8 Hz, 1 H, CH), 3.01-3.19 (s, 3 H, 3 × CH), 3.97 (d, *J* = 10.7 Hz, 1 H, CH_{A}), 4.07 (d, *J* = 10.8 Hz, 1 H, CH_{B}), 6.33 (s, 1 H, NH), 7.18-7.26 (m, 2 H, 2 × Ar-H), 7.29-7.34 (m, 2 H, 2 × Ar-H), 7.50-7.57 (m, 2 H, 2 × Ar-H), 7.71-7.77 (m, 2 H, 2 × Ar-H).

**¹³C NMR** (CDCl₃, 100 MHz): δ = 23.5, 27.1, 27.4, 31.2, 31.5, 34.4, 34.4, 34.5, 35.3, 39.2, 40.2, 46.5, 50.7, 57.1, 58.2, 70.0, 118.8, 126.0, 128.1, 132.3, 135.0, 153.6, 156.8.

**MS** (ESI) *m*/*z* (%) = 567.1 (100) [M+H]⁺.

**HRMS** (ESI) for C₃₃H₄₇N₂O₄S: calculated 567.3251 [M+H]⁺, found 567.3257.

### ((3aS,4R,6R,7aS)-2-((4-(tert-butyl)phenyl)sulfonyl)-5,5-dimethyloctahydro-3aH-4,6-methanoisoindol-3a-yl)methyl [1,1'-biphenyl]-4-ylcarbamate (5):

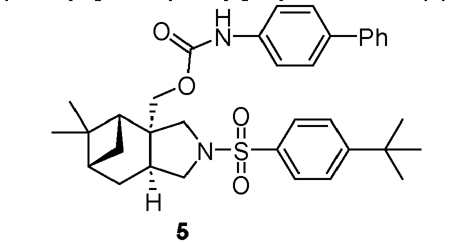

Following general procedure A2: From **Int-10** (39.2 mg, 0.1 mmol) and 4-diphenyl isocyanate (23.4 mg, 1.2 equiv.). Purification by column chromatography (PE/EtOAc 6:1) afforded an orange oil **4** (26.0 mg, 45.9 µmol, 90 % yield).

**¹H NMR** (500 MHz, CDCl₃) δ 7.75 (d, *J* = 8.5 Hz, 2H), 7.59 - 7.56 (m, 2H), 7.56 - 7.52 (m, 4H), 7.46 - 7.42 (m, 2H), 7.42 - 7.38 (m, 2H), 7.37 - 7.31 (m, 1H), 6.47 (s, 1H), 4.10 (d, *J* = 10.5 Hz, 1H), 4.01 (d, *J* = 10.7 Hz, 1H), 3.23 - 3.17 (m, 1H), 3.11 (d, *J* = 9.8 Hz, 1H), 3.09 - 3.03 (m, 2H), 2.35 - 2.26 (m, 2H), 2.25 - 2.17 (m, 1H), 1.98 (dd, *J* = 6.5, 5.0 Hz, 1H), 1.90 (dd, *J* = 5.5, 3.1 Hz, 1H), 1.67 - 1.62 (m, 1H), 1.37 (d, *J* = 10.6 Hz, 1H), 1.31 (s, 9H), 1.24 (s, 3H), 1.02 (s, 3H).

**¹³C NMR** (126 MHz, CDCl₃) δ 156.9, 153.4, 143.3, 140.6, 137.0, 132.3, 128.9, 128.1, 127.9, 127.2, 126.9, 126.1, 119.2, 70.2, 58.2, 57.1, 50.7, 46.5, 40.2, 39.2, 35.3, 34.5, 34.3, 31.2, 27.4, 27.1, 23.5.

**HRMS** (ESI) for C₃₅H₄₂N₂O₄SNa: calculated 609.2763 [M+Na]⁺, found 609.2754.

[α]²⁰_{D} = -6.2° (CHCl₃, *c* = 0.5).

### ((3aS,4R,6R,7aS)-2-((4-(tert-butyl)phenyl)sulfonyl)-5,5-dimethyloctahydro-3aH-4,6-methanoisoindol-3a-yl)methyl phenylcarbamate (6)

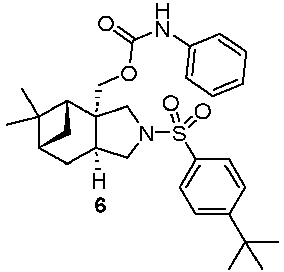

Following general procedure A2: From **Int-10** (20.0 mg, 51.1 µmol) and phenyl isocyanate. Purification by column chromatography (PE/EtOAc 4:1) afforded a colorless solid **6** (23.0 mg, 45.0 µmol, 88 %yield).

**¹H-NMR** (CDCl₃, 500 MHz): δ = 1.01 (s, 3 H, CH₃), 1.22 (s, 3 H, CH₃), 1.30 (s, 9 H, 3 × CH₃), 1.37 (d, *J* = 10.5 Hz, 1 H, CH), 1.60-1.66 (m, 1 H, CH), 1.86-1.91 (m, 1 H, CH), 1.97 (t, *J* = 5.7 Hz, 1 H, CH), 2.16-2.38 (m, 3 H, 3 × CH), 3.02 (d, *J* = 9.8 Hz, 1

H, CH), 3.07 (dd, *J* = 9.2, 2.8 Hz, 1 H, CH), 3.11 (d, *J* = 9.8 Hz, 1 H, CH), 3.16 (dd, *J* = 9.2, 6.7 Hz, 1 H, CH), 3.98 (d, *J* = 10.6 Hz, 1 H, CH_{A}), 4.08 (d, *J* = 10.7 Hz, 1 H, CH_{B}), 6.42 (s, 1 H, NH), 7.02-7.10 (m, 1 H, Ar-H), 7.26-7.38 (m, 4 H, 4 × Ar-H), 7.51-7.59 (m, 2 H, 2 × Ar-H), 7.69-7.79 (m, 2 H, 2 × Ar-H).

**¹³C-NMR** (CDCl₃, 125 MHz): δ = 23.5, 27.1, 27.4, 31.2, 34.3, 34.5, 35.3, 39.2, 40.2, 46.4, 50.6, 57.1, 58.2, 70.1, 118.8, 126.0, 128.1, 129.2, 129.4, 132.2, 137.6, 153.4, 156.8.

**MS** (ESI) *m*/*z* (%) = 511.2 (100) [M+H]⁺.

**HRMS** (ESI) for C₂₉H₃₉N₂O₄S: calculated 511.2625 [M+H]⁺, found 511.2628.

### ((3aS,4R,6R,7aS)-2-((4-(tert-butyl)phenyl)sulfonyl)-5,5-dimethyloctahydro-3aH-4,6-methanoisoindol-3a-yl)methyl (2,4-dimethylphenyl)carbamate (7):

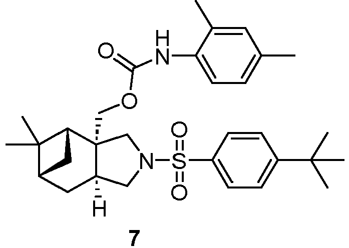

Following general procedure A2: From **Int-10** (39.2 mg, 0.1 mmol) and 1-isocyanato-2,4-dimethylbenzene (17.7 mg, 1.2 equiv.). Purification by column chromatography (PE/EtOAc 4:1) afforded a colorless solid **7** (27 mg, 50.0 µmol, 50 %yield).

**¹H NMR** (500 MHz, , CDCl₃) δ 7.74 (d, *J* = 8.1 Hz, 2H), 7.53 (d, *J* = 8.5 Hz, 2H), 7.47 (s, 1H), 6.98 (d, *J* = 7.3 Hz, 2H), 6.03 (s, 1H), 4.08 (d, *J* = 10.6 Hz, 1H), 4.04 - 3.91 (m, 1H), 3.22 - 2.89 (m, 4H), 2.31 (dd, *J* = 7.7, 5.3 Hz, 1H), 2.29 (s, 3H), 2.25 (s, 1H), 2.23 - 2.19 (m, 1H), 2.18 (s, 3H), 1.95 (t, *J* = 5.7 Hz, 1H), 1.88 (dt, *J* = 5.4, 2.7 Hz, 1H), 1.66 - 1.59 (m, 2H), 1.36 (d, *J* = 10.5 Hz, 1H), 1.29 (s, 9H), 1.22 (s, 3H), 1.01 (s, 3H).

**¹³C NMR** (126 MHz, CDCl₃) δ 156.6, 153.9, 137.6, 132.7, 132.1, 131.1, 127.9, 127.3, 125.9, 122.0 118.7, 69.9, 58., 57.0, 50.5, 46.3, 40.0, 39.0, 35.1, 34.3, 34.1, 31.0, 27.2, 26.9, 23.3, 20.8, 17.7.

**HRMS** (ESI) for C₃₁H₄₃N₂O₄S: calculated 539.2938 [M+H]⁺, found 539.2942.

[α]²⁰_{D} = -14.7° (CHCl₃, *c* = 0.5).

### ((3aS,4R,6R,7aS)-2-((4-(tert-butyl)phenyl)sulfonyl)-5,5-dimethyloctahydro-3aH-4,6-methanoisoindol-3a-yl)methyl (4-fluorophenyl)carbamate (8)

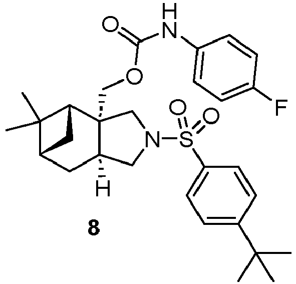

Following general procedure A2: From **Int-10** (20.0 mg, 51.1 µmol) and 4-fluorophenyl isocyanate. Purification by column chromatography (PE/EtOAc 4:1) afforded a colorless solid **8** (22.0 mg, 41.6 µmol, 81 %yield).

**¹H NMR** (CDCl₃, 500 MHz): δ = 1.00 (s, 3 H, CH₃), 1.22 (s, 3 H, CH₃), 1.30 (s, 9 H, 3 × CH₃), 1.33 (d, *J* = 10.6 Hz, 1 H, CH), 1.59-1.67 (m, 1 H, CH), 1.86-1.90 (m, 1 H, CH), 1.95 (t, *J* = 5.7 Hz, 1 H, CH), 2.16-2.22 (m, 1 H, CH), 2.23-2.32 (m, 2 H, 2 × CH), 3.00-3.11 (m, 3 H, 3 × CH), 3.20 (t, *J* = 8.1 Hz, 1 H, CH), 3.98 (d, *J* = 10.6 Hz, 1 H, CH_{A}), 4.08 (d, *J* = 10.7 Hz, 1 H, CH_{B}), 6.46 (s, 1 H, NH), 6.94-7.02 (m, 2 H, 2 × Ar-H), 7.29 (s_{br}, 2 H, 2 × Ar-H), 7.48-7.58 (m, 2 H, 2 × Ar-H), 7.71-7.77 (m, 2 H, 2 × Ar-H).

**¹³C NMR** (CDCl₃, 125 MHz): δ = 23.5, 27.2, 27.4, 31.2, 34.2, 34.5, 35.3, 39.1, 40.1, 46.4, 50.6, 57.0, 58.2, 70.2, 115.8, 115.9, 120.6, 126.0, 128.1, 132.3, 133.7, 153.6, 156.9.

**MS** (ESI) *m*/*z* (%) = 529.1 (100) [M+H]⁺.

**HRMS** (ESI) for C₂₉H₃₈FN₂O₄S: calculated 529.2531 [M+H]⁺, found 529.2535.

### ((3aS,4R,6R,7aS)-2-((4-(tert-butyl)phenyl)sulfonyl)-5,5-dimethyloctahydro-3aH-4,6-methanoisoindol-3a-yl)methyl (4-chlorophenyl)carbamate (9)

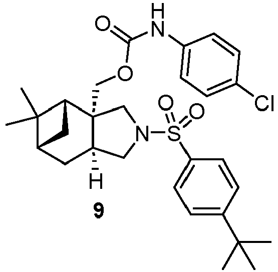

Following general procedure A2: From **Int-10** (20.0 mg, 51.1 µmol) and 4-chlorophenyl isocyanate. Purification by column chromatography (PE/EtOAc 4:1) afforded a colorless solid **9** (26.0 mg, 47.7 µmol, 93 %yield).

**¹H NMR** (CDCl₃, 500 MHz): δ = 1.00 (s, 3 H, CH₃), 1.22 (s, 3 H, CH₃), 1.31 (s, 9 H, 3 × CH₃), 1.31-1.34 (m, 1 H, CH), 1.60-1.64 (m, 1 H, CH), 1.86-1.90 (m, 1 H, CH), 1.95 (t, *J* = 5.8 Hz, 1 H, CH), 2.16-2.34 (m, 3 H, 3 × CH), 3.01-3.10 (m, 3 H, 3 × CH), 3.20 (dd, *J* = 9.2, 6.7 Hz, 1 H, CH), 3.98 (d, *J* = 10.7 Hz, 1 H, CH_{A}), 4.10 (d, *J* =

10.9 Hz, 1 H, CH_{B}), 6.55 (s, 1 H, NH), 7.23-7.32 (m, 4 H, 4 × Ar-H), 7.51-7.61 (m, 2 H, 2 × Ar-H), 7.71-7.78 (m, 2 H, 2 × Ar-H).

**¹³C NMR** (CDCl₃, 125 MHz): δ = 23.5, 27.1, 27.4, 31.2, 34.1, 34.5, 35.3, 39.1, 40.1, 46.3, 50.6, 57.0, 58.2, 70.2, 120.0, 126.1, 128.1, 129.2, 129.2, 132.3, 136.4, 153.3, 156.9.

**MS** (ESI) *m*/*z* (%) = 545.1 (100) [M+H]⁺.

**HRMS** (ESI) for C₂₉H₃₈ClN₂O₄S: calculated 545.2235 [M+H]⁺, found 545.2243.

### ((3aS,4R,6R,7aS)-2-((4-(tert-butyl)phenyl)sulfonyl)-5,5-dimethyloctahydro-3aH-4,6-methanoisoindol-3a-yl)methyl (4-bromophenyl)carbamate (10)

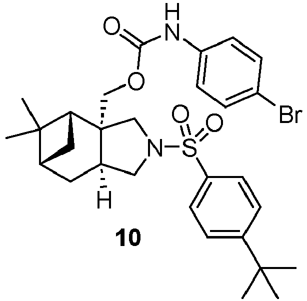

Following general procedure A2: From **Int-10** (20.0 mg, 51.1 µmol) and 4-bromophenyl isocyanate. Purification by column chromatography (PE/EtOAc 4:1) afforded a colorless solid **10** (22.0 mg, 37.7 µmol, 73 % yield).

**¹H NMR** (CDCl₃, 700 MHz): δ = 1.01 (s, 3 H, CH₃), 1.22 (s, 3 H, CH₃), 1.31 (s, 9 H, 3 × CH₃), 1.32-1.36 (m, 1 H, CH), 1.59-1.64 (m, 1 H, CH), 1.86-1.90 (m, 1 H, CH), 1.95 (t, *J* = 5.8 Hz, 1 H, CH), 2.16-2.22 (m, 1 H, CH), 2.24-2.32 (m, 2 H, 2 × CH), 3.02 (dd, *J* = 9.4, 3.2 Hz, 1 H, CH), 3.07 (s, 2 H, 2 × CH), 3.23 (t, *J* = 8.2 Hz, 1 H, CH), 3.98 (d, *J* = 10.6 Hz, 1 H, CH_{A}), 4.10 (d, *J* = 10.6 Hz, 1 H, CH_{B}), 6.57 (s, 1 H, NH), 7.20-7.28 (m, 2 H, 2 × Ar-H), 7.40 (d, *J* = 8.5 Hz, 2 H, 2 × Ar-H), 7.53 (d, *J* = 8.3 Hz, 2 H, 2 × Ar-H), 7.74 (d, *J* = 8.1 Hz, 2 H, 2 × Ar-H).

**¹³C NMR** (CDCl₃, 175 MHz): δ = 23.5, 27.1, 27.4, 31.2, 34.1, 34.5, 35.2, 39.1, 40.2, 46.4, 50.6, 57.0, 58.2, 70.3, 116.3, 120.4, 126.1, 128.1, 132.1, 132.4, 136.9, 153.3, 156.9.

**MS** (ESI) *m*/*z* (%) = 589.0 (100) [M+H]⁺.

**HRMS** (ESI) for C₂₉H₃₈BrN₂O₄S: calculated 589.1730 [M+H]⁺, found 589.1740.

### ((3aS,4R,6R,7aS)-2-((4-(tert-butyl)phenyl)sulfonyl)-5,5-dimethyloctahydro-3aH-4,6-methanoisoindol-3a-yl)methyl (4-iodophenyl)carbamate (11) (iDeg-2)

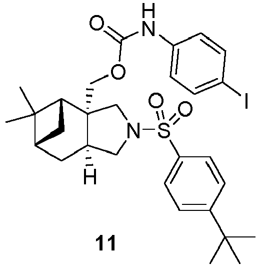

Following general procedure A2: From **Int-10** (20.0 mg, 51.1 µmol) and 4-iodophenyl isocyanate (15 mg, 1.2 equiv.). Purification by column chromatography (PE/EtOAc 4:1) afforded a colorless solid (28.0 mg, 44.0 µmol, 86 % yield).

**1H NMR** (500 MHz, CD₂Cl₂) δ 7.71 (d, J = 8.5 Hz, 2H), 7.61 (d, J = 8.8 Hz, 2H), 7.56 (d, J = 8.6 Hz, 2H), 7.15 (d, J = 8.3 Hz, 2H), 6.54 (s, 1H), 4.11 (d, J = 10.7 Hz, 1H), 3.93 (d, J = 10.7 Hz, 1H), 3.20 (dd, J = 9.3, 7.1 Hz, 1H), 3.04 - 2.96 (m, 3H), 2.37 - 2.28 (m, 1H), 2.28 - 2.17 (m, 2H), 1.98 (dd, J = 6.5, 4.9 Hz, 1H), 1.87 (s, 1H), 1.63 - 1.58 (m, 1H), 1.31 (s, 9H), 1.28 (s, 1H), 1.22 (s, 3H), 1.01 (s, 3H).

**¹³C NMR** (126 MHz, CD₂Cl₂) δ 157.2, 153.4, 138.3, 138.2, 132.5, 128.3, 126.4, 120.9, 86.5, 70.4, 58.5, 57.3, 50.8, 46.6, 40.6, 39.4, 35.4, 34.7, 34.3, 31.2, 27.4, 27.2, 23.5. **MS** (ESI) *m*/*z* (%) = 637.0 (100) [M+H]⁺.

**HRMS** (ESI) for C₂₉H₃₈IN₂O₄S: calculated 637.1592 [M+H]⁺, found 637.1601.

[α]²⁰_{D} = -10.7° (CHCl₃, *c* = 1.0).

### ((3aS,4R,6R,7aS)-2-((4-(tert-butyl)phenyl)sulfonyl)-5,5-dimethyloctahydro-3aH-4,6-methanoisoindol-3a-yl)methyl (2-iodophenyl)carbamate (12)

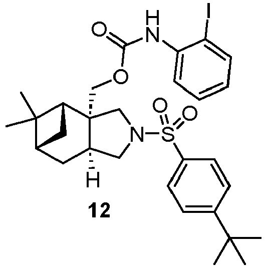

Following general procedure A2: From **Int-10** (20.0 mg, 51.1 µmol) and 2-iodophenyl isocyanate. Purification by column chromatography (PE/EtOAc 6:1) afforded a colorless solid **12** (23.0 mg, 36.1 µmol, 71 %yield).

**¹H NMR** (CDCl₃, 500 MHz): δ = 1.01 (s, 3 H, CH₃), 1.23 (m, 3 H, CH₃), 1.26 (s, 9 H, 3 × CH₃), 1.41 (d, *J* = 10.5 Hz, 1 H, CH), 1.63-1.70 (m, 1 H, CH), 1.86-1.92 (m, 1 H, CH), 1.95-2.00 (m, 1 H, CH), 2.19-2.37 (m, 3 H, 3 × CH), 2.97 (d, *J* = 9.8 Hz,1 H, CH), 3.09-3.20 (s, 3 H, 3 × CH), 3.97 (d, *J* = 10.7 Hz, 1 H, CH_{A}), 4.09 (d, *J* = 10.7 Hz, 1 H, CH_{B}), 6.66 (s, 1 H, NH), 6.82 (td, *J* = 7.9, 1.5 Hz, 1 H, Ar-H), 7.30-7.34 (m, 1 H, Ar-H), 7.51-7.56 (m, 2 H, 2 × Ar-H), 7.72-7.78 (m, 3 H, 3 × Ar-H), 7.85-7.93 (m, 1 H, Ar-H).

**¹³C NMR** (CDCl₃, 125 MHz): δ = 23.5, 27.1, 27.4, 31.1, 34.3, 34.5, 35.3, 39.2, 40.1, 46.4, 50.6, 57.0, 58.2, 70.3, 126.1, 128.1, 129.5, 129.6, 132.0, 138.1, 139.0, 153.3, 156.7.

**MS** (ESI) *m*/*z* (%) = 637.1 (100) [M+H]⁺.

**HRMS** (ESI) for C₂₉H₃₈IN₂O₄S: calculated 637.1592 [M+H]⁺, found 637.1600.

### ((3aS,4R,6R,7aS)-2-((4-(tert-butyl)phenyl)sulfonyl)-5,5-dimethyloctahydro-3aH-4,6-methanoisoindol-3a-yl)methyl (2-bromophenyl)carbamate (13)

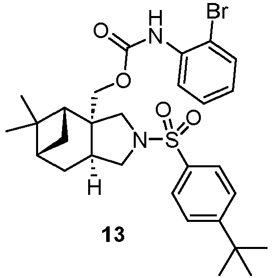

Following general procedure A2: From **Int-10**(20.0 mg, 51.1 µmol) and 2-bromophenyl isocyanate. Purification by column chromatography (PE/EtOAc 6:1) afforded a colorless oil **13** (18.0 mg, 30.5 µmol, 60 %yield).

**¹H NMR** (CDCl₃, 500 MHz): δ = 1.01 (s, 3 H, CH₃), 1.23 (m, 3 H, CH₃), 1.27 (s, 9 H, 3 × CH₃), 1.40 (d, *J* = 10.4 Hz, 1 H, CH), 1.63-1.70 (m, 1 H, CH), 1.86-1.92 (m, 1 H, CH), 1.95-2.01 (m, 1 H, CH), 2.18-2.38 (m, 3 H, 3 × CH), 2.97 (d, *J* = 9.8 Hz,1 H, CH), 3.07-3.20 (s, 3 H, 3 × CH), 3.97 (d, *J* = 10.7 Hz, 1 H, CH_{A}), 4.11 (d, *J* = 10.6 Hz, 1 H, CH_{B}), 6.85 (s, 1 H, NH), 6.92-6.97 (m, 1 H, Ar-H), 7.27-7.32 (m, 1 H, Ar-H), 7.49-7.56 (m, 3 H, 3 × Ar-H), 7.71-7.76 (m, 2 H, 2 × Ar-H), 7.96-8.06 (m, 1 H, Ar-H). **¹³C NMR** (CDCl₃, 125 MHz): δ = 23.5, 27.1, 27.4, 31.1, 34.3, 35.2, 39.2, 40.1, 46.4, 50.5, 57.0, 58.2, 70.3, 124.8, 126.1, 128.1, 128.6, 132.0, 132.5, 135.6, 153.1, 156.8. **MS** (ESI) *m*/*z* (%) = 589.0 (100) [M+H]⁺.

**HRMS** (ESI) for C₂₉H₃₈BrN₂O₄S: calculated 591.1710 [M+H]⁺, found 591.1716.

### ((3aS,4R,6R,7aS)-2-((4-(tert-butyl)phenyl)sulfonyl)-5,5-dimethyloctahydro-3aH-4,6-methanoisoindol-3a-yl)methyl (3-bromophenyl)carbamate (14)

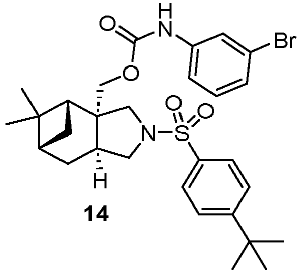

Following general procedure A2: From **Int-10** (20.0 mg, 51.1 µmol) and 3-bromophenyl isocyanate. Purification by column chromatography (PE/EtOAc 6:1) afforded a colorless **oil 14** (25.0 mg, 42.4 µmol, 83 %yield).

**¹H NMR** (CDCl₃, 700 MHz): δ = 1.01 (s, 3 H, CH₃), 1.23 (m, 3 H, CH₃), 1.31 (s, 9 H, 3 × CH₃), 1.34 (d, *J* = 10.5 Hz, 1 H, CH), 1.60-1.65 (m, 1 H, CH), 1.87-1.91 (m, 1 H, CH), 1.93-1.97 (m, 1 H, CH), 2.18-2.22 (m, 1 H, CH), 2.24-2.33 (m, 2 H, 2 × CH), 3.02-3.07 (s, 2 H, 2 × CH), 3.09 (d, *J* = 9.9 Hz, 1 H, CH), 3.21 (dd, *J* = 9.3, 7.0 Hz, 1 H, CH), 4.00 (d, *J* = 10.6 Hz, 1 H, CH_{A}), 4.09 (d, *J* = 10.7 Hz, 1 H, CH_{B}), 6.55 (s, 1 H, NH), 7.15 (t, *J* = 8.0 Hz, 1 H, Ar-H), 7.18-7.20 (m, 1 H, Ar-H), 7.22-7.26 (m, 1 H, Ar-H), 7.52-7.56 (m, 2 H, 2 × Ar-H), 7.59 (s_{br}, 1 H, Ar-H), 7.72-7.76 (m, 2 H, 2 × Ar-H).

**¹³C NMR** (CDCl₃, 175 MHz): δ = 23.5, 27.1, 27.4, 31.2, 34.2, 34.5, 35.4, 39.2, 40.2, 46.4, 50.7, 57.0, 58.2, 70.3, 122.0, 126.1, 126.7, 128.1, 130.5, 132.4, 139.1, 153.1, 156.9.

**MS** (ESI) *m*/*z* (%) = 589.0 (100) [M+H]⁺.

**HRMS** (ESI) for C₂₉H₃₈BrN₂O₄S: calculated 589.1730 [M+H]⁺, found 589.1738.

### Synthesis of compounds 23

### ((3aS,4R,6R,7aS)-2-((4-(tert-butyl)phenyl)sulfonyl)-5,5-dimethyloctahydro-3aH-4,6-methanoisoindol-3a-yl)methyl (4-cyanophenyl)carbamate (23)

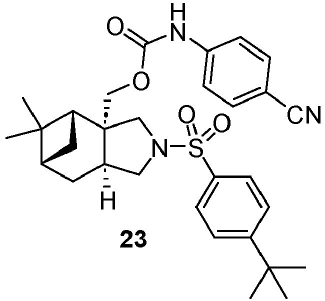

Following general procedure A2: From **Int-10** (20.0 mg, 51.1 µmol) and 4-cyanophenyl isocyanate. Purification by column chromatography (PE/EtOAc 4:1) afforded a colorless solid **23** (26.0 mg, 48.5 µmol, 95 %yield).

**¹H NMR** (CDCl₃, 400 MHz): δ = 1.01 (s, 3 H, CH₃), 1.22 (s, 3 H, CH₃), 1.31 (s, 9 H, 3 × CH₃), 1.32-1.36 (m, 1 H, CH), 1.56-1.64 (m, 1 H, CH), 1.85-1.92 (m, 1 H, CH), 1.97 (t, *J* =5.7 Hz, 1 H, CH), 2.14-2.22 (m, 1 H, CH), 2.23-2.35 (m, 2 H, 2 × CH), 2.97 (dd, *J* = 9.5, 3.2 Hz, 1 H, CH), 3.04 (d, *J* = 9.9 Hz, 1 H, CH), 3.16 (d, *J* = 9.9 Hz, 1 H , CH), 3.31-3.37 (m, 1 H, CH), 4.01 (d, *J* = 10.7 Hz, 1 H, CH_{A}), 4.17 (d, *J* = 10.7 Hz, 1 H, CH_{B}), 7.10 (s, 1 H, NH), 7.48-7.56 (m, 4 H, 4 × Ar-H), 7.56-7.61 (m, 2 H, 2 × Ar-H), 7.71-7.77 (m, 2 H, 2 × Ar-H).

**¹³C NMR** (CDCl₃, 100 MHz): δ = 23.5, 27.2, 27.4, 31.2, 33.8, 34.4, 35.3, 39.1, 40.2, 46.2, 50.6, 57.0, 58.2, 70.6, 106.5, 118.5, 119.0, 126.1, 128.0, 132.5, 133.5, 142.2, 152.9, 157.0.

**MS** (ESI) *m*/*z* (%) = 536.1 (100) [M+H]⁺.

**HRMS** (ESI) for C₃₀H₃₈N₃O₄S: calculated 536.2578 [M+H]⁺, found 536.2578.

### Synthesis of compounds 19, 37 - 53, 55 - 61, 64 - 76

### Synthesis of intermediates Int-11 to Int-38

### ((3aS,4R,6R,7aS)-5,5-dimethyl-2-(phenylsulfonyl)octahydro-3aH-4,6-methanoisoindol-3a-yl)methanol (Int-11)

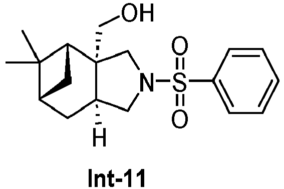

Following general procedure C2: From **Int-9** (39.6 mg, 0.2 mmol) and benzenesulfonyl chloride (1.0 equiv.). Purification by column chromatography (PE/EtOAc 4:1) afforded a colorless solid **Int-11** (90 %yield).

**¹H NMR** (500 MHz, CDCl₃) δ 7.84 -7.79 (m, 2H), 7.64 - 7.57 (m, 1H), 7.58 - 7.51 (m, 2H), 3.49 (d, J = 10.5 Hz, 1H), 3.41 (d, J = 10.5 Hz, 1H), 3.19 (dd, J = 9.1, 7.3 Hz, 1H), 3.06 (d, J = 9.7 Hz, 1H), 3.02 - 2.95 (m, 2H), 2.31 - 2.23 (m, 1H), 2.21 - 2.14 (m, 2H), 1.98 - 1.92 (m, 1H), 1.86 (dt, J = 5.3, 2.7 Hz, 1H), 1.60 (ddd, J = 13.4, 4.4, 2.8 Hz, 1H), 1.29 (d, J = 10.5 Hz, 1H), 1.21 (s, 3H), 0.94 (s, 3H).

**¹³C NMR** (126 MHz, CDCl₃) δ 135.0, 132.9, 129.0, 128.2, 68.1, 58.6, 56.9, 52.3, 45.9, 40.4, 39.2, 34.3, 34.1, 27.5, 27.2, 23.7.

**HRMS** (ESI) for C₁₈H₂₅NO₃SNa: calculated 358.1453 [M+Na]⁺, found 358.1445.

[α]²⁰_{D} = -18.7° (CHCl₃, c = 0.5).

### ((3aS,4R,6R,7aS)-5,5-dimethyl-2-(naphthalen-2-ylsulfonyl)octahydro-3aH-4,6-methanoisoindol-3a-yl)methanol (Int-12)

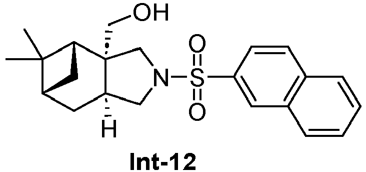

Following general procedure C2: From **Int-9** (329.3 mg, 1.69 mmol) and naphthalene-2-sulfonyl chloride (1.0 equiv.). Purification by column chromatography (PE/EtOAc 4:1) afforded a colorless solid **Int-12** (65 % yield).

**¹H NMR** (700 MHz, CDCl₃) δ 8.38 (s, 1H), 8.01 - 7.96 (m, 2H), 7.93 (d, *J* = 8.1 Hz, 1H), 7.81 (dd, *J* = 8.5, 1.8 Hz, 1H), 7.67 - 7.64 (m, 1H), 7.64 - 7.60 (m, 1H), 3.47 (d, *J* = 10.4 Hz, 1H), 3.39 (d, *J* = 10.4 Hz, 1H), 3.26 (dd, *J* = 9.2, 7.3 Hz, 1H), 3.12 (d, *J* = 9.7 Hz, 1H), 3.06 (dd, *J* = 9.4, 3.2 Hz, 2H), 2.30 - 2.24 (m, 1H), 2.20 - 2.14 (m, 2H), 1.95 (dd, *J* = 6.4, 5.0 Hz, 1H), 1.85 (tt, *J* = 5.7, 3.0 Hz, 1H), 1.61 (ddd, *J* = 13.5, 4.6, 2.9 Hz, 1H), 1.31 (d, *J* = 10.4 Hz, 1H), 1.21 (s, 3H), 0.93 (s, 3H).

**¹³C NMR** (176 MHz, CDCl₃) δ 135.1, 132.4, 132.3, 129.5, 129.5, 129.2, 128.9, 128.1, 127.6, 123.6, 68.1, 58.6, 57.0, 52.4, 45.9, 40.4, 39.2, 34.3, 34.2, 27.5, 27.3, 23.7.

**HRMS** (ESI) for C₂₂H₂₇NO₃SNa: calculated 408.1609 [M+Na]⁺, found 409.1591.

[α]²⁰_{D} = -13.3° (CHCl₃, *c* = 0.5).

### ((3aS,4R,6R,7aS)-5,5-dimethyl-2-(o-tolylsulfonyl)octahydro-3aH-4,6-methanoisoindol-3a-yl)methanol (Int-13):

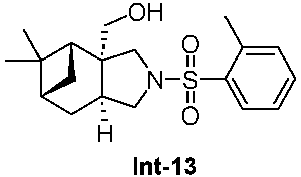

Following general procedure C2: From **Int-9** (39.6 mg, 0.2 mmol) and 2-methylbenzenesulfonyl chloride (1.0 equiv.). Purification by column chromatography (PE/EtOAc 4:1) afforded a colorless solid **Int-13** (52.4mg, 0.15 mmol, 75 %yield).

**¹H NMR** (700 MHz, CDCl₃) δ 7.91 (dd, J = 8.2, 1.4 Hz, 1H), 7.45 (td, J = 7.5, 1.4 Hz, 1H), 7.33 - 7.29 (m, 2H), 3.54 (d, J = 10.6 Hz, 1H), 3.48 (d, J = 10.5 Hz, 1H), 3.41 (dd, J = 9.4, 7.3 Hz, 1H), 3.24 (d, J = 9.8 Hz, 1H), 3.03 (dd, J = 9.3, 3.6 Hz, 1H), 2.96 (d, J = 9.8 Hz, 1H), 2.67 (s, 3H), 2.43 (d, J = 3.1 Hz, 1H), 2.26 (dt, J = 12.9, 2.6 Hz, 1H), 2.23 - 2.20 (m, 1H), 2.17 (ddd, J = 10.5, 5.2, 3.2 Hz, 1H), 1.95 (dd, J = 6.5, 5.0 Hz, 1H), 1.88 - 1.84 (m, 1H), 1.58 - 1.55 (m, 1H), 1.27 (d, J = 10.4 Hz, 1H), 1.21 (s, 3H), 0.96 (s, 3H).

**¹³C NMR** (176 MHz, CDCl₃) δ 138.4, 135.4, 133.0, 132.8, 130.4, 126.2, 68.1, 58.0, 56.2, 52.5, 45.9, 40.4, 39.2, 34.3, 34.2, 27.5, 27.3, 23.7, 21.1.

**HRMS** (ESI) for C₁₉H₂₇NO₃SNa: calculated 372.1609 [M+Na]⁺, found 372.1591.

[α]²⁰_{D} = -2.7° (CHCl₃, c = 1.0).

### ((3aS,4R,6R)-5,5-dimethyl-2-(m-tolylsulfonyl)octahydro-3aH-4,6-methanoisoindol-3a-yl)methanol (Int-14):

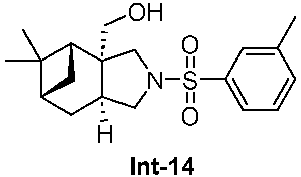

Following general procedure C2: From **Int-9** (39.6 mg, 0.2 mmol) and 3-methylbenzenesulfonyl chloride (1.0 equiv.). Purification by column chromatography (PE/EtOAc 4:1) afforded a colorless solid **Int-14** (78 % yield).

**¹H NMR** (500 MHz, CDCl₃) δ 7.61 (s, 1H), 7.59 (d, J = 7.0 Hz, 1H), 7.44 - 7.38 (m, 2H), 3.49 (d, J = 10.6 Hz, 1H), 3.40 (d, J = 10.6 Hz, 1H), 3.19 (dd, J = 9.1, 7.2 Hz, 1H), 3.05 (d, J = 9.7 Hz, 1H), 2.97 (d, J = 2.4 Hz, 1H), 2.96 (dd, J = 5.4, 3.7 Hz, 1H), 2.43 (s, 3H), 2.30 - 2.23 (m, 1H), 2.20 - 2.13 (m, 2H), 1.95 (dd, J = 6.5, 4.9 Hz, 1H), 1.85 (dq, J = 5.7, 2.9 Hz, 1H), 1.59 (ddd, J = 13.4, 4.5, 2.9 Hz, 1H), 1.29 (d, J = 10.4 Hz, 1H), 1.21 (s, 3H), 0.94 (s, 3H).

**¹³C NMR** (126 MHz, CDCl₃) δ 139.2, 134.8, 133.8, 128.9, 128.5, 125.3, 68.0, 58.5, 56.9, 52.3, 45.9, 40.4, 39.2, 34.2, 34.1, 27.5, 27.2, 23.7, 21.6.

**HRMS** (ESI) for C₁₉H₂₇NO₃SNa: calculated 372.1609 [M+Na]⁺, found 372.1598

[α]²⁰_{D} = -45.3° (CHCl₃, c = 1.0).

### ((3aS,4R,6R,7aS)-5,5-dimethyl-2-tosyloctahydro-3aH-4,6-methanoisoindol-3a-yl) methanol (Int-15)

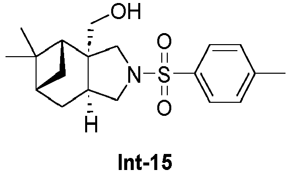

Following general procedure C2: From **Int-9** (39.6 mg, 0.2 mmol) and 4-methylbenzenesulfonyl chloride (1.0 equiv.). Purification by column chromatography (PE/EtOAc 4:1) afforded a colorless solid **Int-15** (80 % yield).

**¹H NMR** (700 MHz, CDCl₃) δ 7.68 (d, J = 8.3 Hz, 2H), 7.32 (d, J = 8.1 Hz, 2H), 3.49 (d, J = 10.5 Hz, 1H), 3.40 (d, J = 10.5 Hz, 1H), 3.17 (dd, J = 9.1, 7.3 Hz, 1H), 3.04 (d, J = 9.7 Hz, 1H), 2.97 - 2.92 (m, 2H), 2.43 (s, 3H), 2.28 - 2.22 (m, 1H), 2.19 - 2.12 (m, 2H), 1.95 (dd, J = 6.4, 5.0 Hz, 1H), 1.84 (tt, J = 5.6, 3.0 Hz, 1H), 1.59 (ddd, J = 13.5, 4.5, 2.8 Hz, 1H), 1.29 (d, J = 10.4 Hz, 1H), 1.21 (s, 3H), 0.94 (s, 3H).

**¹³C NMR** (176 MHz, CDCl₃) δ 143.7, 131.9, 129.7, 128.3, 68.1, 58.5, 56.9, 52.3, 45.9, 40.4, 39.2, 34.2, 34.1, 27.5, 27.2, 23.7, 21.7.

**HRMS** (ESI) for C₁₉H₂₇NO₃SNa: calculated 372.1609 [M+Na]⁺, found 372.1603.

[α]²⁰D = -15.7° (CHCl₃, *c* = 1.0).

### ((3aS,4R,6R,7aS)-2-((4-isopropylphenyl)sulfonyl)-5,5-dimethyloctahydro-3aH-4,6-methanoisoindol-3a-yl)methanol (Int-17)

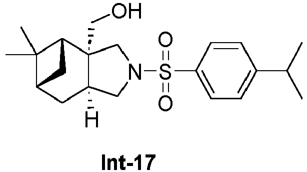

Following general procedure C2: From **Int-9** (39.6 mg, 0.2 mmol) and 4-isopropylbenzenesulfonyl chloride (44.8 ul, 1.0 equiv.). Purification by column chromatography (PE/EtOAc 10:1 to 4:1) afforded a colorless solid **Int-17** (66 mg, 88 % yield).

**1H NMR** (500 MHz, CDCl₃) δ 7.72 (d, J = 8.4 Hz, 2H), 7.37 (d, J = 8.3 Hz, 2H), 3.50 (d, J = 10.5 Hz, 1H), 3.41 (d, J = 10.6 Hz, 1H), 3.20 (dd, J = 9.1, 7.3 Hz, 1H), 3.06 (d, J = 9.7 Hz, 1H), 3.01 - 2.96 (m, 2H), 2.95 (dd, J = 6.1, 3.1 Hz, 1H), 2.30 - 2.23 (m, 1H), 2.20 - 2.12 (m, 2H), 1.96 (dd, J = 6.5, 5.0 Hz, 1H), 1.85 (tt, J = 5.7, 2.9 Hz, 1H), 1.60 (ddd, J = 13.4, 4.4, 2.8 Hz, 1H), 1.29 (d, J = 9.1 Hz, 1H), 1.27 (d, J = 6.9 Hz, 6H), 1.21 (s, 3H), 0.94 (s, 3H).

**13C NMR** (126 MHz, CDCl₃) δ 154.3, 132.3, 128.4, 127.1, 68.1 58.5, 56.9, 52.3, 45.8, 40.4, 39.2, 34.3, 34.2, 34.0, 27.5, 27.2, 23.8, 23.8, 23.7.

**HRMS** (ESI) for C₂₁H₃₁NO₃SNa: calculated 400.1922 [M+Na]⁺, found 400.1917 [α]²⁰_{D} = -64.3° (CHCl₃, *c* = 1.0).

### ((3aS,4R,6R,7aS)-2-([1,1'-biphenyl]-4-ylsulfonyl)-5,5-dimethyloctahydro-3aH-4,6-methanoisoindol-3a-yl)methanol (Int-18)

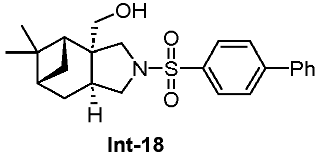

Following general procedure C2: From **Int-9** (39.6 mg, 0.2 mmol) and biphenyl sulfonyl chloride (63.2 mg, 0.2 mmol, 1.0 equiv.). Purification by column chromatography (PE/EtOAc 10 :1 to 4:1) afforded a colorless solid **Int-18** (65.8 mg, 0.16 mmol, 80 % yield).

**¹H NMR** (500 MHz, CDCl₃) δ 7.87 (dd, J = 8.5, 2.0 Hz, 2H), 7.75 (dd, J = 8.5, 2.1 Hz, 2H), 7.66 - 7.60 (m, 2H), 7.52 - 7.45 (m, 2H), 7.43 (dd, J = 7.6, 1.7 Hz, 1H), 3.54 - 3.48 (m, 1H), 3.43 (dd, J = 10.5, 4.1 Hz, 1H), 3.24 (dd, J = 9.1, 7.2 Hz, 1H), 3.11 (d, J = 9.7 Hz, 1H), 3.06 - 2.99 (m, 2H), 2.34 - 2.24 (m, 1H), 2.23 - 2.15 (m, 2H), 1.97 (dd, J = 6.4, 5.0 Hz, 1H), 1.87 (dq, J = 5.7, 2.9 Hz, 1H), 1.63 (ddd, J = 13.4, 4.5, 2.9 Hz, 2H), 1.34 (d, J = 10.4 Hz, 1H), 1.22 (s, 3H), 0.95 (s, 3H).

**¹³C NMR** (126 MHz, CDCl₃) δ 145.7, 139.4, 133.7, 133.6, 129.2, 128.8, 128.6 127.6, 127.5, 68.1, 58.6, 57.0, 52.3, 45.9, 40.4, 39.2, 34.3, 34.2, 27.5, 27.3, 23.7.

**HRMS** (ESI) for C₂₄H₂₉NO₃SNa: calculated 434.1766 [M+Na]⁺, found 434.1757

[α]²⁰_{D} = -17.3° (CHCl₃, *c* = 1.0).

### ((3aS,4R,6R,7aS)-2-((4-cyclohexylphenyl)sulfonyl)-5,5-dimethyloctahydro-3aH-4,6-methanoisoindol-3a-yl)methanol (Int-19)

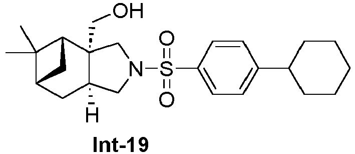

Following general procedure C2: From **Int-9** (29.3 mg, 0.15 mmol) and 4-cyclohexylbenzenesulfonyl chloride (42.7 mg,1.0 equiv.). Purification by column chromatography (PE/EtOAc 10:1 to 4:1) afforded a colorless solid **Int-19** (44.9 mg, 0.11 mmol, 72 %yield).

**¹H NMR** (700 MHz, CDCl₃) δ 7.71 (d, J = 8.3 Hz, 2H), 7.36 (d, J = 8.3 Hz, 2H), 3.51 (d, J = 10.5 Hz, 1H), 3.42 (d, J = 10.5 Hz, 1H), 3.20 (dd, J = 9.1, 7.3 Hz, 1H), 3.06 (d, J = 9.7 Hz, 1H), 3.00 - 2.96 (m, 2H), 2.96 (d, J = 3.7 Hz, 1H), 2.58 (ddd, J = 11.5, 8.7, 3.3 Hz, 1H), 2.30 - 2.23 (m, 1H), 2.20 - 2.13 (m, 2H), 1.95 (dd, J = 6.4, 5.0 Hz, 1H), 1.92 - 1.83 (m, 5H), 1.77 (d, J = 13.3 Hz, 1H), 1.63 - 1.58 (m, 1H), 1.46 - 1.37 (m, 4H), 1.30 (d, J = 10.4 Hz, 1H), 1.27 (dd, J = 12.7, 3.6 Hz, 2H), 1.22 (s, 3H), 0.95 (s, 3H).

**¹³C NMR** (176 MHz, CDCl₃) δ 153.5, 132.3, 128.4, 127.5, 68.2, 58.5, 56.9, 52.3, 45.9, 44.7, 40.4, 39.2, 34.3, 34.2, 34.1, 27.5, 27.2, 26.8, 26.1, 23.8.
**HRMS** (ESI) for C₂₄H₃₅NO₃SNa: calculated 440.2235 [M+Na]⁺, found 440.2217 [α]²⁰_{D} = -10.3° (CHCl₃, *c* = 0.2)

### ((3aS,4R,6R,7aS)-5,5-dimethyl-2-((5,5,8,8-tetramethyl-5,6,7,8-tetrahydronaphthalen-2-yl)sulfonyl)octahydro-3aH-4,6-methanoisoindol-3a-yl)methanol (Int-20)

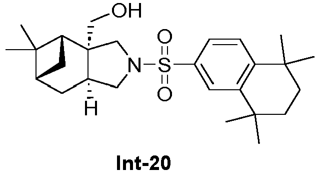

Following general procedure C2: From **Int-9** (48.8 mg, 0.25 mmol) and benzenesulfonyl chloride (71.7mg, 0.25 mmol, 1.0 equiv.). Purification by column chromatography (PE/EtOAc 10:1 to 4:1) afforded a colorless solid **Int-20** (84.1 mg, 0.18 mmol, 75 %yield).

**¹H NMR** (500 MHz, CDCl₃) δ 7.71 (d, J = 2.0 Hz, 1H), 7.53 (dd, J = 8.3, 2.0 Hz, 1H), 7.44 (d, J = 8.3 Hz, 1H), 3.51 (d, J = 10.5 Hz, 1H), 3.43 (d, J = 10.5 Hz, 1H), 3.22 (dd, J = 9.1, 7.3 Hz, 1H), 3.09 (d, J = 9.7 Hz, 1H), 2.96 (d, J = 9.8 Hz, 1H), 2.93 (dd, J = 9.2, 3.8 Hz, 1H), 2.30 - 2.22 (m, 1H), 2.20 - 2.13 (m, 2H), 1.95 (dd, J = 6.5, 5.0 Hz, 1H), 1.86 (td, J = 5.5, 2.9 Hz, 1H), 1.71 (s, 4H), 1.61 - 1.56 (m, 1H), 1.30 (s, 3H), 1.30 (s, 9H), 1.26 (d, J = 10.4 Hz, 1H), 1.21 (s, 3H), 0.95 (s, 3H).

**¹³C NMR** (126 MHz, CDCl₃) δ 150.5, 146.1, 132.0, 127.4, 126.6, 125.1, 68.3, 58.5, 56.9, 52.3, 45.9, 40.4, 39.2, 34.8, 34.8, 34.8, 34.7, 34.2, 34.1, 31.9, 31.9, 31.8, 31.8, 27.5, 27.2, 23.7.

**HRMS** (ESI) for C₂₆H₃₉NO₃SNa: calculated 468.2548 [M+Na]⁺, found 468.2535.

[α]²⁰_{D} = -2.3° (CHCl₃, *c* = 1.0).

### ((3aS,4R,6R,7aS)-5,5-dimethyl-2-((4-neopentylphenyl)sulfonyl)octahydro-3aH-4,6-methanoisoindol-3a-yl)methanol (Int-21)

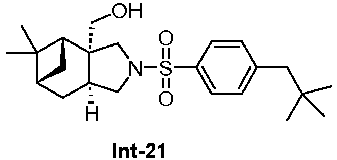

Following general procedure C2: From **Int-9** (58.6 mg, 0.3 mmol) and 4-neopentylbenzenesulfonyl chloride^{[3]} (74.3 mg, 1.0 equiv.). Purification by column chromatography (PE/EtOAc 10:1 to 4:1) afforded a colorless solid **Int-21** (79.2 mg, 0.2 mmol, 65 %yield).

**¹H NMR** (500 MHz, CDCl₃) δ 7.70 (d, J = 8.4 Hz, 2H), 7.28 (d, J = 8.3 Hz, 2H), 3.49 (d, J = 10.6 Hz, 1H), 3.42 (d, J = 10.5 Hz, 1H), 3.20 (dd, J = 9.1, 7.2 Hz, 1H), 3.06 (d, J = 9.7 Hz, 1H), 2.98 - 2.92 (m, 2H), 2.57 (s, 2H), 2.30 - 2.23 (m, 1H), 2.20 - 2.13 (m, 2H), 1.94 (dd, J = 6.5, 5.0 Hz, 1H), 1.85 (tt, J = 5.6, 3.0 Hz, 1H), 1.59 (ddd, J = 13.4, 4.3, 3.0 Hz, 1H), 1.26 (d, J = 10.4 Hz, 1H), 1.21 (s, 3H), 0.92 (d, J = 19.4 Hz, 10H).

**¹³C NMR** (126 MHz, CDCl₃) δ 145.5, 132.1, 130.9, 127.7, 68.2, 58.7, 56.9, 52.3, 50.2, 45.9, 40.4, 39.1, 34.2, 34.0, 32.1, 29.5, 27.5, 27.2, 23.8.

**HRMS** (ESI) for C₂₃H₃₆NO₃S: calculated 406.2410 [M+H]⁺, found 406.2411.

[α]²⁰D = -4.3° (CHCl₃, *c* = 0.5).

### ((3aS,4R,6R,7aS)-5,5-dimethyl-2-((4-(tert-pentyl)phenyl)sulfonyl)octahydro-3aH-4,6-methanoisoindol-3a-yl)methanol (Int-22):

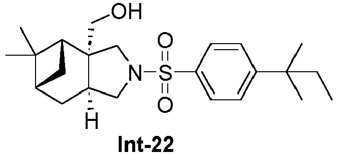

Following general procedure C2: From **Int-9** (58.59 mg, 0.3 mmol) and 4-(tert-pentyl) benzenesulfonyl chloride **Int-39** (74.03 mg, 1.0 equiv.). Purification by column chromatography (PE/EtOAc 4:1) afforded a colorless solid **Int-22** (86.4 mg, 0.21 mmol, 71 % yield).

**¹H NMR** (500 MHz, CDCl₃) δ 7.72 (d, J = 8.5 Hz, 2H), 7.48 (d, J = 8.6 Hz, 2H), 3.50 (d, J = 10.5 Hz, 1H), 3.42 (d, J = 10.6 Hz, 1H), 3.21 (dd, J = 9.1, 7.3 Hz, 1H), 3.07 (d, J = 9.7 Hz, 1H), 2.96 (d, J = 4.0 Hz, 1H), 2.94 (d, J = 3.5 Hz, 1H), 2.30 - 2.22 (m, 1H), 2.20 - 2.13 (m, 2H), 1.94 (dd, J = 6.5, 5.0 Hz, 1H), 1.85 (tt, J = 5.7, 3.0 Hz, 1H), 1.67 (q, J = 7.5 Hz, 2H), 1.60 (ddd, J = 13.4, 4.3, 2.9 Hz, 1H), 1.31 (s, 6H), 1.27 (d, J = 10.5 Hz, 1H), 1.21 (s, 3H), 0.94 (s, 3H), 0.63 (t, J = 7.5 Hz, 3H).

**¹³C NMR** (126 MHz, CDCl₃) δ 155.1, 131.7, 128.0, 126.7, 68.2, 58.6, 56.9, 52.3, 45.9, 40.4, 39.1, 38.6, 37.0, 34.2, 34.0, 28.3, 27.5, 27.2, 23.7, 9.2.

**HRMS** (ESI) for C₂₃H₃₅NO₃SNa: calculated 428.2235 [M+Na]⁺, found 428.2227.

[α]²⁰_{D} = -15.3° (CHCl₃, *c* = 1.0).

### ((3aS,4R,6R,7aS)-5,5-dimethyl-2-((4-(trimethylsilyl)phenyl)sulfonyl)octahydro-3aH-4,6-methanoisoindol-3a-yl)methanol (Int-24)

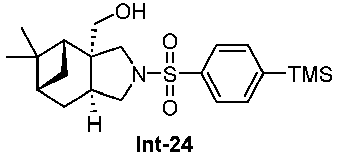

Following general procedure C2: From **Int-9** (58.59, 0.3 mmol) and 4-(trimethylsilyl)benzenesulfonyl chloride **Int-48** (74.6 mg, 0.3 mmol, 1.0 equiv.). Purification by column chromatography (PE/EtOAc 10:1 to 4:1) afforded a colorless solid **Int-24** (72.6 mg, 0.18 mmol, 60 %yield).

**¹H NMR** (500 MHz, Chloroform-d) δ 7.75 (d, J = 8.2 Hz, 2H), 7.67 (d, J = 8.2 Hz, 2H), 3.51 (d, J = 10.6 Hz, 1H), 3.41 (d, J = 10.6 Hz, 1H), 3.20 (dd, J = 9.2, 7.2 Hz, 1H), 3.07 (d, J = 9.7 Hz, 1H), 3.03 - 2.97 (m, 2H), 2.27 (tdd, J = 13.5, 3.2, 2.0 Hz, 1H), 2.22 - 2.11 (m, 2H), 1.96 (dd, J = 6.5, 5.0 Hz, 1H), 1.86 (tt, J = 5.7, 3.0 Hz, 1H), 1.65 - 1.58 (m, 1H), 1.31 (d, J = 10.4 Hz, 1H), 1.22 (s, 3H), 0.95 (s, 3H), 0.30 (s, 9H).

**¹³C NMR** (126 MHz, CDCl₃) δ 147.1, 135.3, 133.9, 127.1, 68.1, 58.5, 56.9, 52.3, 45.9, 40.4, 39.2, 34.3, 34.1, 27.5 27.2, 23.7, -1.2.

**HRMS** (ESI) for C₂₁H₃₄NO₃SSi: calculated 408.2023 [M+H]⁺, found 408.2023.

[α]²⁰_{D} = -10.7° (CHCl₃, *c* = 0.5).

### ((3aS,4R,6R,7aS)-2-((4-(tert-butyl)-2-methylphenyl)sulfonyl)-5,5-dimethyloctahydro-3aH-4,6-methanoisoindol-3a-yl)methanol (Int-26)

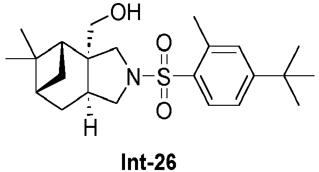

Following general procedure C2: From **Int-9** (58.59 mg, 0.3 mmol) and 4-(*tert*-butyl)-2-methylbenzenesulfonyl chloride **Int-41** (74.0 mg, 0.3 mmol, 1.0 equiv.). Purification by column chromatography (PE/EtOAc 10:1 to 4:1) afforded a colorless solid **Int-26** (80 mg, 0.20mmol, 66 % yield).

**¹H NMR** (500 MHz, CDCl₃) δ 7.83 (d, J = 8.1 Hz, 1H), 7.33 - 7.28 (m, 2H), 3.57 (d, J = 10.6 Hz, 1H), 3.50 (d, J = 10.6 Hz, 1H), 3.42 (dd, J = 9.3, 7.0 Hz, 1H), 3.25 (d, J = 9.8 Hz, 1H), 3.03 (dd, J = 9.4, 3.7 Hz, 1H), 2.99 (d, J = 9.7 Hz, 1H), 2.66 (s, 3H), 2.27 (ddt, J = 12.1, 7.6, 2.1 Hz, 1H), 2.23 - 2.16 (m, 2H), 2.00 - 1.94 (m, 1H), 1.87 (dt, J = 5.4, 2.7 Hz, 1H), 1.59 (d, J = 3.2 Hz, 1H), 1.33 (s, 9H), 1.31 (d, J = 10.5 Hz, 1H), 1.22 (s, 3H), 0.97 (s, 3H).

**¹³C NMR** (126 MHz, CDCl₃) δ 156.6, 138.0, 132.6, 130.4, 129.9, 123.2, 68.3, 58.0, 56.3, 52.4, 45.8, 40.5, 39.2, 35.0, 34.3, 34.1, 31.2, 27.6, 27.3, 23.8, 21.3.

**HRMS** (ESI) for C₂₃H₃₆NO₃S: calculated 406.2410 [M+H]⁺, found 406.2408.

[α]²⁰_{D} = -88.3° (CHCl₃, c = 0.5).

### ((3aS,4R,6R,7aS)-2-((4-(tert-butyl)-2-ethylphenyl)sulfonyl)-5,5-dimethyloctahydro-3aH-4,6-methanoisoindol-3a-yl)methanol (Int-27)

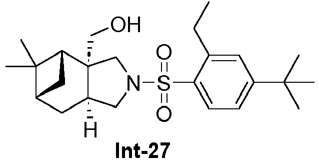

Following general procedure C2: From **Int-9** (58.59 mg, 0.3 mmol) and 4-(*tert*-butyl)-2-ethylbenzenesulfonyl chloride **Int-42** (78.2 mg, 0.3 mmol, 1.0 equiv.). Purification by column chromatography (PE/EtOAc 10:1 to 4:1) afforded a colorless solid **Int-27** (109.4 mg. 0.26 mmol, 87 %yield).

**1H NMR** (500 MHz, CDCl₃) δ 7.82 (d, J = 8.4 Hz, 1H), 7.36 (d, J = 2.1 Hz, 1H), 7.30 (dd, J = 8.4, 2.1 Hz, 1H), 3.56 (d, J = 10.5 Hz, 1H), 3.50 (d, J = 10.7 Hz, 1H), 3.40 (dd, J = 9.3, 7.0 Hz, 1H), 3.25 (d, J = 9.8 Hz, 1H), 3.10 - 3.02 (m, 3H), 2.99 (d, J = 9.8 Hz, 1H), 2.30 - 2.23 (m, 1H), 2.23 - 2.13 (m, 2H), 1.99 - 1.95 (m, 1H), 1.89 - 1.84 (m, 1H), 1.61 - 1.56 (m, 1H), 1.33 (s, 9H), 1.30 (d, J = 10.4 Hz, 1H), 1.28 (t, J = 7.5 Hz, 3H), 1.22 (s, 3H), 0.96 (s, 3H).

**¹³C NMR** (126 MHz, CDCl₃) δ 156.6, 144.3, 132.1, 130.3, 128.1, 123.1, 68.3, 58.0, 56.3, 52.4, 45.8, 40.5, 39.2, 35.1, 34.3, 34.0 31.2, 27.6, 27.2, 26.8, 23.8, 16.3. **HRMS** (ESI) for C₂₄H₃₇NO₃SNa: calculated 442.2392 [M+Na]⁺, found 442.2379.

[α]²⁰_{D} = -32° (CHCl₃, *c* = 1).

### ((3aS,4R,6R,7aS)-2-((2-bromo-4-(tert-butyl)phenyl)sulfonyl)-5,5-dimethyloctahydro-3aH-4,6-methanoisoindol-3a-yl)methanol (Int-28)

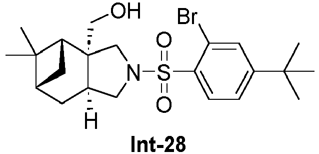

Following general procedure C2: From **Int-9** (29.3 mg, 0.15 mmol) and arenesulfonyl chloride **Int-44** (46.7 mg, 0.15 mmol, 1.0 equiv.). Purification by column chromatography (PE/EtOAc 10:1 to 4:1) afforded a colorless solid **Int-28** (70.6 mg, quanti.).

**¹H NMR** (500 MHz, CDCl₃) δ 7.98 (d, J = 8.4 Hz, 1H), 7.71 (d, J = 1.9 Hz, 1H), 7.42 (dd, J = 8.3, 1.9 Hz, 1H), 3.60 (dd, J = 9.7, 7.1 Hz, 1H), 3.56 (d, J = 10.5 Hz, 1H), 3.52 (d, J = 10.7 Hz, 1H), 3.36 (d, J = 10.1 Hz, 1H), 3.19 (dd, J = 9.5, 3.5 Hz, 1H), 3.10 (d, J = 10.0 Hz, 1H), 2.30 - 2.15 (m, 3H), 1.97 (dd, J = 6.5, 5.0 Hz, 1H), 1.88 (dq, J = 5.5, 2.4 Hz, 1H), 1.68 - 1.61 (m, 1H), 1.35 (d, J = 10.5 Hz, 6H), 1.32 (s, 9H), 1.22 (s, 3H), 0.97 (s, 3H).

**¹³C NMR** (126 MHz, CDCl₃) δ 158.0, 134.6, 133.0, 132.2, 124.7, 120.7, 68.1, 58.6, 56.4, 52.8, 45.7, 40.5, 39.1, 35.2, 34.5, 33.9, 31.0, 27.5, 27.3, 23.8.

**HRMS** (ESI) for C₂₂H₃₂BrNO₃SNa: calculated 492.1184 [M+Na]⁺, found 492.1167.

[α]²⁰_{D} = -12.1° (CHCl₃, c = 1.0).

### ((3aS,4R,6R,7aS)-2-((4-(tert-butyl)-2-methoxyphenyl)sulfonyl)-5,5-dimethyloctahydro-3aH-4,6-methanoisoindol-3a-yl)methanol (Int-29)

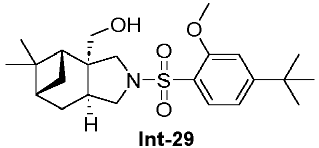

Following general procedure C2: From **Int-9** (58.59mg, 0.2 mmol) and 4-(*tert*-butyl)-2-methoxybenzenesulfonyl chloride **Int-43** (92.0 mg, 0.2 mmol, 1.0 equiv.). Purification by column chromatography (PE/EtOAc 4:1) afforded a colorless solid **Int-29** (90 % yield).

**¹H NMR** (500 MHz, CDCl₃) δ 7.80 (d, J = 8.3 Hz, 1H), 7.04 (dd, J = 8.3, 1.7 Hz, 1H), 6.99 (d, J = 1.7 Hz, 1H), 3.92 (s, 3H), 3.76 - 3.71 (m, 1H), 3.52 (d, J = 5.6 Hz, 1H), 3.51 - 3.47 (m, 1H), 3.29 (d, J = 10.0 Hz, 1H), 3.16 - 3.11 (m, 2H), 2.30 - 2.16 (m, 3H), 1.95 (dd, J = 6.5, 4.9 Hz, 1H), 1.89 - 1.85 (m, 1H), 1.64 - 1.59 (m, 1H), 1.39 (d, J = 10.3 Hz, 1H), 1.33 (s, 9H), 1.22 (s, 3H), 0.96 (s, 3H).

**¹³C NMR** (126 MHz, CDCl₃) δ 159.0, 157.1, 131.6, 123.2, 117.7, 109.8, 68.6, 58.4, 56.5, 56.1, 52.4, 45.9, 40.5, 39.2, 35.5, 34.3, 34.0, 31.2, 27.6, 27.2, 23.8.

**HRMS** (ESI) for C₂₃H₃₅NO₄SNa: calculated 444.2184 [M+Na]⁺, found 444.2180.

[α]²⁰_{D} = -24.0° (CHCl₃, *c* = 1).

### ((3aS,4R,6R,7aS)-2-((4-(tert-butyl)-2-iodophenyl)sulfonyl)-5,5-dimethyloctahydro-3aH-4,6-methanoisoindol-3a-yl)methanol (Int-30)

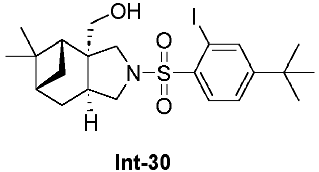

Following general procedure C2: From **Int-9** (29.3 mg, 0.15 mmol) and 4-(tert-butyl)-2-iodobenzenesulfonyl chloride **Int-45** (46.7 mg, 0.15 mmol, 1.0 equiv.). Purification by column chromatography (PE/EtOAc 10: 1 to 4:1) afforded a colorless solid **Int-30** (70.6 mg, quanti.).

**¹H NMR** (500 MHz, CDCl₃) δ 8.06 (d, J = 1.9 Hz, 1H), 8.00 (d, J = 8.3 Hz, 1H), 7.46 (dd, J = 8.4, 1.9 Hz, 1H), δ 3.79 - 3.70 (m, 1H).3.65 - 3.59 (m, 1H), 3.59 (d, J = 10.7 Hz, 1H), 3.54 (d, J = 10.6 Hz, 1H), 3.37 (d, J = 10.1 Hz, 1H), 3.19 (dd, J = 9.7, 3.6 Hz, 1H), 3.07 (d, J = 10.0 Hz, 1H), 2.30 - 2.17 (m, 3H), 1.98 (dd, J = 6.5, 5.0 Hz, 1H), 1.89 (dt, J = 5.6, 2.8 Hz, 1H), 1.69 (dd, J = 9.2, 2.9 Hz, 1H), 1.38 (d, J = 10.4 Hz, 1H), 1.32 (s, 9H), 1.22 (s, 3H), 0.97 (s, 3H).

**¹³C NMR** (126 MHz, CDCl₃) δ 157.6, 140.5, 137.7, 131.6, 125.5, 93.1, 68.2, 58.6, 56.5, 52.8, 45.7, 40.5, 39.1, 35.0, 34.4, 33.7, 31.0, 27.6, 27.4, 23.8.

**HRMS** (ESI) for C₂₂H₃₂INO₃SNa: calculated 540.1045 [M+Na]⁺, found 540.1034.

[α]²⁰_{D} = -9.03° (CHCl₃, c = 1.0).

### ((3aS,4R,6R,7aS)-2-((4-(1-(2-methoxyethoxy)-2-methylpropan-2-yl)phenyl)sulfonyl)-5,5-dimethyloctahydro-3aH-4,6-methanoisoindol-3a-yl)methanol (Int-31)

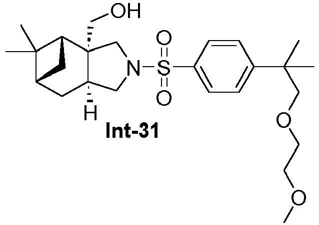

Following general procedure C2: From **Int-9** (48.83 mg, 0.25 mmol) and arenesulfonyl chloride **Int-49** (1.0 equiv.). Purification by column chromatography (PE/EtOAc 4:1) afforded a colorless solid **Int-31** (90 %yield).

**¹H NMR** (700 MHz, CDCl₃) δ 7.73 (d, J = 8.8 Hz, 2H), 7.55 (d, J = 8.8 Hz, 2H), 3.52 (d, J = 9.3 Hz, 1H), 3.48 (d, J = 9.4 Hz, 1H), 3.47 - 3.43 (m, 4H), 3.39 (d, J = 2.9 Hz, 2H), 3.32 (s, 3H), 3.14 (dd, J = 9.1, 7.1 Hz, 1H), 3.06 - 3.02 (m, 1H), 3.02 (d, J = 9.9 Hz, 1H), 2.97 (d, J = 9.7 Hz, 1H), 2.31 - 2.24 (m, 1H), 2.19 (dtd, J = 14.9, 8.0, 7.6, 5.1 Hz, 2H), 1.91 (t, J = 5.8 Hz, 1H), 1.86 (dt, J = 5.2, 2.6 Hz, 1H), 1.64 - 1.60 (m, 1H), 1.38 (s, 1H), 1.36 (s, 7H), 1.21 (s, 3H), 0.94 (s, 3H).

**¹³C NMR** (176 MHz, CDCl₃) δ 153.2, 132.1, 128.0, 126.9, 81.1, 72.0, 71.1, 68.1, 59.2, 58.7, 56.9, 52.4, 46.3, 40.4, 39.8, 39.2, 34.4, 34.4, 27.6, 27.4, 26.0, 26.0, 23.8. **HRMS** (ESI) for C₂₅H₃₉NO₅SNa: calculated: 488.2447 [M+Na]⁺, found 488.2445.

[α]²⁰_{D} = +12.03° (CHCl₃, *c* = 0.5).

### 2-(4-(((3aS,4R,6R,7aS)-3a-(hydroxymethyl)-5,5-dimethyloctahydro-2H-4,6-methanoisoindol-2-yl)sulfonyl)phenyl)-2-methyl-1-morpholinopropan-1-one (Int-32)

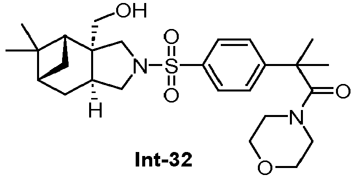

Following general procedure C2: From **Int-9** (58.6 mg, 0.3 mmol) and arenesulfonyl chloride **Int-50** (1.0 equiv.). Purification by column chromatography (PE/EtOAc 2:1) afforded a colorless solid **Int-32** (104.8 mg, 0.21 mmol, 71 %yield).

**¹H NMR** (400 MHz, CDCl₃) δ 7.79 (d, J = 8.5 Hz, 2H), 7.41 (d, J = 8.5 Hz, 2H), 3.60 (s, 2H), 3.47 - 3.39 (m, 3H), 3.25 (d, J = 6.3 Hz, 3H), 3.14 (dd, J = 9.0, 7.0 Hz, 2H), 3.05 - 2.95 (m, 4H), 2.34 - 2.24 (m, 1H), 2.18 (tdd, J = 8.4, 6.6, 3.9 Hz, 2H), 1.93 (dd, J = 6.5, 5.0 Hz, 1H), 1.86 (tt, J = 7.0, 3.1 Hz, 1H), 1.64 - 1.59 (m, 1H), 1.56 (s, 6H), 1.30 (d, J = 10.4 Hz, 1H), 1.21 (s, 3H), 0.94 (s, 3H).

**¹³C NMR** (101 MHz, CDCl₃) δ 174.1, 151.5, 133.3, 129.0, 125.6, 68.0, 58.7, 56.8, 52.6, 47.3, 46.2, 40.4, 39.2, 34.3, 34.3, 28.3, 28.2, 27.5, 27.2, 23.7.

**HRMS** (ESI) for C₂₆H₃₈N₂O₅SNa: calculated: 513.2399 [M+Na]⁺, found 513.2388.

[α]²⁰_{D} = -3.1° (CHCl₃, *c* = 1.0).

### 2-(4-(((3aS,4R,6R,7aS)-3a-(hydroxymethyl)-5,5-dimethyloctahydro-2H-4,6-methanoisoindol-2-yl)sulfonyl)phenyl)-N,N,2-trimethylpropanamide (Int-33)

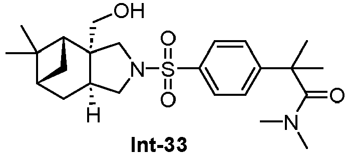

Following general procedure C2: From **Int-9** (39.6 mg, 0.2 mmol) and arenesulfonyl chloride **Int-51** (57.8 mg, 1.0 equiv.). Purification by column chromatography (PE/EtOAc 2:1) afforded a colorless solid **Int-33** (73 mg, 0.16 mmol, 81 %yield).

**¹H NMR** (600 MHz, CDCl₃) δ 7.78 (d, J = 8.5 Hz, 2H), 7.39 (d, J = 8.5 Hz, 2H), 3.48 (d, J = 10.5 Hz, 1H), 3.43 (d, J = 10.4 Hz, 1H), 3.20 (dd, J = 9.1, 7.3 Hz, 1H), 3.08 (d, J = 9.7 Hz, 1H), 3.01 - 2.96 (m, 1H), 2.97 (d, J = 9.6 Hz, 1H), 2.94 (s, 3H), 2.43 (s, 3H), 2.31 - 2.24 (m, 1H), 2.22 - 2.13 (m, 2H), 1.94 (dd, J = 6.4, 5.0 Hz, 1H), 1.86 (tt, J = 5.6, 3.0 Hz, 1H), 1.60 (ddd, J = 13.5, 4.4, 2.9 Hz, 1H), 1.57 (s, 6H), 1.26 (d, J = 10.4 Hz, 1H), 1.21 (s, 3H), 0.95 (s, 3H).

**¹³C NMR** (151 MHz, CDCl₃) δ 175.2, 152.0, 133.0, 129.0, 125.5, 68.1, 58.7, 57.0, 52.3, 47.4, 46.1, 40.4, 39.2, 38.4, 38.2, 37.4, 34.3, 34.1, 28.2, 27.5, 27.2, 23.7. **HRMS** (ESI) for C₂₄H₃₆N₂O₄SNa: calculated: 471.2293 [M+Na]⁺, found 471.2269.

[α]²⁰_{D} = -12.7° (CHCl₃, *c* = 1.0).

### Methyl 2-(4-(((3aS,4R,6R,7aS)-3a-(hydroxymethyl)-5,5-dimethyloctahydro-2H-4,6-methanoisoindol-2-yl)sulfonyl)phenyl)-2-methylpropanoate (Int-34)

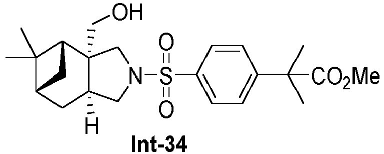

Following general procedure C2: From **Int-9** (78.1 mg, 0.4 mmol) and arenesulfonyl chloride **Int-46** (110.7 mg, 0.4 mmol, 1.0 equiv.). Purification by column chromatography (PE/EtOAc 4:1) afforded a colorless solid **Int-34** (129 mg, 0.3 mmol, 75 %yield).

**¹H NMR** (500 MHz, CDCl₃) δ 7.75 (d, J = 8.5 Hz, 2H), 7.49 (d, J = 8.5 Hz, 2H), 3.66 (s, 3H), 3.49 (d, J = 10.5 Hz, 1H), 3.41 (d, J = 10.6 Hz, 1H), 3.20 (dd, J = 9.1, 7.2 Hz, 1H), 3.07 (d, J = 9.7 Hz, 1H), 2.99 (dd, J = 9.4, 2.8 Hz, 2H), 2.30 - 2.24 (m, 1H), 2.20 - 2.14 (m, 2H), 1.95 (dd, J = 6.5, 5.0 Hz, 1H), 1.87 - 1.84 (m, 1H), 1.60 (s, 6H), 1.29 (d, J = 10.4 Hz, 1H), 1.21 (s, 3H), 0.94 (s, 3H).

**¹³C NMR** (126 MHz, CDCl₃) δ 176.5, 149.8, 133.6, 128.3 126.5, 68.1, 58.5, 56.9, 52.6, 52.4, 47.0, 45.9, 40.4, 39.2, 34.3, 34.1, 27.5, 27.2, 26.5, 23.7.

**HRMS** (ESI) for C₂₃H₃₃NO₅SNa: calculated: 458.1977 [M+Na]⁺, found 458.1971.

[α]²⁰_{D} = -16.0° (CHCl₃, *c* = 1.0).

### Methyl 2-(4-(((3aS,4R,6R,7aS)-3a-(hydroxymethyl)-5,5-dimethyloctahydro-2H-4,6-methanoisoindol-2-yl)sulfonyl)-3-methoxyphenyl)-2-methylpropanoate (Int-35)

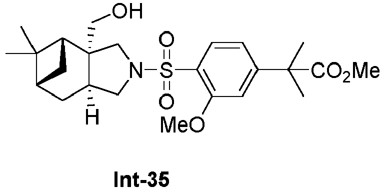

Following general procedure C2: From **Int-9** (58.6 mg, 0.3 mmol) and arenesulfonyl chloride **Int-47** (92 mg, 1.0 equiv.). Purification by column chromatography (PE/EtOAc 10:1 to 4:1) afforded a colorless solid **Int-35** (122.7 mg, 0.26 mmol, 88 % yield).

**¹H NMR** (600 MHz, CDCl₃) δ 7.84 (d, *J* = 8.3 Hz, 1H), 6.99 (dd, *J* = 8.3, 1.7 Hz, 1H), 6.94 (d, *J* = 1.8 Hz, 1H), 3.91 (s, 3H), 3.67 (s, 3H), 3.53 (d, *J* = 10.7 Hz, 1H), 3.52 - 3.47 (m, 2H), 3.30 (d, *J* = 10.1 Hz, 1H), 3.14 (d, *J* = 9.9 Hz, 2H), 2.29 - 2.16 (m, 3H), 1.95 (dd, *J* = 6.4, 4.9 Hz, 1H), 1.87 (tt, *J* = 5.5, 2.9 Hz, 1H), 1.64 - 1.59 (m, 1H), 1.58 (s, 6H), 1.37 (d, *J* = 10.4 Hz, 1H), 1.22 (s, 3H), 0.96 (s, 3H).

**¹³C NMR** (151 MHz, CDCl₃) δ 176.5, 157.2, 151.9, 131.9, 124.9, 117.9, 110.2, 68.5, 58.4, 56.5, 56.1, 52.6, 52. 5, 47.1, 46.0, 40.5, 39.2, 34.4, 34.1, 27.6, 27.2, 26.5, 26.5, 23.8. **HRMS** (ESI) for C₂₄H₃₅NO₆SNa: calculated: 488.2083 [M+Na]⁺, found 488.2068.

[α]²⁰_{D} = +10.1° (CHCl₃, *c* = 0.2).

### Methyl 2-(3-chloro-4-(((3aS,4R,6R)-3a-(hydroxymethyl)-5,5-dimethyloctahydro-2H-4,6-methanoisoindol-2-yl)sulfonyl)phenyl)-2-methylpropanoate (Int-36)

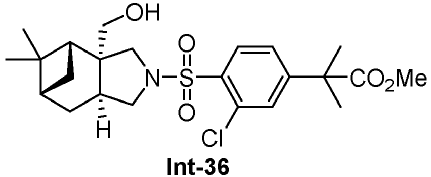

Following general procedure C2: From **Int-9** (25.4 mg, 0.13 mmol) and arenesulfonyl chloride **Int-52** (39 mg, 1.0 equiv.). Purification by column chromatography (PE/EtOAc 10:1 to 4:1) afforded a colorless solid **lnt-36** (47.5 mg, 0.1mmol, 78 % yield).

**¹H NMR** (500 MHz, CDCl₃) δ 7.99 (d, J = 8.3 Hz, 1H), 7.47 (d, J = 1.9 Hz, 1H), 7.33 (dd, J = 8.3, 1.9 Hz, 1H), 3.68 (s, 3H), 3.60 (dd, J = 9.7, 7.1 Hz, 1H), 3.56 (d, J = 10.6 Hz, 1H), 3.50 (d, J = 10.6 Hz, 1H), 3.36 (d, J = 10.1 Hz, 1H), 3.21 (dd, J = 9.7, 3.6 Hz, 1H), 3.13 (d, J = 10.1 Hz, 1H), 2.27 (dd, J = 13.5, 1.6 Hz, 2H), 2.26 - 2.18 (m, 2H), 1.97 (dd, J = 6.5, 5.0 Hz, 1H), 1.89 (dq, J = 5.4, 2.6 Hz, 1H), 1.63 (s, 2H), 1.59 (s, 6H), 1.33 (d, J = 10.5 Hz, 1H), 1.22 (s, 3H), 0.97 (s, 3H).

**¹³C NMR** (126 MHz, CDCl₃) δ 176.0, 151.1, 134.5, 132.8, 132.1, 129.8, 124.7, 68.1, 58.6, 56.4, 52.8, 46.8, 45.8, 40.5, 39.2, 34.6, 34.0, 27.6, 27.3, 26.4, 26.4, 23.8. **HRMS** (ESI) for C₂₃H₃₂ClNO₈SNa: calculated: 492.1587 [M+Na]⁺, found 492.1580.

[α]²⁰_{D} = -12.0° (CHCl₃, *c* = 1.0).

### Methyl 2-(4-(((3aS,4R,6R,7aS)-3a-(hydroxymethyl)-5,5-dimethyloctahydro-2H-4,6-methanoisoindol-2-yl) sulfonyl)-3-methylphenyl)-2-methylpropanoate (Int-37)

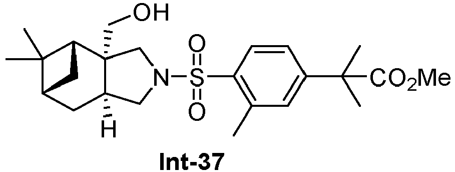

Following general procedure C2: From **Int-9** (117.2 mg, 0.6 mmol) and arenesulfonyl chloride **Int-53** (174.58 mg, 0.6 mmol, 1.0 equiv.). Purification by column chromatography (PE/EtOAc 10:1 to 4:1) afforded a colorless solid **Int-37** (246.5 mg, 0.55 mmol, 91 %).

**¹H NMR** (500 MHz, DMSO-d6) δ 7.83 (d, J = 8.9 Hz, 1H), 7.33 - 7.23 (m, 2H), 3.65 (s, 3H), 3.52 (d, J = 10.3 Hz, 1H), 3.46 (dd, J = 10.6, 3.7 Hz, 1H), 3.37 (dd, J = 9.3, 7.1 Hz, 1H), 3.21 (d, J = 9.7 Hz, 1H), 3.02 (dd, J = 9.2, 3.5 Hz, 1H), 2.96 (d, J = 9.7 Hz, 1H), 2.64 (s, 3H), 2.32 - 2.25 (m, 1H), 2.25 - 2.15 (m, 2H), 1.95 (dd, J = 6.5, 5.0 Hz, 1H), 1.86 (tt, J = 5.7, 3.0 Hz, 1H), 1.57 (d, J = 2.3 Hz, 9H), 1.56 - 1.54 (m, 1H), 1.29 (s, 1H), 1.22 (s, 3H), 0.96 (s, 3H).

**¹³C NMR** (126 MHz, DMSO) δ 176.7, 150.2, 138.7, 134.3, 130.6, 130.6, 123.9, 68.3, 58.3, 56.5, 52.7, 52.6, 47.0, 46.3, 40.8, 39.4, 34.7, 34.4, 27.6, 27.5, 26.5, 23.7, 21.3. **HRMS** (ESI) for C₂₄H₃₅NO₅SNa: calculated: 472.2134 [M+Na]⁺, found 472.2124.

[α]²⁰_{D} = -28.1° (CHCl₃, c = 0.5).

### ((3aS,4R,6R,7aS)-5,5-dimethyl-2-((4-(3-(trifluoromethyl)-3H-diazirin-3-yl)phenyl)sulfonyl)octahydro-3aH-4,6-methanoisoindol-3a-yl)methanol (Int-38)

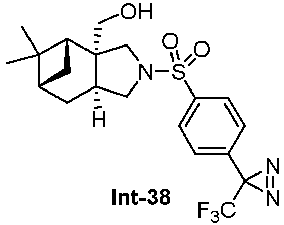

Following general procedure C2: From **Int-9** (35.0 mg, 0.179 mmol) and 4-[3-(trifluoromethyl)-3*H*-diazirin-3-yl]benzene-1-sulfonyl chloride (51.0 mg, 0.179 mmol, 1.0 equiv.). Purification by column chromatography (PE/EtOAc 4:1 to 3:1) afforded a colorless solid **Int-38** (65.0 mg, 0.147 mmol, 82 % yield).

**¹H-NMR** (CDCl₃, 500 MHz): δ = 0.94 (s, 3 H, CH₃), 1.22 (s, 3 H, CH₃), 1.30 (d, *J* = 10.4 Hz,1 H, CH), 1.37-1.41 (m, 1 H, CH), 1.58-1.63 (m, 1 H, CH), 1.85-1.90 (m, 1 H, CH), 1.93-1.97 (m, 1 H, CH), 2.16-2.23 (m, 2 H, 2 × CH), 2.25-2.33 (m, 1 H, CH), 2.99-3.05 (m, 3 H, 3 × CH), 3.15 (dd, *J* = 9.1, 7.1 Hz, 1 H, CH), 3.40-3.44 (m, 1 H, CH_{A}), 3.48 (d, *J* = 9.8 Hz, 1 H, CH_{B}), 7.34 (d, *J* = 8.2 Hz, 2 H, 2 × Ar-H), 7.82-7.86 (m, 2 H, 2 × Ar-H).

**¹³C-NMR** (CDCl₃, 125 MHz): δ = 23.7, 27.3, 27.5, 34.3, 34.4, 39.2, 40.3, 46.0, 52.4, 56.8, 58.5, 68.0, 127.0, 128.6, 134.0, 136.6.

**MS** (ESI) *m*/*z* (%) = 444.0 (100) [M+H]⁺.

**HRMS** (ESI) for C₂₀H₂₅F₃N₃O₃S: calculated 444.1563 [M+H]⁺, found 444.1565.

### Synthesis of intermediates Int-39 to Int-53

### General procedure for the synthesis of arenesulfonyl chlorides:^{[4]}

To a solution of alkyl benzene (1 equiv.) in chloroform, chlorosulfonic acid ( 5 equiv.) was added dropwise at 0 °C over15 min.. The resulting solution was stirred at room temperature for 1 h. Then the reaction mixture was poured onto ice, and the aqueous phase was extracted with chloroform twice. The combined organic layers were then washed with brine, dried over sodium sulfate, and concentrated by rotary evaporation to give the crude product. The resulting oil was then purified by flash column chromatography (hexanes/ethyl acetate = 20:1).

### 4-(tert-pentyl)benzenesulfonyl chloride (Int-39:

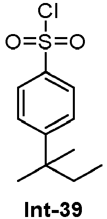

**¹H NMR** (400 MHz, CDCl₃) δ 7.96 (d, *J* = 8.6 Hz, 2H), 7.56 (d, *J* = 8.6 Hz, 2H), 1.70 (q, *J* = 7.4 Hz, 2H), 1.33 (s, 6H), 0.69 (t, *J* = 7.3 Hz, 3H).

**¹³C NMR** (101 MHz, CDCl₃) δ 158.5, 141.6, 127.5, 126.9 39.0, 36.78, 28.3, 9.1.

### 4'-(tert-butyl)-[1,1'-biphenyl]-4-sulfonyl chloride (Int-40):

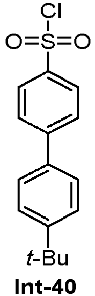

**¹H NMR** (400 MHz, CDCl₃) δ 8.09 (d, J = 8.6 Hz, 2H), 7.80 (d, J = 8.7 Hz, 2H), 7.58 (d, J = 8.4 Hz, 2H), 7.53 (d, J = 8.5 Hz, 2H), 1.38 (s, 9H).

**¹³C NMR** (101 MHz, CDCl₃) δ 152.7, 148.3, 142.7, 135.7, 128.1, 127.7, 127.3, 126.4, 34.9, 31.4.

### 4-(tert-butyl)-2-methylbenzenesulfonyl chloride (Int-41):

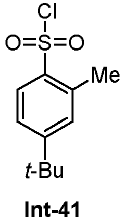

**¹H NMR** (500 MHz, CDCl₃) δ 7.98 (d, *J* = 9.1 Hz, 1H), 7.42 - 7.38 (m, 2H), 2.78 (s, 3H), 1.35 (s, 9H).

**¹³C NMR** (126 MHz, CDCl₃) δ 159.6, 140.4, 137.9, 130.6, 128.9, 123.9, 35.4, 31.1, 20.7.

### 4-(tert-butyl)-2-ethylbenzenesulfonyl chloride (Int-42):

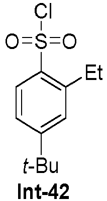

**¹H NMR** (500 MHz CDCl₃) δ 7.98 (d, *J* = 8.5 Hz, 1H), 7.45 (d, *J* = 2.0 Hz, 1H), 7.39 (dd, *J* = 8.6, 2.0 Hz, 1H), 3.18 (q, *J* = 7.5 Hz, 2H), 1.37 (t, *J* = 7.5 Hz, 3H), 1.36 (s, 9H).

**¹³C NMR** (126 MHz, CDCl₃) δ 159.6, 144.0, 140.1, 129.1, 128.8, 123.9, 35.5, 31.1, 26.2, 15.4.

### 4-(tert-butyl)-2-methoxybenzenesulfonyl chloride (Int-43):

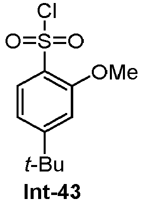

**¹H NMR** (500 MHz, CDCl₃) δ 7.86 (d, *J* = 8.3 Hz, 1H), 7.12 - 7.08 (m, 2H), 4.06 (s, 3H), 1.36 (s, 9H).

**¹³C NMR** (126 MHz, CDCl₃) δ 162.3, 157.3, 129.7, 129.3, 117.7, 110.4, 56.5, 36.0, 31.1.

### 2-bromo-4-(tert-butyl)benzenesulfonyl chloride (Int-44):

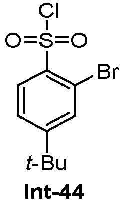

**¹H NMR** (600 MHz, CDCl₃) δ 8.10 (d, *J* = 8.5 Hz, 1H), 7.83 (d, *J* = 1.9 Hz, 1H), 7.52 (dd, *J* = 8.5, 1.9 Hz, 1H), 1.36 (s, 9H).

**¹³C NMR** (151 MHz, CDCl₃) δ 160.8, 140.4, 133.8, 130.8, 125.3, 120.7, 35.7, 31.0.

### 4-(tert-butyl)-2-iodobenzenesulfonyl chloride (Int-45):

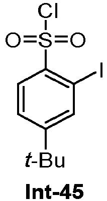

**¹H NMR** (600 MHz, CDCl₃) δ 8.15 (d, *J* = 2.0 Hz, 1H), 8.09 (d, *J* = 8.5 Hz, 1H), 7.57 (dd, *J* = 8.5, 2.0 Hz, 1H), 1.32 (s, 9H).

**¹³C NMR** (151 MHz, CDCl₃) δ 160.1, 143.3, 141.0, 130.3, 126.1, 92.3, 35.2, 30.8.

### Methyl 2-(4-(chlorosulfonyl)phenyl)-2-methylpropanoate(Int-46):

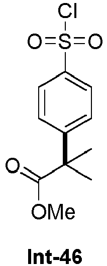

**¹H NMR** (500 MHz, CD₂Cl₂) δ 7.99 (d, *J* = 8.7 Hz, 2H), 7.60 (d, *J* = 8.8 Hz, 2H), 3.66 (s, 3H), 1.61 (s, 6H).

**¹³C NMR** (126 MHz, CD₂Cl₂) δ 176.1, 153.5, 142.8, 127.8, 127.5, 52.8, 47.5, 26.6.

### Methyl 2-(4-(chlorosulfonyl)-3-methoxyphenyl)-2-methylpropanoate (Int-47):

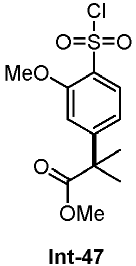

**¹H NMR** (500 MHz, CDCl₃) δ 7.89 (d, *J* = 8.9 Hz, 1H), 7.08 - 7.03 (m, 2H), 4.05 (s, 3H), 3.69 (s, 3H), 1.61 (s, 6H).

**¹³C NMR** (126 MHz, CDCl₃) δ 176.0, 157.4, 155.1, 130.4, 130.0, 117.9, 110.9, 56.7, 52.8, 47.39, 26.4.

### Synthesis of intermediate Int-48^{[5]}

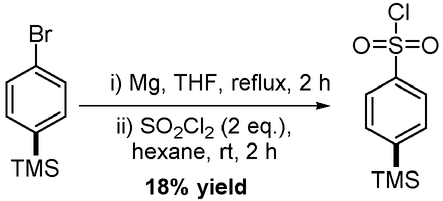

i) Magnesium powder (1.1 equiv.) was added to a 25 mL round-bottom flask containing anhydrous THF (0.1 M) and iodine (0.1 equiv.) under argon pressure. A solution of (4-bromophenyl) trimethylsilane (1 equiv.) in anhydrous THF (0.1 M) was dropped into the suspension of magnesium. After initiatiation of the reaction, the dropping rate of the bromobenzene solution was adjusted to maintain the mixture at reflux. After the addition, heating to reflux was continued for 2 more hours. ii) The Grignard reagent prepared as described above was added slowly to sulphuryl chloride (2.0 equiv.) in hexane at 0 °C. The mixture was stirred at room temperature for 2 h. poured into water, and the aqueous layer was extracted with ethyl acetate (3 times). The combined organic layers were then washed with brine, dried over sodium sulfate, and concentrated under reduced pressure to give the crude product. The resulting oil was purified by flash column chromatography (hexanes/ethyl acetate = 20:1) to give pure arenesulfonyl chloride.

### 4-(trimethylsilyl)benzenesulfonyl chloride (Int-48):

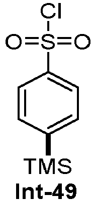

**¹H NMR** (700 MHz, CDCl₃) δ 7.94 (d, *J* = 7.0 Hz, 2H), 7.74 (d, *J* = 8.0 Hz, 2H), 0.33 (s, 9H).

**¹³C NMR** (176 MHz, CDCl₃) δ 151.1, 147.5, 134.5, 125.2, -1.3.

### Synthesis of intermediate Int-49 :

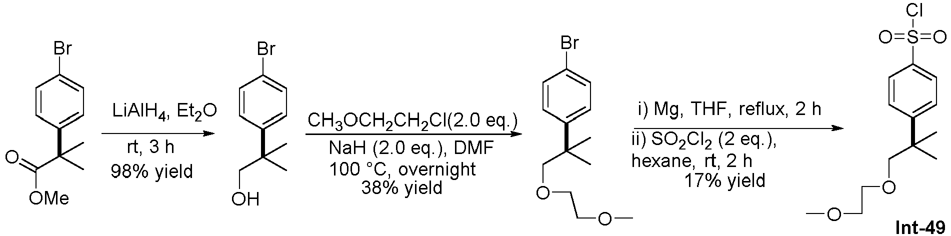

1) To a solution of the ester (10 mmol, 1.00 equiv) in diethyl ether (0.25 M) LiAlH₄ (855 mg, 22.5 mmol, 2.25 equiv) was added in four approximately equal portions at 0 °C over 10 minutes. Then mixture was allowed to warm to room temperature and stirred for an additional 30 minutes. After TLC monitoring showed complete consumption of the methyl ester, the flask was returned to an ice bath and quenched with 1 M aqueous HCl slowly. The primary alcohol product was extracted with diethyl ether (3 times). The combined organic layers were dried over anhydrous MgSO₄, filtered, and concentrated under reduced pressure. The primary alcohol was used in next step without further purification.
2) To a stirred suspension of NaH (400 mg, 10 mmol) in dry DMF (5 mL) was added slowly a solution of the alcohol (1.14 g, 5 mmol) in dry DMF (5 mL) at 0 °C. The mixture was stirred for 30 min at room temperature before CH₂OCH₂CH₂Cl (0.91 mL, 10 mmol) was slowly introduced at 0 °C. After stirring overnight at 95-100 °C, sat. NH₄Cl solution (3 mL) and water (6 mL) were added at 0 °C. The aqueous phase was extracted with tert-butyl methyl ether (2 × 30 mL) and the combined organic layers were washed with brine and dried over MgSO₄. The solvent was removed under reduced pressure and the crude product was purified by flash chromatography (hexanes/EtOAc 30:1 to 10:1) to give the product as a colorless oil (541 mg, 1.89 mmol, 38%yield), which was used for the synthesis of **Int-49** based on the synthesis route of **Int-48.** (**Int-49,** 100 mg, 18% yield).

### 4-(1-(2-methoxyethoxy)-2-methylpropan-2-yl)benzenesulfonyl chloride (Int-49):

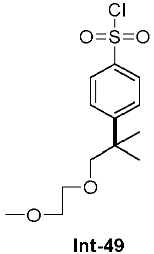

**¹H NMR** (500 MHz, CDCl₃) δ 7.91 (d, J = 8.8 Hz, 2H), 7.62 (d, J = 8.7 Hz, 2H), 3.58 - 3.54 (m, 2H), 3.53 (s, 2H), 3.50 - 3.48 (m, 2H), 3.33 (s, 3H), 1.37 (s, 6H).

**¹³C NMR** (126 MHz, CDCl₃) δ 156.5, 144.8, 127.7, 126.3, 80.4, 72.1, 71.1, 59.2, 40.2, 26.2.

### Synthesis of Intermediate Int-50 and Int-51:

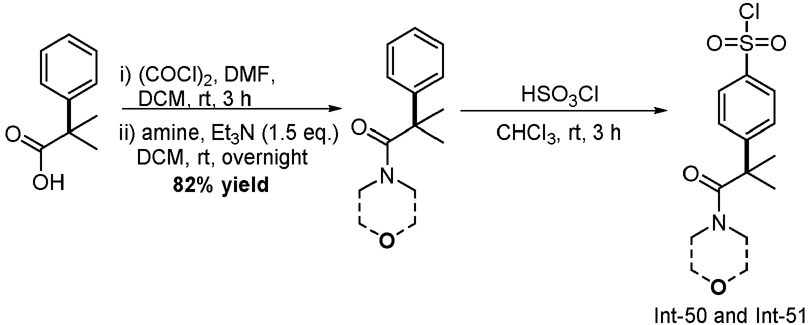

To a solution of the acid (1.0 equiv) in dichloromethane (0.5 M) was added dropwise (COCl)₂ (1.5 equiv) followed by 3 drops of DMF at 0 °C. The reaction mixture was stirred at rt for 4 h. The reaction mixture was then concentrated. The resulting acid chloride was redissolved in dichloromethane (0.3 M), then morphine or dimethylamine (1.1 equiv) and triethylamine (1.5 equiv.) were added. The resulting mixture was stirred overnight at rt, diluted with dichloromethane, washed twice with HCl (1 M), once with sat.. NaHCO₃ solution, and once with brine. The organic layers were dried (Na₂SO₄) and concentrated under reduced pressure to give the crude amides. which were used for the synthesis of **Int-50** and **Int-51** based on the synthesis route of **Int-46.**

### 4-(2-methyl-1-morpholino-1-oxopropan-2-yl)benzenesulfonyl chloride (Int-50):

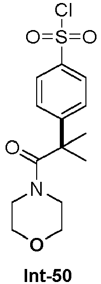

**¹H NMR** (500 MHz, CDCl₃) δ 8.03 (d, J = 8.6 Hz, 2H), 7.49 (d, J = 8.6 Hz, 2H), 3.41 (d, J = 125.0 Hz, 8H), 1.59 (s, 6H).

**¹³C NMR** (126 MHz, CDCl₃) δ 173.4, 154.6, 142.8, 128.0, 126.3, 66.5, 47.6, 46.9, 28.3.

### 4-(1-(dimethylamino)-2-methyl-1-oxopropan-2-yl)benzenesulfonyl chloride(Int-51):

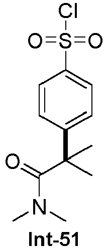

**¹H NMR** (500 MHz, CDCl₃) δ 8.04 (d, *J* = 8.6 Hz, 2H), 7.50 (d, *J* = 8.6 Hz, 2H), 2.98 (s, 3H), 2.51 (s, 3H), 1.61 (s, 6H).

**¹³C NMR** (126 MHz, CDCl₃) δ 174.6, 155.0, 142.5, 127.9, 126.3, 47.7, 38.4, 37.4, 28.2.

### Synthesis of Intermediate Int-52 and Int-53:

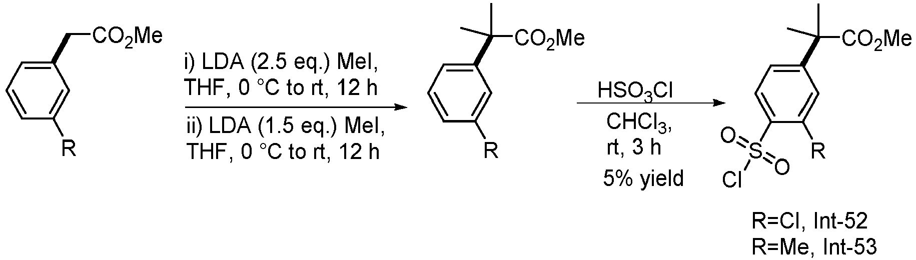

i) To the methyl phenylacetate (10 mmol) in anhydrous THF (10 mL) solution, LDA (2.0 M in THF, 11 mL, 22 mmol) was added dropwise under N₂ atmosphere in an ice bath. Then the solution was stirred at 45 °C for 1 hour. After cooling the reaction mixture to 0 °C again, Mel (22 mmol 2.2 equiv.) was added dropwise. The mixture was stirred at room temperature overnight, quenched with 4N HCl at 0 °C. The phases were separated and the aqueous phase was extracted with EtOAc (3 times). The organic layers were combined, washed with brine, and dried over anhydrous Na₂SO₄. After filtration and concentration, the crude product was purified by flash column chromatography to obtain a pale yellow liquid product, which was used for preparing **Int-52** and **Int-53** based on the general procedure for the synthesis of arenesulfonyl chlorides.

### Methyl 2-(3-chloro-4-(chlorosulfonyl)phenyl)-2-methylpropanoate (Int-52):

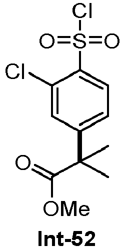

**¹H NMR** (400 MHz, CDCl₃) δ 8.08 (d, *J* = 8.5 Hz, 1H), 7.59 (d, *J* = 1.9 Hz, 1H), 7.44 (dd, *J* = 8.5, 2.0 Hz, 1H), 3.70 (s, 3H), 1.62 (s, 6H).

**¹³C NMR** (101 MHz, CDCl₃) δ 175.4, 154.0, 139.7, 130.6, 130.6, 127.3, 125.2, 52.9, 47.1, 26.4.

### Methyl 2-(4-(chlorosulfonyl)-3-methylphenyl)-2-methylpropanoate (Int-53):

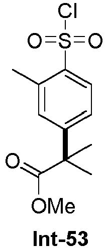

**¹H NMR** (500 MHz, DMSO-*d*₆) δ 8.00 (d, *J* = 9.1 Hz, 1H), 7.40 - 7.34 (m, 2H), 3.66 (s, 3H), 2.76 (s, 3H), 1.59 (s, 6H).

**¹³C NMR** (126 MHz, DMSO) δ 176.2, 153.3, 141.5, 138.7, 131.4, 129.3, 124.7, 52.8, 47.3, 26.5, 20.7.

### ((3aR,4R,6R)-5,5-dimethyl-2-((4-(3-(trifluoromethyl)-3H-diazirin-3-yl)phenyl)-sulfonyl)octahydro-3aH-4,6-methanoisoindol-3a-yl)methyl-(4-iodophenyl)carbamate (Int-54)

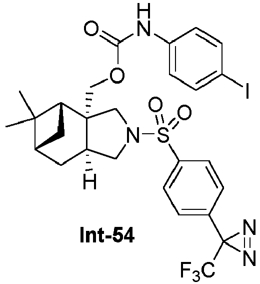

Following general procedure A2: From **Int-38** (50.0 mg, 0.113 mmol) and 4-iodophenyl isocyanate. Purification by column chromatography (PE/EtOAc 6:1) afforded a beige colorled solid **Int-54** (47.0 mg, 68.3 µmol, 61 % yield).

**¹H NMR** (CDCl₃, 500 MHz): δ = 1.01 (s, 3 H, CH₃), 1.23 (s, 3 H, CH₃), 1.26 (d, *J* = 10.6 Hz,1 H, CH), 1.56-1.64 (m, 2 H, 2 × CH), 1.87-1.92 (m, 1 H, CH), 2.17-2.24 (m, 1 H, CH), 2.24-2.37 (m, 2 H, 2 × CH), 2.98-3.10 (m, 3 H, 3 × CH), 3.17-3.25 (m, 1 H, CH), 3.98 (d, *J* = 10.7 Hz, 1 H, CH_{B}), 4.11 (d, *J* = 10.7 Hz, 1 H, CH_{B}), 6.47 (s, 1 H, NH), 7.07-7.19 (m, 2 H, 2 × Ar-H), 7.33 (d, *J* = 8.3 Hz, 2 H, 2 × Ar-H), 7.59-7.64 (m, 2 H, 2 × Ar-H), 7.84 (d, *J* = 8.6 Hz, 2 H, 2 × Ar-H).

**¹³C NMR** (CDCl₃, 125 MHz): δ = 23.5, 27.1, 27.3, 34.1, 34.5, 39.1, 40.1, 46.3, 50.8, 56.9, 58.2, 70.1, 120.6, 127.0, 128.5, 134.2, 136.9, 137.5, 138.1, 153.1.

**MS** (ESI) *m*/*z* (%) = 688.9 (100) [M+H]⁺.

**HRMS** (ESI) for C₂₇H₂₉F₃IN₄O₄S: calculated 689.0901 [M+H]⁺, found 689.0912.

### ((3aS,4R,6R,7aS)-5,5-dimethyl-2-(phenylsulfonyl)octahydro-3aH-4,6-methanoisoindol-3a-yl)methyl (4-iodophenyl)carbamate (37):

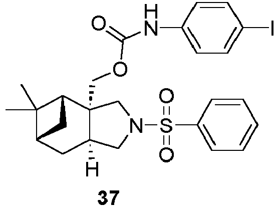

Following general procedure A2: From **Int-11** (38.5 mg, 0.1 mmol) and 1-iodo-4-isocyanatobenzene (29.4 mg, 1.2 equiv.). Purification by column chromatography (PE/EtOAc 20:1 to 10:1) afforded a colorless solid **37** (68 % yield).

**¹H NMR** (500 MHz, CDCl₃) δ 7.85 - 7.79 (m, 2H), 7.61 (d, J = 8.8 Hz, 2H), 7.57 - 7.51 (m, 3H), 7.14 - 7.06 (m, 2H), 6.17 (s, 1H), 4.01 (s, 2H), 3.14 - 3.05 (m, 3H), 2.98 (d, J = 9.8 Hz, 1H), 2.31 (dd, J = 6.8, 3.8 Hz, 2H), 2.27 - 2.18 (m, 1H), 1.90 (d, J = 6.6 Hz, 2H), 1.64 (dd, J = 8.8, 2.3 Hz, 1H), 1.36 (d, J = 10.5 Hz, 1H), 1.23 (s, 3H), 1.00 (s, 3H).

**¹³C NMR** (126 MHz, CDCl₃) δ 152.9, 138.8, 138.1, 137.5, 134.7, 133.0, 130.3, 129.0, 128.4, 120.6, 86.6, 70.4, 58.5, 56.9, 50.72, 46.8, 40.2, 39.1, 34.7, 34.4, 27.4, 27.3, 23.6.

**HRMS** (ESI) for C₂₅H₂₉IN₂O₄SNa: calculated 603.0790 [M+Na]⁺, found 603.0792.

[α]²⁰_{D} = -5.3° (CHCl₃, *c* = 0.5).

### ((3aS,4R,6R,7aS)-5,5-dimethyl-2-(naphthalen-2-ylsulfonyl)octahydro-3aH-4,6-methanoisoindol-3a-yl)methyl (4-iodophenyl)carbamate (38):

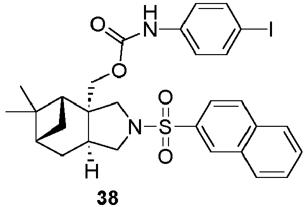

Following general procedure A2: From **Int-12** (38.5 mg, 0.1 mmol) and 1-iodo-4-isocyanatobenzene (29.4 mg, 1.2 equiv.). Purification by column chromatography (PE/EtOAc 20:1 to 10:1) afforded a colorless solid (65 % yield).

**¹H NMR** (500 MHz, Chloroform-*d*) δ 8.39 (s, 1H), 8.00 - 7.97 (m, 1H), 7.95 (d, *J* = 8.5 Hz, 1H), 7.86 - 7.79 (m, 2H), 7.62 (ddd, *J* = 7.0, 4.6, 1.8 Hz, 2H), 7.51 (d, *J* = 8.8 Hz, 2H), 6.80 (d, *J* = 8.6 Hz, 2H), 5.96 (s, 1H), 4.02 (d, *J* = 10.7 Hz, 1H), 3.95 (d, *J* = 10.8 Hz, 1H), 3.19 (d, *J* = 9.9 Hz, 1H), 3.17 - 3.14 (m, 2H), 3.07 (d, *J* = 9.8 Hz, 1H), 2.31 (dd, *J* = 4.9, 3.0 Hz, 2H), 2.26 - 2.17 (m, 1H), 1.89 (d, *J* = 6.2 Hz, 2H), 1.67 - 1.63 (m, 1H), 1.39 (d, *J* = 10.5 Hz, 1H), 1.22 (s, 3H), 0.99 (s, 3H).

**¹³C NMR** (126 MHz, CDCl₃) δ 152.8, 138.8, 137.9, 137.3, 134.9, 132.1, 132.1, 130.3, 129.6, 129.4, 129.1, 128.1, 127.8, 123.7, 120.5, 86.5, 70.4, 58.6, 56.9, 50.8, 46.9, 40.2, 39.1, 34.7, 34.5, 27.4, 27.4, 23.6.

**HRMS** (ESI) for C29H31IN2O4SNa: calculated 653.0947 [M+Na]⁺, found 653.0946.

[α]²⁰_{D} = -6.23 (CHCl₃, *c* = 1.0 ).

### ((3aS,4R,6R,7aS)-5,5-dimethyl-2-(o-tolylsulfonyl)octahydro-3aH-4,6-methanoisoindol-3a-yl)methyl (4-iodophenyl)carbamate (39)

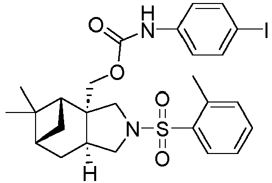

Following general procedure A2: From **Int-13** (52.4 mg, 0.15 mmol) and 1-iodo-4-isocyanatobenzene (66.2 mg, 1.2 equiv.). Purification by column chromatography (PE/EtOAc 20:1 to 10:1) afforded a colorless solid **39** (60 mg, 0.1 mmol, 67% yield).

**1H NMR** (700 MHz, CDCl₃) δ 7.93 (d, J = 7.0 Hz, 1H), 7.60 (d, J = 8.7 Hz, 2H), 7.44 (td, J = 7.5, 1.4 Hz, 1H), 7.32 (t, J = 7.5 Hz, 2H), 7.15 (d, J = 7.9 Hz, 2H), 6.69 (s, 1H), 4.15 (d, J = 10.6 Hz, 1H), 4.03 (d, J = 10.6 Hz, 1H), 3.41 (t, J = 8.3 Hz, 1H), 3.29 (d, J = 10.0 Hz, 1H), 3.08 - 3.01 (m, 2H), 2.67 (s, 3H), 2.35 (dd, J = 7.5, 3.8 Hz, 1H), 2.33 - 2.26 (m, 1H), 2.21 (dtd, J = 10.5, 6.3, 1.9 Hz, 1H), 1.93 - 1.87 (m, 2H), 1.59 - 1.54 (m, 1H), 1.30 (d, J = 10.5 Hz, 1H), 1.23 (s, 3H), 1.02 (s, 3H).

**¹³C NMR** (176 MHz, CDCl₃) δ 153.2, 138.5, 138.1, 137.7, 135.5, 133.1, 132.9, 130.4, 126.2, 120.7, 86.5, 70.6, 57.9, 56.3, 50.9, 46.7, 40.3, 39.1, 34.7, 34.3, 27.4, 27.3, 23.6, 21.0.

**HRMS** (ESI) for C₂₆H₃₁IN₂O₄SNa: calculated 617.0942 [M+Na]⁺, found 617.0942.

[α]²⁰_{D} = + 4.3° (CHCl₃, *c* = 1.0).

### ((3aS,4R,6R,7aS)-5,5-dimethyl-2-(m-tolylsulfonyl)octahydro-3aH-4,6-methanoisoindol-3a-yl)methyl (4-iodophenyl)carbamate (40):

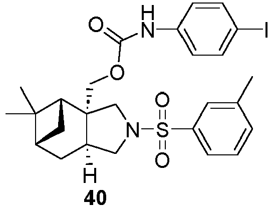

Following general procedure A2: From **Int-14** (52.4 mg, 0.15 mmol) and 1-iodo-4-isocyanatobenzene (29.4 mg, 1.2 equiv.). Purification by column chromatography (PE/EtOAc 20:1 to 10:1) afforded a colorless solid **40** (54 mg, 0.09 mmol, 60% yield). **1H NMR** (700 MHz, CDCl₃) δ 7.65 - 7.59 (m, 4H), 7.43 - 7.39 (m, 1H), 7.33 (d, J = 7.5 Hz, 1H), 7.09 (d, J = 8.2 Hz, 2H), 6.19 (s, 1H), 4.02 (s, 2H), 3.12 - 3.08 (m, 2H), 3.07 (dd, J = 9.3, 2.4 Hz, 1H), 2.99 (d, J = 9.9 Hz, 1H), 2.39 (s, 3H), 2.31 (ddt, J = 9.4, 4.9, 2.5 Hz, 2H), 2.26 - 2.21 (m, 1H), 1.94 - 1.88 (m, 2H), 1.64 (dd, J = 8.8, 2.6 Hz, 1H), 1.38 (d, J = 10.5 Hz, 1H), 1.23 (s, 3H), 1.00 (s, 3H).

**¹³C NMR** (176 MHz, CDCl₃) δ 152.9, 139.3, 138.1, 137.6, 134.7, 133.8, 128.8, 128.7, 125.5, 120.6, 86.7, 70.4, 58.5, 56.9, 50.8, 46.8, 40.2, 39.1, 34.7, 34.4, 27.4, 27.3, 23.6, 21.6.

**HRMS** (ESI) for C₂₆H₃₂IN₂O₄S: calculated 595.1122 [M+H]⁺, found 595.1114.

[α]²⁰_{D} = - 8.3° (CHCl₃, *c* = 1.0).

### ((3aS,4R,6R,7aS)-5,5-dimethyl-2-tosyloctahydro-3aH-4,6-methanoisoindol-3a-yl)methyl (4-iodophenyl)carbamate (41):

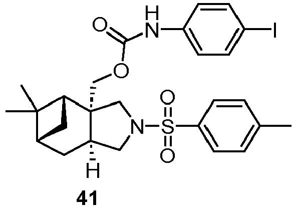

Following general procedure A2: From **Int-15** (104.8 mg, 0.3 mmol) and 1-iodo-4-isocyanatobenzene (88 mg, 1.2 equiv.). Purification by column chromatography (PE/EtOAc 20:1 to 10:1) afforded a colorless solid **40** (135 mg, 0.23 mmol, 76%yield).

**¹H NMR** (500 MHz, CDCl₃) δ 7.70 (d, *J* = 8.2 Hz, 2H), 7.61 (d, *J* = 8.8 Hz, 2H), 7.32 (d, *J* = 7.7 Hz, 2H), 7.10 (d, *J* = 8.3 Hz, 2H), 6.24 (s, 1H), 4.04 (d, *J* = 10.7 Hz, 1H), 3.98 (d, *J* = 10.7 Hz, 1H), 3.12 (dd, *J* = 9.2, 6.8 Hz, 1H), 3.09 (d, *J* = 9.8 Hz, 1H), 3.06 (dd, *J* = 9.0, 2.5 Hz, 1H), 2.98 (d, *J* = 9.9 Hz, 1H), 2.34 (s, 3H), 2.33 - 2.27 (m, 2H), 2.22 (dtd, *J* = 10.5, 6.3, 1.7 Hz, 1H), 1.94 - 1.90 (m, 1H), 1.89 (dt, *J* = 5.1, 2.7 Hz, 1H), 1.66 - 1.62 (m, 1H), 1.37 (d, *J* = 10.5 Hz, 1H), 1.23 (s, 3H), 1.00 (s, 3H).

**¹³C NMR** (126 MHz, CDCl₃) δ 153.0, 143.9, 138.1, 137.6, 132.0, 129.6, 128.4, 120.6, 86.7, 70.4, 58.4, 57.0, 50.7, 46.7, 40.2, 39.1, 34.7, 34.4, 27.4, 27.3, 23.6, 21.7. **HRMS** (ESI) for C₂₆H₃₂IN₂O₄S: calculated 595.1122 [M+H]⁺, found 595.1131.

[α]²⁰_{D} = - 5.3° (CHCl₃, *c* = 1.0).

### ((3aS,4R,6R,7aS)-2-((4-isopropylphenyl)sulfonyl)-5,5-dimethyloctahydro-3aH-4,6-methanoisoindol-3a-yl)methyl (4-iodophenyl)carbamate (43):

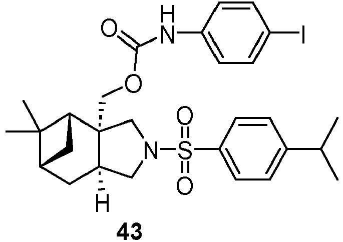

Following general procedure A2: From **Int-17** (56.63 mg, 0.15 mmol) and 1-iodo-4-isocyanatobenzene (66.2 mg, 1.2 equiv.). Purification by column chromatography (PE/EtOAc 20:1 to 10:1) afforded a colorless solid (66.3 mg, 0.11 mmol, 71 %yield).

**¹H NMR** (700 MHz, CDCl₃) δ 7.73 (d, J = 8.4 Hz, 2H), 7.59 (d, J = 8.7 Hz, 2H), 7.37 (d, J = 8.3 Hz, 2H), 7.12 (d, J = 8.2 Hz, 2H), 6.45 (s, 1H), 4.07 (d, J = 10.6 Hz, 1H), 4.00 (d, J = 10.6 Hz, 1H), 3.19 (dd, J = 9.3, 6.9 Hz, 1H), 3.07 (d, J = 9.9 Hz, 1H), 3.06 - 3.02 (m, 2H), 2.93 (p, J = 6.9 Hz, 1H), 2.33 - 2.28 (m, 1H), 2.28 - 2.24 (m, 1H), 2.20 (ddt, J = 10.6, 6.4, 3.2 Hz, 1H), 1.94 (dd, J = 6.5, 4.9 Hz, 1H), 1.88 (dt, J = 5.4, 2.7 Hz, 1H), 1.64 - 1.61 (m, 1H), 1.33 (d, J = 10.5 Hz, 1H), 1.24 (d, J = 6.9 Hz, 3H), 1.23 - 1.21 (d, J = 6.9 Hz, s, 6H), 1.00 (s, 3H).

**¹³C NMR** (176 MHz, CDCl₃) δ 154.6, 153.1, 138.1, 137.6, 132.6, 128.4, 127.2, 120.7, 86.6, 70.3, 58.3, 57.0, 50.7, 46.5, 40.2, 39.1, 34.5, 34.3, 34.2, 27.4, 27.1, 23.8, 23.7 23.5.

**HRMS** (ESI) for C₂₈H₃₇IN₂O₄SNa: calculated 623.1435 [M+Na]⁺, found 623.1443.[α]²⁰_{D} = -6.0° (CHCl₃, *c* = 1.0).

### ((3aS,4R,6R,7aS)-2-([1,1'-biphenyl]-4-ylsulfonyl)-5,5-dimethyloctahydro-3aH-4,6-methanoisoindol-3a-yl)methyl (4-iodophenyl)carbamate (44):

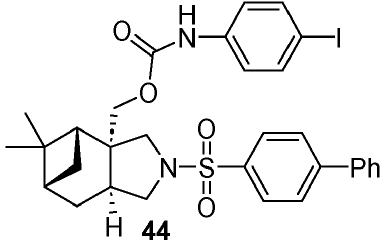

Following general procedure A2: From **Int-18** (61.7 mg, 0.15 mmol) and 1-iodo-4-isocyanatobenzene (66.2 mg, 1.2 equiv.). Purification by column chromatography (PE/EtOAc 20:1 to 10:1) afforded a colorless solid **44** (91 mg, 0.14mmol, 92 %yield). **¹H NMR** (700 MHz, CDCl₃) δ 7.88 (d, J = 8.4 Hz, 2H), 7.75 (d, J = 8.4 Hz, 2H), 7.54 (dd, J = 7.6, 2.0 Hz, 2H), 7.46 - 7.43 (m, 3H), 7.41 (d, J = 8.7 Hz, 2H), 6.93 (d, J = 8.2 Hz, 2H), 6.18 (s, 1H), 4.04 (d, J = 10.6 Hz, 1H), 4.00 (d, J = 10.8 Hz, 1H), 3.16 (d, J = 10.0 Hz, 1H), 3.15 - 3.12 (m, 2H), 3.02 (d, J = 10.0 Hz, 1H), 2.36 - 2.30 (m, 2H), 2.24 (dtd, J = 10.5, 6.2, 1.5 Hz, 1H), 1.95 - 1.89 (m, 2H), 1.70 - 1.63 (m, 1H), 1.42 (d, J = 10.5 Hz, 1H), 1.23 (s, 3H), 1.00 (s, 3H).

**¹³C NMR** (176 MHz, CDCl₃) δ 153.0, 145.8, 138.9, 138.0, 137.3, 133.4, 129.3, 128.9, 128.8, 127.5, 127.3, 120.5, 86.7, 70.5, 58.6, 57.0, 50.8, 46.9, 40.2, 39.1, 34.8, 34.5, 27.4, 27.4, 23.6.

**HRMS** (ESI) for C₃₁H₃₃IN₂O₄SNa: calculated 679.1103 [M+Na]⁺, found 679.1102 [α]²⁰_{D} = -6.0° (CHCl₃, *c* = 1.0).

### ((3aS,4R,6R,7aS)-2-((4-cyclohexylphenyl)sulfonyl)-5,5-dimethyloctahydro-3aH-4,6-methanoisoindol-3a-yl)methyl (4-iodophenyl)carbamate (45)

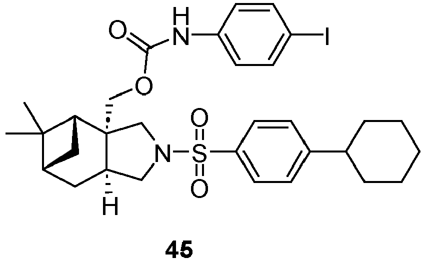

Following general procedure A2: From **Int-19** (41.8 mg, 0.1 mmol) and 1-iodo-4-isocyanatobenzene (29.4 mg, 1.2 equiv.). Purification by column chromatography (PE/EtOAc 20:1 to 10:1) afforded a colorless solid **45** (61 mg, 0.09 mg, 92 %yield).

**¹H NMR** (700 MHz, CD₂Cl₂) δ 7.69 (d, J = 8.3 Hz, 2H), 7.61 (d, J = 8.8 Hz, 2H), 7.36 (d, J = 8.2 Hz, 2H), 7.13 (d, J = 8.2 Hz, 2H), 6.41 (s, 1H), 4.06 (d, J = 10.6 Hz, 1H), 3.92 (d, J = 10.6 Hz, 1H), 3.13 (dd, J = 9.2, 7.1 Hz, 1H), 3.05 (d, J = 9.9 Hz, 1H), 3.03 (dd, J = 9.2, 3.0 Hz, 1H), 2.93 (d, J = 9.9 Hz, 1H), 2.52 - 2.46 (m, 1H), 2.32 (tdd, J = 12.7, 3.0, 1.9 Hz, 1H), 2.29 - 2.25 (m, 1H), 2.21 (dtd, J = 10.4, 6.3, 2.0 Hz, 1H), 1.95 (dd, J = 6.5, 5.0 Hz, 1H), 1.88 (dt, J = 5.4, 2.7 Hz, 1H), 1.83 - 1.77 (m, 3H), 1.76 - 1.71 (m, 2H), 1.61 (ddd, J = 13.1, 4.3, 2.8 Hz, 1H), 1.40 - 1.31 (m, 5H), 1.27 - 1.23 (m, 1H), 1.22 (s, 3H), 1.00 (s, 3H).

**¹³C NMR** (176 MHz, CD₂Cl₂) δ 154.1, 153.3, 138.3, 138.2, 132.4, 128.6, 127.7, 120.8, 86.5, 70.4, 58.7, 57.3, 50.8, 46.8, 44.8, 40.6, 39.3, 34.8, 34.6, 34.5, 34.3, 27.4, 27.4, 27.1, 27.0, 26.3, 23.5.

**HRMS** (ESI) for C₃₁H₃₉IN₂O₄SNa: calculated 685.1572 [M+Na]⁺, found 685.1568.

[α]²⁰_{D} = -4.3° (CHCl₃, *c* = 1.0).

### ((3aS,4R,6R,7aS)-5,5-dimethyl-2-((5,5,8,8-tetramethyl-5,6,7,8-tetrahydronaphthalen-2-yl)sulfonyl)octahydro-3aH-4,6-methanoisoindol-3a-yl)methyl (4-iodophenyl)carbamate) (46)

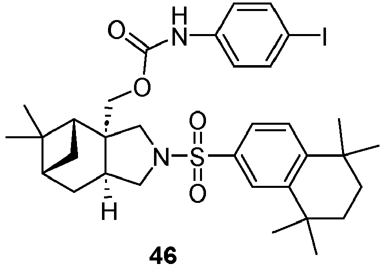

Following general procedure A2: From **Int-20** (66.85 mg, 0.15 mmol) and 1-iodo-4-isocyanatobenzene (45 mg, 0.18 mmol, 1.2 equiv.). Purification by column chromatography (PE/EtOAc 20:1 to 10:1) afforded a colorless solid **46** (81 mg, 78 %yield).

**¹H NMR** (700 MHz, CDCl₃) δ 7.71 (d, J = 2.1 Hz, 1H), 7.59 (d, J = 8.8 Hz, 2H), 7.53 (dd, J = 8.3, 2.0 Hz, 1H), 7.44 (d, J = 8.3 Hz, 1H), 7.13 (d, J = 7.7 Hz, 2H), 6.54 (s, 1H), 4.10 (d, J = 10.6 Hz, 1H), 3.97 (d, J = 10.6 Hz, 1H), 3.19 (dd, J = 9.4, 7.2 Hz, 1H), 3.05 (s, 2H), 2.99 (dd, J = 9.4, 3.4 Hz, 1H), 2.33 - 2.26 (m, 1H), 2.24 (tt, J = 6.9, 3.6 Hz, 1H), 2.18 (ddt, J = 8.2, 6.3, 3.1 Hz, 1H), 1.96 (dd, J = 6.5, 4.9 Hz, 1H), 1.88 (tt, J = 5.6, 3.0 Hz, 1H), 1.68 (d, J = 1.9 Hz, 4H), 1.61 (dd, J = 4.2, 2.9 Hz, 1H), 1.30 (d, J = 8.6 Hz, 4H), 1.29 (s, 3H), 1.27 (s, 8H), 1.26 (s, 8H), 1.22 (s, 3H), 1.00 (s, 3H). **¹³C NMR** (176 MHz, CDCl₃) δ 153.2, 150.7, 146.3, 138.1, 137.6, 132.2, 127.4, 126.6, 125.0, 120.7, 86.7, 70.3, 58.1, 57.1, 50.5, 46.4, 40.2, 39.2, 34.8, 34.7, 34.7, 34.7, 34.4, 34.1, 31.9, 31.8, 31.7, 27.4, 27.0, 23.5.

**HRMS** (ESI) for C₃₃H₄₃IN₂O₄SNa: calculated 691.2061 [M+Na]⁺, found 691.2071.

[α]²⁰_{D} = -12.3° (CHCl₃, *c* = 1).

### ((3aS,4R,6R,7aS)-5,5-dimethyl-2-((4-neopentylphenyl)sulfonyl)octahydro-3aH-4,6-methanoisoindol-3a-yl)methyl (4-iodophenyl)carbamate (47)

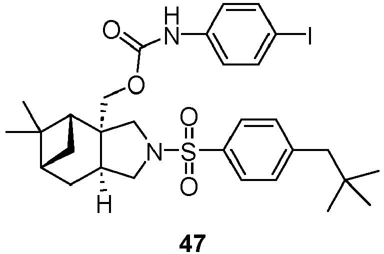

Following general procedure A2: From **Int-21** (60.84 mg, 0.15 mmol) and 1-iodo-4-isocyanatobenzene (45 mg, 0.18 mmol, 1.2 equiv.). Purification by column chromatography (PE/EtOAc 20:1 to 10:1) afforded a colorless solid **47** (80 mg, 0.12 mmol, 82 %yield).

**¹H NMR** (500 MHz, CDCl₃) δ 7.71 (d, J = 8.2 Hz, 2H), 7.60 (d, J = 8.7 Hz, 2H), 7.26 (d, J = 8.2 Hz, 2H), 7.10 (d, J = 8.2 Hz, 2H), 6.37 (s, 1H), 4.07 (d, J = 10.7 Hz, 1H), 3.99 (d, J = 10.6 Hz, 1H), 3.18 (dd, J = 9.2, 6.5 Hz, 1H), 3.10 - 2.99 (m, 3H), 2.45 (s, 2H), 2.33 - 2.27 (m, 2H), 2.23 - 2.16 (m, 1H), 1.92 (dd, J = 6.5, 5.0 Hz, 1H), 1.91 - 1.86 (m, 1H), 1.64 - 1.58 (m, 4H), 1.30 (d, J = 10.4 Hz, 1H), 1.22 (s, 3H), 1.00 (s, 3H), 0.87 (s, 9H).

**¹³C NMR** (126 MHz, CDCl₃) δ 153.0, 145.7, 138.1, 137.6, 132.4, 130.9, 127.7, 120.6, 86.6, 70.3, 58.4, 57.0, 50.7, 50.1, 46.6, 40.2, 39.1, 34.5, 34.2, 32.1, 29.4, 27.4, 27.1, 23.6.

**HRMS** (ESI) for C₃₀H₄₀IN₂O₄S: calculated 651.1748 [M+H]⁺, found 651.1759.

[α]²⁰_{D} = -4.3° (CHCl₃, *c* = 0.5).

### ((3aS,4R,6R,7aS)-5,5-dimethyl-2-((4-(tert-pentyl)phenyl)sulfonyl)octahydro-3aH-4,6-methanoisoindol-3a-yl)methyl (4-iodophenyl)carbamate (48)

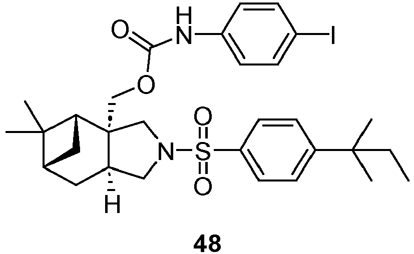

Following general procedure A2: From **Int-22** (60.8 mg, 0.15 mmol) and 1-iodo-4-isocyanatobenzene (45 mg, 1.2 equiv.). Purification by column chromatography (PE/EtOAc 20:1 to 10:1) afforded a colorless solid **48** (62.2 mg, 0.1 mmol, 64 %yield).

**¹H NMR** (500 MHz, CDCl₃) δ 7.74 (d, J = 8.5 Hz, 2H), 7.59 (d, J = 8.9 Hz, 2H), 7.48 (d, J = 8.5 Hz, 2H), 7.13 (d, J = 8.3 Hz, 2H), 6.53 (s, 1H), 4.09 (d, J = 10.7 Hz, 1H), 4.00 (d, J = 10.6 Hz, 1H), 3.25 (dd, J = 9.2, 6.8 Hz, 1H), 3.10 - 3.02 (m, 2H), 3.04 - 2.97 (m, 2H), 2.33 - 2.23 (m, 2H), 2.17 (dtd, J = 10.5, 6.3, 1.6 Hz, 1H), 1.94 (dd, J = 6.4, 4.9 Hz, 1H), 1.88 (td, J = 5.4, 2.3 Hz, 1H), 1.64 (q, J = 7.6 Hz, 2H), 1.59 (d, J = 3.0 Hz, 1H), 1.28 (s, 3H), 1.28 (s, 3H), 1.26 (s, 1H), 1.22 (s, 3H), 1.00 (s, 3H), 0.62 (J = 7.4 Hz, 3H).

**¹³C NMR** (126 MHz, CDCl₃) δ 155.5, 153.2, 138.1, 137.6, 132.1, 128.0, 126.7, 120.7, 93.1, 70.2, 58.3, 57.0, 50.6, 46.3, 40.1, 39.1, 38.6, 37.0, 34.4, 34.0, 28.3, 28.2, 27.4, 27.0, 23.5, 9.2.

**HRMS** (ESI) for C₃₀H₄₀IN₂O₄S: calculated 651.1748 [M+H]⁺, found 651.1757.

[α]²⁰_{D} = -3.3° (CHCl₃, *c* = 1.0).

### ((3aS,4R,6R,7aS)-5,5-dimethyl-2-((4-(trimethylsilyl)phenyl)sulfonyl)octahydro-3aH-4,6-methanoisoindol-3a-yl)methyl (4-iodophenyl)carbamate (50)

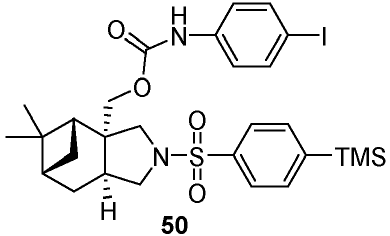

Following general procedure A2: From **Int-24** (61.15 mg, 0.15 mmol) and 1-iodo-4-isocyanatobenzene (73.5 mg, 1.2 equiv.). Purification by column chromatography (PE/EtOAc 20:1 to 10:1) afforded a colorless solid **50** (63mg, 0.10 mmol, 64 %yield). **¹H NMR** (700 MHz, CD₂Cl₂) δ 7.75 (d, J = 6.7 Hz, 2H), 7.70 (d, J = 6.6 Hz, 2H), 7.62 - 7.58 (m, 2H), 7.14 (d, J = 8.2 Hz, 2H), 6.53 (d, J = 6.9 Hz, 1H), 4.10 (d, J = 10.6 Hz, 1H), 3.93 (d, J = 10.6 Hz, 1H), 3.20 (t, J = 8.3 Hz, 1H), 3.08 - 2.98 (m, 3H), 2.35 - 2.29 (m, 1H), 2.25 (tt, J = 7.0, 3.7 Hz, 1H), 2.20 (dt, J = 11.4, 6.4 Hz, 1H), 1.98 (t, J = 5.8 Hz, 1H), 1.88 (q, J = 5.0, 4.3 Hz, 1H), 1.60 (d, J = 13.4 Hz, 1H), 1.29 (d, J = 10.4 Hz, 1H), 1.22 (s, 3H), 1.00 (s, 3H), 0.27 (s, 9H).

**¹³C NMR** (176 MHz, CD₂Cl₂) δ 153.4, 147.7 138.3 138.2, 135.6, 134.2, 127.2, 120.9, 86.5, 70.4, 58.5, 57.3, 50.9, 46.6, 40.6, 39.4, 34.7, 34.4, 27.4, 27.3, 23.5, -1.4. **HRMS** (ESI) for C₂₈H₃₇IN₂O₄SSiNa: calculated 675.1186 [M+Na]⁺, found 675.1176.

[α]²⁰_{D} = -2.3° (CHCl₃, *c* = 1.0).

### ((3aS,4R,6R,7aS)-2-((4-(tert-butyl)-2-methylphenyl)sulfonyl)-5,5-dimethyloctahydro-3aH-4,6-methanoisoindol-3a-yl)methyl (4-iodophenyl)carbamate (52)

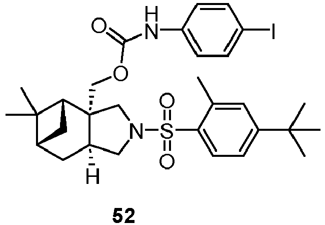

Following general procedure A2: From **Int-26** (60.84 mg, 0.15 mmol) and 1-iodo-4-isocyanatobenzene (45 mg, 0.18 mmol, 1.2 equiv.). Purification by column chromatography (PE/EtOAc 20:1 to 10:1) afforded a colorless solid **52** (69.6mg, 0.11 mmol, 71 %yield).

**1H NMR** (500 MHz, CD₂Cl₂) δ 7.85 (d, J = 8.7 Hz, 1H), 7.60 (d, J = 8.8 Hz, 2H), 7.33 - 7.28 (m, 2H), 7.18 (d, J = 8.4 Hz, 2H), 6.90 (s, 1H), 4.18 (d, J = 10.7 Hz, 1H), 4.05 (d, J = 10.6 Hz, 1H), 3.48 (t, J = 8.4 Hz, 1H), 3.37 (d, J = 10.0 Hz, 1H), 3.04 (d, J = 10.0 Hz, 1H), 3.00 (dd, J = 9.5, 3.5 Hz, 1H), 2.66 (s, 3H), 2.35 (dt, J = 7.3, 3.5 Hz, 1H), 2.34 - 2.26 (m, 1H), 2.21 (dtd, J = 10.4, 6.3, 1.9 Hz, 1H), 1.95 - 1.87 (m, 2H), 1.57 - 1.53 (m, 1H), 1.31 (s, 9H), 1.29 (d, J = 8.5 Hz, 1H), 1.23 (s, 3H), 1.03 (s, 3H).

**¹³C NMR** (176 MHz, CD₂Cl₂) δ 157.1, 153.5, 138.4, 138.3, 138.3, 133.0, 130.4, 130.3, 123.5, 120.8, 86.4, 70.6, 58.0, 56.6, 51.0, 46.7, 40.6, 39.3, 35.2, 34.8, 34.4, 31.1, 27.4, 27.4, 23.6, 21.2.

**HRMS** (ESI) for C₃₀H₃₉IN₂O₄SNa: calculated 651.1748 [M+Na]⁺, found 651.1752.

[α]²⁰_{D} = +10.7° (CHCl₃, *c* = 0.5).

### ((3aS,4R,6R,7aS)-2-((4-(tert-butyl)-2-ethylphenyl)sulfonyl)-5,5-dimethyloctahydro-3aH-4,6-methanoisoindol-3a-yl)methyl (4-iodophenyl)carbamate (53)

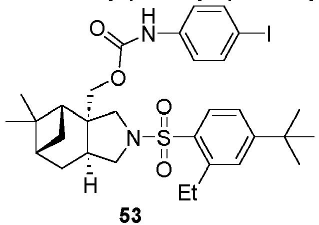

Following general procedure A2: From **Int-27** (42 mg, 0.1 mmol) and 1-iodo-4-isocyanatobenzene (29.4 mg, 0.12 mmol, 1.2 equiv.). Purification by column chromatography (PE/EtOAc 20:1 to 10:1) afforded a colorless solid **53** (78 % yield).

**¹H NMR** (500 MHz, CD₂Cl₂) δ 7.80 (d, J = 8.4 Hz, 1H), 7.61 (d, J = 8.7 Hz, 2H), 7.40 (d, J = 2.1 Hz, 1H), 7.33 (dd, J = 8.4, 2.1 Hz, 1H), 7.19 (d, J = 8.4 Hz, 2H), 6.87 (s, 1H), 4.09 (d, J = 2.1 Hz, 2H), 3.41 (dd, J = 9.4, 6.7 Hz, 1H), 3.26 (d, J = 10.0 Hz, 1H), 3.08 - 3.01 (m, 2H), 3.01 - 2.96 (m, 2H), 2.30 (dt, J = 9.5, 2.9 Hz, 2H), 2.20 (dtd, J = 10.4, 6.2, 1.5 Hz, 1H), 1.98 - 1.93 (m, 1H), 1.91 - 1.86 (m, 1H), 1.55 (d, J = 2.7 Hz, 1H), 1.31 (s, 8H), 1.29 (s, 3H), 1.31 - 1.24 (m, 4H), 1.22 (s, 3H), 1.02 (s, 3H).

**¹³C NMR** (126 MHz, CD₂Cl₂) δ 157.2, 153.5, 144.6 138.4, 138.3, 132.5, 130.5, 128.6, 123.4, 120.9, 86.6, 70.6, 58.1, 56.6, 51.0, 46.7, 40.6, 39.3, 35.3, 34.9, 34.3, 31.2, 27.4, 27.3, 27.00, 23.6, 16.3.

**HRMS** (ESI) for C₃₁H₄₁IN₂O₄SNa: calculated 687.1729 [M+Na]⁺, found 687.1727.

[α]²⁰_{D} = -8.3° (CHCl₃, *c* = 1).

### ((3aS,4R,6R,7aS)-2-((4-(tert-butyl)-2-methoxyphenyl)sulfonyl)-5,5-dimethyloctahydro-3aH-4,6-methanoisoindol-3a-yl)methyl (4-iodophenyl)carbamate (55)

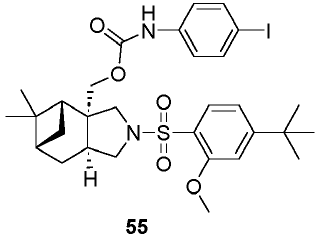

Following general procedure A2: From **Int-29** (39.4 mg, 0.1 mmol) and 1-iodo-4-isocyanatobenzene (29.4 mg, 1.2 equiv.). Purification by column chromatography (PE/EtOAc 20:1 to 10:1) afforded a colorless solid **55** (60 %yield).

**¹H NMR** (500 MHz, CDCl₃) δ 7.81 (d, J = 8.2 Hz, 1H), 7.59 (d, J = 8.8 Hz, 2H), 7.15 (d, J = 8.3 Hz, 2H), 7.04 (dd, J = 8.3, 1.7 Hz, 1H), 6.98 (d, J = 1.8 Hz, 1H), 6.71 (s, 1H), 4.11 (s, 2H), 3.91 (s, 3H), 3.55 (dd, J = 9.8, 6.3 Hz, 1H), 3.35 (d, J = 10.2 Hz, 1H), 3.18 (d, J = 10.2 Hz, 1H), 3.15 (dd, J = 10.0, 2.9 Hz, 1H), 2.29 (dd, J = 6.0, 3.3 Hz, 2H), 2.21 (dtd, J = 10.4, 6.2, 1.5 Hz, 1H), 1.96 (dd, J = 6.5, 5.0 Hz, 1H), 1.90 (dd, J = 5.5, 3.0 Hz, 1H), 1.64 - 1.60 (m, 1H), 1.39 (d, J = 10.5 Hz, 1H).

**¹³C NMR** (126 MHz, CDCl₃) δ 159.0, 157.0, 153.3, 138.0, 137.6, 131.4, 123.5, 120.5, 117.6, 109.7, 86.3, 70.4, 57.9, 56.5, 56.0, 50.7, 46.3, 40.1, 39.0, 35.4, 34.7, 33.9, 31.1, 27.3, 26.9, 23.5.

**HRMS** (ESI) for C₃₀H₃₉IN₂O₅SNa: calculated 689.1522 [M+Na]⁺, found 689.1537.

[α]²⁰_{D} = -12.1° (CHCl₃, *c* = 0.5).

### ((3aS,4R,6R,7aS)-2-((2-bromo-4-(tert-butyl)phenyl)sulfonyl)-5,5-dimethyloctahydro-3aH-4,6-methanoisoindol-3a-yl)methyl (4-iodophenyl)carbamate (56)

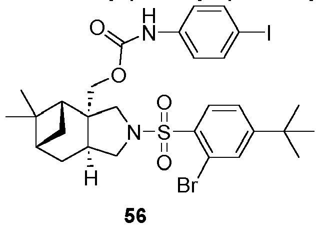

Following general procedure A2: From **Int-28** (47 mg, 0.1 mmol) and 1-iodo-4-isocyanatobenzene (29.4 mg, 1.2 equiv.). Purification by column chromatography (PE/EtOAc 20:1 to 10:1) afforded a colorless solid **56** (47 mg, 0.07 mmol, 66 %yield). **¹H NMR** (600 MHz, CDCl₃) δ 8.02 (d, J = 8.3 Hz, 1H), 7.72 (d, J = 1.9 Hz, 1H), 7.59 (d, J = 8.8 Hz, 2H), 7.42 (dd, J = 8.3, 1.9 Hz, 1H), 7.18 (d, J = 8.2 Hz, 2H), 6.97 (s, 1H), 4.24 (d, J = 10.6 Hz, 1H), 4.05 (d, J = 10.6 Hz, 1H), 3.76 - 3.66 (m, 1H), 3.49 (d, J = 10.3 Hz, 1H), 3.19 (d, J = 10.3 Hz, 1H), 3.14 (dd, J = 10.0, 4.4 Hz, 1H), 2.41 (dd, J = 11.9, 6.5 Hz, 1H), 2.29 (dt, J = 13.5, 2.5 Hz, 1H), 2.26 - 2.19 (m, 1H), 1.92 (dd, J = 6.2, 2.6 Hz, 2H), 1.64 (ddd, J = 13.5, 4.1, 2.5 Hz, 1H), 1.36 (d, J = 10.5 Hz, 1H), 1.31 (s, 10H), 1.24 (s, 3H), 1.03 (s, 3H).

**¹³C NMR** (151 MHz, CDCl₃) δ 158.2, 153.4, 138.1, 137.9, 135.0, 133.0, 132.2, 124.8, 120.7, 120.7, 86.4, 70.8, 58.5, 56.8, 51.2, 46.5, 40.3, 39.0, 35.3, 34.8, 33.7, 31.0, 27.4, 27.3, 23.7.

**HRMS** (ESI) for C₂₉H₃₆BrIN₂O₄SNa: calculated 737.0522 [M+Na]⁺, found 737.0516.

[α]²⁰_{D} = -36.1° (CHCl₃, c = 0.5).

### ((3aS,4R,6R,7aS)-2-((4-(tert-butyl)-2-iodophenyl)sulfonyl)-5,5-dimethyloctahydro-3aH-4,6-methanoisoindol-3a-yl)methyl (4-iodophenyl)carbamate (57)

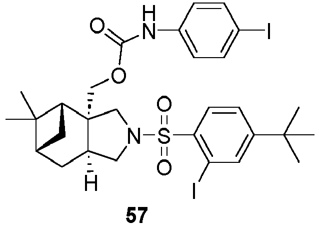

Following general procedure A2: From **Int-30** (51.75 mg, 0.1 mmol) and 1-iodo-4-isocyanatobenzene (29.4 mg, 1.2 equiv.). Purification by column chromatography (PE/EtOAc 20:1 to 10:1) afforded a colorless solid **57** (32 mg, 42 % yield).

**¹H NMR** (600 MHz, CDCl₃) δ 8.07 (d, J = 1.9 Hz, 1H), 8.04 (d, J = 8.3 Hz, 1H), 7.60 (d, J = 8.8 Hz, 2H), 7.47 (dd, J = 8.3, 1.9 Hz, 1H), 7.19 (d, J = 8.2 Hz, 2H), 6.99 (s, 1H), 4.28 (d, J = 10.5 Hz, 1H), 4.05 (d, J = 10.5 Hz, 1H), 3.70 (t, J = 8.9 Hz, 1H), 3.54 (d, J = 10.3 Hz, 1H), 3.20 (d, J = 10.4 Hz, 1H), 3.11 (dd, J = 10.0, 4.7 Hz, 1H), 2.43 (d, J = 9.9 Hz, 1H), 2.27 (t, J = 10.8 Hz, 1H), 2.25 (dd, J = 8.3, 2.6 Hz, 1H), 1.93 (d, J = 6.1 Hz, 2H), 1.71 - 1.65 (m, 1H), 1.40 (d, J = 10.6 Hz, 1H), 1.31 (s, 9H), 1.24 (s, 3H), 1.04 (s, 3H).

**¹³C NMR** (151 MHz, CDCl₃) δ 157.9, 153.5, 140.5, 138.1, 138.0, 131.7, 125.6, 120.7, 93.3, 86.4, 71.0, 58.6, 57.2, 51.4, 46.6, 40.5, 39.0, 35.1, 34.9, 33.7, 31.1, 27.6, 27.5, 23.8.

**HRMS** (ESI) for C₂₉H₃₆I₂N₂O₄SNa: calculated 785.0383 [M+Na]⁺, found 785.0385 [α]²⁰_{D} = -80.0° (CHCl₃, *c* = 0.5).

### ((3aS,4R,6R,7aS)-2-((4-(1-(2-methoxyethoxy)-2-methylpropan-2-yl)phenyl)sulfonyl)-5,5-dimethyloctahydro-3aH-4,6-methanoisoindol-3a-yl)methyl (4-iodophenyl)carbamate (58)

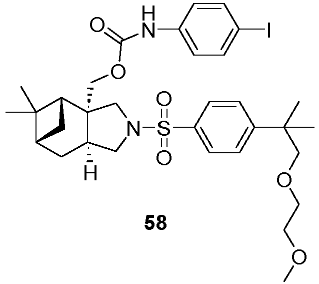

Following general procedure A2: From **Int-31** (46.57 mg, 0.1 mmol) and 1-iodo-4-isocyanatobenzene (29.4 mg, 1.2 equiv.). Purification by column chromatography (PE/EtOAc 20:1 to 10:1) afforded a colorless solid **58** (75 % yield).

**¹H NMR** (700 MHz, CDCl₃) δ 7.71 (d, J = 8.1 Hz, 2H), 7.60 (d, J = 7.2 Hz, 2H), 7.58 - 7.55 (m, 2H), 7.15 (d, J = 8.2 Hz, 2H), 6.67 (s, 1H), 4.07 (d, J = 10.8 Hz, 1H), 3.98 (d, J = 10.7 Hz, 1H), 3.52 (d, J = 9.2 Hz, 1H), 3.50 - 3.46 (m, 3H), 3.42 (t, J = 4.8 Hz, 2H), 3.26 (d, J = 1.4 Hz, 3H), 3.15 (t, J = 8.2 Hz, 1H), 3.04 (d, J = 9.9 Hz, 1H), 3.00 (dd, J = 9.5, 3.6 Hz, 2H), 2.32 (t, J = 11.9 Hz, 1H), 2.28 (dd, J = 7.3, 3.7 Hz, 1H), 2.20 (dt, J = 11.6, 6.5 Hz, 1H), 1.96 (t, J = 5.8 Hz, 1H), 1.87 (d, J = 5.7 Hz, 1H), 1.62 - 1.59 (m, 1H), 1.33 (s, 1H), 1.31 (s, 3H), 1.30 (s, 3H), 1.22 (s, 3H), 1.01 (s, 3H).

**¹³C NMR** (176 MHz, CDCl₃) δ 153.9, 153.5, 138.3, 138.3, 132.7, 128.1, 127.3, 121.1, 86.4, 80.9, 72.2, 71.3, 70.3, 59.0, 58.6, 57.2, 51.0, 46.8, 40.6, 40.0, 39.4, 34.7, 34.5, 27.4, 27.3, 26.1, 23.5.

**HRMS** (ESI) for C₃₂H₄₃IN₂O₆SNa: calculated: 733.1784 [M+Na]⁺, found 733.1777.

[α]²⁰_{D} = +12.0° (CHCl₃, *c* = 0.5).

### ((3aS,4R,6R,7aS)-5,5-dimethyl-2-((4-(2-methyl-1-morpholino-1-oxopropan-2-yl)phenyl)sulfonyl)octahydro-3aH-4,6-methanoisoindol-3a-yl)methyl (4-iodophenyl)carbamate (59)

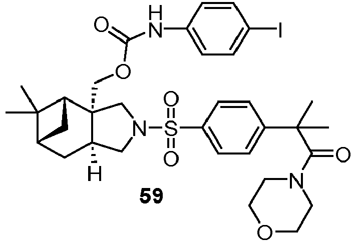

Following general procedure A2: From **Int-32** (73.6 mg, 0.15 mmol) and 1-iodo-4-isocyanatobenzene (45 mg, 0.18 mmol, 1.2 equiv.). Purification by column chromatography (PE/EtOAc 20:1 to 10:1) afforded a colorless solid **59** (73.5 mg, 67 %yield).

**¹H NMR** (400 MHz, CD₂Cl₂) δ 7.77 (d, J = 8.5 Hz, 2H), 7.61 (d, J = 8.8 Hz, 2H), 7.42 (d, J = 8.5 Hz, 2H), 7.16 (d, J = 8.4 Hz, 2H), 6.74 (s, 1H), 4.16 (d, J = 10.8 Hz, 1H), 3.89 (d, J = 10.7 Hz, 1H), 3.64 (d, J = 3.1 Hz, 1H), 3.35 (s, 4H), 3.24 (dd, J = 9.4, 7.2 Hz, 2H), 2.99 (d, J = 1.9 Hz, 3H), 2.33 - 2.27 (m, 1H), 2.24 (dq, J = 6.9, 3.5 Hz, 1H), 2.18 (dt, J = 10.6, 5.9 Hz, 1H), 2.03 - 1.98 (m, 1H), 1.88 (q, J = 3.2, 2.8 Hz, 1H), 1.61 - 1.58 (m, 1H), 1.51 (s, 6H), 1.25 (d, J = 13.3 Hz, 1H), 1.22 (s, 3H), 1.01 (s, 3H).

**¹³C NMR** (101 MHz, CD₂Cl₂) δ 173.9, 156.8, 152.2, 139.3, 133.6, 131.7, 129.1, 126.1, 120.9, 85.7, 70.1, 58.4, 57.4, 50.9, 47.4, 46.4, 40.5, 39.4, 34.6, 34.2, 28.4, 28.2, 27.4, 27.2, 23.4.

**HRMS** (ESI) for C₃₃H₄₂IN₃O₆SNa: calculated: 758.1737 [M+Na]⁺, found 758.1735.

[α]²⁰_{D} = -4.0° (CHCl₃, *c* = 0.5).

### ((3aS,4R,6R,7aS)-2-((4-(1-(dimethylamino)-2-methyl-1-oxopropan-2-yl)phenyl)sulfonyl)-5,5-dimethyloctahydro-3aH-4,6-methanoisoindol-3a-yl)methyl (4-iodophenyl)carbamate (60)

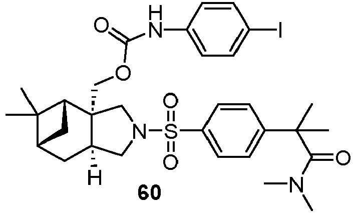

Following general procedure A2: From **Int-33** (44.9 mg, 0.1 mmol) and 1-iodo-4-isocyanatobenzene (29.4 mg, 0.12 mmol, 1.2 equiv.). Purification by column chromatography (PE/EtOAc 20:1 to 10:1) afforded a colorless solid **60** (68 % yield).

**¹H NMR** (700 MHz, CDCl₃) δ 7.78 (d, J = 8.5 Hz, 2H), 7.60 (d, J = 8.8 Hz, 2H), 7.39 (d, J = 8.5 Hz, 2H), 7.14 (d, J = 8.2 Hz, 2H), 6.59 (s, 1H), 4.13 (d, J = 10.6 Hz, 1H), 3.96 (d, J = 10.6 Hz, 1H), 3.29 (dd, J = 9.4, 7.1 Hz, 1H), 3.08 (d, J = 9.9 Hz, 1H), 3.02 (d, J = 9.9 Hz, 1H), 3.00 - 2.98 (m, 1H), 2.92 (s, 3H), 2.42 (s, 3H), 2.33 - 2.28 (m, 1H), 2.26 (ddt, J = 10.5, 6.9, 3.7 Hz, 1H), 2.19 - 2.14 (m, 1H), 1.97 (dd, J = 6.5, 4.9 Hz, 1H), 1.88 (dq, J = 5.4, 2.6 Hz, 1H), 1.59 (dt, J = 12.9, 3.4 Hz, 1H), 1.54 (s, 3H), 1.53 (s, 3H), 1.24 (d, J = 17.2 Hz, 1H), 1.22 (s, 3H), 1.01 (s, 3H).

**¹³C NMR** (176 MHz, CDCl₃) δ 175.0, 153.2, 152.3, 138.1, 137.6, 133.4, 128.9, 125.6, 120.6, 86.7, 70.1, 58.3, 57.1, 50.7, 47.4, 46.2, 40.1, 39.1, 38.2, 37.3, 34.4, 33.9, 28.4, 28.0, 27.4, 27.0, 23.5.

**HRMS** (ESI) for C₃₁H₄₀IN₃O₅S: calculated: 716.1631 [M+Na]⁺, found 716.1630.

[α]²⁰_{D} = -8.1° (CHCl₃, *c* = 0.5).

### ((3aS,4R,6R,7aS)-2-((4-(1-(dimethylamino)-2-methylpropan-2-yl)phenyl)sulfonyl)-5,5-dimethyloctahydro-3aH-4,6-methanoisoindol-3a-yl)methyl (4-iodophenyl)carbamate (61)

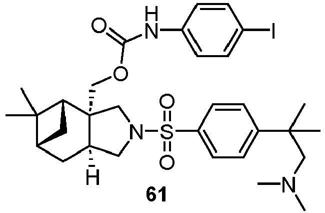

A solution of **Int-33** (89.7 mg, 0.2 mmol) in THF (3 mL) was treated dropwise with lithium aluminum hydride (8 mg) while the mixture was stirred under a nitrogen atmosphere. The reaction was stirred for 5 h and then quenched with water (1 mL). The reaction was filtered through a glass fiber pad, and the filtrate was evaporated. The residue was partitioned between 2 N aqueous NaOH and ethyl acetate. The organic phase was dried (Na₂SO₄), evaporated, and afforded crude oil, which was used in next step without further purification (54 mg, 0.12 mmol, 62%yield). Following general procedure A2: From crude oil obtained from above step (52.16 mg, 0.12 mmol) and 1-iodo-4-isocyanatobenzene (35.3 mg, 1.2 equiv.). Purification by column chromatography (PE/EtOAc 15:1 to 10:1 to 5:1) afforded a colorless solid **61** (58 %yield).

**¹H NMR** (500 MHz, CDCl₃) δ 7.73 (d, J = 8.5 Hz, 2H), 7.60 (d, J = 8.7 Hz, 2H), 7.56 (d, J = 8.5 Hz, 2H), 7.14 (d, J = 8.2 Hz, 2H), 6.54 (s, 1H), 4.08 (d, J = 10.7 Hz, 1H), 3.99 (d, J = 10.7 Hz, 1H), 3.26 - 3.19 (m, 1H), 3.08 - 3.01 (m, 2H), 3.01 - 2.96 (m, 1H), 2.46 (d, J = 6.7 Hz, 2H), 2.32 - 2.23 (m, 2H), 2.16 (ddt, J = 7.8, 6.2, 3.2 Hz, 1H), 2.00 (s, 6H), 1.93 (dd, J = 6.4, 4.9 Hz, 1H), 1.87 (dd, J = 5.5, 3.2 Hz, 1H), 1.60 - 1.56 (m, 1H), 1.33 (s, 3H), 1.32 (s, 3H), 1.28 (d, J = 4.8 Hz, 1H), 1.21 (s, 3H), 1.01 (s, 3H). **¹³C NMR** (126 MHz, CDCl₃) δ 154.7, 153.2, 138.1, 137.7, 132.2, 127.9, 127.0, 120.8, 86.7, 72.6, 70.2, 58.3, 57.0, 50.7, 48.3, 46.4, 40.2, 40.0, 39.1, 34.4, 34.0, 27.4, 27.3, 27.1, 27.0, 23.5.

**HRMS** (ESI) for C31H42IN3O4SNa: calculated: 680.2013 [M+Na]⁺, found 680.2012.

[α]²⁰_{D} = 16.0° (CHCl₃, c = 0.5).

### Ethyl 2-(4-(((3aS,4R,6R,7aS)-3a-((((4-iodophenyl)carbamoyl)oxy)methyl)-5,5-dimethyloctahydro-2H-4,6-methanoisoindol-2-yl)sulfonyl)phenyl)-2-methylpropanoate (64)

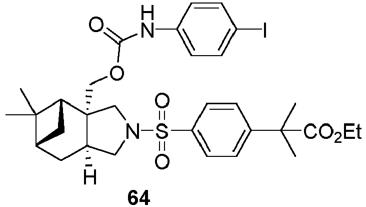

**¹H NMR** (500 MHz, CDCl₃) δ 7.77 (d, J = 8.5 Hz, 2H), 7.59 (d, J = 8.8 Hz, 2H), 7.51 (d, J = 8.6 Hz, 2H), 7.13 (d, J = 8.3 Hz, 2H), 6.58 (s, 1H), 4.16 - 4.06 (m, 3H), 3.99 (d, J = 10.9 Hz, 1H), 3.23 - 3.12 (m, 1H), 3.09 - 3.02 (m, 3H), 2.36 - 2.26 (m, 2H), 2.20 (dtd, J = 10.6, 6.4, 1.5 Hz, 1H), 1.92 (dd, J = 6.5, 5.0 Hz, 1H), 1.89 (dq, J = 5.4, 2.6 Hz, 1H), 1.60 (s, 1H), 1.59 (s, 3H), 1.59 (s, 3H), 1.33 (d, J = 10.5 Hz, 1H), 1.22 (s, 3H), 1.17 (t, J = 7.1 Hz, 3H), 1.02 (s, 3H).

**¹³C NMR** (126 MHz, CDCl₃) δ 176.0, 153.2, 150.1, 137.9, 137.5, 133.4, 128.1, 126.5, 120.9, 86.6, 70.1, 61.3, 58.2, 56.8, 50.7, 46.8, 46.5, 40.1, 39.0, 34.2, 34.1, 27.3, 27.0, 26.4, 26.2, 23.4, 14.0.

**HRMS** (ESI) for C₃₁H₃₉IN₂O₆SNa: calculated 695.1657 [M+Na]⁺, found 695.1646.

[α]²⁰_{D} = +8.1° (CHCl₃, c = 1.0).

### 2-(4-(((3aS,4R,6R,7aS)-3a-((((4-iodophenyl)carbamoyl)oxy)methyl)-5,5-dimethyloctahydro-2H-4,6-methanoisoindol-2-yl)sulfonyl)phenyl)-2-methylpropanoic acid (65)

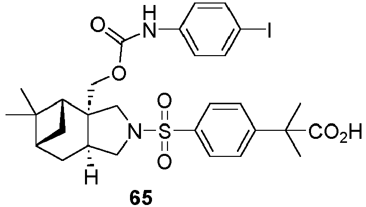

To a solution of **Int-34** (0.24 mmol, 1.0 equiv.) in ethanol (0.1M), cooled in an ice bath, a solution of sodium hydroxide (32 mg, 0.24 mmol, 3 equiv.) in ethanol (0.1 M) was added dropwise. The resulting mixture was refluxed for 2-6 h, and then the solution was acidified with 2 N HCl to pH = 2. Extraction of the resulting mixture with diethyl ether, drying of the combined organic extracts over magnesium sulfate and evaporation of the solvent afforded carboxylic acid without purification for next step. Following general procedure A3: From the crude carboxylic acid (63.2 mg, 0.15 mmol) and 1-iodo-4-isocyanatobenzene (44.1 mg, 0.18 mmol, 1.2 eq). Purification by column chromatography (DCM : MeOH=50:1 to 20:1) afforded a colorless solid **65** (45.6mg, 0.07 mmol, two steps, totally 46% yield).

**¹H NMR** (400 MHz, CD₂Cl₂) δ 7.76 (d, J = 8.5 Hz, 2H), 7.60 (d, J = 8.8 Hz, 2H), 7.57 (d, J = 8.5 Hz, 2H), 7.14 (d, J = 8.3 Hz, 2H), 6.69 (s, 1H), 4.06 (d, J = 10.8 Hz, 1H), 3.96 (d, J = 10.8 Hz, 1H), 3.17 (dd, J = 9.3, 6.9 Hz, 1H), 3.06 - 2.97 (m, 3H), 2.37 - 2.16 (m, 3H), 2.01 - 1.96 (m, 1H), 1.88 (dq, J = 5.4, 2.6 Hz, 1H), 1.61 (m, 1H), 1.60 (s, 6H), 1.28 (d, J = 10.4 Hz, 1H),1.22 (s, 3H), 1.00 (s, 3H)

**¹³C NMR** (101 MHz, CD₂Cl₂) δ 179.9, 152.7, 149.7, 138.3, 138.3, 134.0, 128.5, 127.1, 121.1, 86.6, 70.3, 58.6, 57.3, 50.9, 46.9, 46.6, 40.5, 39.3, 34.6, 34.3, 27.4, 27.3, 26.3, 23.5.

**HRMS** (ESI) for C₂₉H₃₅IN₂O₆S Na: calculated: 689.1158 [M+Na]⁺, found 689.1155.

[α]²⁰_{D} = +6.0° (CHCl₃, *c* = 1.0).

### Methyl 2-(4-(((3aS,4R,6R,7aS)-3a-((((4-iodophenyl)carbamoyl)oxy)methyl)-5,5-dimethyloctahydro-2H-4,6-methanoisoindol-2-yl)sulfonyl)-3-methoxyphenyl)-2-methylpropanoate (66)

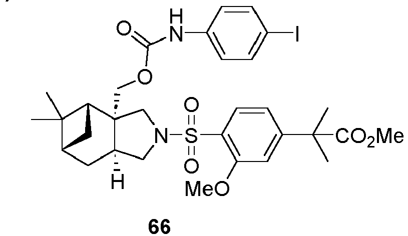

Following general procedure A2: From **Int-35** (67.7 mg, 0.15 mmol) and 1-iodo-4-isocyanatobenzene (73.5 mg, 1.5 equiv.). Purification by column chromatography (PE/EtOAc 20:1 to 10:1) afforded a colorless solid **66** (78 % yield).

**¹H NMR** (500 MHz, CDCl₃) δ 7.90 (d, J = 8.9 Hz, 1H), 7.60 (d, J = 8.7 Hz, 2H), 7.20 (d, J = 8.3 Hz, 2H), 7.07 (d, J = 2.6 Hz, 1H), 7.01 (s, 1H), 6.85 (dd, J = 8.9, 2.6 Hz, 1H), 4.22 (d, J = 10.6 Hz, 1H), 4.02 (d, J = 10.6 Hz, 1H), 3.84 (s, 3H), 3.65 (s, 3H), 3.55 (t, J = 8.6 Hz, 1H), 3.40 (d, J = 10.1 Hz, 1H), 3.10 (d, J = 10.1 Hz, 1H), 3.04 (dd, J = 9.7, 3.8 Hz, 1H), 2.37 (tt, J = 7.8, 4.1 Hz, 1H), 2.30 (dt, J = 13.3, 2.5 Hz, 1H), 2.27 - 2.21 (m, 1H), 1.92 (dd, J = 7.3, 2.5 Hz, 2H), 1.70 (s, 6H), 1.57 - 1.53 (m, 1H), 1.34 (d, J = 10.5 Hz, 1H), 1.24 (s, 3H), 1.03 (s, 3H).

**¹³C NMR** (126 MHz, CDCl₃) δ 177.2, 162.6, 153.4, 146.7, 138.0, 137.9, 133.5, 129.5, 120.7, 115.2, 110.7, 86.4, 70.8, 58.1, 56.6, 55.6, 52.2, 50.9, 47.3, 46.8, 40.3, 39.1, 34.7, 34.1, 28.6, 28.6, 27.4, 27.4, 23.7.

**HRMS** (ESI) for C₃₁H₃₉IN₂O₇SNa: calculated: 711.1595 [M+Na]⁺, found 711.1607.

[α]²⁰_{D} = -30.0° (CHCl₃, *c* = 1.0).

### 2-(4-(((3aS,4R,6R,7aS)-3a-((((4-iodophenyl)carbamoyl)oxy)methyl)-5,5-dimethyloctahydro-2H-4,6-methanoisoindol-2-yl)sulfonyl)-3-methoxyphenyl)-2-methylpropanoic acid (67)

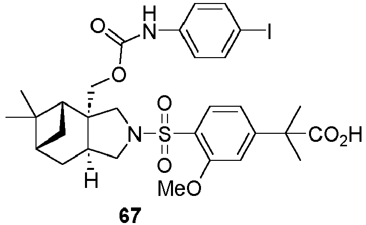

To a solution of **Int-35** (60.5mg, 0.13 mmol, 1.0 equiv.) in ethanol (0.1M), cooled in an ice bath, a solution of sodium hydroxide (17.3 mg, 0.43 mmol, 3 equiv.) in ethanol (0.1 M) was added dropwise. The resulting mixture was refluxed for 2-6 h, and then the solution was acidified with 2 N HCl to pH = 2. Extraction of the resulting mixture with diethyl ether, drying of the combined organic extracts over magnesium sulfate and evaporation of the solvent afforded carboxylic acid without purification for next step.

Following general procedure A3: From the crude carboxylic acid ( 45.2 mg, 0.1mmol) and 1-iodo-4-isocyanatobenzene (29.4 mg, 0.12 mmol, 1.2 eq). Purification by column chromatography (DCM : MeOH=50:1 to 20:1) afforded a colorless solid **67** (50 mg, 0.07 mmol, two steps, totally 72 % yield).

**¹H NMR** (500 MHz, CD₂Cl₂) δ 7.81 (d, J = 8.2 Hz, 1H), 7.60 (d, J = 8.8 Hz, 2H), 7.16 (d, J = 8.6 Hz, 2H), 7.06 (dd, J = 8.2, 1.7 Hz, 1H), 7.01 (d, J = 1.8 Hz, 1H), 6.84 (s, 1H), 4.12 (d, J = 10.6 Hz, 1H), 4.01 (d, J = 10.7 Hz, 1H), 3.89 (s, 3H), 3.50 (dd, J = 9.9, 6.8 Hz, 1H), 3.27 (d, J = 10.3 Hz, 1H), 3.15 (d, J = 10.4 Hz, 1H), 3.14 - 3.10 (m, 1H), 2.36 - 2.31 (m, 1H), 2.28 (td, J = 6.6, 3.1 Hz, 1H), 2.25 - 2.19 (m, 1H), 1.99 - 1.97 (m, 1H), 1.88 (dt, J = 5.4, 2.7 Hz, 1H), 1.61 (d, J = 3.5 Hz, 1H), 1.59 (s, 6H), 1.34 (d, J = 10.4 Hz, 1H), 1.23 (s, 5H), 1.02 (s, 3H).

**¹³C NMR** (126 MHz, CD₂Cl₂) δ 170.5, 157.5, 151.8, 145.4, 138.4, 138.3, 131.9, 125.3, 120.9, 118.3, 110.7, 87.3, 70.5, 60.7, 58.3, 56.9, 56.3, 51.1, 46.6, 40.6, 39.4, 35.0, 34.3, 30.1, 27.4, 27.2, 26.4, 26.4, 23.5.

**HRMS** (ESI) for C₃₀H₃₇IN₂O₇SNa: calculated: 719.1264 [M+Na]⁺, found 719.1261.

[α]²⁰_{D} = -12.3°(CHCl₃, c = 0.5).

### Methyl 2-(3-chloro-4-(((3aS,4R,6R,7aS)-3a-((((4-iodophenyl)carbamoyl)oxy) methyl)-5,5-dimethyloctahydro-2H-4,6-methanoisoindol-2-yl)sulfonyl)phenyl)-2-methylpropanoate (68)

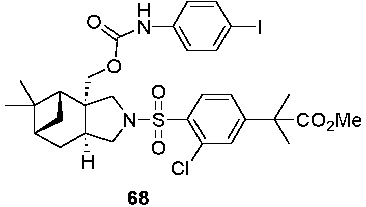

Following general procedure A2: From **Int-36** (14.1 mg, 0.03 mmol) and 1-iodo-4-isocyanatobenzene (12 mg, 0.05mmol, 1.2 equiv.). Purification by column chromatography (PE/EtOAc 20:1 to 10:1) afforded a colorless solid **68** (10 mg, 0.01 mmol, 47 % yield).

**¹H NMR** (600 MHz, CDCl₃) δ 8.01 (d, J = 8.3 Hz, 1H), 7.60 (d, J = 8.7 Hz, 2H), 7.48 (d, J = 1.9 Hz, 1H), 7.34 (dd, J = 8.4, 1.9 Hz, 1H), 7.18 (d, J = 8.2 Hz, 2H), 6.87 (s, 1H), 4.23 (d, J = 10.6 Hz, 1H), 4.05 (d, J = 10.6 Hz, 1H), 3.67 (s, 3H), 3.46 (d, J = 10.3 Hz, 1H), 3.21 (d, J = 10.3 Hz, 1H), 3.16 (dd, J = 10.0, 4.3 Hz, 1H), 2.39 (ddt, J = 11.1, 7.8, 4.0 Hz, 1H), 2.29 (ddt, J = 13.0, 10.4, 2.4 Hz, 1H), 2.24 (dtd, J = 10.5, 6.2, 1.9 Hz, 1H), 1.93 (t, J = 5.5 Hz, 2H), 1.66 - 1.61 (m, 2H), 1.58 (s, 9H), 1.33 (d, J = 10.6 Hz, 2H), 1.24 (s, 5H), 1.04 (s, 3H).

**¹³C NMR** (151 MHz, CDCl₃) δ 176.0, 153.4, 151.3, 138.1, 137.8, 134.8, 132.7, 132.1, 129.9, 124.8, 120.7, 86.5, 70.7, 58.5, 56.8, 52.8, 51.2, 46.8, 46.5, 40.3, 39.0, 34.8, 33.8, 29.9, 27.4, 27.3, 26.4, 26.4, 23.7.

**HRMS** (ESI) for C₃₀H₃₆ClIN₂O₆SNa: calculated: 737.0925 [M+Na]⁺, found 737.0932.

[α]²⁰_{D} = -16.2° (CHCl₃, c = 0.25).

### 2-(3-chloro-4-(((3aS,4R,6R,7aS)-3a-((((4-iodophenyl)carbamoyl)oxy)methyl)-5,5-dimethyloctahydro-2H-4,6-methanoisoindol-2-yl)sulfonyl)phenyl)-2-methylpropanoic acid (69)

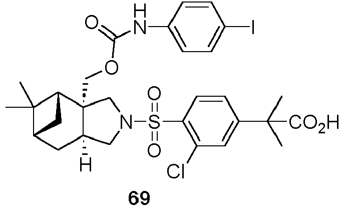

To a solution of **Int-36** (23.5 mg, 0.05 mmol, 1.0 equiv.) in ethanol (0.1M), cooled in an ice bath, a solution of sodium hydroxide (7 mg, 0.17 mmol, 3 equiv.) in ethanol (0.1 M) was added dropwise. The resulting mixture was refluxed for 2-6 h, and then the solution was acidified with 2 N HCl to pH = 2. Extraction of the resulting mixture with diethyl ether, drying of the combined organic extracts over magnesium sulfate and evaporation of the solvent afforded carboxylic acid without purification for next step.

Following general procedure A3: From the crude carboxylic acid ( 20.6 mg, 0.05mmol) and 1-iodo-4-isocyanatobenzene (15 mg, 1.2 eq). Purification by column chromatography (DCM : MeOH=50:1 to 20:1) afforded a colorless solid **69** (7 mg, 0.01 mmol, two steps, totally 20 % yield).

**¹H NMR** (600 MHz, CDCl₃) δ 8.03 (d, *J* = 8.4 Hz, 1H), 7.60 (d, *J* = 8.7 Hz, 2H), 7.53 (d, *J* = 1.9 Hz, 1H), 7.40 (dd, *J* = 8.4, 1.9 Hz, 1H), 7.17 (s, 2H), 6.88 (s, 1H), 4.20 (d, *J* = 10.8 Hz, 1H), 4.05 (d, *J* = 10.7 Hz, 1H), 3.68 (s, 1H), 3.44 (s, 1H), 3.21 (d, *J* = 10.3 Hz, 1H), 3.17 (dd, *J* = 10.0, 4.3 Hz, 1H), 2.37 (d, *J* = 8.4 Hz, 1H), 2.33 - 2.26 (m, 2H), 2.27 - 2.20 (m, 2H), 1.93 (d, *J* = 6.3 Hz, 3H), 1.64 (d, *J* = 4.2 Hz, 1H), 1.65 - 1.58 (m, 6H), 1.33 (d, *J* = 10.6 Hz, 1H), 1.24 (s, 3H), 1.03 (s, 3H).

**¹³C NMR** (151 MHz, CDCl₃) δ 177.5, 150.6, 138.1, 137.8, 135.0, 132.8, 132.2, 130.0, 124.8, 120.7, 83.6, 70.7, 58.5, 53.6, 51.2, 46.5, 46.3, 40.2, 39.0, 34.7, 33.8, 29.8, 27.5, 27.3, 26.37, 26.2, 23.7.

**HRMS** (ESI) for C₂₉H₃₄ClIN₂O₆SNa: calculated: 723.0728 [M+Na]⁺, found 723.0772.

[α]²⁰_{D} = -24.1° (CHCl₃, c = 0.5).

### 2-(4-(((3aS,4R,6R,7aS)-3a-((((4-iodophenyl)carbamoyl)oxy)methyl)-5,5-dimethyloctahydro-2H-4,6-methanoisoindol-2-yl)sulfonyl)-3-methylphenyl)-2-methylpropanoic acid (70)

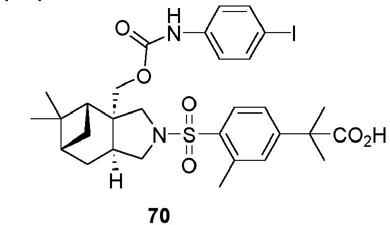

To a solution of **Int-37** (179.8 mg, 0.4 mmol, 1.0 equiv.) in ethanol (0.1M), cooled in an ice bath, a solution of sodium hydroxide (48 mg, 1.2 mmol, 3 equiv.) in ethanol (0.1 M) was added dropwise. The resulting mixture was refluxed for 2-6 h, and then the solution was acidified with 2 N HCl to pH = 2. Extraction of the resulting mixture with diethyl ether, drying of the combined organic extracts over magnesium sulfate and evaporation of the solvent afforded carboxylic acid without purification for next step.

Following general procedure A3: From the crude carboxylic acid (196 mg, 0.4 mmol) and 1-iodo-4-isocyanatobenzene (118 mg, 0.48 mmol, 1.2 eq). Purification by column chromatography (DCM : MeOH=50:1 to 20:1) afforded a colorless solid **70** (153 mg, 0.22 mmol, two steps, totally 50 % yield).

**¹H NMR** (600 MHz, CD₂Cl₂) δ 7.88 - 7.85 (m, 1H), 7.64 - 7.59 (m, 2H), 7.37 - 7.32 (m, 2H), 7.18 (d, J = 7.8 Hz, 2H), 6.84 (s, 1H), 4.09 (s, 2H), 3.42 (d, J = 9.8 Hz, 1H), 3.25 (d, J = 10.0 Hz, 1H), 3.04 (d, J = 10.1 Hz, 1H), 3.02 (dd, J = 9.7, 2.9 Hz, 1H), 2.64 (s, 3H), 2.32 (td, J = 6.2, 5.4, 3.1 Hz, 2H), 2.24 - 2.19 (m, 1H), 1.96 (d, J = 1.5 Hz, 1H), 1.89 (d, J = 3.3 Hz, 1H), 1.59 (s, 6H), 1.58 - 1.55 (m, 1H), 1.29 (d, J = 10.4 Hz, 1H), 1.23 (s, 3H), 1.03 (s, 3H).

**¹³C NMR** (151 MHz, CD₂Cl₂) δ 177.9, 153.6, 149.5, 138.8, 138.3, 134.5, 130.7, 124.0, 120.9, 86.4, 70.6, 58.1, 56.6, 51.1, 46.7, 46.6, 40.6, 39.3, 34.8, 34.3, 27.4, 27.4, 26.4, 26.4, 23.6, 21.2.

**HRMS** (ESI) for C₃₀H₃₇IN₂O₆SNa: calculated: 703.1315 [M+Na]⁺, found 703.1316.

[α]²⁰_{D} = -8.1° (CHCl₃, c = 0.5).

### ((3aS,4R,6R,7aS)-2-((4-(tert-butyl)-2-methylphenyl) sulfonyl)-5,5-dimethylocta-hydro-3aH-4,6-methanoisoindol-3a-yl)methyl (4-ethynylphenyl)carbamate (71)

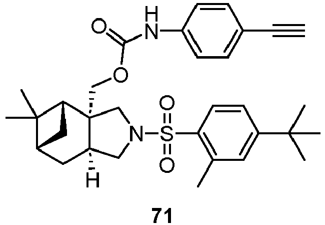

Following general procedure D: From **52** (89 mg, 0.14 mmol) and trimethylsilylacetylene (42 ul,0.28 mmol, 2.0 equiv.). Purification by column chromatography (PE/EtOAc 15:1 to 10:1 to 5:1) afforded a colorless solid (53 % yield).

**¹H NMR** (700 MHz, CDCl₃) δ 7.86 (d, J = 8.1 Hz, 1H), 7.44 (d, J = 8.7 Hz, 2H), 7.35 (d, J = 8.2 Hz, 2H), 7.33 - 7.29 (m, 2H), 6.83 (s, 1H), 4.17 (d, J = 10.6 Hz, 1H), 4.09 (d, J = 10.5 Hz, 1H), 3.48 (t, J = 8.6 Hz, 1H), 3.37 (d, J = 10.2 Hz, 1H), 3.06 (d, J = 10.1 Hz, 1H), 3.03 (s, 1H), 3.02 (dd, J = 9.6, 3.8 Hz, 1H), 2.66 (s, 3H), 2.37 - 2.32 (m, 1H), 2.32 - 2.26 (m, 1H), 2.22 (ddd, J = 10.5, 5.2, 3.2 Hz, 1H), 1.94 (dd, J = 6.5, 4.9 Hz, 1H), 1.91 (dq, J = 5.3, 2.7 Hz, 1H), 1.60 - 1.55 (m, 1H), 1.31 (s, 10H), 1.23 (s, 3H), 1.03 (s, 3H).

**¹³C NMR** (151 MHz, CD₂Cl₂) δ 157.1, 153.4, 139.1, 138.3, 133.3, 133.0, 130.4, 130.3, 123.5, 118.5, 117.0, 83.6, 76.8, 70.6, 58.0, 56.6, 51.0, 46.7, 40.6, 39.3, 35.2, 34.9, 34.4, 31.1, 27.4, 27.4, 23.6, 21.2.

**HRMS** (ESI) for C₃₂H₄₀N₂O₄SNa: calculated: 571.2606 [M+Na]⁺, found 571.2602.

[α]²⁰_{D} = +12.3° (CHCl₃, c = 0.5).

### ((3aS,4R,6R,7aS)-2-((4-(tert-butyl)-2-ethylphenyl)sulfonyl)-5,5-dimethylocta-hydro-3aH-4,6-methanoisoindol-3a-yl)methyl(4-ethynylphenyl) carbamate (72)

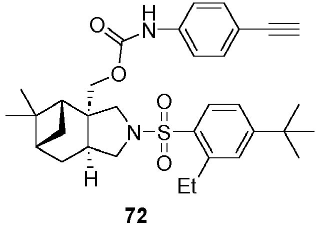

Following general procedure D: From **53** (29.9 mg, 0.04 mmol) and trimethylsilylacetylene (42 ul,0.28 mmol, 2.0 equiv.). Purification by column chromatography (PE/EtOAc 15:1 to 10:1 to 5:1) afforded a colorless solid (53 %yield).

**¹H NMR** (500 MHz, CD₂Cl₂) δ 7.81 (d, J = 8.4 Hz, 1H), 7.61 (d, J = 8.7 Hz, 2H), 7.40 (d, J = 2.1 Hz, 1H), 7.33 (dd, J = 8.4, 2.1 Hz, 1H), 7.19 (d, J = 8.3 Hz, 2H), 6.87 (s, 1H), 4.09 (d, J = 2.1 Hz, 2H), 3.41 (dd, J = 9.4, 6.8 Hz, 1H), 3.26 (d, J = 10.0 Hz, 1H), 3.07 - 3.02 (m, 2H), 3.01 - 2.96 (m, 2H), 2.31 (dt, J = 6.4, 3.4 Hz, 2H), 2.23 - 2.17 (m, 1H), 1.96 (dd, J = 6.5, 4.9 Hz, 1H), 1.88 (td, J = 5.3, 3.0 Hz, 1H), 1.55 (d, J = 2.9 Hz, 1H), 1.32 (s, 9H), 1.29 (s, 2H), 1.27 (t, J = 7.5 Hz, 4H), 1.23 (s, 3H), 1.02 (s, 3H). **¹³C NMR** (151 MHz, CD₂Cl₂) δ 157.2, 153.4, 144.6, 139.1, 133.3, 132.5, 130.5, 128.6, 123.4, 118.5, 117.0, 83.7, 76.8, 70.6, 58.1, 56.6, 51.0, 46.7, 40.6, 39.4, 35.3, 34.9, 34.3, 31.2, 27.4, 27.3, 27.0, 23.6, 16.3.

**HRMS** (ESI) for C₃₃H₄₂N₂O₄SNa: calculated: 585.2763 [M+Na]⁺, found 585.2753.

[α]²⁰_{D} = +12.0° (CHCl₃, c = 0.5).

### ((3aS,4R,6R,7aS)-2-((4-(tert-butyl)-2-cyclopropylphenyl)sulfonyl)-5,5-dimethyl-octahydro-3aH-4,6-methanoisoindol-3a-yl)methyl(4-ethynylphenyl)carbamate (73)

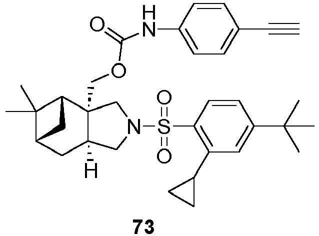

Following general procedure D: From **54** (33.8 mg, 0.05 mmol) and trimethylsilylacetylene (14 ul,0.1 mmol, 2.0 equiv.). Purification by column chromatography (PE/EtOAc 15:1 to 10:1 to 5:1) afforded a colorless solid (60 %yield).

**¹H NMR** (500 MHz, CD₂Cl₂) δ 7.84 (d, J = 8.4 Hz, 1H), 7.47 - 7.40 (m, 2H), 7.36 (d, J = 8.6 Hz, 2H), 7.28 (dd, J = 8.4, 2.0 Hz, 1H), 6.93 (s, 1H), 6.89 (d, J = 2.0 Hz, 1H), 4.11 (s, 2H), 3.47 - 3.41 (m, 1H), 3.29 (d, J = 10.0 Hz, 1H), 3.09 (s, 1H), 3.05 (d, J = 10.2 Hz, 1H), 3.03 - 3.00 (m, 1H), 2.87 (tt, J = 8.4, 5.2 Hz, 1H), 2.35 - 2.28 (m, 2H), 2.20 (dtd, J = 10.4, 6.3, 1.6 Hz, 1H), 1.97 (dd, J = 6.5, 5.0 Hz, 1H), 1.89 (td, J = 5.3,

3.1 Hz, 1H), 1.57 (dd, J = 8.9, 2.8 Hz, 1H), 1.35 (d, J = 10.4 Hz, 1H), 1.28 (s, 9H), 1.23 (s, 3H), 1.10 (ddt, J = 9.3, 5.2, 2.1 Hz, 2H), 1.03 (s, 3H), 0.84 - 0.80 (m, 2H). **¹³C NMR** (126 MHz, CD₂Cl₂) δ 157.2, 153.1, 144.3, 139.1, 133.3, 133.3, 130.7, 128.5, 122.7, 121.7, 118.6, 111.9, 83.6, 76.8, 70.6, 58.0, 56.7, 51.0, 46.7, 40.6, 39.4, 35.4, 34.9, 34.4, 31.1, 27.4, 27.3, 23.5, 14.4, 12.6, 11.7, 11.6.

**HRMS** (ESI) for C₃₄H₄₂N₂O₄SNa: calculated: 597.2763 [M+Na]⁺, found 597.2743.

[α]²⁰_{D} = +18.7° (CHCl₃, c = 0.25).

### 2-(4-(((3aS,4R,6R,7aS)-3a-((((4-ethynylphenyl)carbamoyl)oxy)methyl)-5,5-dimethyloctahydro-2H-4,6-methanoisoindol-2-yl)sulfonyl)phenyl)-2-methylpropanoic acid (74)

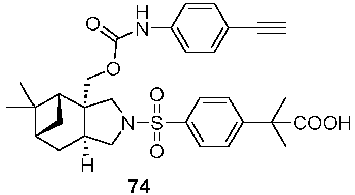

Following general procedure D: From **65** (46.8 mg, 0.07 mmol) and trimethylsilylacetylene (11.3 ul,0.1 mmol, 2.0 equiv.). Purification by column chromatography (DCM:MeOH =50:1 to 20:1 to 5:1) afforded a colorless solid (45 %yield).

**¹H NMR** (700 MHz, CDCl₃) δ 7.78 (d, J = 8.4 Hz, 2H), 7.55 (d, J = 8.6 Hz, 2H), 7.43 (d, J = 8.7 Hz, 2H), 7.29 (s, 2H), 6.60 (s, 1H), 4.03 (d, J = 12.7 Hz, 2H), 3.22 - 3.14 (m, 1H), 3.09 (d, J = 9.9 Hz, 1H), 3.07 - 3.00 (m, 3H), 2.32 (dt, J = 10.3, 2.6 Hz, 1H), 2.30 - 2.26 (m, 1H), 2.20 (dtd, J = 10.4, 6.2, 1.8 Hz, 1H), 1.95 (dd, J = 6.5, 4.9 Hz, 1H), 1.89 (dq, J = 5.5, 2.7 Hz, 1H), 1.64 - 1.62 (m, 1H), 1.62 (d, J = 1.4 Hz, 6H), 1.33 (d, J = 10.6 Hz, 1H), 1.23 (s, 3H), 1.01 (s, 3H).

**¹³C NMR** (176 MHz, CDCl₃) δ 179.5, 153.2, 149.2, 138.2, 134.0, 133.2, 130.1, 128.4, 126.7, 125.9, 118.5, 117.3, 83.4, 70.2, 58.3, 57.0, 50.8, 46.6, 46.5, 40.2, 39.2, 34.4, 34.1, 27.4, 27.1, 26.3, 23.5.

**HRMS** (ESI) for C₃₁H₃₆N₂O₆S: calculated: 587.2192 [M+Na]⁺, found 587.2179.

[α]²⁰_{D} = -136.1° (CHCl₃, c = 0.25).

### 2-(4-(((3aS,4R,6R,7aS)-3a-((((4-ethynylphenyl)carbamoyl)oxy)methyl)-5,5-dimethyloctahydro-2H-4,6-methanoisoindol-2-yl)sulfonyl)-3-methylphenyl)-2-methylpropanoic acid (75)

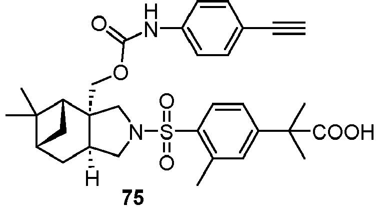

Following general procedure D: From **70** (110 mg, 0.16 mmol) and trimethylsilylacetylene (45.2 ul,0.32 mmol, 2.0 equiv.). Purification by column chromatography (DCM:MeOH =50:1 to 20:1 to 5:1) afforded a colorless solid (67 %yield).

**¹H NMR** (700 MHz, CD₂Cl₂) δ 7.86 (d, J = 8.8 Hz, 1H), 7.43 (d, J = 8.7 Hz, 2H), 7.36 (s, 1H), 7.34 (d, J = 6.4 Hz, 2H), 6.93 (s, 1H), 4.10 (s, 2H), 3.41 (s, 1H), 3.26 (d, J = 10.2 Hz, 1H), 3.09 (s, 1H), 3.07 - 3.00 (m, 2H), 2.64 (s, 3H), 2.32 (td, J = 7.3, 3.1 Hz, 2H), 2.21 (dtd, J = 10.5, 6.4, 1.6 Hz, 1H), 1.96 (dd, J = 6.4, 5.0 Hz, 1H), 1.91 - 1.88 (m, 1H), 1.59 (s, 6H), 1.56 (s, 1H), 1.29 (d, J = 10.4 Hz, 1H), 1.23 (s, 3H), 1.03 (s, 3H).

**¹³C NMR** (151 MHz, CD₂Cl₂) δ 178.1, 153.5, 149.7, 139.0, 138.8, 134.6, 133.3, 130.7, 130.7, 126.1, 124.0, 118.6, 117.1, 83.6, 76.8, 70.6, 58.1, 56.7, 51.1, 46.7, 46.6, 40.6, 39.3 34.8, 34.4, 27.4, 26.4, 26.4, 23.6, 21.2.

**HRMS** (ESI) for C₃₂H₃₈N₂O₆SNa: calculated: 601.2348 [M+Na]⁺, found 601.2341.

[α]²⁰_{D} = -72.4° (CHCl₃, *c* = 0.25).

### ((3aS,4R,6R,7aS)-5,5-dimethyl-2-((4-(3-(trifluoromethyl)-3H-diazirin-3-yl)phenyl) sulfonyl)octahydro-3aH-4,6-methanoisoindol-3a-yl)methyl (4-ethynylphenyl) carbamate (76):

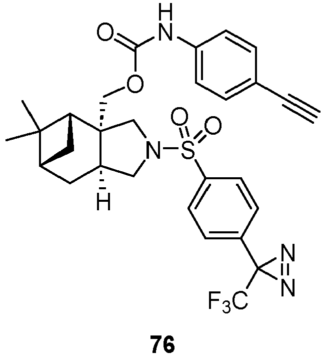

Following general procedure D: From **Int-54** (40.0 mg, 58.1 µmol) and trimethylsilylacetylene (16 µL, 0.116 mmol, 2.0 equiv.). Column chromatography (PE/EtOAc 8:1) afforded a beige colored oil (14.0 mg, 23.9 µmol, 41 % over 2 steps). **¹H-NMR** (CDCl₃, 700 MHz): δ = 1.02 (s, 3 H, CHs), 1.23 (s, 3 H, CHs), 1.27 (d, *J* = 10.6 Hz,1 H, CH), 1.58-1.68 (m, 1 H, CH), 1.88-1.92 (m, 1 H, CH), 1.95-1.98 (m, 1 H, CH), 2.19-2.24 (m, 1 H, CH), 2.26-2.34 (m, 2 H, 2 × CH), 2.99-3.10 (m, 4 H, 4 × CH), 3.22 (dd, *J* = 9.3, 6.8 Hz, 1 H, CH), 4.00 (d, *J* = 10.7 Hz, 1 H, CHs), 4.12 (d, *J* = 10.7 Hz, 1 H, CHs), 6.50 (s, 1 H, NH), 7.28-7.35 (m, 4 H, 4 × Ar-H), 7.45 (d, *J* = 8.6 Hz, 2 H, 2 × Ar-H), 7.84 (d, *J* = 8.4 Hz, 2 H, 2 × Ar-H).

**¹³C-NMR** (CDCl₃, 175 MHz): δ = 23.5, 27.1, 27.4, 34.1, 34.5, 39.2, 40.1, 46.4, 50.8, 57.0, 58.2, 70.2, 83.4, 121.1, 122.6, 127.0, 128.5, 133.3, 134.2, 137.0, 138.1, 153.0. **MS** (ESI) *m*/*z* (%) = 586.9 (100) [M+H]⁺.

**HRMS** (ESI) for C₂₉H₃₀F₃N₄O₄S: calculated 587.1934 [M+H]⁺, found 587.1942.

### Synthesis of 18:

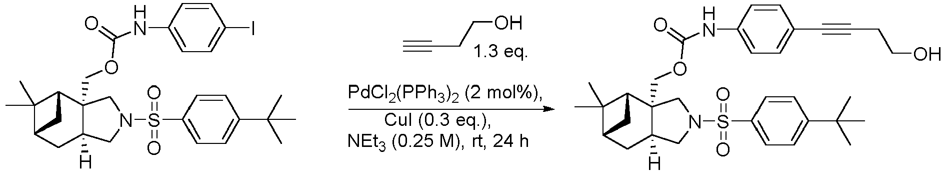

To a solution of **11 (iDeg-2)** (63.7 mmol, 0.1 mmol,1.0 equiv.), PdCl₂(PPh₃)₂ (7 mg, 0.1 equiv.), Cul (5.7 mg, 0.3 equiv.) in EtsN (0.25 M, 0.4) was added but-3-yn-1-ol (10 ul, 1.3 equiv.) and the mixture was stirred at room temperature under Ar atmosphere. After completion, the resulting mixture was concentrated under reduced pressure and the residue was subjected to column chromatography on silica gel (PE:EA=10:1 to 3:1) to give the desired coupling product **18.**

### ((3aS,4R,6R,7aS)-2-((4-(tert-butyl)phenyl)sulfonyl)-5,5-dimethyloctahydro-3aH-4,6-methanoisoindol-3a-yl)methyl (4-(4-hydroxybut-1-yn-1-yl)phenyl)carbamate (18)

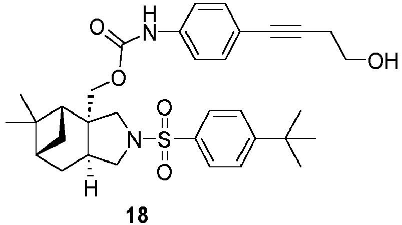

**¹H NMR** (600 MHz, CDCl₃) δ 7.74 (d, J = 8.5 Hz, 2H), 7.53 (d, J = 8.5 Hz, 2H), 7.34 (d, J = 8.6 Hz, 2H), 7.28 (s, 2H), 6.66 (s, 1H), 4.07 (d, J = 10.7 Hz, 1H), 3.99 (d, J = 10.9 Hz, 1H), 3.81 (t, J = 6.3 Hz, 2H), 3.22 (dd, J = 9.2, 6.8 Hz, 1H), 3.06 (d, J = 2.1 Hz, 2H), 3.03 (dd, J = 9.3, 3.0 Hz, 1H), 2.68 (t, J = 6.3 Hz, 2H), 2.28 (ddt, J = 13.1, 7.1, 3.0 Hz, 2H), 2.18 (ddd, J = 10.5, 6.1, 1.7 Hz, 1H), 2.15 (s, 1H), 1.95 (dd, J = 6.5, 4.9 Hz, 1H), 1.88 (dd, J = 5.5, 3.0 Hz, 1H), 1.61 (dt, J = 12.3, 3.2 Hz, 1H), 1.31 (s, 9H), 1.29 (d, J = 7.2 Hz, 1H), 1.22 (s, 3H), 1.00 (s, 3H).

**¹³C NMR** (151 MHz, CDCl₃) δ 156.9, 153.2, 137.5, 132.6, 132.3, 128.1, 126.1, 118.4, 85.8, 82.2, 70.3, 61.3, 58.2, 57.0, 50.6, 46.4, 40.2, 39.1, 35.3, 34.5, 34.1, 31.2, 27.4, 27.1, 23.9, 23.5.

**HRMS** (ESI) for C₃₃H₄₂N₂O₅SNa: calculated: 601.2702 [M+Na]⁺, found 601.2704.

[α]²⁰_{D} = -6.0° (CHCl₃, c = 1.0).

### General procedure for the synthesis of 54

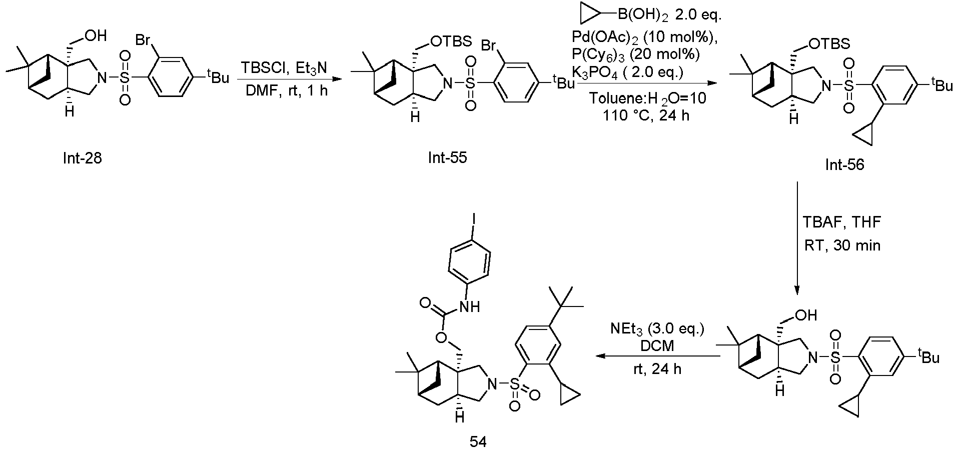

### (3aS,4R,6R,7aS)-2-((2-bromo-4-(tert-butyl)phenyl)sulfonyl)-3a-(((tert-butyldimethylsilyl)oxy)methyl)-5,5-dimethyloctahydro-1H-4,6-methanoisoindole (Int-55)

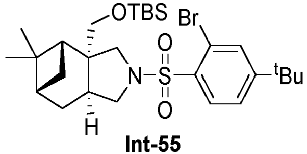

A solution of **Int-28** (90.1 mg, 0.2 mmol), Et₃N(83.4 uL, 0.6 mmol, 3equiv.), and TBSCI (60.3 mg, 0.4 mmol,2 equiv.) in DMF (5 ML) was stired for 20 minutes under an argon atmosphere, Afterwards, the reaction mixture was diluted with water and the product was extract with Et₂O, The organic layer was washed with water and brine, dried over anhydrous NaSO4, the residue was purified by chromatography (PE:EA=10:1) to afford solid product **Int-55** (98.8 mg, 0.17 mmol, 85% yield).

**¹H NMR** (700 MHz, CDCl₃) δ 7.97 (d, J = 8.3 Hz, 1H), 7.71 (d, J = 1.9 Hz, 1H), 7.41 (dd, J = 8.4, 1.9 Hz, 1H), 3.44 (dd, J = 9.1, 6.9 Hz, 1H), 3.43 (d, J = 9.6 Hz, 1H), 3.31 (d, J = 9.6 Hz, 1H), 3.29 - 3.27 (m, 1H), 3.17 (d, J = 9.6 Hz, 1H), 3.10 (d, J = 9.6 Hz, 1H), 2.28 (dd, J = 13.1, 10.6 Hz, 1H), 2.25 - 2.17 (m, 2H), 1.88 (d, J = 6.2 Hz, 2H), 1.67 (ddd, J = 12.9, 4.9, 2.0 Hz, 1H), 1.46 (d, J = 10.3 Hz, 1H), 1.32 (s, 9H), 1.21 (s, 3H), 0.94 (s, 3H), 0.76 (s, 9H), -0.11 (d, J = 8.5 Hz, 6H).

**¹³C NMR** (176 MHz, CDCl₃) δ 157.7, 134.3, 133.2, 132.4, 124.6, 120.8, 68.2, 58.9, 56.4, 52.9, 46.6, 40.5, 39.3, 35.2, 34.6, 34.5, 31.1, 27.6, 27.4, 26.0, 23.7, 18.2, -5.4, - 5.5.

**HRMS** (ESI) for C28H46BrNO3SSiNa: calculated 606.2049 [M+Na]⁺, found 606.2040.

[α]²⁰_{D} = -176.12° (CHCl₃, c = 0.25).

### (3aS,4R,6R,7aS)-2-((4-(tert-butyl)-2-cyclopropylphenyl)sulfonyl)-3a-(((tert-butyldimethylsilyl)oxy)methyl)-5,5-dimethyloctahydro-1H-4,6-methanoisoindole (Int-56)

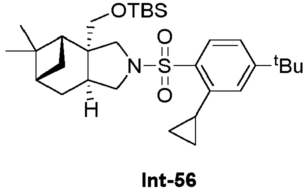

In a 10 mL sealed tube, **Int-55** (292.4 mg, 0.5 mmol), cyclopropylboronic acid (85.8 mg,2.0 eq), K₃PO₄ (265.3 mg, 1.25 mmol), Pd(OAc)₂ (11.3 mg, 10 mol%), P(Cy)₃ (28 mg, 20 mol%) were added successively under Ar. Then, toluene/H₂O = 10/1 (5:0.5 mL) was added. The tube was sealed with a Teflon lined cap and the reaction mixture was stirred at 110 °C for 24 h in an oil bath. After cooling to room temperature, the mixture was concentrated under vacuum and the residue was purified by column chromatography on silica gel (PE:EA=10:1) to give the corresponding products **Int-56** (98.8 mg, 0.17 mmol, 85%yield).

**¹H NMR** (700 MHz, CDCl₃) δ 7.83 (d, J = 8.4 Hz, 1H), 7.23 (dd, J = 8.4, 2.0 Hz, 1H), 6.85 (d, J = 2.0 Hz, 1H), 3.42 (d, J = 9.6 Hz, 1H), 3.29 (d, J = 9.6 Hz, 1H), 3.24 (dd, J = 8.8, 6.7 Hz, 1H), 3.14 (dd, J = 8.9, 2.2 Hz, 1H), 3.10 (d, J = 9.4 Hz, 1H), 3.05 (d, J = 9.4 Hz, 1H), 2.99 - 2.95 (m, 1H), 2.32 - 2.25 (m, 1H), 2.23 (ddt, J = 8.5, 6.1, 3.0 Hz, 1H), 2.17 (dtd, J = 10.3, 6.2, 2.0 Hz, 1H), 1.87 (d, J = 6.2 Hz, 2H), 1.62 (ddd, J = 13.1, 4.7, 2.3 Hz, 1H), 1.52 (d, J = 10.3 Hz, 1H), 1.28 (s, 9H), 1.21 (s, 3H), 1.10 - 1.08 (m, 2H), 0.94 (s, 3H), 0.81 - 0.78 (m, 2H), 0.76 (s, 9H), -0.10 (s, 3H), -0.13 (s, 3H).

**¹³C NMR** (176 MHz, CDCl₃) δ 155.1, 142.7, 131.5, 129.5 121.1, 120.1, 67.1, 57.1, 55.3, 51.3, 45.6 39.3, 38.2, 34.0, 33.6, 33.3, 30.0, 26.4, 26.0, 24.8, 22.5, 17.1, 11.3, 10.5, 10.4, -6.5, -6.6.

**HRMS** (ESI) for C28H46BrNO3SSiNa: calculated 568.3257 [M+Na]⁺, found 568.3248. [α]²⁰_{D} = -72.2° (CHCl₃, c = 0.5).

### ((3aS,4R,6R,7aS)-2-((4-(tert-butyl)-2-cyclopropylphenyl)sulfonyl)-5,5-dimethyl-octahydro-3aH-4,6-methanoisoindol-3a-yl)methyl (4-iodophenyl)carbamate (54)

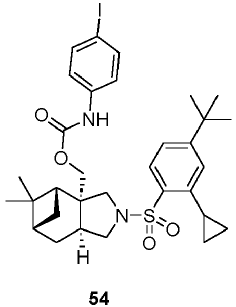

To a solution of **Int-55** ((169.2 mg, 0.3 mmol, 1.0 equiv.) in anhydrous THF (6 ml) were added dropwise of 1.0 M TBAF solution in THF (0.39 mL, 0.39 mmol, 1.3 equiv.). After stirring for 1 h at ambient temperature, the reaction was quenched with water, extracted with DCM, washed with brine, dried over MgSO4, and concentrated in vacuo. The residue were purified by column chromatography (EtOAc:Hexanes, gradient from 15:85 to 40:60) to afford the alcohol (quanti.).

Following general procedure A2: From the alcohol (151.1 mg, 0.35 mmol) and 1-iodo-4-isocyanatobenzene (102.9 mg, 0.42 mmol, 1.2 equiv.). Purification by column chromatography (PE/EtOAc 20:1 to 10:1) afforded a colorless solid **54** (170 mg, 0.25 mmol, 72 %yield).

**¹H NMR** (600 MHz, CD₂Cl₂) δ 7.84 (d, J = 8.4 Hz, 1H), 7.60 (d, J = 8.8 Hz, 2H), 7.27 (dd, J = 8.4, 2.0 Hz, 1H), 7.18 (d, J = 8.3 Hz, 2H), 6.89 (d, J = 2.0 Hz, 2H), 4.09 (s, 2H), 3.46 - 3.39 (m, 1H), 3.29 (d, J = 10.0 Hz, 1H), 3.04 (d, J = 10.0 Hz, 1H), 3.01 (dd, J = 9.6, 2.7 Hz, 1H), 2.89 - 2.84 (m, 1H), 2.36 - 2.26 (m, 2H), 2.19 (dtd, J = 10.4, 6.3, 1.6 Hz, 1H), 1.96 (dd, J = 6.5, 5.0 Hz, 1H), 1.88 (dt, J = 5.3, 2.8 Hz, 1H), 1.57 (dd, J = 9.0, 2.8 Hz, 1H), 1.35 (d, J = 10.4 Hz, 1H), 1.27 (s, 9H), 1.22 (s, 3H), 1.11 - 1.08 (m, 2H), 1.02 (s, 3H), 0.84 - 0.81 (m, 2H).

**¹³C NMR** (151 MHz, CD₂Cl₂) δ 157.2, 153.5, 144.3, 138.4, 138.3, 133.3, 130.7, 122.7, 121.7, 120.9, 86.3, 70.6, 58.0, 56.6, 51.0, 46.7, 40.6, 39.4, 35.4, 34.9, 34.4, 31.1, 27.4, 27.3, 23.5, 12.6, 11.7, 11.6.

**HRMS** (ESI) for C32H41IN2O4SNa: calculated 699.1729 [M+Na]⁺, found 699.1730.

[α]²⁰_{D} = -22.1° (CHCl₃, c = 1.0).

### Synthesis of 63:

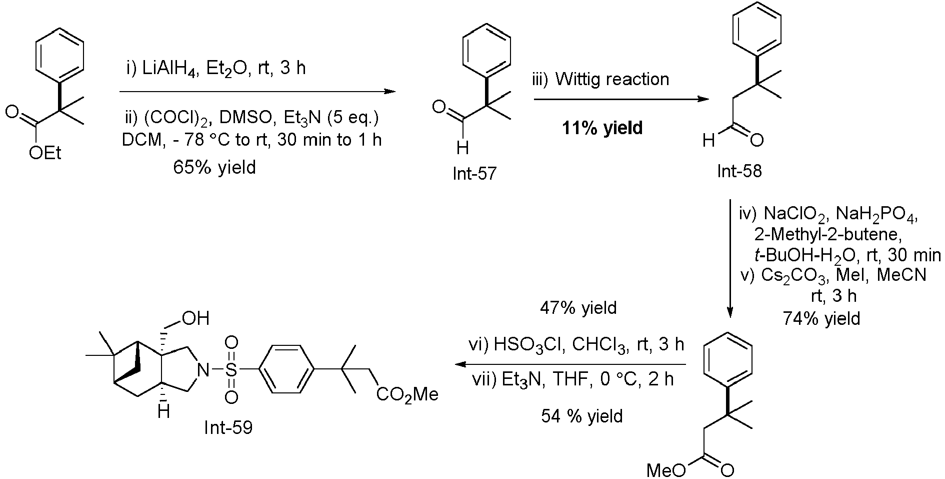

i) To a solution of ester (10 mmol, 1.00 equiv) in diethyl ether (0.25 M) was added LiAlH₄ (22.5 mmol, 2.25 equiv) in four approximately equal portions over 10 minutes at °C. The reaction was allowed to warm to room temperature and stirred for an additional 30 minutes. After TLC monitoring showed complete consumption of the methyl ester, the flask was returned to an ice bath and quenched with 1 M aqueous HCl. The primary alcohol product was extracted with diethyl ether (3 times). The combined organic layers were dried over anhydrous MgSO4, filtered, and concentrated under reduced pressure. The obtained alcohol was used in next step without further purification.
ii) To a solution of (COCl)₂ (0.94 mL, 12.0 mmol) in DCM (26 mL) was added DMSO (1.7 mL, 24 mmol) at -78 °C. After being stirred for 30 min, the alcohol (1.43 mg, 9.5 mmol) was added to the reaction mixture. The resulting mixture was stirred for 1 h at the same temperature. Then, to the mixture was added Et₃N (6.6 mL, 48 mmol), and warmed to room temperature. After being stirred for 1 h, the reaction was quenched by water, and extracted with DCM twice. The extract was washed with water and brine respectively, dried, and concentrated to afford the residue. Purification by column chromatography (PE to PE:EA=50:1) afforded the desired product (0.92g, 65%yield) as a colorless oil Int-57.
iii) A solution of n-BuLi (4.7 mL, 7.5 mmol) was added slowly to a stirred suspension of (methoxymethyl)triphenylphosphonium chloride (2.57 g, 7.5 mmol) in THF (16 mL) at 0°C. After stirring the dark red mixture at same temparture with 30 min, a solution of aldehyde Int-**57** (922.8 mg, 6.5 mmol) in THF (5 mL) was added . After stirring for 24 h, 2N HCl (1 mL of an aqueous solution) was added slowly to the yellow mixture which was stirred for further 3.5 h. NaHCOs (saturated aqueous solution) was added, the crude product was extracted with DCM (2 times) and the combined extracts were dried over MgSO₄. Purification by flash chromatography (gradient; 5, 20% EtOAc/hexanes) gave a desired product (**Int-58**, 108 mg, 11%yield).
iv) **Int-58** (108 mg, 0.66 mmol) was added into a solution of sodium chlorite (4.62mmol, 7 equiv.), monobasic sodium phosphate (3.48 mmol, 5equiv.), and 2-methyl-2-butene (169 qL) in a 20:1 tert-butyl alcohol-water mixture (1mL) at room temperature. After 30 min, the solvents were evaporated, and the residue was dissolved in 1 M hydrochloric acid and extracted with ether (3 times). The combined extracts were washed with brine , dried, filtered through silica gel, and evaporated to afford the crude carboxyl acid without further purification.
v) The yellow crude carboxylic acid was dissolved in MeCN (2 mL) and treated with an excess of Mel (2.0 equiv.) and Cs₂CO₃ (3.0 equiv.). The mixture was stirred at room temperature for 3 h. then water was added, the mixture was washed by EA for 3 times, the organic phase was washed by brine and then dried by NaSO₄. The unconsumed Mel was removed by evaporation, and the reaction mixture was filtered through silica gel and evaporated, No other furhter purification was required for the crude ester.
vi) Following general synthesis rout for preparing **Int-46** : From the above obtained ester (96 mg, 0.5 mmol) to afford the desired methyl 3-(4-(chlorosulfonyl)phenyl)-3-methylbutanoate,which was used in next step immediately (68 mg, 50 % yield).
vii) Following general procedure C2: From **Int-9** (48.8 mg, 0.25 mmol) and methyl 3-(4-(chlorosulfonyl)phenyl)-3-methylbutanoate (1.0equiv.). Purification by column chromatography (PE/EtOAc 4:1) afforded a colorless solid **Int-59** (60.6 mg, 0.13 mmol, 54 %yield).

### 2-methyl-2-phenylpropanal (Int-57):

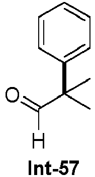

**¹H NMR** (500 MHz, CDCl₃) δ 9.51 (s, 1H), 7.41 - 7.36 (m, 2H), 7.31 - 7.27 (m, 3H), 1.47 (s, 6H).

**¹³C NMR** (126 MHz, CDCl₃) δ 202.4, 141.4, 129.0, 127.4, 126.9, 50.6, 22.6.

### 3-Methyl-3-phenylbutanal (Int-58):

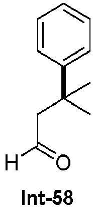

**¹H NMR** (500 MHz, CDCl₃) δ 9.51 (s, 1H), 7.41 - 7.37 (m, 2H), 7.35 (dd, *J* = 8.7, 6.9 Hz, 2H), 7.25 - 7.20 (m, 1H), 2.68 (d, *J* = 3.1 Hz, 2H), 1.47 (s, 6H).

**¹³C NMR** (126 MHz, CDCl₃) δ 203.2, 147.5, 128.7, 126.5, 125.7, 56.7, 36.9, 29.5.

### Methyl 3-(4-(((3aS,4R,6R,7aS)-3a-(hydroxymethyl)-5,5-dimethyloctahydro-2H-4,6-methanoisoindol-2-yl)sulfonyl)phenyl)-3-methylbutanoate (Int-59)

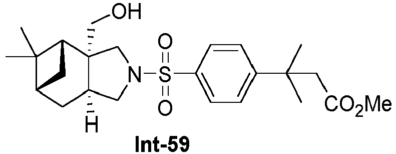

**¹H NMR** (500 MHz, CDCl₃) δ 7.74 (d, J = 8.5 Hz, 2H), 7.51 (d, J = 8.5 Hz, 2H), 3.50 (s, 3H), 3.42 - 3.34 (m, 2H), 3.16 (dd, J = 9.0, 7.0 Hz, 1H), 3.03 (dd, J = 9.1, 3.0 Hz, 1H), 2.99 (d, J = 9.7 Hz, 1H), 2.90 (d, J = 9.7 Hz, 1H), 2.66 (d, J = 2.0 Hz, 2H), 2.30 - 2.24 (m, 1H), 2.24 - 2.15 (m, 2H), 1.89 (dd, J = 6.5, 5.0 Hz, 1H), 1.86 (dq, J = 5.3, 2.7 Hz, 1H), 1.62 (ddd, J = 12.8, 4.2, 2.6 Hz, 1H), 1.48 (s, 3H), 1.47 (s, 3H), 1.33 (d, J = 10.4 Hz, 1H), 1.20 (s, 3H), 0.93 (s, 3H).

**¹³C NMR** (126 MHz, CDCl₃) δ 171.8, 153.3, 132.1, 128.2, 126.3, 68.1, 58.8, 57.0, 52.4, 51.5, 48.3, 46.2, 40.4, 39.2, 37.7, 34.3, 29.0, 28.9, 27.6, 27.5, 23.7.

**HRMS** (ESI) for C₂₄H₃₅NO₅SNa: calculated: 472.2134 [M+Na]⁺, found 472.2122 [α]²⁰_{D} = -14.1° (CHCl₃, c = 1.0).

### Methyl 3-(4-(((3aS,4R,6R,7aS)-3a-((((4-iodophenyl)carbamoyl)oxy)methyl)-5,5-dimethyloctahydro-2H-4,6-methanoisoindol-2-yl)sulfonyl)phenyl)-3-methylbutanoate (62)

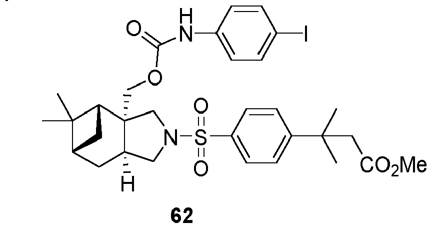

Following general procedure A2: From **Int-59** (22.5 mg, 0.05 mmol) and 1-iodo-4-isocyanatobenzene (22 mg, 1.5equiv.). Purification by column chromatography (PE/EtOAc 20:1 to 10:1) afforded a colorless solid **62** (0.03 mmol, 60 % yield).

**¹H NMR** (700 MHz, CDCl₃) δ 7.74 (d, J = 8.5 Hz, 2H), 7.58 (d, J = 8.7 Hz, 2H), 7.52 (d, J = 8.5 Hz, 2H), 7.14 (d, J = 8.3 Hz, 2H), 6.86 (s, 1H), 3.45 (s, 3H), 3.10 (d, J = 6.5 Hz, 1H), 3.08 (d, J = 2.5 Hz, 1H), 3.02 (dd, J = 9.1, 6.6 Hz, 1H), 2.90 (d, J = 9.7 Hz, 1H), 2.75 (d, J = 15.1 Hz, 1H), 2.65 (d, J = 15.1 Hz, 1H), 2.35 (ddd, J = 9.3, 3.6, 1.9 Hz, 1H), 2.33 - 2.30 (m, 1H), 2.22 (dtd, J = 10.4, 6.3, 1.8 Hz, 1H), 1.89 (t, J = 2.8 Hz, 1H), 1.88 - 1.86 (m, 1H), 1.63 (dt, J = 12.7, 3.3 Hz, 1H), 1.54 (s, 2H), 1.49 (s, 3H), 1.46 (m, 4H), 1.23 (s, 3H), 1.04 (s, 3H).

**¹³C NMR** (176 MHz, CDCl₃) δ 171.9, 153.6, 153.5, 138.0, 137.9, 132.3, 128.2, 126.3, 121.1, 86.6, 69.9, 58.5, 56.8, 51.6, 50.9, 47.9, 47.0, 40.2, 39.2, 37.6, 34.5, 34.2, 29.7, 28.6, 27.4, 27.1, 23.5.

**HRMS** (ESI) for C₃₁H₃₉IN₂O₆SNa- calculated: 717.1471 [M+Na]⁺, found 717.1477.

[α]²⁰_{D} = +64.0° (CHCl₃, c = 0.5).

### 3-(4-(((3aS,4R,6R,7aS)-3a-((((4-iodophenyl)carbamoyl)oxy)methyl)-5,5-dimethyloctahydro-2H-4,6-methanoisoindol-2-yl)sulfonyl)phenyl)-3-methylbutanoic acid (63)

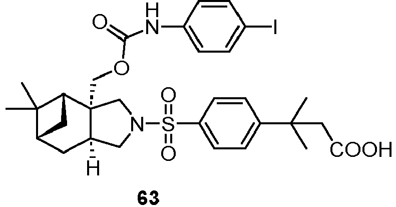

To a solution of **Int-59** (0.08 mmol, 1.0 equiv.) in ethanol (0.1M), cooled in an ice bath, a solution of sodium hydroxide (10 mg, 0.24 mmol, 3 equiv.) in ethanol (0.1 M) was added dropwise. The resulting mixture was refluxed for 6 h, and then the solution was acidified with 2 N HCl to pH = 2. Extraction of the resulting mixture with diethyl ether, drying of the combined organic extracts over magnesium sulfate and evaporation of the solvent afforded carboxylic acid without purification for next step. Following general procedure A3: From the crude carboxylic acid ( 0.08 mmol) and 1-iodo-4-isocyanatobenzene (22.1 mg, 1.2 eq). Purification by column chromatography (DCM:MeOH = 50:1 to 20:1) afforded a colorless solid **63** (0.04 mmol, totally 50 % yield for 2 steps).

**1H NMR** (700 MHz, CDCl₃) δ 7.73 (s, 2H), 7.67 - 7.61 (m, 2H), 7.55 (d, J = 8.0 Hz, 2H), 7.19 - 7.09 (m, 2H), 6.78 (s, 1H), 4.08 - 3.96 (m, 2H), 3.21 (d, J = 13.4 Hz, 2H), 3.14 (d, J = 9.9 Hz, 1H), 2.71 (s, 2H), 2.67 (s, 1H), 2.36 (s, 1H), 2.29 - 2.23 (m, 2H), 1.93 (s, 2H), 1.70 (d, J = 13.3 Hz, 1H), 1.51 (s, 4H), 1.26 (s, 2H), 1.03 (s, 3H).

**13C NMR** (176 MHz, CDCl₃) δ 172.5, 153.0, 151.9, 138.2, 138.1, 132.0, 128.0, 126.5, 121.1, 87.0, 70.3, 59.8, 58.1, 55.9, 50.9, 47.9, 43.5, 43.5, 40.1, 39.3, 34.4, 27.5, 27.1, 23.5, 22.0.

**HRMS** (ESI) for C₃₀H₃₇IN₂O₆SNa: calculated: 703.1315 [M+Na]⁺, found 703.1315.

[α]²⁰_{D} = + 40.0° (CHCl₃, *c* = 0.5).

### References:

1. Mutti, S.; Daubié, C.; Decalogne, F.; Fournier, R.; Rossi, P., Enantiospecific synthesis of RPR 107880: A new non peptide substance P antagonist. Tetrahedron Letters 1996, 37 (18), 3125-3128.
2. Sheffler, D. J.; Nedelcovych, M. T.; Williams, R.; Turner, S. C.; Duerk, B. B.; Robbins, M. R.; Jadhav, S. B.; Niswender, C. M.; Jones, C. K.; Conn, P. J.; Daniels, R. N.; Lindsley, C. W., Novel GlyT1 inhibitor chemotypes by scaffold hopping. Part 2: Development of a [3.3.0]-based series and other piperidine bioisosteres. Bioorganic & Medicinal Chemistry Letters 2014, 24 (4), 1062-1066.
3. Wang, Z.; Xu,W.; Song, T.; Guo, Z.; Liu, L.; Fan, Y.; Wang, A.; Zhang, Z. Fragment- based design, synthesis, and biological evaluation of 1-Substituted-indole-2-carboxylic acids as selective mcl-1 inhibitors, Arch. Pharm. (Weinheim) 2017, 1, 350.
4. Zhong, L.-J, Xiong, Z.-Q, Ouyang, X.-H, Li, Y, Song, R.-J, Sun, Q, Lu, X, Li, J.-H. Intermolecular 1,2-Difunctionalization of Alkenes Enabled by Fluoroamide-Directed Remote Benzyl C(sp3)-H Functionalization. J. Am. Chem. Soc. 2022, 144, 1, 339-348 (2022).
5. Bhattacharya, S. N.; Eaborn, C.; Walton, D. R. M. J. Chem. Soc. C, 1968, 1265-1267.
6. Barral, K.; Moorhouse, A. D.; Moses, J. E., Efficient Conversion of Aromatic Amines into Azides: A One-Pot Synthesis of Triazole Linkages. Organic Letters 2007, 9 (9), 1809-1811.

### Biological Examples

### Material and Methods

### Material

BxPC3 cells, HEK293T and HeLa cells were purchased from DSMZ GmbH (Germany), BT549 and SKOV3 cells purchased from ATCC (USA). KBM7 iCas9 cells were a gift from J. Zuber (IMP, Vienna). Lenti-X 293T lentiviral packaging cells were obtained from Clontech. pCMV3-IDO1 was obtained from Sino Biological US Inc. pSpCas9(BB)-2A-GFP (PX458) plasmid was a gift from Feng Zhang (Addgene plasmid # 48138; http://n2t.net/addgene:48138; RRID:Addgene_48138¹)). pCMVR8.74 was a gift from Didier Trono (Addgene plasmid # 22036; http://n2t.net/addgene:22036 ; RRID:Addgene_22036). pMD2.G was a gift from Didier Trono (Addgene plasmid # 12259; http://n2t.net/addgene:12259 ; RRID:Addgene_12259). Fetal bovine serum (FBS), penicillin/streptomycin, anti-IDO1 (14-9750-80) and anti-vinculin (V9131) antibodies were obtained from ThermoFisher, USA. DMEM, McCoy's medium, sodium pyruvate, and non-essential amino acids were obtained from PAN biotech, Germany. Where indicated, DMEM was obtained from Gibco, USA. IMDM was obtained from Gibco, USA. 1 mM L-tryptophan was obtained from Sigma. IFN-γ was purchased from 50 ng/mL, PeproTech. anti-IDO1 (ab211017) and anti-β-actin (ab8227) antibodies were obtained from abcam. Mouse Thy1.1 antibody was purchased from BioLegend. IRDye-conjugated secondary antibodies were obtained from LI-COR Biosciences. Succinyl acetone was obtained from TCI. Doxycycline was obtained from PanReac AppliChem.

### Cell Culture

BxPC3 cells were cultured in RPMI-1640 (PAN biotech, Germany) containing 10% heat-inactivated fetal bovine serum (FBS). BT549, HeLa and HEK293T cells were cultured in DMEM (PAN biotech, Germany) supplemented with 10% FBS, sodium and non-essential amino acids. SKOV3 and U2OS wt and knockout cells were cultured in McCoys containing 10% heat-inactivated FBS. Lenti-X 293T lentiviral packaging cells were cultured in DMEM (Gibco) supplemented with 100 U/mL penicillin/streptomycin and 10% heat-inactivated FBS. IDO1-deficient SKOV3 cells were produced using the CRISPR-Cas9 system. For this, 100,000 cell/well in 6-well plates were transfected using Lipofectamine 3000 with the PX458 plasmid containing three separated sgRNA located in *IDO1.* sgRNA sequences: 5'-CACACGCTATGGAAAACTCC-3' (SEQ ID No.: 1); 5'-AGCCCACTTCTTCATCAATA-3' (SEQ ID No.: 2); 5'-TACCATATTGATGAAGAAGT-3' (SEQ ID No.: 3). 48 hours later, the cells were sorted for single cells based on GFP positivity. Single clones were expanded and successful knockout of *IDO1* was verified using immunoblotting. KBM7 iCas9 cells were cultured in IMDM supplemented with 100 U/mL penicillin/streptomycin, 10% heat-inactivated FBS and 1 mM L-tryptophan. Cell lines were maintained in a humidified atmosphere at 37°C and 5 % CO₂. Regular mycoplasma testing confirmed cell lines as mycoplasma-negative.

### Example B-1

### Automated Kyn Screening Assay

The Kyn screening assay was performed as previously described⁷. BxPC3 cells were seeded in phenol red-free RPMI 1640 medium in 1536-well or 384-well plates (Greiner #782086, Corning #3770) 24 h prior to the addition of compounds, IFN-γ (50 ng/mL, PeproTech) and L-Trp (380 µM, Sigmal-Aldrich). After 48 h of incubation, first cell viability was accessed by the addition of Hoechst-33342. Afterwards, trichloroacetic acid (TCA, Sigma-Aldrich) was added to a final concentration of 7 % and incubated for 10 min prior to a centrifugation step of 10 min at 1620 × g. Kyn levels were determined by means of the Kyn sensor⁷ at a final concentration of 17.5 µM in sensor buffer (50 mM H₃PO₄ and 120 mM NaCl (pH=1)). Fluorescence intensity (excitation: 535 nm, emission: 595 nm) was measured using a Spectramax Paradigm reader (Molecular Devices). Data were normalized to the values for cells that were treated with DMSO. All HTS data was analyzed using the Quattro Software Suite (Quattro Research GmbH).

### Manual Kyn Assay

For the manual assay, BxPC3 or SKOV3 cells were seeded in clear 96-well or 384-well plates, respectively, 24 h prior to compound treatment and IFN-γ (50 or 5 ng/mL, respectively) and L-Trp (380 µM) addition as described before.⁷ 48 h after treatment, TCA was added and Kyn levels were detected by means of 2 % w/v of p-DMAB (Ehrlich reagent) in acetic acid as described by Braun et al²⁹. The absorbance of the Ehrlich reagent was detected at 492 nm and at 650 nm as background control was measured on Spark^{®} multimode microplate plate reader (Tecan, Austria). Kyn levels were determined by subtracting the absorbance value at 650 nm and presented relative to the DMSO control. Dose-response curves and IC₅₀ values were generated and fitted with GraphPad Prism 6.0 (GraphPad software, USA) using four-parameter variable slope non-linear regression curve fit.

For label-free Kyn detection, the cell supernatant was analyzed via LC-MS/MS upon the addition of 30 % TCA. Kyn levels were measured by HPLC-MS/MS using the LTQ Velos Pro and Dionex HPLC (Thermo Fisher Scientific). Data were analyzed using Thermo Xcalibur^{™} (Thermo Fisher Scientific) and presented GraphPad Prism 6.0 (GraphPad software, USA). Values are presented relative to the DMSO control.

**Table B-1: Structure-activity relationship for myrtanol based compounds using the Kyn assay in BxPC-3 cells. Cells were incubated with IFN-γ, Trp and the compounds for 48 h prior to detection of Kyn levels.**

| Compounds having an activity designated as "A" provided an IC₅₀ ≤ 0.2 µM; compounds having an activity designated as "B" provided an 0.2 µM < IC₅₀ ≤ 1.0 µM; compounds having an activity designated as "C" provided an 1.0 µM < IC₅₀ ≤ 5.0 µM; compounds having an activity designated as "D" provided an 5. µM < IC₅₀ ≤ 10.0 µM | | | |
|---|---|---|---|
| 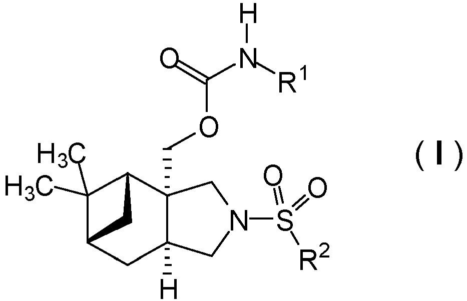 | | | |
| **Cpd No.** | **R¹** | **R²** | Kyn assay IC₅₀ ± **S**D [µM] |
| 1 (iDeg-1) | 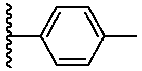 | 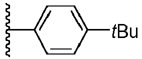 | B |
| **2** | 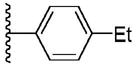 | 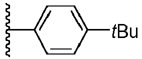 | C |
| **6** | 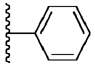 | 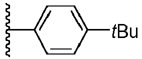 | D |
| **7** | 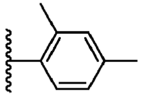 | 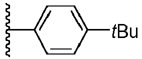 | C |
| **9** | 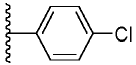 | 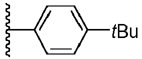 | B |
| **10** | 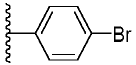 | 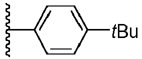 | B |
| **11** | 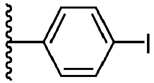 | 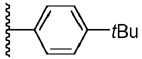 | A |

| **Cpd No.** | **R¹** | **R²** | Kyn assay IC₅₀ ± SD [µM] |
|---|---|---|---|
| **12** | 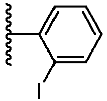 | 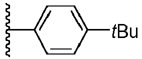 | B |
| **13** | 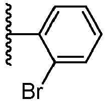 | 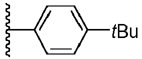 | B |
| **14** | 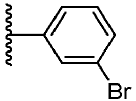 | 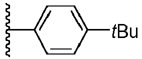 | D |
| **18** | 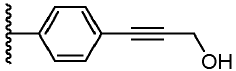 | 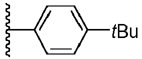 | C |
| **19** (iDeg-3) | 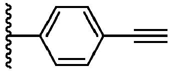 | 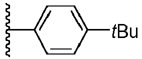 | A |
| **20** | 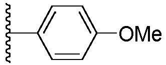 | 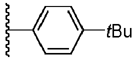 | C |
| **23** | 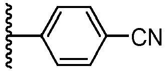 | 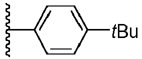 | C |
| **30** | 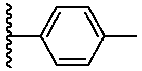 | 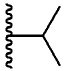 | C |
| **31** | 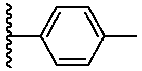 | 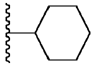 | D |
| **32** | 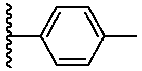 | 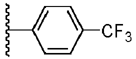 | C |
| **34** | 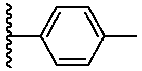 | 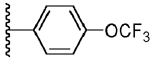 | C |
| **35** | 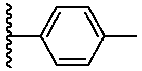 | 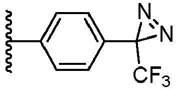 | C |
| **36** | 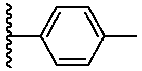 | 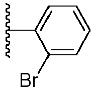 | D |
| **37** | 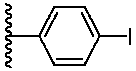 | 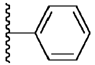 | C |
| **38** | 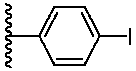 | 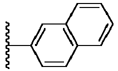 | B |
| **39** | 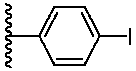 | 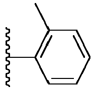 | B |
| **40** | 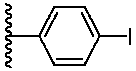 | 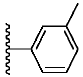 | C |
| **41** | 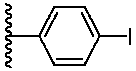 | 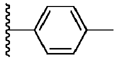 | B |
| **43** | 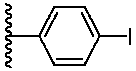 | 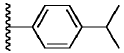 | A |
| **44** | 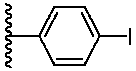 | 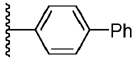 | B |
| **45** | 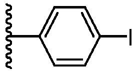 | 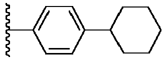 | C |
| **46** | 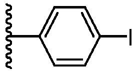 | 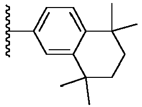 | B |
| **47** | 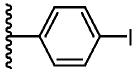 | 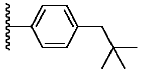 | B |
| **48** | 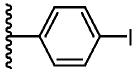 | 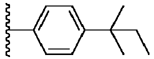 | A |
| **50** | 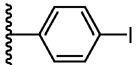 | 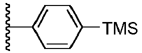 | B |
| **52** (iDeg-4) | 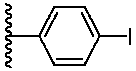 | 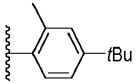 | A |
| **53** | 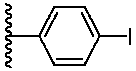 | 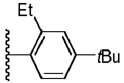 | A |
| **54** | 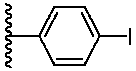 | 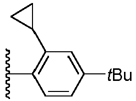 | A |
| **55** | 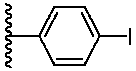 | 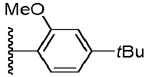 | A |
| **56** | 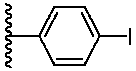 | 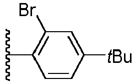 | A |
| **57** | 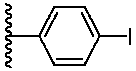 | 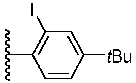 | A |
| **58** | 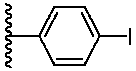 | 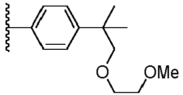 | A |
| **59** | 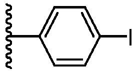 | 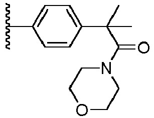 | D |
| **60** | 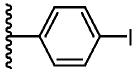 | 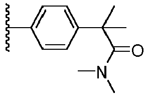 | B |
| **61** | 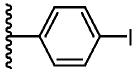 | 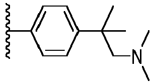 | C |
| **62** | 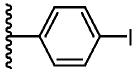 | 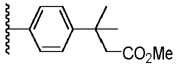 | A |
| **63** | 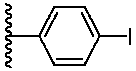 | 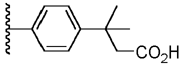 | A |
| **64** | 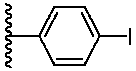 | 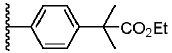 | B |
| **65** (iDeg-5) | 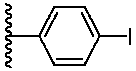 | 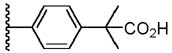 | A |
| **66** | 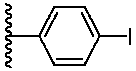 | 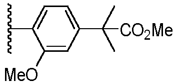 | A |
| **67** | 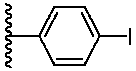 | 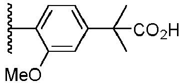 | A |
| **68** | 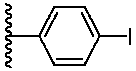 | 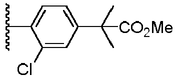 | A |
| **69** | 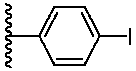 | 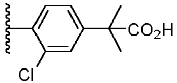 | A |
| **70** | 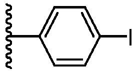 | 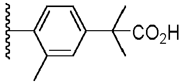 | A |
| **71** | 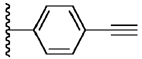 | 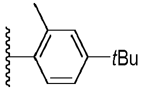 | A |
| **72** (iDeg-8) | 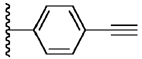 | 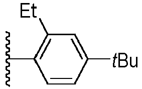 | A |
| 73 | 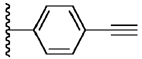 | 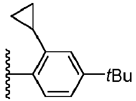 | A |
| **74** (iDeg-7) | 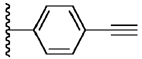 | 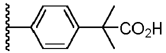 | A |
| **75** (iDeg-9) | 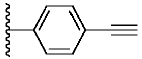 | 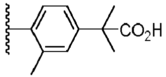 | A |
| **76** | 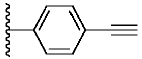 | 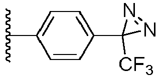 | A |

Initially, the sulfonamide substituent **R²** was kept constant and the urethane substituent **R¹** was varied (**Table B-1**). It was found, that omission (compound **6**) or combination with an ortho-substituent (compound **7**) is tolerated in the inhibitory activity. Replacement of the *para*-CH₃ by Cl, Br or I led to higher potency which also increased with the size of the substituent. Halogens were also tolerated in *ortho-*position (compounds **12** and **13**), but introduction of a halogen or a different substituent into the *meta*-position led to light loss of activity (compound **14**). Additional exploration of the *para*-substituent (compounds **18-20**) revealed that in this position limited structure variation is possible but may yield potent compounds. Thus, introduction of an acetylene substantially increased potency (compound **19**)**.**

These investigations showed that within the investigated group the most favorable substituents **R¹** are *p*-CHs-phenyl (compound **1;** iDeg-1), *p*-I-phenyl (compound **11;** iDeg-2) and *p*-C=CH phenyl (compound **19;** iDeg-3). Therefore, these substituents were employed for exploration of the structure of sulfonamide substituent **R²**. Replacement of the *p*-tert-butyl group in compound **1** by different substituents of exchange of the aromatic substituent to aliphatic analogs did not improve compound potency (compounds **30-32, 34-36**). However, exploration of **R²** starting from compound **11** (**R¹** = *p*-I-phenyl) and compound **19** (**R¹** = *p*-C≡CH phenyl) yielded more potent compounds.

Exchange of the *tert*-butyl group in the para position of the phenyl group by H, different aliphatic or aromatic compounds initially did not yield improved compounds (see compounds **37-41, 43-48, 50**), but closure of an aliphatic ring (compound **46**), change to a *neo-*pentyl group (compound **47**) and addition of one methyl substituent to the *tert*-butyl group (compound **48**) indicated that further structural modification might yield improved compounds. In addition, it was observed that combination of the *tert*-butyl group with different *ortho*-substituents enhanced potency several-fold (compounds **52-57**). The inventors combined these observations and further varied the structure of the *para-*substituent by derivatizing one of the methyl substituents of the *tert*-butyl group, initially in the absence of a further *ortho*-substituent (compounds **58-65),** followed by introduction of different *ortho*-substituents (compounds **66-70**). Most of the compounds in this series were approximately equipotent to *ortho-para-*disubstituted compounds **52** - **57.** Notably, replacement of one methyl group in the *tert-*butyl substituent by a carboxylic acid as in **65** was beneficial, and if combined with an *ortho*-methyl group yielded a very potent compound (see compound **70**)**.** Similar derivatization of **19** (R¹ = *para*-C=CH phenyl) yielded potent compounds with *ortho*-substituted *para-tert* butyl-phenyl sulfonamide such as **71** and **72.** Finally, also for alkinyl-substituted urethanes, replacement of one methyl group in the tert-butyl substituent of the sulfonamide by a carboxylic acid (compound **74**;) and additional introduction of an *ortho*-substituent (compound **75**) delivered potent compounds. Through this series of structure optimization, finally compounds such as **11, 19, 52, 65, 71, 72, 74, 75** were identified that displayed activity in the cell-based kynurenine assay in the low nanomolar range.

### Example B-2

### Live-cell Analysis

SKOV3 WT and IDO1-deficient cells were seeded in 48-well-plates (8000 cells/well, Corning, 3548) for 2D experiments or in a 96 ultra-low attachment plates (5000 cells/well, S-bio, MS-9096UZ) for 3D experiments. After seeding, cells were treated with 10 ng/mL (2D) or 1 ng/mL (3D) of IFN-γ and CellTox Green (Promega, G8731) was added in the culture media (1:6000). The following day, cells were treated with either DMSO or 5 µM of compound **1** (iDeg-1). Compound **1** was freshly added every three days. Cells were monitored by live phase-contrast microscopy using the IncuCyte S3 system (Sartorius) with the 10X objective, capturing images every day up to 4 days (2D) and 6 days (3D). Cell death was quantified by counting the numbers of green fluorescence positive cells (CellTox Green positive) normalized to the confluence of each respective image (2D) or by measuring the green signal mean intensity of each spheroid (3D).

### Immunoblotting

To analyze IDO1 levels, IFN-γ-stimulated BxPC3 cells (50 ng/mL IFN-γ), U2OS cells (5 ng/mL IFN-γ), HEK^{rhIDO1} (HEK293T cells electroporated with rhIDO1), SKOV3 and BT549 cells were treated with compounds for the indicated times (6 h to 24 h) followed by cell lysis (150 mM sodium chloride in 50 mM Tris (pH 8.0) and 1% NP-40 Alternative, protease and phosphatase inhibitors). Protein concentrations were determined (DC protein assay, Bio-Rad) followed by addition of 1 × SDS loading buffer and protein separation by a 10% SDS-PAGE. Separated protein samples were transferred to a polyvinylidene difluoride (PVDF) membrane using a wet-tank blotting system (Bio-Rad). IDO1 as well as vinculin as a loading were detected using the primary antibodies anti-IDO1 (ab211017, abcam or 14-9750-80, Thermo Fisher), anti-vinculin (V9131, Thermo Fisher) and anti-β-actin (ab8227, abcam) in 5 % milk in PBS-buffered saline with 0.05 % (v/v) Tween-20 (PBS-T). Primary antibodies were incubated at 4°C overnight and detected with IRDye-conjugated secondary antibodies (LI-COR Biosciences). IDO1 protein level in BT549 and SKOV3 cells was detected with HRP conjugated secondary antibody and SuperSignal^{™} West Dura Extended Duration Substrat (Thermo Fisher Scientific). Washing steps were performed with PBS-T. Protein bands were visualized by ChemiDoc^{™} MP (Bio-Rad) using a fluorescent readout. Band intensities were quantified using Image Lab software.

### Reverse Transcription-Quantitative PCR

BxPC3 cells (100,000 cells/well) or SKOV3 cells (150,000 cell/well) were seeded in a 24-well plate or 12-well plate, respectively. BxPC3 cells were incubated for 24 h prior to treatment with the indicated compound concentrations and 50 ng/mL IFN-γ. SKOV3 cells were directly treated with or without 10 µM succinyl acetone for 24 h prior to the addition of hemin for further 24 h. Total RNA was isolated by means of RNeasy Mini Kit as described by the manufacturer (Qiagen). Obtained RNA was reverse transcribed into cDNA by means of QuantiTect Reverse transcription kit according to the manufacturer's instructions (Qiagen) prior to the qPCR using QuantiFast SYBR green PCR kit (Bio-Rad). Gene-specific primers for *IDO1* (fw: 5'-GCCTGATCTCATAGAGTCTGGC-3' (SEQ ID No.: 4) and rv: 5'-TGCATCCCAGAACTAGACGTGC-3' (SEQ ID No.: 5)) and GAPDH (fw: 5'-GTCTCCTCTGACTTCAACAGCG-3' (SEQ ID No.: 6) and rv: 5'-ACCACCCTGTTGCTGTAGCCAA-3' (SEQ ID No.: 7)) as a reference gene were used. qPCR was performed using CFX96 Real-Time PCR Detection System (Bio-Rad). Data were analyzed using the CFX Manager (Bio-Rad). *IDO1* expression levels in each sample were normalized to the levels of the reference gene *GAPDH.* Relative quantification was performed using the 2^{-ΔΔCt} methods.

### IDO1 Promoter-Dependent Reporter Gene Assay

For the assay, the pXPG-IDO1 plasmid348 containing a firefly luciferase (Fluc) gene under the control of the full-length *IDO1* promoter (kindly provided by Gina M. Doody, Leeds, UK) was used as a reporter. The *Renilla* (Rluc) luciferase expression plasmid pRL-TK vector (Promega Corporation, US) was used as a control. HEK293T cells were transiently reverse-transfected with the plasmids using Lipofectamine^{™} 2000 (Invitrogen, US) following a modified protocol of the manufacturer's guidelines. 4 µg of the Fluc plasmid and 300 ng of the Rluc plasmid were mixed in serum-free Opti-MEM in a 1:3 ratio with Lipofectamine^{™} 2000. The mixture was added to 9 mL cell suspension at a cell density of 2.78 × 10⁵ cells/mL. 25,000 cells were seeded per well in a clear 96-well plate and incubated for 24 h. On the next day, *IDO1* promoter-mediated Fluc expression was induced by addition of 50 ng/mL IFN-γ with simultaneous compound treatment. Cells were incubated for 48 h prior to determining the activity of Fluc and Rlus using the Dual-Glo^{®} Luciferase Assay System with the Spark^{®} Multimode Microplate Reader (Tecan, AT). Fluc / Rluc ratios were normalized to the values of the DMSO control (set to 100 %).

### CHX Pulse Assay

BxPC3 cells (200,000 cells/well) were seeded in a 12-well plate and 50 ng/mL IFN-γ were added. After 24 h, 5 µM CHX was added 30 min prior to the addition of 5 µM compound 1 followed by cell lysis preparation and immunoblotting.

### TUBE Pulldown

Tandem ubiquitin binding entity protein (TUBE) was expressed from pGex-6P-1-GST-4xUBA-His (UBA: Ubiquitin-associated domains) in *E*. *coli* and purified by means of affinity purification using a Ni-NTA column. TUBE protein expression was induced by IPTG for 4 h at 30 °C. Afterwards cells were lysed in buffer containing 20 mM sodium phosphate (pH 7.4), 500 mM NaCl, 20 mM imidazole, 1 mM PMSF (Phenylmethylsulfonylfluorid, 39107.01, Serva) and 1 mM 2-mercaptoethanol and TUBE protein was separated using a Ni-NTA affinity chromatography. The TUBE protein was eluted by 20 mM sodium phosphate (pH 7.4) buffer containing 500 mM imidazole and 1 mM 2-mercaptoethanol and subsequently subjected to a size exclusion column (HiLoad 16/600 Superdex 200 pg) in 20 mM potassium phosphate buffer (pH 7.4, supplemented with 300 mM NaCl and 1mM DTE and 2 % Glycerol) to yield pure TUBE3.

For the TUBE pulldown, BxPC3 cells were seeded in either 10 or 15 cm² cell culture dishes and after cell attachment treated with 40 ng/mL IFN-γ to induce IDO1 expression overnight. Next, the medium was aspired and medium containing the respective concentration of compound **1** (iDeg-1) was added and incubated for 6 h at 37 °C and 5 % CO₂. In case of proteasome inhibition, 450 nM Carfilzomib (CFZ, ab216469, abcam) was added 1 h prior to addition of compound **1** (iDeg-1). After compound incubation, cell lysates were prepared in TUBE lysis buffer (20 mM Na₂HPO₄, 20 mM NaH₂PO₄, 1 % NP-40 Alternative, 2 mM EDTA and 10 mM N-ethylmaleimide (NEM, freshly prepared)) supplemented with 100 µg/mL TUBE protein. Samples were kept on ice for 30 min followed by a centrifugation step for 30 min at 16,000 × g at 4°C. Protein concentrations were determined to adjust protein concentration prior to the addition of 40 µL GST magnetic beads (ThermoFisher). Samples were rotated for 2 h at 4 °C followed by four washing steps using ice-cold PBS-T for 5 min. To elute ubiquitinated protein species, 20 µL 1x SDS sample buffer were added to the beads and incubated for 15 min at 55 °C. Afterwards, all samples were boiled for 5 min at 98 °C prior the separation by an SDS-PAGE and immunoblotting for IDO1 and vinculin as a control.

### Electroporation of rhIDO1 into HEK293T Cells

HEK239T (3,000,000 cell/per electroporation) cells were electroporated with 80 µg rhIDO1 utilizing the NeonTM transfection kit according to the manufactures description (ThermoFisher). For electroporation, 1000 V and 2 pulses for 35 ms were applied using the Neon^{™} transfection system pipette station. Afterwards, cells were washed in pre-warmed PBS followed by resuspension in 3 mL trypsin/EDTA solution and incubated for 3 min at 37 °C. Electroporated cells were washed again and resuspended in phenol red-free DMEM growth medium (900,000 cells/mL). For the Kyn assay in HEK^{rhIDO1} cells, cells were plated in 96-well plates and supplemented with 150 µM L-Trp and the respective compound concentrations for 24 h prior read out as described above for the manual Kyn assay. For immunoblotting, electroporated cells were seeded in a 12-well plate and treated with the indicated compounds for 6, 14 or 24 h prior to immunoblotting as described above. For co-treatment, cells were pre-treated with the proteasome inhibitor CFZ or the neddylation inhibitor MLN4924 for 30 min prior to compound **1** (iDeg-1) addition.

### HEK293T-based Kyn Assay

HEK293T (270,000 cell/mL) cells were transfected with 0.5 µg pCMV3-IDO1 (Sino Biological US Inc.) using Lipofectamine 2000 (Thermo Fisher) while seeding in a 96-well plate followed by incubation overnight. Next, 500 µM L-Trp and compounds at the indicated concentrations were added and cells were incubated for 24 h prior to the detection of Kyn levels using p-DMAB as described above for the manual Kyn assay.

### Proteome Profiling

HEK239T cells were electroporated with rhIDO1 as described above and treated with 10 µM compound **1** (iDeg-1) or DMSO for 6 h prior to lysate preparation by means of four freeze-thaw cycles in PBS containing 0.4 % NP-40 Alternative. Protein concentration was determined and 200 µg of protein lysates were subjected to the sample preparation and MS analysis. Protein lysates were dissolved in equal volume of 100 mM triethylammonium bicarbonate (TEAB) buffer supplemented with 7.5 µL of a 200 mM Tris(2-carboxyethyl)phosphine (TCEP) and incubated at 55°C for 1 h. Afterwards, 7.5 µL of 375 mM iodoacetamide were added and samples were incubated for another 30 min at room temperature in the dark prior to acetone-based protein precipitation (incubation at -20 °C overnight). On the next day, samples were centrifuged at 8,000 × g and 4 °C, the supernatant was aspired and protein pellets were dried at room temperature. Pellets were dissolved in 50 mM TEAB containing Trypsin/Lys-C (25:1 protein:protease ratio (w/w), pre-dissolved in 1 mM HCl (Trypsin/Lys-C Mix, Mass spec. grade, 5x from Promega Corporation)) and incubation at 37 °C overnight, while shaking. On the next day, samples were dried in a vacuum concentrator at 30 °C and subjected to MS.

### Mass Spectrometry (MS)

All solvents used during MS measurements were LC-MS grade. Digested samples were first dissolved in 120 µL of 20 mM ammonium formate (NH₄COOH) at pH 11 in an ultra-sonicator for 15 min followed a 15 min incubation step, while rotating and a subsequent centrifugation step (8,000 × g for 3 min at room temperature). 50 µL of each sample was separated into 10 fractions by means of a XBridge C18 column (130 Å, 3.5 µm, 1mm × 150 mm) using a U3000 capHPLC system (Thermo Fisher Scientific, Germany) under high pH conditions to reduce sample complexity an increase the number of quantified proteins. Separation was performed at a flow rate of 50 µl/min starting with 95% solvent A (20 mM NH₄COO pH 11 in H₂O) / 5% solvent B (40% 20 mM NH₄COO pH 11, 60% acetonitrile in H₂O) isocratic for the first 10 min, followed by a linear gradient increasing solvent B up to 25 % over 5 min. Afterwards, a second linear gradient was applied, increasing solvent B up to 65 % in 60 min, followed by a third gradient increasing solvent B up to 100% over 10 min. Fractions were detected at a wavelength of 214 nm.

The 10 fractions were collected 15 and 100 min (30 s per fraction, circular fractionation using 10 vials) and dried in a SpeedVac at 30°C until complete dryness for the final analysis by nanoHPLC-MS/MS. The dried samples were then dissolved in 10 µL of 0.1% TFA in water prior to the injection of 9 µL onto an UltiMateTM 3000 RSLCnano system (Thermo Fisher Scientific, Germany) online coupled to a Q Exactive^{™} HF Hybrid Quadrupole-Orbitrap Mass Spectrometer equipped with a nanospray source (Nanospray Flex Ion Source, Thermo Fisher Scientific).

To finally detect the peptides within the samples a mass range of m/z 300 to 1650 was attained with a resolution of 60000 for full scan prior to 15 high energy collision dissociation (HCD) MS/MS scans of the most intense at least doubly charged ions using a resolution of 15 000 and an NCE energy of 27%. The obtained data were analyzed using MaxQuant software (v.1.6.17.0)⁹ including the Andromeda search algorithm and using the human reference proteome of the Uniprot database. A full enzymatic trypsin cleavage search was conducted allowing for two miscleavages. Carbamidomethylation was chosen as fixed modification and oxidation of methionine and acetylation of the N-terminus as variable modifications.

For the first analysis, the mass accuracy for full mass spectra was set to 20 ppm while in the second search it was set to 4.5 ppm and for the MS/MS spectra to 20 ppm. A false discovery rate of 1 % was selected for peptide and protein identification and only proteins for which at least two peptides were quantified were chosen for further validation. Relative protein quantification was achieved by means of the label-free quantification algorithm implemented in MaxQuant.

To consider the differences of IDO1 concentration after electroporation of IDO1 into the HEK293T cells, all LFQ-intensities were normalized to those of the DMSO control of the same biological replicate by dividing the LFQ-intensity of the sample through that of the corresponding DMSO control (resulting in a value of 1 for the DMSO-controls themselves).

Statistical data analysis was completed using Perseus¹⁰ v.1.6.14.0. The normalized label-free quantification (LFQ) intensities were log-transformed (log2) and grouped. Only proteins were retained for further analysis when quantified at least two times in at least one of the groups. Missing values were ascribed to '0' and a t-test (FDR=0.01, s0=0.5) was conducted. Results were exported into an Excel file (xlsx) and visualized using Perseus. Detailed analysis procedure is described by Cox *et al.* and Tyanova *et al*.^{9,10}

Reduction of the kynurenine level in the cell-based model employed for initial SAR exploration can be due to inhibition of enzymatic activity, degradation of IDO1, or both (**Table B-2**). In order to differentiate between these different activities, we explored the modes of action of selected compounds in an *in vitro* assay employing recombinant human IDO1 (rhIDO1) monitoring enzymatic activity only, and we characterized the degradation efficiency by means of Western blotting, determining maximum degradation levels at 0.1µM (Dₘₐₓ) and half maximum degradation concentration (DC₅₀) (**Table B-2)**.

**Table B-2 in vitro inhibition and degradation of IDO1 by selected compounds. For the analysis of in vitro enzymatic IDO1 activity, recombinant human rhIDO1 protein (rhIDO1) was pre-incubated with the compounds at concentrations up to 59 µM at 37°C for 40 min prior to detection of Kyn levels using p-DMAB. Data are mean values ± SD (n = 4, except for compounds 1, 11, and 19 (n=2)). IC₅₀ values were determined if inhibition at 59 µM was higher than 50%. Inactive: no inhibition observed up to a concentration of 59 µM. IDO degradation was monitored in BxPC3 cells that were treated with 50 ng/ml IFN-γ for 24 h prior to washout, addition of compound (100 nM) for 24 h and detection of IDO1 protein levels using immunoblotting. Data are mean values of n biological replicates. Dₘₐₓ corresponds to maximum levels of IDO1 degradation at 0.1 µM.**

| **Cpd No.** | rhIDO1 enzymatic activity | | Dₘₐₓ [%] (n) *^{b}* | DC₅₀ ± SD [nM] *^{b,d}* |
|---|---|---|---|---|
| | Inhibition [%] *^{a,b}* | IC₅₀ ± SD [µM] *^{b}* | | |
| **1** (iDeg-1) | inactive | - | n.d | n.d |
| **11** (iDeg-2) | inactive | - | 37 ± 14 (3) | n.d |
| **19** (iDeg-3) | 49 ± 0.6 | - | 62 ± 10 (5) | n.d |
| **52** (iDeg-4) | 48 ± 6 | - | 65 ± 10(14) | n.d |
| **53** | 37 ± 9 | - | 56 ± 4 (3) | n.d |
| **54** | 35 ± 2 | - | 55 ± 4 (3) | n.d |
| **55** | n.d | n.d | 59 ± 16 (3) | n.d |
| **56** | 12 ± 7 | - | 56 ± 4 (3) | n.d |
| **57** | n.d | n.d | 55 ± 5 (3) | n.d |
| **63** | 67 ± 6 | 1.9 ± 0.5 | 36 ± 2 (3) | n.d |
| **65** (iDeg-5) | 72 ± 4 | 0.82 ± 0.14 | 46 ± 21 (5) | n.d |
| **67** | n.d | n.d | - (5)*^{c}* | n.d |
| **68** | inactive | - | n.d | n.d |
| **69** | 55 ± 2 | 0.27 ± 0.02 | 20 ± 12 (3) | n.d |
| **70** | 62 ± 1 | 0.11 ± 0.02 | 18 ± 19 (3) | n.d |
| **71** (iDeg-6) | 30 ± 5 | - | 74 ± 13 (8) | 6.5 ± 3.0 |
| **72** (iDeg-8) | 62 ± 5 | 4.0 ± 3.3 | 75 ± 7 (3) | 3.0 ± 3.5 |
| **73** | 37 ± 4 | - | 71 ± 11 (3) | n.d |
| **74** (iDeg-7) | 78 ± 3 | 0.31 ± 0.06 | 54 ± 8 (5) | n.d |
| **75** (iDeg-9) | n.d | n.d | 27 ± 14 (3) | n.d |

| | | | | |
|---|---|---|---|---|
| **a,** [%] in vitro inhibition at a concentration of 59 uM. Data are mean values (n = 4), except for compounds **1, 11,** and **19** (n=2). **b,** n.d: not determined. "-" **c,** No degradation observed at 100 nM. **d,** Half-maximal degradation concentration DC₅₀ values. Data are mean values ± SD (compound **71,** n = 4 except for 50 and 500 nM (n = 3)); compound **72,** n = 3 except for 0.1 nM (n = 2)). | | | | |

Under the conditions of the enzymatic assay compounds **1** and **11** which both embody a *tert*-butyl sulfonamide and a *para*-methyl or *para-*iodo-phenyl urethane respectively were not active up to 59µM concentration. But since compound **11** was 7-fold more potent in the Kyn assay than compound **1,** the inventors characterized degradation which revealed a maximum degradation level of 37% for compound **11.** compound **19;** *tert*-butyl-phenyl sulfonamide combined with alkinyl-phenyl urethane), and compound **52;** (*ortho*-methyl-*para*-*tert*-butyl-phenyl sulfonamide combined with iodo-phenyl urethane) were weak inhibitors but better degraders with Dₘₐₓ >60%). Variation of the *ortho*-substituent in the para-*tert*-butylphenyl sulfonamide in compounds **53-57** diminished both, enzyme inhibition and degradation efficiency (Dₘₐₓ 55-59%). Replacement of one methyl group in the tert-butylphenyl sulfonamide in compound **52** by a carboxylic acid yielded more potent inhibitors, including comopund **65,** but degradation efficiency was lower (compounds **63** and **65;** Dₘₐₓ of 36-46%), and introduction of a further ortho-substituent into the sulfonamide aryl group did not improve activity (compounds **67-69**). Notably, compounds **69** and **70** are potent inhibitors, but are not efficient in the cellular assay monitoring IDO1 degradation, which may in part be due to possibly limited cell penetration of these carboxylic acids. This may also hold true for compounds **74** and **75.** Highest degradation efficiency was displayed by compounds **71,** and **72** and their close analog **73.** These compounds embody an alkinyl-phenyl urethane and an *ortho-*substituted *para-tert*-butylphenyl sulfonamide, and were not identified as particularly potent inhibitors. For compounds **71** and **72,** the inventors determined that their half maximal degradation concentration is in the low nanomolar range, hence they are efficient degraders.

This analysis revealed compound **72** as compound with the best balance of properties. It shows an IC₅₀ value of 11 nM in the Kyn assay, inhibits the enzyme *in vitro* with IC₅₀ = 4 µM, reached a degradation efficiency of 75% at 100 nM concentration and its half maximal degradation concentration is 3 nM.

### IDO1 Immunoprecipitation Followed by MS Analysis

BxPC3 cells were seeded in 10 cm² dishes and stimulated with 40 ng/mL IFN-γ overnight at 37 °C and 5 % CO₂. On the next day, fresh medium was added containing 20 µM of compound **1** (iDeg-1) or DMSO a as control and incubated for 8 h prior cell detachment and cell lysis using immunoprecipitation lysis buffer (20 mM Tris-HCl pH 7.5, 100 mM NaCl, 0.1 mM EDTA and 0.5 % NP-40 Alternative) supplemented with fresh N-ethylmaleimide as well as phosphatase (PhosSTOP phosphatase inhibitors, Sigma-Aldrich) and protease inhibitors (cOmplete^{™} protease inhibitor cocktail, Sigma-Aldrich). Three freeze-and-thaw cycles were performed for cell lysis followed by centrifugation at 14,000 × g for 20 min at 4 °C and determination of the protein concentration (DC protein assay, Bio-Rad). 800 µg lysate was subjected to the immunoprecipitation.

For the following steps, a magnetic rack was used to separate magnetic beads from its supernatant. First, cell lysates were pre-cleared using 25 µL of Pierce Protein A/G Magnetic beads per sample for 1 h at 4°C while rotating. Afterwards, pre-cleared lysates were added to pre-coated Pierce Protein A/G Magnetic beads with IDO1 antibody (ab211017, abcam) followed by continuous rotation for 2 h at 4 °C. Beads were washed three times with immunoprecipitation wash buffer (50 mM PIPES pH 7.4, 150 mM NaCl, 5 mM MgCl₂, 0.5 mM EGTA, 0.1 % NP-40 alternative, 0.1 % Triton X-100, 0.1 % Tween^{®} 20 and 1 mM DTT) for 5 min while rotating. Subsequently, beads were transferred to a fresh sample tube after being washed using three times 500 µL PBS prior to further washing steps with 500 µL PBS (thrice) to remove detergents. Afterwards, beads were resuspended in 50 µL denaturation buffer (50 mM Tris pH 7.5, 8 M Urea and 1 mM DTT) and incubated for 30 min while shaking prior to addition of 5.55 µL 50 mM chloroacetamide solution for another 30 min while shaking. For on bead digestion, 1 µL LysC (stock solution of 0.5 µg/µL in water) was added for 1 h at 37 °C while shaking. The LysC digestion supernatant was transferred into a fresh sample tube and to the remaining beads 165 µL of a 50 mM Tris (pH 7.5) solution containing 0.25 µg trypsin was added and incubated for another 1 h at 37 °C while shaking. The two digestion solutions were combined and supplemented with additional 0.5 µg trypsin and the digestion was continued overnight at 37 °C while shaking. The remaining beads were discarded. On the next day, the addition of 20 µL 10 % TFA stopped the digestion and samples were desalted by means of stage tip purification. A double layer of C18 chromatography matrix was placed into 200 µL pipette tip and the matrix was activated by adding 100 µL of 100 % methanol. The matrix was washed once with buffer A (0.1 % formic acid, 80 % acetonitrile in H₂O) and twice with buffer B (0.1 % formic acid in H₂O). For each washing step, a centrifugation in a tip centrifuge was performed. Afterwards, samples were subjected to the tip washed once with buffer B prior sample elution using twice 20 µL buffer A. The samples were dried in a vacuum concentrator at 30 °C and analyzed by MS.

All solvents used during MS measurements were LC-MS grade. The samples were dissolved in 20 µl of 0.1% TFA in water and 3 µl were subjected to nanoHPLC-MS/MS analysis. Therefore, the samples were injected onto UltiMateTM 3000 RSLCnano system (ThermoFisher scientific, Germany) online coupled to a Q Exactive^{™} Plus Hybrid Quadrupole-Orbitrap Mass Spectrometer equipped with a nanospray source (Nanospray Flex Ion Source, Thermo Scientific). Samples were desalted by means of a pre-column cartridge (5 µm, 100 Å, 300 µm ID * 5 mm, Dionex, Germany) using 0.1% TFA in water and a flow rate of 30 µL/min for 5 min with eluent flow to waste followed by back-flushing of the sample during the whole analysis from the pre-column to the PepMap100 RSLC C18 nano-HPLC column (2 µm, 100 Å, 75 µm ID × 50 cm, nanoViper, Dionex, Germany). Therefore, a linear gradient starting with 95% solvent A (0.1% formic acid in H₂O) and 5% solvent B (0.1% formic acid in acetonitrile) was used which was increased to 30% solvent B over 90 min (flow rate 300 nL/min). Afterwards the column was washed (95% solvent B as highest acetonitrile concentration) and re-equilibrated to starting conditions. To finally detect the peptides within the samples the nano-HPLC was online coupled to the Quadrupole-Orbitrap Mass Spectrometer using a standard uncoated SilicaTip (ID 20 µm, Tip-ID 10 µM, New Objective, Woburn, MA, USA). A mass range of m/z 300 to 1650 was attained with a resolution of 70000 for full scan prior to 10 high energy collision dissociation (HCD) MS/MS scans of the most intense at least doubly charged ions using a resolution of 17,500 and an NCE energy of 25 %.

The obtained data were analyzed using MaxQuant software (v.1.6.17.0)⁹ including the Andromeda search algorithm and using a database containing the IDO1 (Uniprot accession number P14902) or the ubiquitin (Uniprot accession number P0CG48) sequence together with typical contaminants included in the MaxQuant software. The search was performed for full enzymatic trypsin cleavages allowing two miscleavages. For protein modifications carbamidomethylation was chosen as fixed and oxidation of methionine, acetylation of the N-terminus, and GlyGly of lysine as variable modifications. The mass accuracy for full mass spectra was set to 20 ppm (first search) and 4.5 ppm (second search), respectively and for MS/MS spectra to 20 ppm. The false discovery rates for peptide and protein identification were set to 1%. Results were exported into a txt-file and visualized using Prism 6.0 (GraphPad, USA). Detailed analysis procedure is described by Cox *et al*.⁹

### Cellular Thermal Shift Assay in Intact Cells (In Cell CETSA) ¹¹

SKOV3 cells were seeded into T-75 flask (or a 6-well plate for the isothermal dose-response experiment) 48 h prior to compound addition. On day 3, the indicated compound **1** (iDeg-1) concentrations or DMSO as a control were added for 1 h and incubated at 37 °C and 5 % CO₂ prior to cell detachment by means of trypsin. Cells were washed twice with PBS (4 °C, 300 × g). Afterwards, the cell pellet was resuspended in PBS and equally divided into ten different PCR samples tubes that were subjected to the gradient heat treatment (3 min) by means of the Mastercycler ep gradient (37, 40.8, 44.1, 47.4, 50.0, 53.4, 55.7, 56.3, 61.0 and 63.7°C).

For the isothermal dose-response of compound **1,** cells were heat-treated at a constant temperature of 50 °C. Afterwards, SKOV3 cells were placed on ice and cell lysis was performed by adding 0.4 % NP-40 prior to freeze-thaw lysis. After cell lysis, samples were centrifuged at 100,000 × g at 4 °C for 20 min to separate denaturated protein. Supernatants were collected and subjected to an SDS-PAGE and immunoblotting followed by immunostaining for IDO1. Obtained band intensities were quantified using Image Lab software and normalized to the intensities of the bands at 37°C. Melting temperatures in presence or absence of the test compound were determined via non-linear regression using Prism 6.0 (GraphPad, USA).

### Protein Translation Assay in Cells

BxPC3 cells (100,000 cells/well) were seeded in a 96-well plate and incubated at 37 °C and 5 % CO₂ overnight prior to addition of 25 ng/mL IFN-γ and the respective compounds for 24 h. Afterwards, cells were washed three times with pre-warmed PBS followed by Protein translation analysis using the Click-iT HPG Alexa Fluor 488 Protein Synthesis Assay kit (Thermo Fisher). Therefore, fresh medium containing the respective compounds as well as 50 µM L-homopropargylglycine (L-HPG) to tag newly synthesized proteins were added to the cells followed by incubation for 45 min at 37 °C and 5 % CO₂. Next, cells were fixed using 4 % para-formaldehyde and L-HPG was fluorescently label with Alexa 488 reagent provided by the assay kit using a click reaction as described by the manufacturer (Click-iT HPG Alexa Fluor 488 Protein Synthesis assay, Thermo Fisher). Cell nuclei were stained using the nuclei stain provided in the assay kit. Images were acquired at 10 × magnification using the microscope Axiovert 200M (Zeiss). Images were analyzed by means of MetaXpress software. The L-HPG-mediated fluorescence intensity as well as a cell count analysis were performed and the obtained data were normalized to the cell count and visualized by Fiji ImageJ. Data were plotted using Prism 6.0 (GraphPad, USA).

### In vitro IDO1 Translation Assay

Full length IDO1 cDNA was cloned into the pT7CFE vector (pT7CFE1-IDO1) and utilized in the *in vitro* translation assay by means of 1-Step Human Coupled DNA IVT kit (Thermo Fisher). To obtain proteins required for protein translation, HeLa cell lysate was prepared according to the manual of the manufacturer. Obtained lysates were combined with pT7CFE1-IDO1 plasmid and supplemented with the provided reagents of the 1-Step Human Coupled DNA IVT kit (Thermo Fisher). The reaction mixture was incubated for 3 h at 30 °C followed by immunoblotting for IDO1.

### Cloning and Purification of Recombinant IDO1 Protein and KLHDC3-EloBIC Complex

Full length human IDO1 cDNA was cloned into the pGEX6p-2rbs vector using EcoRI and Sall restriction sites. For protein translation assays, full length IDO1 cDNA was cloned into the pT7CFE vector (pT7CFE1-IDO1). Human recombinant IDO1 protein was expressed in *E.coli* according to Littlejohn *et al. ¹²* yielding pure and active IDO1 protein. IDO1 protein expression in *E.coli* was induced by the addition of 200 µM IPTG and cells were incubated at 18°C overnight. Harvested cells were resuspended in lysis buffer, disrupted by sonication and the lysate was centrifuged at 13,000 × g for 35 min at 10 °C. The supernatant was applied to a GST Trap HP column to enrich GST-tagged IDO1 equilibrated in 50 mM Tris-HCl, 150 mM NaCl and 1 mM DTE, pH 7.0. To obtain untagged full length IDO1, GST was cleaved from IDO1 overnight using PreScission protease. Eluted IDO1 protein was further purified using a size exclusion column (26/60 G75 HiLoad) to yield pure rhIDO1.

For hIDO1crystallization, a truncated version (5-400) of IDO1 was cloned into the pGEX6p-2rbs vector using the EcoRI and Sall restriction sites. Heme was removed by overnight incubation with 166 mM 2-mercaptoethanesulfonate (MESNA) to obtain apo-IDO1. Heme occupancy was determined by UV/VIS spectrophotometry. For the ubiquitination assays, IDO1 (residues 5-C) was cloned into a pRSF Duet based vector with a N-terminal His-TEV tag and purified as described above.

Expression constructs for KLHDC3-EloB/C (both monomeric and tetrameric versions) were previously described¹³. Briefly, KLHDC3, EloB, and His-TEV-EloC were first cloned into pLib via Gibson assembly. Cassettes were then generated via PCR as described (Weissman) and Gibson assembled into pBig1a to generate a single vector for co-expression of all components. Proper assembly into pBig1a was confirmed by Pmel and Swal restriction digestion.

KLHDC3-EloB/C was expressed in Hi5 insect cells with an N-terminal 6XHis tag on EloC as previously described¹³. Tagged proteins were purified from cell lysates by Ni affinity chromatography. Nickel elutions were directly loaded onto a HiTrap Q-HP column and eluted with a gradient of 2 %-50 % NaCl. Protein containing fractions were pooled, concentrated, and further purified by size exclusion chromatography in 25 mM HEPES, 200 mM NaCl, 1 mM DTT pH 7.5.

### Protein Modifications

To introduce a cysteine for fluorescent labeling of UB and K0UB we mutated the protein kinase A site in the pGEX2TK backbone converting the PKA site from RRASV to RRACV¹⁴. UB or UBKO purified from this expression construct was labeled with AlexaFluor-647-Maleimide or Fluorescein-5-Maleimide respectively as previously described¹⁴. Briefly, DTT was added to UB or UBKO at a final concentration of 10 mM and incubated on ice for 20 minutes to completely reduce cysteines for labeling. DTT was removed by buffer exchange over a NAP-5 column (GE Healthcare) in labeling buffer (25 mM HEPES, 200 mM NaCl). Labeling reactions consisted of UB or UBKO at 150 µM final concentration and were initiated by the addition of 600 µM AlexaFluor-647-Maleimide or Fluorescein-5-Maleimide (4X excess over labeling target and <5% final DMSO concentration). Reactions were incubated at room temperature for 2 h and quenched by the addition of DTT to 10 mM. Quenched reactions were desalted over a PD-10 column in labeling buffer containing 1mM DTT to remove unreacted probe. Desalted protein was concentrated and further purified over a Sephadex SD75 column

### IDO1 Enzymatic Assay

Full-length rhIDO protein (1 µM) in 50 mM potassium phosphate buffer (50 mM KH₂PO₄ and 50 mM K₂HPO₄, pH 6.5) was incubated with the respective compounds for 40 min at 37 °C prior to the addition of 10 mM ascorbic acid, 10 µM methylene blue, 2 mM Trp and 100 µg/mL catalase (Sigma-Aldrich, C100). Samples were incubated for 60 min followed by the addition of trichloroacetic acid to a final concentration of 7 % and further incubation for 30 min at 37 °C. Kyn levels were detected by the addition of an equal volume of 2 % (w/v) p-DMAB in acetic acid and absorbance was measured at 492 nm and 650 nm as a background control. Values are presented relative to the DMSO control. Dose-response curves and IC₅₀ values were generated and fitted with GraphPad Prism 6.0 (GraphPad software, USA) using four-parameter variable slope non-linear regression curve fit.

### Differential Scanning Fluorimetry (nanoDSF)

Full-length rhIDO1 (10 µM) in 100 mM potassium phosphate buffer (pH 6.5) was incubated with the respective compound or DMSO as a control at 37 °C for 3 h. Afterwards, the protein solution was loaded onto a capillary followed by the measurement of fluorescence intensities at 350 nm and 330 nm every 0.057°C from 20 °C up to 90 °C using the Prometheus NT.48 device. Melting temperatures were calculated by the Prometheus BT.48 software and melting curves with the maximum reporting Tm were visualized by GraphPad Prism 6.0 (GraphPad software, USA).

### UV/VIS analysis

Full-length rhIDO1 (10 µM) in 100 mM potassium phosphate buffer (pH 6.5) was incubated with the respective compounds or DMSO as a control for 3 h at 37°C followed by a centrifugation step for 10 min at 1000 × g. Subsequently, the UV/VIS spectra of the samples were monitored between 250 to 650 nm in 1 nm increments using UV transparent microplates (UV-STAR^{®}, Greiner AG, AT) by the Spark^{®} multimode microplate reader (Tecan, Austria). Absorbance values were normalized to the absorbance at 280 nm (A₂₈₀) and ratios were plotted by GraphPad Prism 6.0 (GraphPad software, USA).

### Isothermal Titration Calorimetry

All experiments were performed on a MicroCal AutolTC200. KLHDC3-EloB/C was buffer exchanged by desalting over a NAP5 column in ITC buffer (25 mM HEPES, 150 mM NaCl, 1 mM 2-mercaptoethanol, pH 7.5). Lyophilized peptides were dissolved in ITC buffer to a final concentration of 1 mM. Titrations contained KLHDC3-EloB/C in the sample cell at 24 µM and 240 µM test peptide in the syringe. Titrations were performed at 25°C with one injection of 0.4 µL, followed by 12 injections of 3 µL. Experiments were performed n = 2 times, and data evaluated with MicroCal PEAQ software.

### Co-crystallization of Apo-IDO1 Protein with compound 11 (iDeg-2)

10 mg/mL (225 mM) of truncated IDO1 protein was incubated with 450 mM iDeg-2 (1.8% (v/v) DMSO) in binding buffer (25 mM Tris pH 8.0, 100 µM tris(2-carboxyethyl)phosphine (TCEP) at 37°C for 2 h. 0.4 µL of the rhIDO1: compound **11** (iDeg-2) solution was mixed with 0.2 µL of precipitant solution in a sitting drop setup (iQ plates, SPT Labtech, UK) at 20 °C. Crystals were obtained in wells with precipitant solution containing 41 % (v/v) PEG200 in 50 mM MES pH 6.95 Crystals appeared in one day as thin bars. After 5 days, crystals were harvested and frozen in liquid nitrogen using the precipitant solution as cryoprotectant. X-ray diffraction data collection was carried out at ESRF (Grenoble, FR) on beamline ID30B. Data were processed with autoPROC and STARANISO (Global Phasing Limited)¹⁵. The structure was solved by molecular replacement with Phaser¹⁶ from the Phenix suite¹⁷ using as initial model the coordinates of PDB 8abx. Iterative refinement cycles were carried out using PHENIX and COOT¹⁸. The figures were generated using the PyMOL Molecular Graphics System (Version 2.5.4, Schrödinger, LLC). LigPlot+¹⁹ was used to analyze protein ligand interactions. The data collection and refinement statistics are presented in Table B-3.

**Table B-3: Data collection and refinement statistics of apo-IDO1 with compound 11 (iDeg-2) structure. Values in parentheses correspond to the highest-resolution shell.**

| | | **apo-IDO1 + compound 11 (iDeg-2)** |
|---|---|---|
| **Data collection** | | |
| Space group | | P 2₁ 2₁ 2 |
| Cell dimensions | | |
| | *a*, *b, c* (Å) | 104.84 110.42 |
| | | 36.14 |
| | α, β, γ (°) | 90 90 90 |
| Resolution range (Å) | | 34.73-1.60 (1.66-1.60) |
| Total reflections | | 97114 (2512) |
| Unique reflections | | 48558 (5574) |
| *Rₘₑₐₛ* | | 0.048 (0.612) |
| *R*ₚᵢₘ | | 0.034 (0.433) |
| CC1/2 | | 1.00 (0.69) |
| *I* / σ*I* | | 13.57 (1.42) |
| Completeness (%) | | 99.96 (99.95) |
| Redundancy | | 2.00 (0.45) |

| **Refinement** | | |
|---|---|---|
| Resolution (Å) | | 1.6 |
| No. reflections | | 56419 (5572) |
| *R*_{work} / *R*_{free} | | 0.171 / 0.189 |
| No. of non -H atoms | | 3272 |
| | Protein | 2927 |
| | Ligand/ion | 231 |
| | Water | 243 |
| *B*-factors | | 29.13 |
| | Protein | 27.87 |
| | Ligand | 44.16 |
| | Water | 38.04 |

| R.m.s. deviations | | |
|---|---|---|
| | Bond lengths (Å) | 0.12 |
| | Bond angles (°) | 1.67 |

The co-crystal structure of apo-IDO1 in complex with iDeg-2 at 1.6 Å resolution revealed that compound 11 (iDeg-2) occupies the heme binding site in apo-IDO1 and induces a large conformational rearrangement (Fig. 2a-d). The phenyl carbamate occupies the previously identified lipophilic pocket A in the distal heme site, while the pyrrolidine and the sulfonyl group occupy the heme-binding pocket. The monoterpene scaffold only slightly protrudes into the D-pocket, and the tert-butyl phenyl group is located in the solvent-exposed B-pocket (Fig. 2b). Binding to this pocket has been observed before only for holo-IDO1 inhibitors (Fig. 2c). Compound **11** (iDeg-2) binding occurs though a large number of hydrophobic interactions (Y126, C129, F163, V167, E171, F214, I217, D226, G262, S263, A264, Q266, S267, F270, H346 and I349), a water-bridged hydrogen bond between the carbamate nitrogen and the hydroxyl group of S167 and a hydrogen bond between the sulphonyl oxygen of iDeg-2 and H346, which coordinates heme in holo-IDO1 (Fig. 2e-f). In the presence of iDeg-2, there was no detectable electron density for the C-terminal K-helix (Fig. 2d). An overlay of the structures of IDO1 in complex with compound **11** and the apo-IDO1 inhibitor linrodostat revealed substantial shifts in helices B, C, H and J and a distortion in the J-helix in the compound **11** complex. The J-helix C-terminus moves toward the binding pocket, while the N-terminus shifts away from the K-helix interface, most likely weakening the interaction of the K-helix with the J- and H-helices, and the K-helix, bearing lysine 389, becomes flexible. Previously reported IDO1 inhibitors do not alter the overall structure of IDO1 compared to holo-IDO1, indicating that this unique binding mode of the iDegs results in a novel conformational rearrangement in IDO1.

### Ubiauitination Assays

The use of pulse-chase assays allowed comparing the paths of UB transfer starting from UBE2R2 or UBE2D2. First, UBE2R2 or UBE2D2 was pulse-labeled by incubating a mixture of UBA1 (0.3 µM), E2 (10 µM), and fluorescently labeled UB or KOUB (15 µM) in 25 mM HEPES, 100 mM NaCl, 100 mM MgCl₂, 2 mM ATP, pH 7.5 at room temperature for 13 min. Pulse-loading reactions were quenched by the addition of EDTA to 50 mM and incubated on ice for 5 min. Ubiquitylation chase reactions consisted of mixing the E2-*UB thioester conjugate (0.4 µM final concentration) with the indicated pre-equilibrated NEDD8-CUL2-RBX1-KLHDC3-EloB/C (0.3 µM final concentration) with a threefold excess of IDO1 variants in 25 mM HEPES, 100 mM NaCl, 50 mM EDTA, 0.5 mg/mL BSA, pH 7.5 at room temperature. Experiments following loading of Apo IDO1 with small molecules consisted of incubating IDO1 (15 µM final concentration) with a twofold excess of the indicated test molecules for 1.5 h at 37°C. Mixtures were pelleted for 10 min at 14,000 rpm to remove insoluble material. A UV-Vis spectrum of the resulting supernatant confirmed heme incorporation and the samples were directly diluted into ubiquitylation reactions. Reactions were quenched at the indicated times with 2X SDS-PAGE sample buffer and separated on 4-12 % Bis-Tris gradient gels and scanned for fluorescence on a Typhoon imager. All experiments, except where indicated, utilized a monomeric version of KLHDC3-EloB/C.

### Reporter and sgRNA Generation

The N-terminal stability reporter was generated starting from the previously reported C-terminal stability vector.^{20,21} The backbone was digested using Sall and Mlul (NEB), while the mCherry and TagBFP inserts were amplified using Phusion^{®} High-Fidelity DNA Polymerase following the manufacturer's instructions and using the primers as indicated. Fragments were subsequently assembled using NEBuilder ^{®} HiFi DNA Assembly Cloning Kit. The Mlul restriction site was maintained and utilized for the insertion of the various IDO1 constructs amplified with the primers as indicated and again assembled using the NEBuilder ^{®} HiFi Kit. As starting material for the genetic versions of IDO1, the Addgene plasmid #187026 (pcDNA3.1-IDO1-p2a-eGFP gifted by Geert van den Bogaart)²¹ was used. The custom-made sgRNA library targeting elements of the ubiquitin proteasome system was generated as previously described in Hsia et al²⁰. Single sgRNAs were designed using the VBC score²³ and ordered as custom oligos (Sigma). After phosphorylation (T4 PNK, NEB) and annealing of the oligos, the NEBridge^{®} Golden Gate Assembly Kit (BsmBl-v2) was used for insertion of the respective sgRNAs. sgRNAs were cloned into pLenti-U6-sgRNA- IT-EF1αs-THY1,1-P2A-NeoR.²⁰ Insertion of the correct DNA sequence was verified by Sanger Sequencing (Microsynth, Austria).

### Virus Production, Transductions and Cell Line Generation for CRISPR-Cas9-based Screen

Lenti-X cells were transfected at 70 % confluence with the reporter or sgRNA plasmids as well as the two packaging plasmids (pCMVR8.74 helper, and pMD2.G envelope) using polyethylenimine (PEI MAX^{®} MW 40,000, Polysciences). pCMVR8.74 was a gift from Didier Trono (Addgene plasmid # 22036; http://n2t.net/addgene:22036 ; RRID:Addgene_22036). pMD2.G was a gift from Didier Trono (Addgene plasmid # 12259; http://n2t.net/addgene:12259 ; RRID:Addgene_12259). The virus was collected and filtered using a 0.45 µm polyethersulfone filter. One million KBM7 iCas9 cells or KBM7 iCas9 IDO1 N-terminal stability reporter cells per 2 mL were transduced with varying volumes of virus solution and incubated with 8 µg/mL polybrene for 24 h prior to cell expansion. Cells were selected either via FACS using a CytoFLEX SRT Benchtop Cell Sorter (for reporter cell lines: BFP and mCherry positive cells) or using G418 (1 mg/mL, Gibco) for sgRNA harboring cell lines, which was added 72 h after the transduction. Knockout (KO) cell lines were generated by selection of the respective sgRNA (sgAAVS1 = CTRL or sgRNA1/2 KLHDC3 for KO1/KO2) for at least 14 days with G418 followed by subsequent induction with doxycycline (0.4 µg/mL, PanReac AppliChem) and cell recovery.

### Flow-cytometric IDO1 Reporter Assay

IDO1 KBM7 reporter cells were treated for the times, compounds and concentrations indicated in the respective figure legends or as specified in the text. All compounds were diluted starting from DMSO stock solutions. BFP and mCherry levels were measured using an LSR Fortessa (BD Biosciences) with the BD FACSDiva software (v9.0). To quantify the changes across conditions the mean BFP and mCherry values were exported after gating for singlets and reporter positive cells utilizing FlowJo 10.6.2.. Normalization was performed either against the respective *wt IDO1* reporter cells treated with DMSO (Fig. 3h), their respective co-treatment (Fig. 4b,) or for each respective genotype (Fig. 4a,b, Fig. 3i, Fig. 5f,i).

### FACS-based CRISPR Cas9 Screen

The screen, library preparation and sequencing analysis was performed as previously described.²⁰ In brief, 120 million KBM7 iCas9 IDO1 N-terminal stability reporter cells were transduced at an MOI of ~ 0.12 sgRNA positive cells (> 1,000-fold library representation, Fig. 6e) with the customized ubiquitin focused library. Transduced cells were selected with G418 (1 mg/mL, Gibco) for 14 days, expanded and Cas9 expression was induced with doxycycline (0.4 µg/mL, PanReac AppliChem). Three days after Cas9 induction, 50 million cells per condition and replicate were treated with DMSO, compound **1,** or **11** (iDeg-1 or iDeg-2). The experiment was performed in two biological replicates. Cells were washed with PBS, stained with APC anti-mouse Thy1.1 antibody (1:400, 202526, BioLegend) and Human TruStain FcX Fc Receptor Blocking Solution (1:1000, 422302, BioLegend) for 5 min at 4°C. Subsequently cells were fixed with BD Fixation buffer 4 % (Thermo Scientific^{™} Pierce^{™}) for 45 min at 4°C. All steps were performed protected from light. Cells were washed twice with PBS and stored in FACS buffer (PBS, 5 % FBS and 1 mM EDTA).

For sorting, cells were strained using a 35-µm nylon mesh and sorted on a BD FACSAria Fusion (70 µm nozzle, BD Biosciences) with the BD FACSDiva software (v8.0.2). Singlets, Cas9 and sgRNA positive cells were selected following the scheme shown in Fig. 6a-d. Next, fractions with different levels of BFP were enriched: the 5 % highest or lowest BFP expressing cells were used for the respective HIGH and LOW fractions, and 30 % of the population's center was selected for the MID fractions. For each replicate, cells corresponding to at least a 1,000-fold library representation were sorted.

To quantify sgRNA levels per each fraction and sample next generation sequencing (NGS) libraries were prepared. Genomic DNA was isolated by cell lysis (10 mM Tris-HCl, 150 mM NaCl, 10 mM EDTA, 0.1 % SDS) and proteinase K treatment (New England Biolabs) overnight at 55°C shaking at 1200 rpm, followed by 2 h of DNAse-free RNAse (Thermo Fisher Scientific) incubation at 37°C. Next, DNA was extracted by two rounds of phenol extraction and subsequent isopropanol precipitation. Barcoded NGS libraries for each sorted population were generated using a two-step PCR protocol using AmpliTaq Gold polymerase (Invitrogen). The resulting PCR products were purified after each step using Mag-Bind TotalPure NGS beads (Omega Biotek). The final NGS libraries were pooled and sequenced on a NovaSeq 6000 platform (Illumina).

Sequencing reads were trimmed using fastx-toolkit (v0.0.14), aligned using Bowtie2 (v2.4.5) and quantified using featureCounts (v2.0.1). The utilized workflows are available at https://github.com/ZuberLab/crispr-process-nf/tree/566f6d46bbcc2a3f49f51bbc96b9820f408ec4a3 and https://github.com/ZuberLab/crispr-mageck-nf/tree/c75a90f670698bfa78bfd8be-786d6e5d6d4fc455. To calculate gene-level enrichment, the sorted populations (HIGH or LOW) were compared to the MID populations using the median-normalized read counts.

### References:

7 Hennes, E. et al. Cell-Based Identification of New IDO1 Modulator Chemotypes. Angewandte Chemie (International ed. in English) 60, 9869-9874 (2021)
8 Livak, K. J. & Schmittgen, T. D. Analysis of relative gene expression data using real-time quantitative PCR and the 2(-Delta Delta C(T)) Method. Methods 25, 402-408 (2001).
9 Cox, J. & Mann, M. MaxQuant enables high peptide identification rates, individualized p.p.b.-range mass accuracies and proteome-wide protein quantification. Nature Biotechnology 26, 1367-1372 (2008).
10 Tyanova, S. & Cox, J. Perseus: A Bioinformatics Platform for Integrative Analysis of Proteomics Data in Cancer Research. Methods Mol Biol 1711, 133-148 (2018).
11 Kawatkar, A. et al. CETSA beyond Soluble Targets: a Broad Application to Multipass Transmembrane Proteins. ACS Chemical Biology 14, 1913-1920 (2019).
12 Littlejohn, T. K. et al. Expression and purification of recombinant human indoleamine 2, 3-dioxygenase. Protein Expr Purif 19, 22-29 (2000).
13 Scott, D. C. et al. E3 ligase autoinhibition by C-degron mimicry maintains C-degron substrate fidelity. Mol Cell 83, 770-786 e779 (2023).
14 Scott, D. C. et al. Structure of a RING E3 trapped in action reveals ligation mechanism for the ubiquitin-like protein NEDD8. Cell 157, 1671-1684 (2014).
15 Vonrhein, C. et al. Data processing and analysis with the autoPROC toolbox. Acta Crystallogr D 67, 293-302 (2011).
16 McCoy, A. J. et al. Phaser crystallographic software. J Appl Crystallogr 40, 658-674 (2007).
17 Liebschner, D. et al. Macromolecular structure determination using X-rays, neutrons and electrons: recent developments in Phenix. Acta Crystallogr D Struct Biol 75, 861-877 (2019).
18 Emsley, P., Lohkamp, B., Scott, W. G. & Cowtan, K. Features and development of Coot. Acta Crystallogr D Biol Crystallogr 66, 486-501 (2010).
19 Laskowski, R. A. & Swindells, M. B. LigPlot+: Multiple Ligand-Protein Interaction Diagrams for Drug Discovery. J Chem Inf Model 51, 2778-2786 (2011).
20 Hsia, O. et al. Targeted protein degradation via intramolecular bivalent glues. Nature 627, 204-211 (2024).
21 Xue, G. et al. Discovery of a Drug-like, Natural Product-Inspired DCAF11 Ligand Chemotype. Nat Commun 14, 7908 (2023).
22 Maassen, S. et al. Mitochondrial interaction of fibrosis-protective 5-methoxy tryptophan enhances collagen uptake by macrophages. Free Radical Bio Med 188, 287-297 (2022).
23 Michlits, G. et al. Multilayered VBC score predicts sgRNAs that efficiently generate loss-of-function alleles. Nat Methods 17, 708-716 (2020).

## Claims

1. A compound of the formula (**I**) 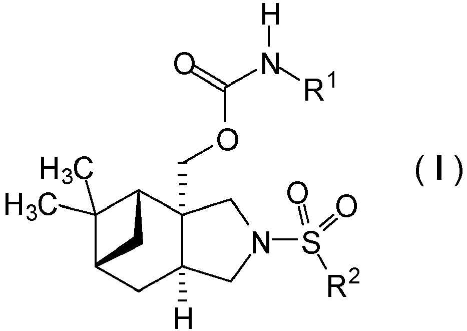 wherein
**R¹** represents 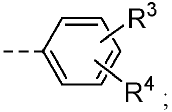
**R²** represents C₃-C₆ alkyl, C₃-C₆ cycloalkyl, 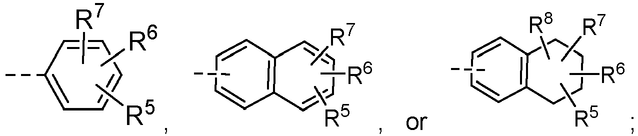
wherein C₃-C₆-cycloalkyl may be substituted with 1 to 4 substituents selected from **R⁵**, **R⁶, R⁷** and **R⁸**;
**R³**, and **R⁴** represent independently of each other -H, -Br, -Cl, -I, -CN, -CH₃, -C₂H₅, -CHF₂, -CF₃, -OCH₃, -OCHF₂, -OCF₃, -NH₂, -NHCH₃, -CH=CH₂, -CH₂-CH=CH₂, -C(CH₃)=CH₂, -CH=CH-CH₃, -CH=CH-C₂H₅, -CH=CH-C₂H₄OH, -CH=CH-C₃H₇, -C≡CH, -C≡C-CH₃, -C≡C-C₂H₅, -C≡C-C₂H₄OH, -C≡C-C₃H₇, -CH₂-C≡CH, -CH₂-C≡C-CH₃, -CH₂-C≡C-C₂H₅, -CH(CH₃)-C≡CH, -CH₂-C≡C-C₃H₇, or -C≡C-CH₂-CH(CH₃)₂;
**R⁵**, **R⁶**, **R⁷**, and **R⁸** represent independently of each other -H, -CH₃, -C₂H₅, -C₃H₇, -CH(CH₃)₂, -C₄H₉, -CH₂-CH(CH₃)₂, -CH(CH₃)-C₂H₅, -C(CH₃)₂**R⁹**, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -cyclo-C₃H₅, -cyclo-C₄H₇, -cyclo-C₅H₉, -cyclo-C₆H₁₁, -cyclo-C₇H₁₃, -cyclo-C₈H₁₅, -cycloC₃H₅O, -CH₂F, -CHF₂, -CF₃, -CH₂-CH₂F, -CH₂-CHF₂, -CH₂-CF₃, -Ph, -CH₂-Ph, -CH₂-CH₂-Ph, -CH=CH-Ph, -Si(CH₃)₃, -Si(CH₃)₂CH₂CH₃, -Si(CH₃)₂CH(CH₃)₂, -Si(CH₂CH₃)₃, -Si(CH₃)₂(C(CH₃)₃), -OH, -OCH₃, -OC₂H₅, -OC₃H₇, -O-cyclo-C₃H₅, -OCH(CH₃)₂, -OC(CH₃)₃, -OC₄H₉, -OPh, -OCH₂-Ph, -OCPh₃, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -F, -Cl, -Br, -I, -CN, -COCH₃, -COC₂H₅, -COC₃H₇, -CO-cyclo-C₃H₅, -COCH(CH₃)₂, -COC(CH₃)₃, -COOH, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCH₂F, -OCHF₂, -OCF₃, -CH₂-OCF₃, -C₂H₄-OCF₃, -C₃H₆-OCF₃, -CH₂-OCHF₂, -C₂H₄-OCHF₂, -C₃H₆-OCHF₂, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CO-NHC₃H₇, -NH-C(=NH)-NH₂, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂], -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CON(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH=CH-CH=CH-CH₃, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)_{2]}=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH(CH₃)-CH₂-C≡CH, -CH(CH₃)-C≡C-CH₃, -C₂H₄-CH(CH₃)-C≡CH, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -C≡CH, -C≡C-CH₃, -CH₂-C≡CH, -C₂H₄-C≡CH, -CH₂-C≡C-CH₃, -C≡=C-C₂H₅, -C₃H₆-C≡CH, -C₂H₄-C≡C-CH₃, -CH₂-C=C-C₂H₅, -C≡C-C₃H₇, -CH(CH₃)-C≡CH, -C₄H₈-C≡-CH, -C₂H₄-C≡C-C₂H₅, -CH₂-C≡C-C₃H₇, -C≡C-C₄H₉, -C≡C-CH₂-CH(CH₃)₂, -CH(CH₃)-C₂H₄-C≡CH, -CH₂-CH(CH₃)-C≡C-CH₃, -C(CH₃)(C₂H₅)-C≡CH, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -CH₂-C≡C-C≡C-CH₃, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -CH₂-CH(C≡CH)₂, -C≡C-C≡H, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -C≡C-C(CH₃)₃, -C≡C-CH₂-C≡C-CH₃, -C≡C-C≡C-C₂H₅, 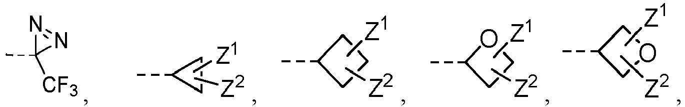 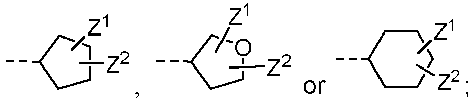
**R⁹** represents -CH₃, -CH₂F, -CHF₂, -CF₃, -C₂H₅, -C₃H₇, -CH(CH₃)₂, -C₄H₉, -CO₂H, -CO₂CH₃, -CO₂CH₂CH₃, -CO₂CH₂CH₂CH₃, -CO₂CH(CH₃)₂, -C0₂C(CH₃)₃, -CO₂-cyclo-C₃H₅, -CO₂-CH₂-cyclo-C₃H₅, -CH₂CO₂H, -CH₂CO₂CH₃, -CH₂CO₂CH₂CH₃, -CONH₂, -CONHCH₃, -CONHCH₂CH₃, -CONHCH₂CH₂CH₃, -CONHCH(CH₃)₂, -CONHC(CH₃)₃, -CON(CH₃)₂, -CON(CH₃)(CH₂CH₃), -CON(CH₂CH₃)₂, -CON(CH₂CH₂CH₃)₂, CON(CH(CH₃)₂)₂, -CH₂N(CH₃)₂, -CH₂N(CH₃)(CH₂CH₃), -CH₂N(CH₂CH₃)₂, -CH₂N(CH₂CH₂CH₃)₂, -CH₂N(CH(CH₃)₂)₂, -CH₂O(CH₂)₂OCH₃, or
or 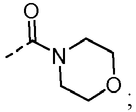
**Z¹,** and **Z²** represent indepenently of each other -H, -F, -Cl, -Br, -CH₃, -C₂H₅, -CH₂F, -CHF₂, -CF₃, -OCH₃, -OC₂H₅, -OCH₂F, -OCHF₂, -OCF₃, -CH₂-CF₃, or-CF₂-CF₃;
or an enantiomer, a diastereomer, a tautomer, a mixture of enantiomers, a mixture of diastereomers, a mixture of tautomers, a hydrate, a solvate, a pharmaceutically acceptable salt of the above-mentioned compound.

2. The compound according to claim 1, wherein 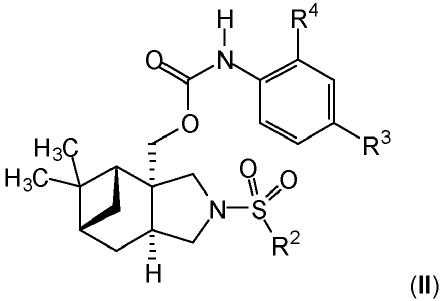 and **R², R³** and **R⁴** have the same meanings as defined in claim 1.

3. The compound according to claim 1 or 2, wherein 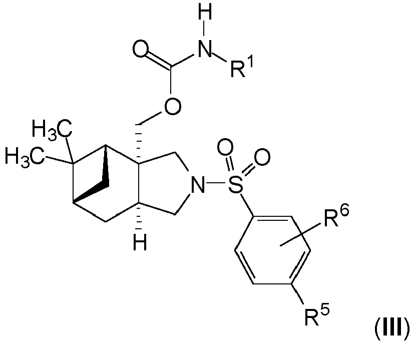 and **R¹, R⁵** and **R⁶** have the same meanings as defined in claim 1.

4. The compound according to any one of the claims 1 - 3, wherein 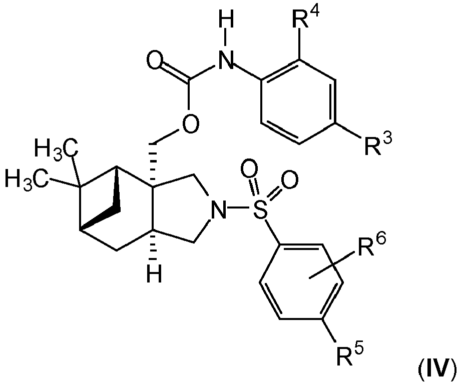 and **R³, R⁴, R⁵** and **R⁶** have the same meanings as defined in claim 1.

5. The compound according to any one of the claims 1 - 4, wherein the compound has any one of the following formulae (**IV-1**) to (**IV-4**) and (**V-1**) - (**V-4**): 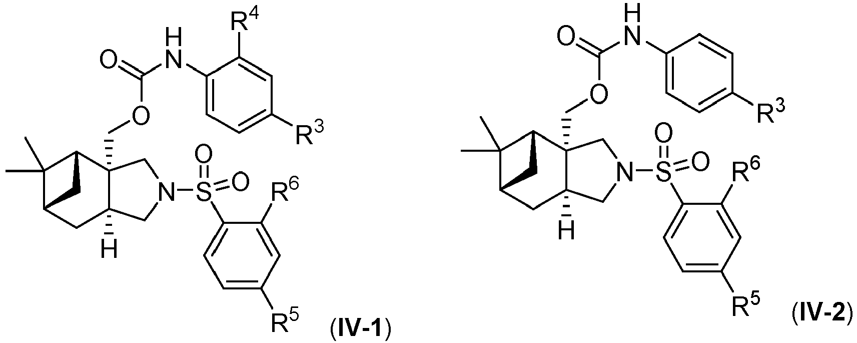 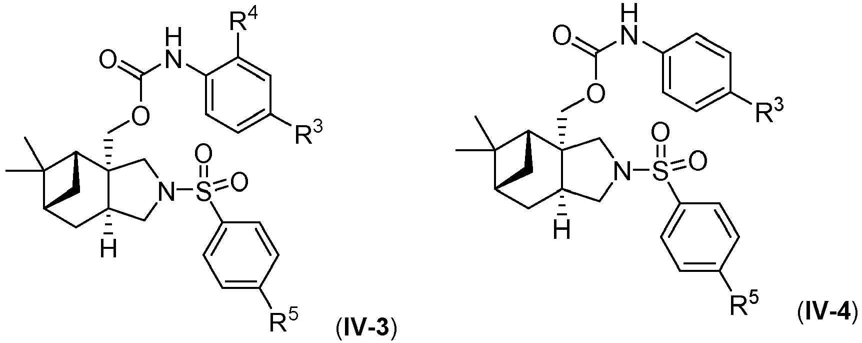 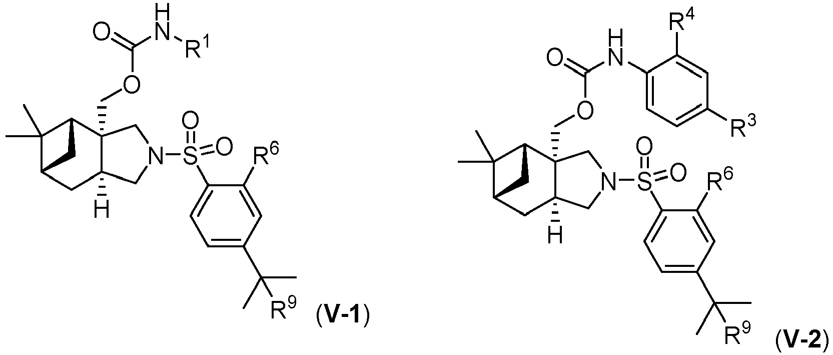 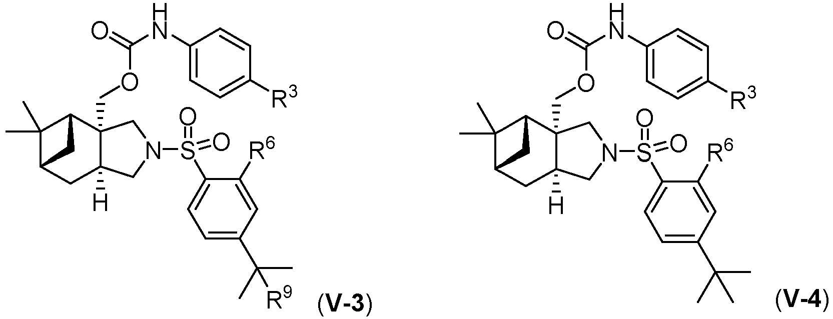 wherein **R³, R⁴, R⁶, R⁶**and **R⁹** have the same meanings as defined in claim 1.

6. The compound according to any one of the claims 1 - 4, wherein
**R¹** represents 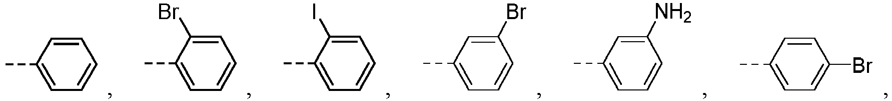 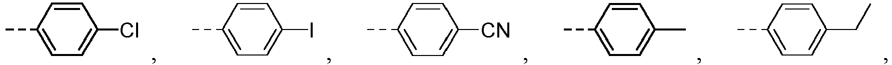 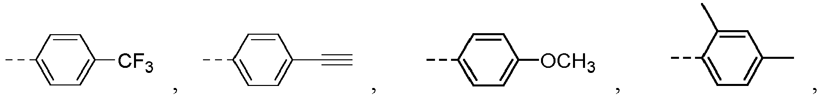 or 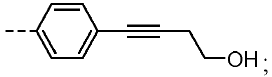
**R²** represents , 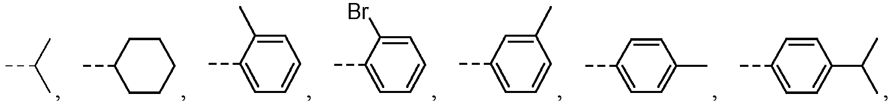 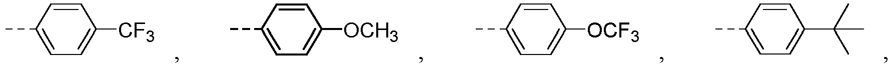 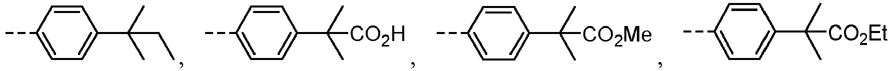 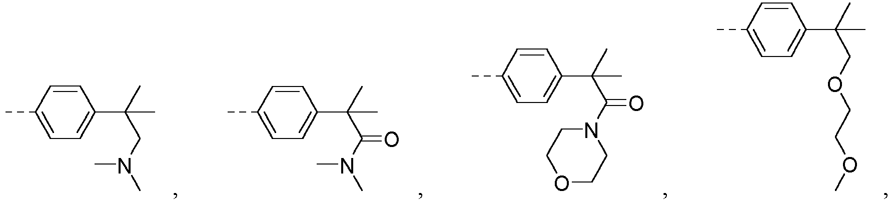 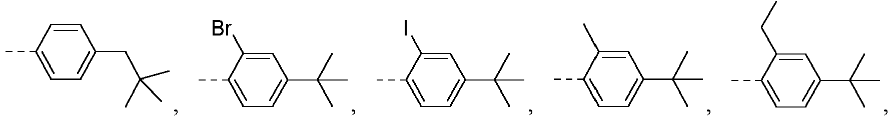 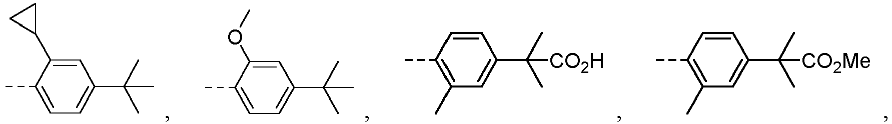 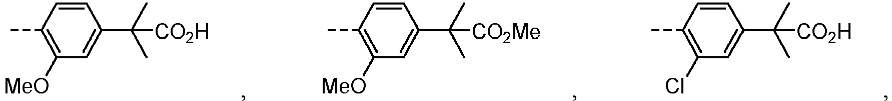 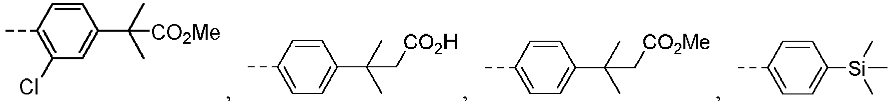 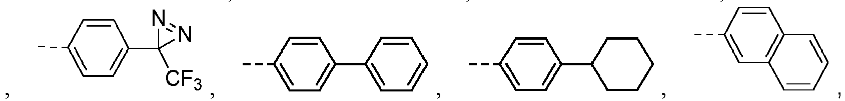 or

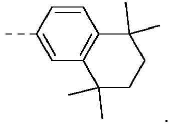

7. A compound selected from the group consisting of:
| **Cpd No.** | **IUPAC-Name** |
|---|---|
| **1** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl *p*-tolylcarbamate |
| **2** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-ethylphenyl)carbamate |
| **3** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-isopropylphenyl)carbamate |
| **4** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-(*tert*-butyl)phenyl)carbamate |
| **5** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl [1,1'-biphenyl]-4-ylcarbamate |
| **6** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl phenylcarbamate |
| **7** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (2,4-dimethylphenyl)carbamate |
| **8** | ((3aS,4R,6R,7aS)-2-((4-(*tert*-butyl)phenyl)sulfonyl)-5,5-dimethyloctahydro-3a*H*-4,6-methanoisoindol-3a-yl)methyl (4-fluorophenyl)carbamate |
| **9** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-chlorophenyl)carbamate |
| **10** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-bromophenyl)carbamate |
| **11** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-iodophenyl)carbamate |
| **12** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (2-iodophenyl)carbamate |
| **13** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (2-bromophenyl)carbamate |
| **14** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (3-bromophenyl)carbamate |
| **15** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (3-aminophenyl)carbamate |
| **18** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-(4-hydroxybut-1-yn-1-yl)phenyl) carbamate |
| **19** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-(4-ethynylphenyl)carbamate |
| **20** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-methoxyphenyl)carbamate |
| **21** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-(trifluoromethyl)phenyl)carbamate |
| **23** | ((3a*S*,4*R*,6*R*,7a*S*)-2-((4-(*tert*-butyl)phenyl)sulfonyl)-5,5-dimethyloctahydro-3a*H*-4,6-methanoisoindol-3a-yl)methyl (4-cyanophenyl)carbamate |
| **30** | ((3a*S*,4*R*,6*R*,7a*S*)-2-(isopropylsulfonyl)-5,5-dimethyloctahydro-3a*H*-4,6-methanoisoindol-3a-yl) methyl *p*-tolylcarbamate |
| **31** | ((3a*S*,4*R*,6*R*,7a*S*)-2-(cyclohexylsulfonyl)-5,5-dimethyloctahydro-3a*H*-4,6-methanoisoindol-3a-yl)methyl *p*-tolylcarbamate |
| **32** | ((3a*S*,4*R*,6*R*,7a*S*)-5,5-dimethyl-2-((4-(trifluoromethyl)phenyl)sulfonyl) octahydro-3a*H*-4,6-methanoisoindol-3a-yl)methyl *p*-tolylcarbamate |
| **34** | ((3a*S*,4*R*,6*R*,7a*S*)-5,5-dimethyl-2-((4-(trifluoromethoxy)phenyl)sulfonyl) octahydro-3a*H*-4,6-methanoisoindol-3a-yl)methyl *p*-tolylcarbamate |
| **35** | ((3a*S*,4*R*,6*R*,7a*S*)-5,5-dimethyl-2-((4-(3-(trifluoromethyl)-3*H*-diazirin-3-yl)phenyl)sulfonyl)octahydro-3aH-4,6-methanoisoindol-3a-yl)methyl *p-*tolylcarbamate |
| **36** | ((3a*S*,4*R*,6*R*,7a*S*)-2-((2-bromophenyl)sulfonyl)-5,5-dimethyloctahydro-3a*H-*4,6-methanoisoindol-3a-yl)methyl *p*-tolylcarbamate |
| **37** | ((3a*S*,4*R*,6*R*,7a*S*)-5,5-dimethyl-2-(phenylsulfonyl)octahydro-3a*H*-4,6-methanoisoindol-3a-yl)methyl (4-iodophenyl)carbamate |
| **38** | ((3a*S*,4*R*,6*R*,7a*S*)-5,5-dimethyl-2-(naphthalen-2-ylsulfonyl)octahydro-3a*H-*4,6-methanoisoindol-3a-yl)methyl (4-iodophenyl)carbamate |
| **39** | ((3a*S*,4*R*,6*R*,7a*S*)-5,5-dimethyl-2-(o-tolylsulfonyl)octahydro-3a*H*-4,6-methanoisoindol-3a-yl)methyl (4-iodophenyl)carbamate |
| **40** | ((3a*S*,4*R*,6*R*,7a*S*)-5,5-dimethyl-2-(m-tolylsulfonyl)octahydro-3a*H*-4,6-methanoisoindol-3a-yl)methyl (4-iodophenyl)carbamate |
| **41** | ((3a*S*,4*R*,6*R*,7a*S*)-5,5-dimethyl-2-tosyloctahydro-3a*H*-4,6-methanoisoindol-3a-yl)methyl (4-iodophenyl)carbamate |
| **43** | ((3a*S*,4*R*,6*R*,7a*S*)-2-((4-isopropylphenyl)sulfonyl)-5,5-dimethyloctahydro-3a*H-*4,6-methanoisoindol-3a-yl)methyl (4-iodophenyl)carbamate |
| **44** | ((3a*S*,4*R*,6*R*,7a*S*)-2-([1,1'-biphenyl]-4-ylsulfonyl)-5,5-dimethyloctahydro-3a*H-*4,6-methanoisoindol-3a-yl)methyl (4-iodophenyl)carbamate |
| **45** | ((3a*S*,4*R*,6*R*,7a*S*)-2-((4-cyclohexylphenyl)sulfonyl)-5,5-dimethyloctahydro-3a*H*-4,6-methanoisoindol-3a-yl)methyl (4-iodophenyl)carbamate |
| **46** | ((3a*S*,4*R*,6*R*,7a*S*)-5,5-dimethyl-2-((5,5,8,8-tetramethyl-5,6,7,8-tetrahydronaphthalen-2-yl)sulfonyl)octahydro-3aH-4,6-methanoisoindol-3a-yl)methyl (4-iodophenyl)carbamate |
| **47** | ((3a*S*,4*R*,6*R*,7a*S*)-5,5-dimethyl-2-((4-neopentylphenyl)sulfonyl)octahydro-3a*H*-4,6-methanoisoindol-3a-yl)methyl (4-iodophenyl)carbamate |
| **48** | ((3a*S*,4*R*,6*R*,7a*S*)-5,5-dimethyl-2-((4-(*tert*-pentyl)phenyl)sulfonyl)octahydro-3a*H*-4,6-methanoisoindol-3a-yl)methyl (4-iodophenyl)carbamate |
| **50** | ((3a*S*,4*R*,6*R*,7a*S*)-5,5-dimethyl-2-((4-(trimethylsilyl)phenyl)sulfonyl)octahydro-3a*H*-4,6-methanoisoindol-3a-yl)methyl (4-iodophenyl)carbamate |
| **52** | ((3a*S*,4*R*,6*R*,7a*S*)-2-((4-(*tert*-butyl)-2-methylphenyl)sulfonyl)-5,5-dimethyloctahydro-3a*H*-4,6-methanoisoindol-3a-yl)methyl (4-iodophenyl) carbamate |
| **53** | ((3a*S*,4*R*,6R*,*7a*S*)-2-((4-(*tert*-butyl)-2-ethylphenyl)sulfonyl)-5,5-dimethyloctahydro-3a*H*-4,6-methanoisoindol-3a-yl) methyl (4-iodophenyl) carbamate |
| **54** | ((3a*S*,4*R*,6*R*,7a*S*)-2-((4-(*tert*-butyl)-2-cyclopropylphenyl)sulfonyl)-5,5-dimethyloctahydro-3a*H*-4,6-methanoisoindol-3a-yl) methyl (4-iodophenyl) carbamate |
| **55** | ((3a*S*,4*R*,6*R*,7a*S*)-2-((4-(*tert*-butyl)-2-methoxyphenyl)sulfonyl)-5,5-dimethyloctahydro-3a*H*-4,6-methanoisoindol-3a-yl)methyl (4-iodophenyl) carbamate |
| **56** | ((3a*S*,4*R*,6*R*,7a*S*)-2-((2-bromo-4-(*tert*-butyl)phenyl)sulfonyl)-5,5-dimethyloctahydro-3a*H*-4,6-methanoisoindol-3a-yl)methyl (4-iodophenyl) carbamate |
| **57** | ((3a*S*,4*R*,6*R*,7a*S*)-2-((4-(*tert*-butyl)-2-iodophenyl)sulfonyl)-5,5-dimethyloctahydro-3aH-4,6-methanoisoindol-3a-yl)methyl (4-iodophenyl) carbamate |
| **58** | ((3a*S*,4*R*,6*R*,7a*S*)-2-((4-(1-(2-methoxyethoxy)-2-methylpropan-2-yl)phenyl)sulfonyl)-5,5-dimethyloctahydro-3a*H*-4,6-methanoisoindol-3a-yl)methyl (4-iodophenyl)carbamate |
| **59** | ((3a*S*,4*R*,6*R*,7a*S*)-5,5-dimethyl-2-((4-(2-methyl-1-morpholino-1-oxopropan-2-yl)phenyl)sulfonyl)octahydro-3a*H*-4,6-methanoisoindol-3a-yl)methyl (4-iodophenyl)carbamate |
| **60** | ((3a*S*,4*R*,6*R*,7a*S*)-2-((4-(1-(dimethylamino)-2-methyl-1-oxopropan-2-yl)phenyl)sulfonyl)-5,5-dimethyloctahydro-3aH-4,6-methanoisoindol-3a-yl)methyl (4-iodophenyl)carbamate |
| **61** | ((3a*S*,4*R*,6*R*,7a*S*)-2-((4-(1-(dimethylamino)-2-methylpropan-2-yl)phenyl)sulfonyl)-5,5-dimethyloctahydro-3a*H*-4,6-methanoisoindol-3a-yl)methyl (4-iodophenyl)carbamate |
| **64** | Ethyl 2-(4-(((3a*S*,4*R*,6*R*,7a*S*)-3a-((((4-iodophenyl)carbamoyl)oxy)methyl)-5,5-dimethyloctahydro-2*H*-4,6-methanoisoindol-2-yl)sulfonyl)phenyl)-2-methylpropanoate |
| **65** | 2-(4-(((3a*S*,4*R*,6*R*,7a*S*)-3a-((((4-iodophenyl)carbamoyl)oxy)methyl)-5,5-dimethyloctahydro-2*H*-4,6-methanoisoindol-2-yl)sulfonyl)phenyl)-2-methylpropanoic acid |
| **66** | Methyl 2-(4-(((3a*S*,4*R*,6*R*,7a*S*)-3a-((((4-iodophenyl)carbamoyl)oxy)methyl)-5,5-dimethyloctahydro-2*H*-4,6-methanoisoindol-2-yl)sulfonyl)-3-methoxyphenyl)-2-methylpropanoate |
| **67** | 2-(4-(((3a*S*,4*R*,6*R*,7a*S*)-3a-((((4-iodophenyl)carbamoyl)oxy)methyl)-5,5-dimethyloctahydro-2*H*-4,6-methanoisoindol-2-yl)sulfonyl)-3-methoxyphenyl)-2-methylpropanoic acid |
| **68** | Methyl 2-(3-chloro-4-(((3a*S*,4*R*,6*R*,7a*S*)-3a-((((4-iodophenyl)carbamoyl)oxy) methyl)-5,5-dimethyloctahydro-2*H*-4,6-methanoisoindol-2-yl)sulfonyl)phenyl)-2-methylpropanoate |
| **69** | 2-(3-chloro-4-(((3a*S*,4*R*,6*R*,7aS*)*-3a-((((4-iodophenyl)carbamoyl)oxy)methyl)-5,5-dimethyloctahydro-2H-4,6-methanoisoindol-2-yl)sulfonyl)phenyl)-2-methylpropanoic acid |
| **70** | 2-(4-(((3a*S*,4*R*,6*R*,7a*S*)-3a-((((4-iodophenyl)carbamoyl)oxy)methyl)-5,5-dimethyloctahydro-2H-4,6-methanoisoindol-2-yl)sulfonyl)-3-methylphenyl)-2-methylpropanoic acid |
| **71** | ((3a*S*,4*R*,6*R*,7a*S*)-2-((4-(*tert*-butyl)-2-methylphenyl)sulfonyl)-5,5-dimethyloctahydro-3a*H*-4,6-methanoisoindol-3a-yl)methyl (4-ethynylphenyl)carbamate |
| **72** | ((3a*S*,4*R*,6*R*,7a*S*)-2-((4-(*tert*-butyl)-2-ethylphenyl)sulfonyl)-5,5-dimethyloctahydro-3a*H*-4,6-methanoisoindol-3a-yl)methyl(4-ethynylphenyl) carbamate |
| **73** | ((3a*S*,4*R*,6*R*,7a*S*)-2-((4-(*tert*-butyl)-2-cyclopropylphenyl)sulfonyl)-5,5-dimethyloctahydro-3aH-4,6-methanoisoindol-3a-yl)methyl (4-ethynylphenyl) carbamate |
| **74** | 2-(4-(((3a*S*,4*R*,6*R*,7a*S*)-3a-((((4-ethynylphenyl)carbamoyl)oxy)methyl)-5,5-dimethyloctahydro-2*H*-4,6-methanoisoindol-2-yl)sulfonyl)phenyl)-2-methylpropanoic acid |
| **75** | 2-(4-(((3a*S*,4*R*,6*R*,7a*S*)-3a-((((4-ethynylphenyl)carbamoyl)oxy)methyl)-5,5-dimethyloctahydro-2*H*-4,6-methanoisoindol-2-yl)sulfonyl)-3-methylphenyl)-2-methylpropanoic acid |
| **76** | ((3a*S*,4*R*,6*R*,7a*S*)-5,5-dimethyl-2-((4-(3-(trifluoromethyl)-3*H*-diazirin-3-yl)phenyl)sulfonyl)octahydro-3a*H*-4,6-methanoisoindol-3a-yl)methyl (4-ethynylphenyl)carbamate |
or an enantiomer, a diastereomer, a tautomer, a mixture of enantiomers, a mixture of diastereomers, a mixture of tautomers, a hydrate, a solvate, a pharmaceutically acceptable salt thereof.

8. A pharmaceutical composition comprising at least one compound according to any one of the claims 1 - 7 as an active ingredient, together with at least one pharmaceutically acceptable carrier, excipient and/or diluent.

9. The pharmaceutical composition according to claim 8 further comprising at least one active agent selected from anti-microtubule agents, platinum coordination complexes, alkylating agents, antibiotic agents, topoisomerase II inhibitors, antimetabolites, topoisomerase I inhibitors, hormones and hormone analogues, signal transduction pathway inhibitors, nonreceptor tyrosine kinase angiogenesis inhibitors, immunotherapeutic agents (such as an anti- tumour vaccine, an oncolytic virus, an immune stimulatory antibody such as anti-CTLA4, anti- PD1 , anti-PDL-1 , anti-OX40, anti-41BB, anti-CD27, anti-CD40, anti-LAG3, anti-TIM3, and anti-GITR, a novel adjuvant, a peptide, a cytokine, a chimeric antigen receptor T cell therapy (CAR-T), a small molecule immune modulator, tumour microenvironment modulators, and anti-angiogenic agents, proapoptotic agents and cell cycle signalling inhibitors.

10. A compound according to any one of the claims 1 - 7, a pharmaceutically acceptable salt thereof, or a pharmaceutical composition according to claim 8 or 9 for use as a medicament.

11. A compound according to any one of the claims 1 - 7 for use, a pharmaceutically acceptable salt thereof for use, or the pharmaceutical composition according to claim 8 or 9 for use in the prophylaxis or treatment of a disease and/or a disorder caused by and/or associated with indolamine 2,3-dioxygenase 1 (IDO1),
wherein the disease and/or the disorder selected from: an inflammatory condition, an infectious disease, a cancer, a central nervous system disease or disorder, a disease or disorder relating to female reproductive health, coronary heart disease, chronic renal failure, post anaesthesia cognitive dysfunction, and cataracts.

12. The compound for use, the pharmaceutically acceptable salt thereof for use, or the pharmaceutical composition for use according to claim 11,
wherein the inflammatory condition is a condition relating to immune B cell, T cell, dendritic cell, natural killer cell, macrophage, and/or neutrophil dysregulation;
wherein the infectious disease is selected from a bacterial infection and a viral infection, preferably a gut infection, sepsis, and sepsis induced hypotension;
wherein the cancer is a cancer selected from: a solid or liquid tumour including cancer of the eye, brain (such as gliomas, glioblastomas, meduUablastomas, craniopharyngioma, ependymoma, and astrocytoma), spinal cord, kidney, mouth, lip, throat, oral cavity, nasal cavity, small intestine, colon, parathyroid gland, gall bladder, head and neck, breast, bone, bile duct, cervix, heart, hypopharyngeal gland, lung, bronchus, liver, skin, ureter, urethra, testicles, vagina, anus, laryngeal gland, ovary, thyroid, oesophagus, nasopharyngeal gland, pituitary gland, salivary gland, prostate, pancreas, adrenal glands; an endometrial cancer, oral cancer, melanoma, neuroblastoma, gastric cancer, an angiomatosis, a hemangioblastoma, a pheochromocytoma, a pancreatic cyst, a renal cell carcinoma, Wilms' tumour, squamous cell carcinoma, sarcoma, osteosarcoma, Kaposi sarcoma, rhabdomyosarcoma, hepatocellular carcinoma, PTEN Hamartoma-Tumor Syndromes (PHTS) (such as Lhermitte-Duclos disease, Cowden syndrome, Proteus syndrome, and Proteus-like syndrome), leukaemias and lymphomas (such as acute lymphoblastic leukaemia, chronic lymphocytic leukaemia, acute myelogenous leukaemia, chronic myelogenous leukaemia, hairy cell leukaemia, T-cell prolymphocytic leukemia (T-PLL), large granular lymphocytic leukemia, adult T-cell leukemia, juvenile myelomonocytic leukaemia, Hodgkin lymphoma, non-Hodgkin lymphoma, mantle lymphoma, follicular lymphoma, primary effusion lymphoma, AIDS-related lymphoma, Hodgkin lymphoma, diffuse B cell lymphoma, Burkitt lymphoma, and cutaneous T-cell lymphoma);
wherein the central nervous system disease or disorder is selected from amyotrophic lateral sclerosis (AML), Huntington's disease, Alzheimer's disease, pain, a psychiatric disorder, multiple sclerosis, Parkinson's disease, and HIV related neurocognitive decline;
wherein the disease or disorder relating to female reproductive health is endometriosis, or the condition relating to female reproductive health is contraception or abortion.

13. The compound for use, the pharmaceutically acceptable salt thereof for use, or the pharmaceutical composition for use according to claim 11 or 12,
wherein the cancer is selected from acute myeloid leukemia (AML), a small-cell lung cancer, a melanoma, an ovarian cancer, a colorectal cancer, a pancreatic cancer, an endometrial cancer, and a skin papilloma.

14. A method for producting a compound of the formula (**I**) comprising:
**Step A1-1**) performing a condensation reaction between a compound **1a*** and an isocyanate comopund **2*** (O=C=N-R¹) 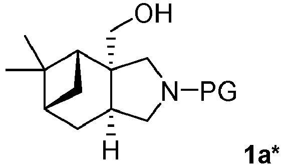
to obtain a compound **3a*** 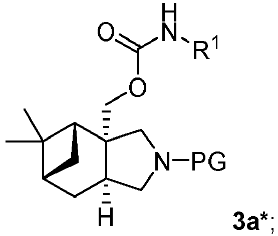
wherein PG is an amine protecting group;
**Step A1-2**) deprotecting the amine protecting group PG from the compound **3a*** to obtain a compound **3b*** 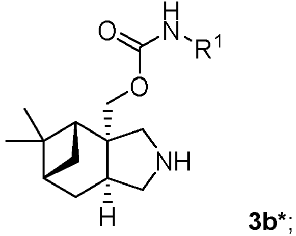
**Step B1**) performing a coupling reaction of compound **3b*** with a sulfonyl chloride compound **4*** (Cl-SO₂-R²) to obtain a compound of the formula (**I**). 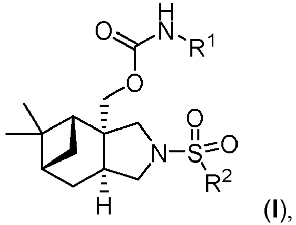 wherein **R¹** and **R²** have the same meanings as defiend in claim 1;
or
**Step A2**) performing a coupling reaction of compound **1b*** with a sulfonyl chloride compound **4* (**Cl-SO₂-R²) 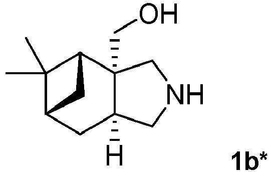 to obtain a sulfonyl amide compound **3c***; 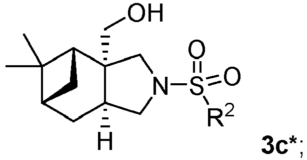
**Step B2**) performing a condensation reaction between a compound **3c*** and an isocyanate comopund **4*** (O=C=N-R¹) to obtain a compound of the formula (**I**). 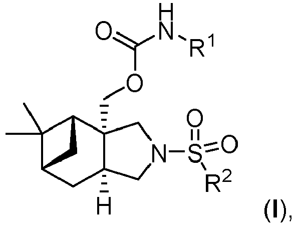 wherein **R¹** and **R²** have the same meanings as defiend in claim 1.

15. An intermediate compound selelected from compounds **3a***, **3b***, and **3c***; 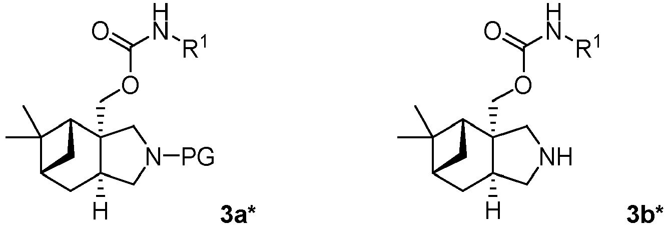 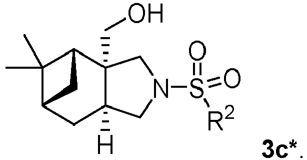 wherein **R¹** and **R²** have the same meaings as defined in Claim 1, and PG is an amine protecting group.
